# EUROPEAN PATENT APPLICATION

(11) **EP 4 011 885 A1**
(43) Date of publication of application: **15.06.2022**
(21) Application number: 20849599.4
(22) Date of filing: 03.08.2020
(51) Int. Cl.: C07D 495/04, C07D 491/048, C07D 487/04, C07D 471/04, A61K 31/444, A61K 31/4545, A61K 31/4709, A61K 31/5377, A61K 31/506, A61P 35/00

(54) **OXO-PYRIDINE FUSION RING DERIVATIVE AND PHARMACEUTICAL COMPOSITION COMPRISING SAME**

(30) Priority: 02.08.2019 KR 20190094455
(71) Applicant: Wellmarker Bio Co., Ltd., Seoul 05855 (KR)
(72) Inventor: RYU, Hyun, Seoul 05855 (KR); LEE, Jung-Eun, Seoul 05855 (KR); KO, Young-Ok, Seoul 05855 (KR); KIM, Yong-Seok, Seoul 05855 (KR); PARK, Hye-Bin, Seoul 05855 (KR)
(74) Representative: HGF
(86) International application number: PCT/KR2020/010204
(87) International publication number: WO 2021/025407

(57) **Abstract**

An oxo-pyridine fusion ring derivative compound represented by chemical formula 1 or a pharmaceutically acceptable salt thereof according to the present invention effectively inhibits the activity of RON and can not only effectively suppress the cell growth of cancer cell lines in which RON is activated, but also can effectively kill the cancer cell lines. Therefore, the oxo-pyridine fusion ring derivative compound represented by chemical formula 1 or a pharmaceutically acceptable salt thereof according to the present invention can be advantageously used for preventing or treating a disease associated with protein kinase activity.

## Description

### Technical Field

The present invention relates to an oxo-pyridine fused ring derivative or a pharmaceutically acceptable salt thereof, and a pharmaceutical composition comprising the same as an active ingredient.

### Background Art

Protein kinases are enzymes involved in cell metabolism by phosphorylating other proteins to regulate activity, location, and function of the proteins in question. Protein kinases include Ab1, ACK, ALK, Arg, ARK5, Aurora, Ax1, Bmx, BTK, CDK, CHK, c-Kit, c-MET, c-RAF, c-SRC, EGFR, FAK, Fes, FGFR, Flt3, GSK3, IGF, IKK, JAK, Lck, LIMK, Lyn, MEK, Mer, MK-2, P38alpha, PDGFR, PDK, Pim, PKA, PKB, PKCR, Plk-1/3, Ret, RON, Ros, Rse, Tie, Trk, Tyro3, VEGFR, YES, and the like. Dysfunction of these protein kinases is closely related to cancer, immune diseases, neurological diseases, metabolic diseases, and infection mechanism that causes diseases.

Among these, abnormal c-MET activation in cancer cells is closely related to worsening prognosis of anticancer therapy, and overexpression and mutation of c-MET have been observed in several carcinomas such as non-small cell lung cancer. Accordingly, treatment methods for inhibiting the activity of c-MET have been continuously studied.

Meanwhile, RON (Recepteur d'Origine Nantais) refers to a protein receptor belonging to the c-MET family, and is a receptor for macrophage-stimulating protein (MSP) which is secreted by the liver and regulates the action of macrophages (Zhou YQ, He C, Chen YQ, Wang D, Wang MH, Oncogene 2003, 22(2):186-197). The activity of RON also has an important function in the development, progression, and metastasis of tumors. In particular, overexpression or hyperactivity thereof in colorectal cancer and breast cancer has been reported to contribute to inducing tumor invasion and metastasis and inhibiting cell death (Faham N. et al., Cold Spring Harb Symp Quant Biol. 2016; Wagh PK. et al., Adv Cancer Res. 2008; Wang MH. et al., Acta Pharmacologica Sinica. 2006; Camp ER. et al., Ann Surg Oncol. 2005).

In addition, although the activity of RON is regulated by macrophage stimulating protein (MSP) that is a ligand, it may also be regulated by alternative splicing, which is one of the important processes for regulating gene expression in eukaryotes. For example, RONΔ155, RONΔ160, and RONΔ165, which are mutant forms generated by exon skipping caused by alternative splicing, are constitutively activated even without a ligand.

Accordingly, there is a need to develop anticancer agents and antibody anticancer agents, which are based on molecules that inhibit the activity of RON, for various carcinomas including colorectal cancer.

### Disclosure of Invention

### Technical Problem

As a result of studying compounds that can be used as protein kinase inhibitors, the present inventors have found that an oxo-pyridine fused ring derivative compound having a specific structure effectively inhibits protein kinase activity and can be useful for prevention or treatment of a related disease, and thus have completed the present invention.

Accordingly, an object of the present invention is to provide a novel oxo-pyridine fused ring derivative compound that inhibits protein kinase activity and thus can be useful for treatment of a disease related thereto, and a pharmaceutical composition comprising the same.

### Solution to Problem

To achieve the above object, in an aspect of the present invention, there is provided an oxo-pyridine fused ring derivative compound represented by Formula 1, or a pharmaceutically acceptable salt thereof.

In another aspect of the present invention, there is provided a pharmaceutical composition for preventing or treating a disease related to protein kinase activity, comprising, as an active ingredient, the compound represented by Formula 1 or a pharmaceutically acceptable salt thereof.

### Advantageous Effects of Invention

The oxo-pyridine fused ring derivative compound represented by Formula 1 according to the present invention or a pharmaceutically acceptable salt thereof can effectively inhibit the activity of RON, so that not only cell growth of a cancer cell line, in which RON is activated, is effectively inhibited but also the cancer cell line is effectively killed. Accordingly, the oxo-pyridine fused ring derivative compound represented by Formula 1 according to the present invention or a pharmaceutically acceptable salt thereof can be useful for prevention or treatment of a disease related to protein kinase activity.

### Brief Description of Drawings

FIG. 1 illustrates a graph showing cell death (%) observed in a case where the cancer cell line KM12C, in which RON is activated, is treated with each of the compounds of Examples 76, 125, 134, 185, 187, and 188 of the present invention at a concentration of 5 µM.
FIG. 2 illustrates a graph showing cell death (%) observed in a case where the cancer cell line HT29, in which mutated RON (Δ160) is expressed, is treated with each of the compounds of Examples 76, 125, 134, 185, 187, and 188 of the present invention at a concentration of 5 µM.
FIG. 3 illustrates a graph showing cell death (%) observed in a case where the cancer cell line SW620, in which mutated RON (Δ155) is expressed, is treated with each of the compounds of Examples 76, 125, 134, 185, 187, and 188 of the present invention at a concentration of 5 µM.

### Best Mode for Carrying out the Invention

Hereinafter, the present invention will be described in detail.

As used herein, the term "halogen" refers to F, Cl, Br, or I unless otherwise stated.

The term "alkyl", unless otherwise specified, refers to a linear or branched saturated hydrocarbon moiety. For example, "C₁₋₁₀ alkyl" refers to an alkyl group having a skeleton of 1 to 10 carbon atoms. Specifically, the C₁₋₁₀ alkyl may include methyl, ethyl, n-propyl, i-propyl, n-butyl, i-butyl, t-butyl, n-pentyl, i-pentyl, t-pentyl, sec-pentyl, neopentyl, hexyl, heptyl, octyl, nonyl, decyl, and the like.

The term "haloalkyl" refers to an alkyl group substituted with one or more halogen atoms. Specifically, the haloalkyl may be an alkyl group substituted with two or more halogen atoms, the halogen atoms being the same or different.

The term "alkoxy", unless otherwise specified, refers to a group having the formula - O-alkyl, in which the alkyl is an alkyl group as defined above and is attached to a parent compound through an oxygen atom. The alkyl portion of the alkoxy group may have 1 to 20 carbon atoms (that is, C₁-C₂₀ alkoxy), 1 to 12 carbon atoms (that is, C₁-C₁₂ alkoxy), or 1 to 6 carbon atoms (that is, C₁-C₆ alkoxy). Examples of suitable alkoxy groups include methoxy (-O-CH3 or -OMe), ethoxy (-OCH2CH3 or -OEt), t-butoxy (-OC(CH3)3 or -O-tBu), and the like.

The term "aryl" refers to an aromatic hydrocarbon radical derived by the removal of one hydrogen atom from a single carbon atom of a parent aromatic ring system. For example, the aryl group may have 6 to 20 carbon atoms, 6 to 14 carbon atoms, or 6 to 10 carbon atoms.

The term "cycloalkyl" refers to a saturated monocycle or polycycle that contains only carbon atoms in the ring. The cycloalkyl may have 3 to 7 carbon atoms as a monocycle, 7 to 12 carbon atoms as a bicycle, and up to about 20 carbon atoms as a polycycle.

The term "heterocyclic ring" refers to an aromatic or non-aromatic ring having one or more heteroatoms, which may be saturated or unsaturated and may be monocyclic or polycyclic. For example, "4- to 10-membered heterocyclic ring" refers to a heterocyclic ring having a skeleton of a total of 4 to 10 atoms including at least one heteroatom and carbon atoms. Specifically, examples of the 4- to 10-membered heterocyclic ring may include azetidine, diazetidine, pyrrolidine, pyrrole, imidazolidine, imidazole, pyrazolidine, pyrazole, oxazolidine, oxazole, isoxazolidine, isoxazole, thiazolidine, thiazole, isothiazolidine, isothiazole, piperidine, pyridine, piperazine, diazine, morpholine, thiomorpholine, azepane, diazepane, and the like.

The term "heteroaryl" refers to an aromatic heterocyclyl having one or more heteroatoms in the ring. Non-limiting examples of the heteroaryl include pyridinyl, pyrrolyl, oxazolyl, indolyl, isoindolyl, furinyl, furanyl, thienyl, benzofuranyl, benzothiophenyl, carbazolyl, imidazolyl, thiazolyl, isoxazolyl, pyrazolyl, isothiazolyl, quinolyl, isoquinolyl, pyridazyl, pyrimidyl, pyrazyl, and the like (each of which may have one or more substituents on the ring).

The term "heterocycloalkyl" refers to a non-aromatic heterocyclyl having one or more heteroatoms in the ring. The heterocycloalkyl may have one or more carbon-carbon double bonds or carbon-heteroatom double bonds in the ring as long as the ring is not rendered aromatic by their presence. Non-limiting examples of the heterocycloalkyl include azetidinyl, aziridinyl, pyrrolidinyl, piperidinyl, piperazinyl, homopiperazinyl, morpholino, thiomorpholino, tetrahydrofuranyl, tetrahydrothiofuranyl, tetrahydropyranyl, pyranyl, and the like (each of which may have one or more substituents on the ring).

The term "heteroatom" refers to an atom other than carbon (C), and may specifically be a nitrogen (N), oxygen (O), or sulfur (S) atom. The above-mentioned heterocyclic ring, heteroaryl, and heterocycloalkyl contain one or more heteroatoms and may, for example, contain 1, 1 to 2,1 to 3, or 1 to 4 heteroatoms.

The term "substitution" refers to replacement of a hydrogen atom in a molecular structure with a substituent such that a chemically stable compound results from the substitution while not exceeding valence on the designated atom. For example, "group A is substituted with substituent B" or "group A has substituent B" means that a hydrogen atom bonded to an atom, such as carbon, which constitutes a skeleton of group A, is replaced with substituent B so that the group A and the substituent B form a covalent bond. Thus, it is substantially difficult or impossible for a group having no removable hydrogen atom to have a substituent. From this viewpoint, in a case where a range of combinations of various groups, which include groups that hardly have a substituent, with substituents are exemplified in the present specification, it should be interpreted that combinations of the groups, for which it is obvious that no substitution is possible, with the substituents are excluded from the range.

In an aspect of the present invention, there is provided a compound represented by Formula 1, or a pharmaceutically acceptable salt thereof: 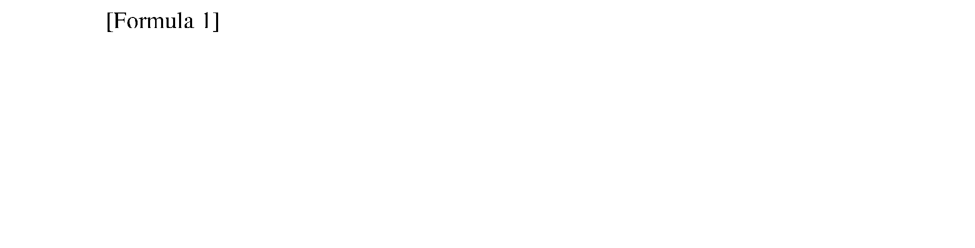 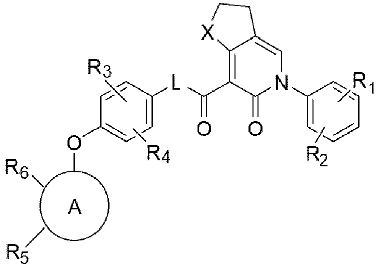 in the formula,
L is -NH- or -CH₂-,
R₁ to R₄ are each independently hydrogen, halogen, hydroxy, cyano, C₁₋₄ alkyl, C₁₋₄ alkoxy, C₁₋₄ haloalkyl, C₂₋₄ alkenyl, C₂₋₄ alkynyl, C₃₋₇ cycloalkyl, C₆₋₁₀ aryl, 5- to 9-membered heteroaryl, or 3- to 9-membered heterocycloalkyl,
X is O, S, -CH(-Rx)-, or -N(-Rx)-,
Rx is hydrogen, C₁₋₄ alkyl, C₁₋₄ alkoxy, C₁₋₄ haloalkyl, C₂₋₄ alkenyl, C₂₋₄ alkynyl, C₆₋₁₀ aryl, C₆₋₁₀ aryl-C₁₋₄ alkyl, or 3- to 9-membered heterocycloalkyl,
A is a 3- to 12-membered heterocyclic ring, and
R₅ and R₆ are each independently hydrogen, nitro, amino, halogen, hydroxy, cyano, C₁₋₆ alkyl, C₁₋₆ haloalkyl, C₂₋₆ alkenyl, C₂₋₆ alkynyl, C₁₋₆ alkoxy, amino-C₁₋₆ alkoxy, aminocarbonyl, C₁₋₆ alkylaminocarbonyl, diC₁₋₆ alkylaminocarbonyl, C₁₋₆ alkylcarbonylamino, C₁₋₆ alkylamino, C₁₋₆ alkylamino-C₁₋₆ alkoxy, C₆₋₁₀ aryl, C₆₋₁₀ aryl-C₁₋₄ alkyl, or 5- to 9-membered heteroaryl,
wherein R₅ and R₆ are each independently optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3- to 9-membered cycloalkyl; or 3- to 9-membered heterocycloalkyl,
the cycloalkyl or the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heterocyclic ring, the heteroaryl, and the heterocycloalkyl each independently contain one or more heteroatoms selected from the group consisting of N, O, and S.

In addition, the C₁₋₆ alkyl may include C₁₋₃ alkyl, C₃₋₆ alkyl, and the like. In addition, the C₁₋₆ alkoxy may include C₁₋₃ alkoxy, C₃₋₆ alkoxy, and the like.

In addition, the 3- to 12-membered heterocyclic ring may include a 4- to 12-membered heterocyclic ring, a 4- to 10-membered heterocyclic ring, a 4- or 7-membered heterocyclic ring, a 7- to 12-membered heterocyclic ring, and the like. In addition, the heterocyclic ring may include an aromatic ring or a non-aromatic ring. As an example, the 3- to 12-membered heterocyclic ring may contain one or more aromatic rings. For example, the heterocyclic ring may be quinoline, quinazoline, pyridine, pyrimidine, thienopyridine, pyrrolopyridine, pyrazolopyridine, imidazopyridine, pyrrolopyrimidine, dihydropyrrolopyrimidine, furopyridine, pyrazolopyrimidine, purine, indazole, azetidine, diazetidine, pyrrolidine, pyrrole, imidazolidine, imidazole, pyrazolidine, pyrazole, oxazolidine, oxazole, isoxazolidine, isoxazole, thiazolidine, thiazole, isothiazolidine, isothiazole, piperidine, pyridine, piperazine, diazine, morpholine, thiomorpholine, azepane, diazepane, or the like.

According to an embodiment, in Formula 1, R₁ to R₄ may each independently be hydrogen, C₁₋₄ haloalkyl, or halogen. Here, the halogen may be F, Cl, Br, or I. Specifically, R₁ may be hydrogen, trifluoromethyl, or fluoro; R₂ may be hydrogen; R₃ may be fluoro; and R₄ may be hydrogen.

According to another embodiment, in Formula 1, X may be O or -CH(-Rx)- and Rx may be hydrogen or C₁₋₆ alkyl.

According to yet another embodiment, in Formula 1, A may be quinoline, quinazoline, pyridine, pyrimidine, thienopyridine, pyrrolopyridine, pyrazolopyridine, imidazopyridine, pyrrolopyrimidine, dihydropyrrolopyrimidine, furopyridine, pyrazolopyrimidine, purine, or indazole.

The thienopyridine may be represented by the following structural formula:

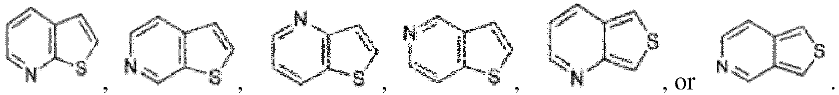

The pyrrolopyridine may be represented by the following structural formula:

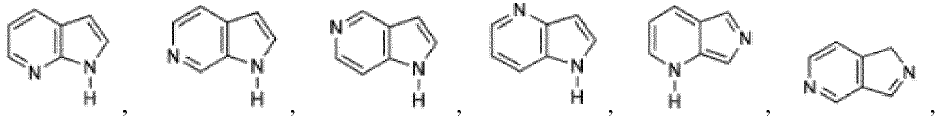

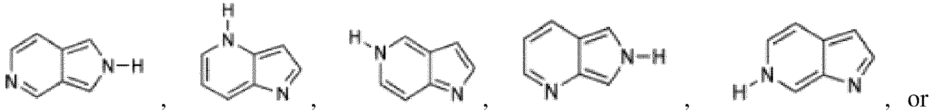

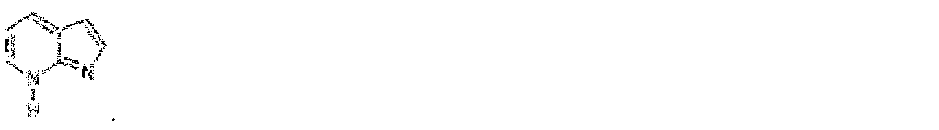

The pyrrolopyrimidine may be represented by the following structural formula:

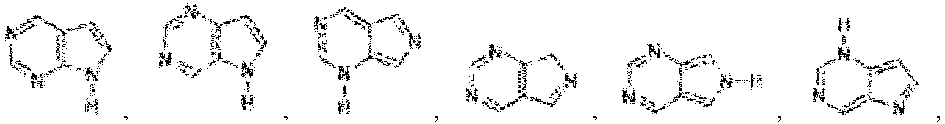

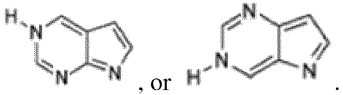

The dihydropyrrolopyrimidine may be represented by the following structural

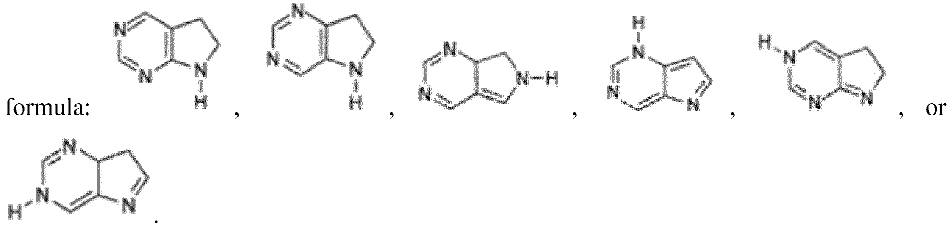

The pyrazolopyridine may be represented by the following structural formula:

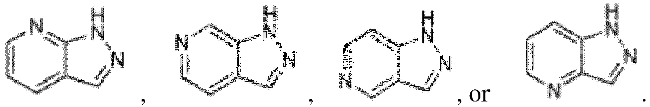

The pyrazolopyrimidine may be represented by the following structural formula:

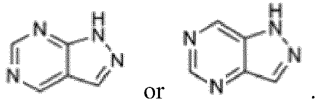

The imidazopyridine may be represented by the following structural formula:

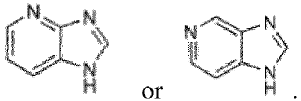

The furopyridine may be represented by the following structural formula:

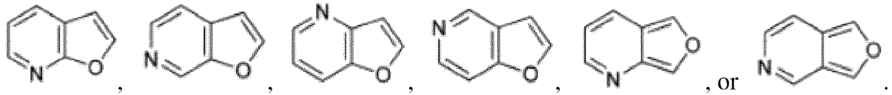

The purine may be represented by the following structural formula:

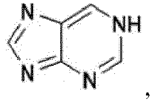

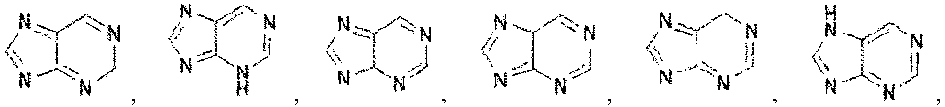

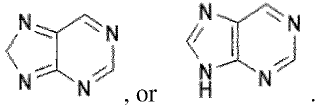

According to still yet another embodiment, in Formula 1, R₅ and R₆ are each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylcarbonylamino, C₁₋₆ alkylcarbonylamino, C₁₋₆ alkylamino, C₁₋₆ alkylamino-C₁₋₆ alkoxy, or 5- to 9- membered heteroaryl, wherein R₅ and R₆ may each independently be optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3- to 9-membered cycloalkyl; or 3- to 9-membered heterocycloalkyl; the cycloalkyl or the heterocycloalkyl may optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl; and the heteroaryl and the heterocycloalkyl may each independently contain one or more heteroatoms selected from the group consisting of N, O, and S.

Specifically, the heteroaryl may be pyridinyl, imidazolyl, or pyrazolyl; the heterocycloalkyl may be azetidinyl, pyrrolidinyl, tetrahydropyranyl, morpholino, morpholinyl, dioxidothiomorpholino, piperazinyl, piperidinyl, or oxetanyl; and the cycloalkyl may be cyclobutyl, cyclopentyl, or cyclohexyl.

In addition, in a case where the heteroaryl or the heterocycloalkyl contains one or more N atoms, substitution may be made at any one of the N atomic positions. However, there is no particular limitation thereon.

According to still yet another embodiment, in Formula 1, R₁ and R₂ are each independently hydrogen, C₁₋₄ haloalkyl, or halogen; R₃ and R₄ are each independently hydrogen or halogen; X is O or -CH(-Rx)- and Rx is hydrogen or C₁₋₄ alkyl; A is quinoline, quinazoline, pyridine, pyrimidine, thienopyridine, pyrrolopyridine, pyrazolopyridine, imidazopyridine, pyrrolopyrimidine, dihydropyrrolopyrimidine, furopyridine, pyrazolopyrimidine, purine, or indazole; and R₅ and R₆ are each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylcarbonylamino, C₁₋₆ alkylcarbonylamino, C₁₋₆ alkylamino, C₁₋₆ alkylamino-C₁₋₆ alkoxy, or 5- to 9-membered heteroaryl, wherein R₅ and R₆ may each independently be optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3- to 9-membered cycloalkyl; or 3- to 9-membered heterocycloalkyl; the cycloalkyl or the heterocycloalkyl may optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl; and the heteroaryl and the heterocycloalkyl may each independently contain one or more heteroatoms selected from the group consisting of N, O, and S.

According to still yet another embodiment, in Formula 1, R₅ and R₆ are each independently hydrogen, nitro, amino, halogen, hydroxy, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylaminocarbonyl, C₁₋₆ alkylcarbonylamino, C₁₋₆ alkylamino, C₁₋₆ alkylamino-C1- 6 alkoxy, C₆₋₁₀ aryl, C₆₋₁₀ aryl-C₁₋₄ alkyl, or 5- to 9-membered heteroaryl, wherein the amino, the alkyl, the alkoxy, the aryl, and the heteroaryl may each independently be optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3- to 9-membered cycloalkyl; or 3- to 9-membered heterocycloalkyl; the cycloalkyl or the heterocycloalkyl may optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl; and the heterocyclic ring, the heteroaryl and the heterocycloalkyl may each independently contain one or more heteroatoms selected from the group consisting of N, O, and S.

According to still yet another embodiment, in Formula 1, R₅ and R₆ may not be, at the same time, C₆₋₁₀ aryl, C₆₋₁₀ aryl-C₁₋₄ alkyl, or 5- to 9-membered heteroaryl. According to still yet another embodiment, in Formula 1, R₅ and R₆ may not be substituted, at the same time, with C₁₋₆ alkyl or C₁₋₆ alkylamino-C₁₋₆ alkyl, substituted with any one of 3- to 9-membered cycloalkyl and 3- to 9-membered heterocycloalkyl; 3- to 9-membered cycloalkyl; or 3- to 9-membered heterocycloalkyl. As a specific example, in a case where R₅ contains a ring such as aryl or heteroaryl, R₆ may not contain this ring at the same time. In addition, in a case where R₅ is substituted with a group containing a ring such as cycloalkyl or heterocycloalkyl, R₆ may not be substituted, at the same time, with a group containing this ring.

As a more specific example, R₅ may be C₆₋₁₀ aryl, C₆₋₁₀ aryl-C₁₋₄ alkyl, or 5- to 9-membered heteroaryl; and R₆ may be hydrogen, nitro, amino, halogen, hydroxy, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylaminocarbonyl, C₁₋₆ alkylcarbonylamino, C₁₋₆ alkylamino, or C₁₋₆ alkylamino-C₁₋₆ alkoxy. Here, R₅ may be optionally substituted with C₁₋₆ alkyl; or C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl. In addition, R₆ may be optionally substituted with 3- to 9-membered cycloalkyl or 3- to 9-membered heterocycloalkyl. Here, the cycloalkyl or the heterocycloalkyl may optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl; and the heteroaryl and the heterocycloalkyl may each independently contain one or more heteroatoms selected from the group consisting of N, O, and S.

According to still yet another embodiment, in Formula 1, R₅ and R₆ are each independently hydrogen, amino, halogen, hydroxy, C₁₋₆ alkoxy, aminocarbonyl, C₁₋₆ alkylaminocarbonyl, diC₁₋₆ alkylaminocarbonyl, C₁₋₆ alkylcarbonylamino, cyano, C₁₋₄ haloalkyl, C₁₋₆ alkyl, 5- or 9-membered heteroaryl, Ax-(CH₂)a-L1-(CH₂)_{b}-L2-, or Ax-(CH2)a-L1-(CH₂)_{b}-L2-pyridinyl; Ax is C₃₋₆ cycloalkyl or 3-6 membered heterocycloalkyl; L1 and L2 are each independently a single bond, -O-, -NH-, -C(=O)-NH-, or -NH-C(=O)-; and a and b are each independently an integer of 0 to 3, provided that in a case where b is 0, L2 is a single bond, wherein the cycloalkyl, the heteroaryl, and the heterocycloalkyl may each independently optionally have one or two substituents selected from the group consisting of halogen, oxo, cyano, hydroxy, hydroxymethyl, C₁₋₆ alkyl, methoxy, methoxymethyl, dimethylamino, and methoxyethylaminomethyl; the heteroaryl and the heterocycloalkyl may each independently contain one or more heteroatoms selected from the group consisting of N, O, and S; and the heteroaryl and the heterocycloalkyl may each independently contain one or more heteroatoms selected from the group consisting of N, O, and S.

According to still yet another embodiment, the compound of Formula 1 may have a structure of Formula 1a: 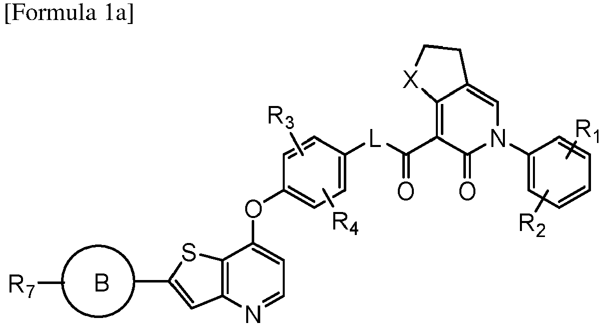 in the formula,
X, L, R₁, R₂, R₃, and R₄ are as defined in Formula 1,
B is a 5- or 6-membered aromatic heterocyclic ring containing 1 or 2 N atoms, and
R₇ is C₁₋₆ alkyl; or C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl,
wherein the cycloalkyl or the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆alkoxy-C₁₋₆ alkyl, and
the heterocycloalkyl contains 1 to 4 heteroatoms selected from the group consisting of N, O, and S.

Specific examples of the compound represented by Formula 1a are as follows:
1) N-(3-fluoro-4-((2-(5-(((2-methoxyethyl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
2) N-(3-fluoro-4-((2-(5-(((2-methoxyethyl)amino)methyl)pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
3) N-(3-fluoro-4-((2-(pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
4) N-(3-fluoro-4-((2-pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
5) N-(3-fluoro-4-((2-(5-(morpholinomethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
6) N-(3-fluoro-4-((2-(5-(morpholinomethyl)pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
7) N-(3-fluoro-4-((2-(5-morpholinomethyl)pyridin-2-yl)thieno[3,2-b]pyridin-7-yl)phenyl)-2-(4-fluorophenyl)-3-oxo-3,5,6,7-tetrahydro-2H-cyclopenta[c]pyridine-4-carboxamide;
8) N-(3-fluoro-4-((2-(1-methyl-1H-imidazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
9) N-(3-fluoro-4-((2-(1-methyl-1H-imidazol-4-yl)thieno[3,2-b]pyridin-7-yl)oxy)phenyl)-6-oxo-5-phenyl-2,3,5,6-tetrahydrofuro[3,2-c]pyridine-7-carboxamide;
10) N-(3-fluoro-4-((2-(1-methyl-1H-pyrazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
11) N-(3-fluoro-4-((2-(5-((3-hydroxyazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
12) N-(3-fluoro-4-((2-(5-((3-(hydroxymethyl)azetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
13) N-(3-fluoro-4-((2-(5-((3-hydroxy-3-methylazetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
14) N-(3-fluoro-4-((2-(5-((3-methoxyazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
15) N-(3-fluoro-4-((2-(5-((3-(methoxymethyl)azetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
16) N-(3-fluoro-4-((2-(5-((3-methoxy-3-methylazetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
17) N-(3-fluoro-4-((2-(5-((3-fluoroazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
18) N-(4-((2-(5-((3,3-difluoroazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
19) N-(4-((2-(5-((3-cyanoazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
20) N-(4-((2-(5-((3-(dimethylamino)azetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
21) N-(3-fluoro-4-((2-(5-((3-methylazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
22) N-(4-((2-(5-((3,3-dimethylazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
23) N-(3-fluoro-4-((2-(5-((3-hydroxypyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
24) N-(3-fluoro-4-((2-(5-((3-hydroxy-3-methylpyrrolidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
25) N-(3-fluoro-4-((2-(5-((3-methoxypyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
26) N-(3-fluoro-4-((2-(5-(pyrrolidin-1-ylmethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
27) N-(3-fluoro-4-((2-(5-((3-fluoropyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
28) N-(4-((2-(5-((3,3-difluoropyrrolidin-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide;
29) N-(3-fluoro-4-((2-(5-((4-hydroxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
30) N-(3-fluoro-4-((2-(5-((3-hydroxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
31) N-(3-fluoro-4-((2-(5-((4-methoxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
32) N-(3-fluoro-4-((2-(5-((3-methoxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
33) N-(3-fluoro-4-((2-(5-(piperidin-1-ylmethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
34) N-(3-fluoro-4-((2-(5-((4-fluoropiperidm-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
35) N-(4-((2-(5-((4,4-difluoropiperidm-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide;
36) N-(3-fluoro-4-((2-(5-((4-methylpiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
37) N-(4-((2-(5-((4,4-dimethylpiperidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
38) N-(3-fluoro-4-((2-(5-((((3-hydroxycyclohexyl)methyl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide; and
39) N-(3-fluoro-4-((2-(5-(((tetrahydro-2H-pyran-4-yl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide.

According to still yet another embodiment, the compound of Formula 1 may have a structure of Formula 1b: 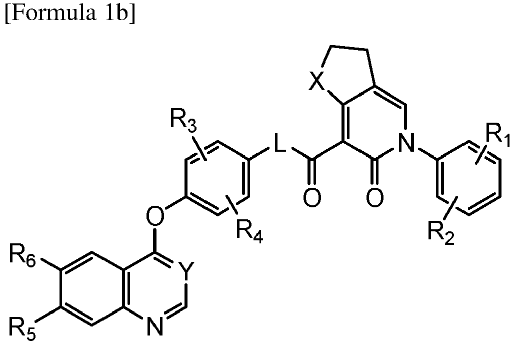 in the formula,
X, L, R₁, R₂, R₃, and R₄ are as defined in Formula 1,
Y is -N= or -CH=, and
R₅ and R₆ are each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylcarbonylamino, C₁₋₆ alkylcarbonylamino, C₁₋₆ alkylamino, or C₁₋₆ alkylamino-C₁₋₆ alkoxy,
wherein R₅ and R₆ are each independently optionally substituted with 3- to 9-membered cycloalkyl or 3- to 9-membered heterocycloalkyl,
the cycloalkyl or the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heterocycloalkyl contains 1 to 4 heteroatoms selected from the group consisting of N, O, and S.

Specific examples of the compound represented by Formula 1b are as follows:
40) N-(4-((6,7-dimethoxyquinolin-4-yl)oxy)-3-fluorophenyl)-5-(4-fluorophenyl)-6-oxo-2,3,5,6-tetrahydrofuro[3,2-c]pyridine-7-carboxamide;
41) N-(4-((6,7-dimethoxyquinolin-4-yl)oxy)-3-fluorophenyl)-6-oxo-5-phenyl-2,3,5,6-tetrahydrofuro[3,2-c]pyridine-7-carboxamide;
42) N-(4-((6,7-dimethoxyquinolin-4-yl)oxy)-3-fluorophenyl)-6-oxo-5-(4-(trifluoromethyl)phenyl)-2,3,5,6-tetrahydrofuro[3,2-c]pyridine-7-carboxamide;
43) N-(4-((6,7-dimethoxyquinolin-4-yl)oxy)-3-fluorophenyl)-6-oxo-5-(3-fluorophenyl)-2,3,5,6-tetrahydrofuro[3,2-c]pyridine-7-carboxamide;
44) N-(3-fluoro-4-((6-methoxyquinolin-4-yl)oxy)phenyl)-5-(4-fluorophenyl)-6-oxo-2,3,5,6-tetrahydrofuro[3,2-c]pyridine-7-carboxamide;
45) N-(3-fluoro-4-((7-methoxyquinolin-4-yl)oxy)phenyl)-5-(4-fluorophenyl)-6-oxo-2,3,5,6-tetrahydrofuro[3,2-c]pyridine-7-carboxamide;
46) N-(4-((6,7-dimethoxyquinazolin-4-yl)oxy)-3-fluorophenyl)-5-(4-fluorophenyl)-6-oxo-2,3,5,6-tetrahydrofuro[3,2-c]pyridine-7-carboxamide;
47) N-(4-((6-carbamoyl-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
48) N-(3-fluoro-4-((7-methoxy-6-(methylcarbamoyl)quinolin-4-yl)oxy)phenyl)-5-(4-fluorophenyl)-6-oxo-2,3,5,6-tetrahydrofuro[3,2-c]pyridine-7-carboxamide;
49) N-(4-((6-(dimethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
50) N-(3-fluoro-4-((7-methoxy-6-((2-morpholinoethyl)carbamoyl)quinolin-4-yl)oxy)phenyl)-5-(4-fluorophenyl)-6-oxo-2,3,5,6-tetrahydrofuro[3,2-c]pyridine-7-carboxamide;
51) N-(4-((6-(ethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
52) N-(4-((6-acetamido-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
53) N-(3-fluoro-4-((7-methoxy-6-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide;
76) N-(3-fluoro-4-((6-methoxy-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
77) N-(3-fluoro-4-((6-methoxy-7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide;
78) N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
79) N-(3-fluoro-4-((6-methoxy-7-(2-morpholinoethoxy)quinolin-4-yl)oxy)phenyl)-6-oxo-5-phenyl-2,3,5,6-tetrahydrofuro[3,2-c]pyridine-7-carboxamide;
80) N-(3-fluoro-4-((6-methoxy-7-(3-(4-methylpiperazin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
81) N-(3-fluoro-4-((7-(3-(3-hydroxyazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
82) N-(3-fluoro-4-((7-(3-(3-hydroxy-3-methylazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
83) N-(3-fluoro-4-((7-(3-(3-(hydroxymethyl)azetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
84) N-(3-fluoro-4-((6-methoxy-7-(3-(3-methoxyazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
85) N-(3-fluoro-4-((6-methoxy-7-(3-(3-methoxy-3-methylazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
86) N-(3-fluoro-4-((7-(3-(3-fluoroazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
87) N-(4-((7-(3-(3,3-difluoroazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
88) N-(4-((7-(3-(3-cyanoazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
89) N-(3-fluoro-4-((6-methoxy-7-(3-(3-methylazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
90) N-(3-fluoro-4-((7-(3-(3-hydroxypyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
91) N-(3-fluoro-4-((7-(3-(3-hydroxy-3-methylpyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
92) N-(3-fluoro-4-((6-methoxy-7-(3-(3-methoxypyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
93) N-(3-fluoro-4-((6-methoxy-7-(3-(pyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
94) N-(3-fluoro-4-((7-(3-(3-fluoropyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
95) N-(4-((7-(3-(3,3-difluoropyrrolidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
96) N-(3-fluoro-4-((7-(3-(4-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
97) N-(3-fluoro-4-((7-(3-(3-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
98) N-(3-fluoro-4-((7-(3-(4-hydroxy-4-methylpiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
99) N-(3-fluoro-4-((6-methoxy-7-(3-(4-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
100) N-(3-fluoro-4-((6-methoxy-7-(3-(3-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
101) N-(3-fluoro-4-((6-methoxy-7-(3-(4-oxopiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
102) N-(4-((7-(3-(1,1-dioxidothiomorpholino)propoxy)-6-methoxyqumolin-4-yl)oxy)-3-fluorophenyl)-5-(4-fluorophenyl)-6-oxo-2,3,5,6-tetrahydrofuro[3,2-c]pyridine-7-carboxamide;
103) N-(3-fluoro-4-((6-methoxy-7-(3-(piperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
104) N-(3-fluoro-4-((7-(3-(4-fluoropiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
105) N-(4-((7-(3-(4,4-difluoropiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
106) N-(3-fluoro-4-((6-methoxy-7-(3-(4-methylpiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
107) N-(4-((7-(3-(4,4-dimethylpiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
108) N-(3-fluoro-4-((7-(3-((3-hydroxycyclobutyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
109) N-(3-fluoro-4-((6-methoxy-7-(3-((3-methoxycyclobutyl)amino)propoxy)quinolin-4-yl)oxy)phenyl)-5-(4-fluorophenyl)-6-oxo-2,3,5,6-tetrahydrofuro[3,2-c]pyridine-7-carboxamide;
110) N-(3-fluoro-4-((6-methoxy-7-(3-(oxetan-3-ylamino)propoxy)quinolin-4-yl)oxy)phenyl)-5-(4-fluorophenyl)-6-oxo-2,3,5,6-tetrahydrofuro[3,2-c]pyridine-7-carboxamide;
111) N-(3-fluoro-4-((6-methoxy-7-(3-((oxetan-3-ylmethyl)amino)propoxy)quinolin-4-yl)oxy)phenyl)-5-(4-fluorophenyl)-6-oxo-2,3,5,6-tetrahydrofuro[3,2-c]pyridine-7-carboxamide;
112) N-(3-fluoro-4-((7-(3-((3-hydroxycyclopentyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
113) N-(3-fluoro-4-((7-(3-((3-hydroxycyclohexyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
114) N-(3-fluoro-4-((7-(3-(((3-hydroxycyclohexyl)methyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
115) N-(3-fluoro-4-((6-methoxy-7-(3-((tetrahydro-2H-pyran-4-yl)amino)propoxy)quinolin-4-yl)oxy)phenyl)-5-(4-fluorophenyl)-6-oxo-2,3,5,6-tetrahydrofuro[3,2-c]pyridine-7-carboxamide;
116) N-(3-fluoro-4-((6-methoxy-7-(3-(((tetrahydro-2H-pyran-4-yl)methyl)amino)propoxy)quinolin-4-yl)oxy)phenyl)-5-(4-fluorophenyl)-6-oxo-2,3,5,6-tetrahydrofuro[3,2-c]pyridine-7-carboxamide;
117) N-(3-fluoro-4-((7-(2-(3-hydroxyazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
118) N-(3-fluoro-4-((7-(2-(3-hydroxy-3-methylazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
119) N-(3-fluoro-4-((7-(2-(3-(hydroxymethyl)azetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
120) N-(3-fluoro-4-((6-methoxy-7-(2-(3-methoxyazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
121) N-(3-fluoro-4-((6-methoxy-7-(2-(3-methoxy-3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
122) N-(3-fluoro-4-((6-methoxy-7-(2-(3-(methoxymethyl)azetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
123) N-(3-fluoro-4-((7-(2-(3-fluoroazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
124) N-(4-((7-(2-(3,3-difluoroazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
125) N-(4-((7-(2-(3-ethynylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
126) N-(3-fluoro-4-((6-methoxy-7-(2-(3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
127) N-(4-((7-(2-(3,3-dimethylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
128) N-(4-((7-(2-(3-(dimethylamino)azetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
129) N-(3-fluoro-4-((7-(2-(3-hydroxypyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
130) N-(3-fluoro-4-((7-(2-(3-hydroxy-3-methylpyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
131) N-(3-fluoro-4-((6-methoxy-7-(2-(3-methoxypyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
132) N-(3-fluoro-4-((6-methoxy-7-(2-(pyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
133) N-(3-fluoro-4-((7-(2-(3-fluoropyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
134) N-(4-((7-(2-(3,3-difluoropyrrolidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
135) N-(3-fluoro-4-((7-(2-(4-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
136) N-(3-fluoro-4-((7-(2-(3-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
137) N-(3-fluoro-4-((7-(2-(4-hydroxy-4-methylpiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
138) N-(3-fluoro-4-((6-methoxy-7-(2-(4-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
139) N-(3-fluoro-4-((6-methoxy-7-(2-(3-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
140) N-(3-fluoro-4-((6-methoxy-7-(2-(4-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
141) N-(3-fluoro-4-((6-methoxy-7-(2-(3-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
142) N-(4-((7-(2-(1,1-dioxidothiomorpholino)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
143) N-(3-fluoro-4-((6-methoxy-7-(2-(piperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
144) N-(3-fluoro-4-((7-(2-(4-fluoropiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
145) N-(4-((7-(2-(4,4-difluoropiperidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
146) N-(3-fluoro-4-((6-methoxy-7-(2-(4-methylpiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
147) N-(4-((7-(2-(4,4-dimethylpiperidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
148) N-(3-fluoro-4-((7-(2-((3-hydroxycyclobutyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
149) N-(3-fluoro-4-((6-methoxy-7-(2-((3-methoxycyclobutyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
150) N-(3-fluoro-4-((6-methoxy-7-(2-(oxetan-3-ylamino)ethoxy)quinolin-4-yl)oxy)phenyl)-5-(4-fluorophenyl)-6-oxo-2,3,5,6-tetrahydrofuro[3,2-c]pyridine-7-carboxamide;
151) N-(3-fluoro-4-((6-methoxy-7-(2-((oxetan-3-ylmethyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
152) N-(3-fluoro-4-((7-(2-((4-hydroxycyclohexyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
153) N-(3-fluoro-4-((7-(2-((3-hydroxycyclohexyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
154) N-(3-fluoro-4-((7-(2-(((3-hydroxycyclohexyl)methyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
155) N-(3-fluoro-4-((6-methoxy-7-(2-((tetrahydro-2H-pyran-4-yl)amino)ethoxy)quinolin-4-yl)oxy)phenyl)-5-(4-fluorophenyl)-6-oxo-2,3,5,6-tetrahydrofuro[3,2-c]pyridine-7-carboxamide;
156) N-(3-fluoro-4-((6-methoxy-7-(2-((4-methoxycyclohexyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
157) N-(3-fluoro-4-((7-(2-morpholinylethoxy)quinolin-4-yl)oxy)phenyl)-5-(4-fluorophenyl)-6-oxo-2,3,5,6-tetrahydrofuro[3,2-c]pyridine-7-carboxamide;
158) N-(3-fluoro-4-((7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
159) N-(3-fluoro-4-((7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide;
160) N-(3-fluoro-4-((7-(3-(4-methylpiperazin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
161) 7-(2-(3-fluoro-4-((7-(3-(piperidin-1-yl)propoxy)quinolin-4-yl)oxy)phenyl)acetyl)-5-(4-fluorophenyl)-3,5-dihydrofuro[3,2-c]pyridin-6(2H)-one;
162) 7-(2-(3-fluoro-4-((7-(3-(4-methylpiperidin-1-yl)propoxy)quinolin-4-yl)oxy)phenyl)acetyl)-5-(4-fluorophenyl)-3,5-dihydrofuro[3,2-c]pyridin-6(2H)-one;
163) N-(3-fluoro-4-((6-(methylcarbamoyl)-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
164) N-(3-fluoro-4-((6-(methylcarbamoyl)-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
165) N-(3-fluoro-4-((6-methoxy-7-(methylcarbamoyl)quinolin-4-yl)oxy)phenyl)-5-(4-fluorophenyl)-6-oxo-2,3,5,6-tetrahydrofuro[3,2-c]pyridine-7-carboxamide;
166) N-(3-fluoro-4-((7-(methylcarbamoyl)-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
167) N-(3-fluoro-4-((6-fluoro-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
168) N-(3-fluoro-4-((6-fluoro-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
169) N-(3-fluoro-4-((7-(2-morpholinoethoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide;
170) N-(3-fluoro-4-((7-(3-morpholinopropoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide;
171) N-(4-((6-chloro-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide;
172) N-(4-((6-chloro-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide;
173) N-(4-((6-cyano-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide;
174) N-(4-((6-cyano-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide;
175) N-(3-fluoro-4-((7-methoxy-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
176) N-(3-fluoro-4-((7-methoxy-6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
177) N-(3-fluoro-4-((6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
178) N-(3-fluoro-4-((6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
179) N-(4-((6-amino-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
180) N-(3-fluoro-4-((7-methoxy-6-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide;
181) N-(4-((7-amino-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
182) N-(3-fluoro-4-((6-methoxy-7-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide;
183) N-(3-fluoro-4-((6-methoxy-7-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide;
184) N-(4-((7-acetamido-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
185) N-(3-fluoro-4-((7-methoxy-6-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide;
186) N-(3-fluoro-4-((7-methoxy-6-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide;
187) N-(3-fluoro-4-((6-methoxy-7-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide; and
188) N-(3-fluoro-4-((6-methoxy-7-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide.

According to still yet another embodiment, the compound of Formula 1 may have a structure of Formula 1c: 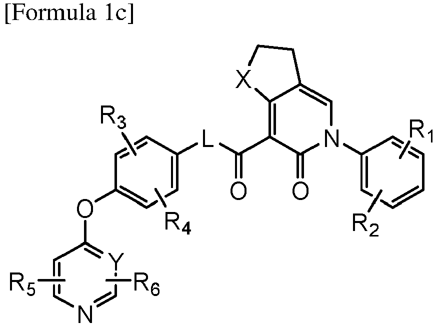 in the formula,
X, L, R₁, R₂, R₃, and R₄ are as defined in Formula 1,
Y is -N= or -CH=, and
R₅ and R₆ are each independently hydrogen, amino, halogen, aminocarbonyl, or C₁₋₆ alkylamino,
wherein R₅ and R₆ are each independently optionally substituted with 3- to 9-membered heterocycloalkyl,
the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heterocycloalkyl contains 1 to 4 heteroatoms selected from the group consisting of N, O, and S.

Specific examples of the compound represented by Formula 1c are as follows:
54) N-(4-((2-carbamoyl-3-chloropyridin-4-yl)oxy)-3-fluorophenyl)-5-(4-fluorophenyl)-6-oxo-2,3,5,6-tetrahydrofuro[3,2-c]pyridine-7-carboxamide;
55) N-(4-((2-amino-3-chloropyridin-4-yl)oxy)-3-fluorophenyl)-5-(4-fluorophenyl)-6-oxo-2,3,5,6-tetrahydrofuro[3,2-c]pyridine-7-carboxamide;
56) N-(4-((2-aminopyridin-4-yl)oxy)-3-fluorophenyl)-5-(4-fluorophenyl)-6-oxo-2,3,5,6-tetrahydrofuro[3,2-c]pyridine-7-carboxamide;
57) N-(4-((2-aminopyrimidin-4-yl)oxy)-3-fluorophenyl)-5-(4-fluorophenyl)-6-oxo-2,3,5,6-tetrahydrofuro[3,2-c]pyridine-7-carboxamide;
58) N-(4-((6-aminopyrimidin-4-yl)oxy)-3-fluorophenyl)-5-(4-fluorophenyl)-6-oxo-2,3,5,6-tetrahydrofuro[3,2-c]pyridine-7-carboxamide;
59) N-(3-fluoro-4-((4-((tetrahydro-2H-pyran-4-yl)amino)pyridin-2-yl)oxy)phenyl)-5-(4-fluorophenyl)-6-oxo-2,3,2,6-tetrahydrofuro[3,2-c]pyridine-7-carboxamide;
60) N-(3-fluoro-4-((2-((tetrahydro-2H-pyran-4-yl)amino)pyridin-4-yl)oxy)phenyl)-5-(4-fluorophenyl)-6-oxo-2,3,5,6-tetrahydrofuro[3,2-c]pyridine-7-carboxamide;
61) N-(3-fluoro-4-((6-((1-methylpiperidin-4-yl)amino)pyrimidin-4-yl)oxy)phenyl)-5-(4-fluorophenyl)-6-oxo-2,3,5,6-tetrahydrofuro[3,2-c]pyridine-7-carboxamide;
62) N-(4-((3-chloro-2-((3-morpholinopropyl)amino)pyridin-4-yl)oxy)-3-fluorophenyl)-5-(4-fluorophenyl)-6-oxo-2,3,5,6-tetrahydrofuro[3,2-c]pyridine-7-carboxamide; and
63) N-(3-fluoro-4-((2-((1-methylpiperidin-4-yl)amino)pyridin-4-yl)oxy)phenyl)-5-(4-fluorophenyl)-6-oxo-2,3,5,6-tetrahydrofuro[3,2-c]pyridine-7-carboxamide.

According to still yet another embodiment, in the compound of Formula 1, A may be pyrrolopyridine, pyrazolopyridine, imidazopyridine, pyrrolopyrimidine, dihydropyrrolopyrimidine, furopyridine, pyrazolopyrimidine, purine, or indazole; and R₅ and R₆ may each independently be hydrogen or 5- to 9-membered heteroaryl, wherein the heteroaryl may contain one or more heteroatoms selected from the group consisting of N, O, and S.

Specific examples of the compound according to the embodiment are as follows:
64) N-(3-fluoro-4-((1-methyl-6-(1H-pyrazol-4-yl)-1H-indazol-5-yl)oxy)phenyl)-5-(4-fluorophenyl)-2-oxo-2,3,5,6-tetrahydrofuro[3,2-c]pyridine-7-carboxamide;
65) N-(4-((1H-pyrrolo[2,3-b]pyridin-4-yl)oxy)-3-fluorophenyl)-5-(4-fluorophenyl)-6-oxo-2,3,5,6-tetrahydrofuro[3,2-c]pyridine-7-carboxamide;
66) N-(4-((3H-imidazo[4,5-b]pyridin-7-yl)oxy)-3-fluorophenyl)-5-(4-fluorophenyl)-6-oxo-2,3,5,6-tetrahydrofuro[3,2-c]pyridine-7-carboxamide;
67) N-(3-fluoro-4-((2-methyl-3H-imidazo[4,5-b]pyridin-7-yl)oxy)phenyl)-5-(4-fluorophenyl)-6-oxo-2,3,5,6-tetrahydrofuro[3,2-c]pyridine-7-carboxamide;
68) N-(4-((1H-pyrazolo[3,4-b]pyridin-4-yl)oxy)-3-fluorophenyl)-5-(4-fluorophenyl)-6-oxo-2,3,5,6-tetrahydrofuro[3,2-c]pyridine-7-carboxamide;
69) N-(3-fluoro-4-((furo[3,2-b]pyridin-7-yl)oxy)phenyl)-5-(4-fluorophenyl)-6-oxo-2,3,5,6-tetrahydrofuro[3,2-c]pyridine-7-carboxamide;
70) N-(3-fluoro-4-((2-methyl-1H-pyrrolo[2,3-b]pyridin-4-yl)oxy)-3-fluorophenyl)-5-(4-fluorophenyl)-6-oxo-2,3,5,6-tetrahydrofuro[3,2-c]pyridine-7-carboxamide;
71) N-(4-((7H-pyrrolo[2,3-d]pyrimidin-4-yl)oxy)-3-fluorophenyl)-5-(4-fluorophenyl)-6-oxo-2,3,5,6-tetrahydrofuro[3,2-c]pyridine-7-carboxamide;
72) N-(4-((9H-purin-6-yl)oxy)-3-fluorophenyl)-5-(4-fluorophenyl)-6-oxo-2,3,5,6-tetrahydrofuro[3,2-c]pyridine-7-carboxamide;
73) N-(4-((1H-pyrazolo[3,4-d]pyrimidin-4-yl)oxy)-3-fluorophenyl)-5-(4-fluorophenyl)-6-oxo-2,3,5,6-tetrahydrofuro[3,2-c]pyridine-7-carboxamide;
74) N-(4-((6,7-dihydro-5H-pyrrolo[2,3-d]pyrimidin-4-yl)oxy)-3-fluorophenyl)-5-(4-fluorophenyl)-6-oxo-2,3,5,6-tetrahydrofuro[3,2-c]pyridine-7-carboxamide; and
75) N-(3-fluoro-4-(pyrazolo[1,5-a]pyrimidin-7-yloxy)phenyl)-5-(4-fluorophenyl)-6-oxo-2,3,5,6-tetrahydro[3,2-c]pyridine-7-carboxamide.

The present invention includes a pharmaceutically acceptable salt of the compound represented by Formula 1.

The pharmaceutically acceptable salt should have low toxicity to humans and should not have any negative effect on the biological activity and physicochemical properties of its parent compound.

For example, the pharmaceutically acceptable salt may be an acid addition salt formed with a pharmaceutically acceptable free acid.

As the free acid, an inorganic acid or an organic acid may be used, wherein the inorganic acid may be hydrochloric acid, sulfuric acid, nitric acid, phosphoric acid, perchloric acid, bromic acid, or the like, and the organic acid may be acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, or the like.

The acid addition salt may be prepared by a conventional method, for example, by dissolving the compound of Formula 1 in an excess amount of acid aqueous solution, and causing the salt thus formed to precipitate using a water-miscible organic solvent such as methanol, ethanol, acetone, or acetonitrile.

In addition, the pharmaceutically acceptable salt may be an alkali metal salt (such as sodium salt) or an alkaline earth metal salt (such as potassium salt).

The alkali metal salt or the alkaline earth metal salt may be obtained, for example, by dissolving the compound of Formula 1 in an excess amount of alkali metal hydroxide or alkaline earth metal hydroxide solution, filtering the undissolved compound salt, and then evaporating and drying the filtrate.

In addition, the compounds of the present invention may have a chiral carbon center, and thus may exist in the form of R or S isomers, racemic compounds, individual enantiomers or a mixture thereof, or individual diastereomers or a mixture thereof. All such stereoisomers and mixtures thereof may be included in the scope of the present invention.

In addition, the compound of the present invention may include hydrates and solvates of the compound of Formula 1. The hydrates and the solvates may be prepared using known methods, and are preferably non-toxic and water-soluble. In particular, the hydrates and the solvates may preferably be those obtained by binding, to the compound, 1 to 5 molecules of water and alcoholic solvent (in particular, ethanol or the like), respectively.

Specific examples of the compound represented by Formula 1 of the present invention are as follows, and pharmaceutically acceptable salts thereof are also included in the scope of the present invention:
1) N-(3-fluoro-4-((2-(5-(((2-methoxyethyl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
2) N-(3-fluoro-4-((2-(5-(((2-methoxyethyl)amino)methyl)pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
3) N-(3-fluoro-4-((2-(pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
4) N-(3-fluoro-4-((2-(pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
5) N-(3-fluoro-4-((2-(5-(morpholinomethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
6) N-(3-fluoro-4-((2-(5-(morpholinomethyl)pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
7) N-(3-fluoro-4-((2-(5-morpholinomethyl)pyridin-2-yl)thieno[3,2-b]pyridin-7-yl)phenyl)-2-(4-fluorophenyl)-3-oxo-3,5,6,7-tetrahydro-2H-cyclopenta[c]pyridine-4-carboxamide;
8) N-(3-fluoro-4-((2-(1-methyl-1H-imidazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
9) N-(3-fluoro-4-((2-(1-methyl-1H-imidazol-4-yl)thieno[3,2-b]pyridin-7-yl)oxy)phenyl)-6-oxo-5-phenyl-2,3,5,6-tetrahydrofuro[3,2-c]pyridine-7-carboxamide;
10) N-(3-fluoro-4-((2-(1-methyl-1H-pyrazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
11) N-(3-fluoro-4-((2-(5-((3-hydroxyazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
12) N-(3-fluoro-4-((2-(5-((3-(hydroxymethyl)azetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
13) N-(3-fluoro-4-((2-(5-((3-hydroxy-3-methylazetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
14) N-(3-fluoro-4-((2-(5-((3-methoxyazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
15) N-(3-fluoro-4-((2-(5-((3-(methoxymethyl)azetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
16) N-(3-fluoro-4-((2-(5-((3-methoxy-3-methylazetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
17) N-(3-fluoro-4-((2-(5-((3-fluoroazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
18) N-(4-((2-(5-((3,3-difluoroazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
19) N-(4-((2-(5-((3-cyanoazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
20) N-(4-((2-(5-((3-(dimethylamino)azetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
21) N-(3-fluoro-4-((2-(5-((3-methylazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
22) N-(4-((2-(5-((3,3-dimethylazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
23) N-(3-fluoro-4-((2-(5-((3-hydroxypyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
24) N-(3-fluoro-4-((2-(5-((3-hydroxy-3-methylpyrrolidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
25) N-(3-fluoro-4-((2-(5-((3-methoxypyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
26) N-(3-fluoro-4-((2-(5-(pyrrolidin-1-ylmethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
27) N-(3-fluoro-4-((2-(5-((3-fluoropyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
28) N-(4-((2-(5-((3,3-difluoropyrrolidin-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide;
29) N-(3-fluoro-4-((2-(5-((4-hydroxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
30) N-(3-fluoro-4-((2-(5-((3-hydroxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
31) N-(3-fluoro-4-((2-(5-((4-methoxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
32) N-(3-fluoro-4-((2-(5-((3-methoxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
33) N-(3-fluoro-4-((2-(5-(piperidin-1-ylmethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
34) N-(3-fluoro-4-((2-(5-((4-fluoropiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
35) N-(4-((2-(5-((4,4-difluoropiperidin-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide;
36) N-(3-fluoro-4-((2-(5-((4-methylpiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
37) N-(4-((2-(5-((4,4-dimethylpiperidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
38) N-(3-fluoro-4-((2-(5-((((3-hydroxycyclohexyl)methyl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
39) N-(3-fluoro-4-((2-(5-(((tetrahydro-2H-pyran-4-yl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
40) N-(4-((6,7-dimethoxyquinolin-4-yl)oxy)-3-fluorophenyl)-5-(4-fluorophenyl)-6-oxo-2,3,5,6-tetrahydrofuro[3,2-c]pyridine-7-carboxamide;
41) N-(4-((6,7-dimethoxyquinolin-4-yl)oxy)-3-fluorophenyl)-6-oxo-5-phenyl-2,3,5,6-tetrahydrofuro[3,2-c]pyridine-7-carboxamide;
42) N-(4-((6,7-dimethoxyquinolin-4-yl)oxy)-3-fluorophenyl)-6-oxo-5-(4-(trifluoromethyl)phenyl)-2,3,5,6-tetrahydrofuro[3,2-c]pyridine-7-carboxamide;
43) N-(4-((6,7-dimethoxyquinolin-4-yl)oxy)-3-fluorophenyl)-6-oxo-5-(3-fluorophenyl)-2,3,5,6-tetrahydrofuro[3,2-c]pyridine-7-carboxamide;
44) N-(3-fluoro-4-((6-methoxyquinolin-4-yl)oxy)phenyl)-5-(4-fluorophenyl)-6-oxo-2,3,5,6-tetrahydrofuro[3,2-c]pyridine-7-carboxamide;
45) N-(3-fluoro-4-((7-methoxyquinolin-4-yl)oxy)phenyl)-5-(4-fluorophenyl)-6-oxo-2,3,5,6-tetrahydrofuro[3,2-c]pyridine-7-carboxamide;
46) N-(4-((6,7-dimethoxyquinazolin-4-yl)oxy)-3-fluorophenyl)-5-(4-fluorophenyl)-6-oxo-2,3,5,6-tetrahydrofuro[3,2-c]pyridine-7-carboxamide;
47) N-(4-((6-carbamoyl-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
48) N-(3-fluoro-4-((7-methoxy-6-(methylcarbamoyl)quinolin-4-yl)oxy)phenyl)-5-(4-fluorophenyl)-6-oxo-2,3,5,6-tetrahydrofuro[3,2-c]pyridine-7-carboxamide;
49) N-(4-((6-(dimethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
50) N-(3-fluoro-4-((7-methoxy-6-((2-morpholinoethyl)carbamoyl)quinolin-4-yl)oxy)phenyl)-5-(4-fluorophenyl)-6-oxo-2,3,5,6-tetrahydrofuro[3,2-c]pyridine-7-carboxamide;
51) N-(4-((6-(ethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
52) N-(4-((6-acetamido-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
53) N-(3-fluoro-4-((7-methoxy-6-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide;
54) N-(4-((2-carbamoyl-3-chloropyridin-4-yl)oxy)-3-fluorophenyl)-5-(4-fluorophenyl)-6-oxo-2,3,5,6-tetrahydrofuro[3,2-c]pyridine-7-carboxamide;
55) N-(4-((2-amino-3-chloropyridin-4-yl)oxy)-3-fluorophenyl)-5-(4-fluorophenyl)-6-oxo-2,3,5,6-tetrahydrofuro[3,2-c]pyridine-7-carboxamide;
56) N-(4-((2-aminopyridin-4-yl)oxy)-3-fluorophenyl)-5-(4-fluorophenyl)-6-oxo-2,3,5,6-tetrahydrofuro[3,2-c]pyridine-7-carboxamide;
57) N-(4-((2-aminopyrimidin-4-yl)oxy)-3-fluorophenyl)-5-(4-fluorophenyl)-6-oxo-2,3,5,6-tetrahydrofuro[3,2-c]pyridine-7-carboxamide;
58) N-(4-((6-aminopyrimidin-4-yl)oxy)-3-fluorophenyl)-5-(4-fluorophenyl)-6-oxo-2,3,5,6-tetrahydrofuro[3,2-c]pyridine-7-carboxamide;
59) N-(3-fluoro-4-((4-((tetrahydro-2H-pyran-4-yl)amino)pyridin-2-yl)oxy)phenyl)-5-(4-fluorophenyl)-6-oxo-2,3,2,6-tetrahydrofuro[3,2-c]pyridine-7-carboxamide;
60) N-(3-fluoro-4-((2-((tetrahydro-2H-pyran-4-yl)amino)pyridin-4-yl)oxy)phenyl)-5-(4-fluorophenyl)-6-oxo-2,3,5,6-tetrahydrofuro[3,2-c]pyridine-7-carboxamide;
61) N-(3-fluoro-4-((6-((1-methylpiperidin-4-yl)amino)pyrimidin-4-yl)oxy)phenyl)-5-(4-fluorophenyl)-6-oxo-2,3,5,6-tetrahydrofuro[3,2-c]pyridine-7-carboxamide;
62) N-(4-((3-chloro-2-((3-morpholinopropyl)amino)pyridin-4-yl)oxy)-3-fluorophenyl)-5-(4-fluorophenyl)-6-oxo-2,3,5,6-tetrahydrofuro[3,2-c]pyridine-7-carboxamide;
63) N-(3-fluoro-4-((2-((1-methylpiperidin-4-yl)amino)pyridin-4-yl)oxy)phenyl)-5-(4-fluorophenyl)-6-oxo-2,3,5,6-tetrahydrofuro[3,2-c]pyridine-7-carboxamide;
64) N-(3-fluoro-4-((1-methyl-6-(1H-pyrazol-4-yl)-1H-indazol-5-yl)oxy)phenyl)-5-(4-fluorophenyl)-2-oxo-2,3,5,6-tetrahydrofuro[3,2-c]pyridine-7-carboxamide;
65) N-(4-((1H-pyrrolo[2,3-b]pyridin-4-yl)oxy)-3-fluorophenyl)-5-(4-fluorophenyl)-6-oxo-2,3,5,6-tetrahydrofuro[3,2-c]pyridine-7-carboxamide;
66) N-(4-((3H-imidazo[4,5-b]pyridin-7-yl)oxy)-3-fluorophenyl)-5-(4-fluorophenyl)-6-oxo-2,3,5,6-tetrahydrofuro[3,2-c]pyridine-7-carboxamide;
67) N-(3-fluoro-4-((2-methyl-3H-imidazo[4,5-b]pyridin-7-yl)oxy)phenyl)-5-(4-fluorophenyl)-6-oxo-2,3,5,6-tetrahydrofuro[3,2-c]pyridine-7-carboxamide;
68) N-(4-((1H-pyrazolo[3,4-b]pyridin-4-yl)oxy)-3-fluorophenyl)-5-(4-fluorophenyl)-6-oxo-2,3,5,6-tetrahydrofuro[3,2-c]pyridine-7-carboxamide;
69) N-(3-fluoro-4-((furo[3,2-b]pyridin-7-yl)oxy)phenyl)-5-(4-fluorophenyl)-6-oxo-2,3,5,6-tetrahydrofuro[3,2-c]pyridine-7-carboxamide;
70) N-(3-fluoro-4-((2-methyl-1H-pyrrolo[2,3-b]pyridin-4-yl)oxy)-3-fluorophenyl)-5-(4-fluorophenyl)-6-oxo-2,3,5,6-tetrahydrofuro[3,2-c]pyridine-7-carboxamide;
71) N-(4-((7H-pyrrolo[2,3-d]pyrimidin-4-yl)oxy)-3-fluorophenyl)-5-(4-fluorophenyl)-6-oxo-2,3,5,6-tetrahydrofuro[3,2-c]pyridine-7-carboxamide;
72) N-(4-((9H-purin-6-yl)oxy)-3-fluorophenyl)-5-(4-fluorophenyl)-6-oxo-2,3,5,6-tetrahydrofuro[3,2-c]pyridine-7-carboxamide;
73) N-(4-((1H-pyrazolo[3,4-d]pyrimidin-4-yl)oxy)-3-fluorophenyl)-5-(4-fluorophenyl)-6-oxo-2,3,5,6-tetrahydrofuro[3,2-c]pyridine-7-carboxamide;
74) N-(4-((6,7-dihydro-5H-pyrrolo[2,3-d]pyrimidin-4-yl)oxy)-3-fluorophenyl)-5-(4-fluorophenyl)-6-oxo-2,3,5,6-tetrahydrofuro[3,2-c]pyridine-7-carboxamide;
75) N-(3-fluoro-4-(pyrazolo[1,5-a]pyrimidin-7-yloxy)phenyl)-5-(4-fluorophenyl)-6-oxo-2,3,5,6-tetrahydro[3,2-c]pyridine-7-carboxamide;
76) N-(3-fluoro-4-((6-methoxy-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
77) N-(3-fluoro-4-((6-methoxy-7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide;
78) N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
79) N-(3-fluoro-4-((6-methoxy-7-(2-morpholinoethoxy)quinolin-4-yl)oxy)phenyl)-6-oxo-5-phenyl-2,3,5,6-tetrahydrofuro[3,2-c]pyridine-7-carboxamide;
80) N-(3-fluoro-4-((6-methoxy-7-(3-(4-methylpiperazin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
81) N-(3-fluoro-4-((7-(3-(3-hydroxyazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
82) N-(3-fluoro-4-((7-(3-(3-hydroxy-3-methylazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
83) N-(3-fluoro-4-((7-(3-(3-(hydroxymethyl)azetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
84) N-(3-fluoro-4-((6-methoxy-7-(3-(3-methoxyazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
85) N-(3-fluoro-4-((6-methoxy-7-(3-(3-methoxy-3-methylazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
86) N-(3-fluoro-4-((7-(3-(3-fluoroazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
87) N-(4-((7-(3-(3,3-difluoroazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
88) N-(4-((7-(3-(3-cyanoazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
89) N-(3-fluoro-4-((6-methoxy-7-(3-(3-methylazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
90) N-(3-fluoro-4-((7-(3-(3-hydroxypyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
91) N-(3-fluoro-4-((7-(3-(3-hydroxy-3-methylpyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
92) N-(3-fluoro-4-((6-methoxy-7-(3-(3-methoxypyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
93) N-(3-fluoro-4-((6-methoxy-7-(3-(pyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
94) N-(3-fluoro-4-((7-(3-(3-fluoropyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
95) N-(4-((7-(3-(3,3-difluoropyrrolidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
96) N-(3-fluoro-4-((7-(3-(4-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
97) N-(3-fluoro-4-((7-(3-(3-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
98) N-(3-fluoro-4-((7-(3-(4-hydroxy-4-methylpiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
99) N-(3-fluoro-4-((6-methoxy-7-(3-(4-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
100) N-(3-fluoro-4-((6-methoxy-7-(3-(3-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
101) N-(3-fluoro-4-((6-methoxy-7-(3-(4-oxopiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
102) N-(4-((7-(3-(1,1-dioxidothiomorpholino)propoxy)-6-methoxyqumolin-4-yl)oxy)-3-fluorophenyl)-5-(4-fluorophenyl)-6-oxo-2,3,5,6-tetrahydrofuro[3,2-c]pyridine-7-carboxamide;
103) N-(3-fluoro-4-((6-methoxy-7-(3-(piperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
104) N-(3-fluoro-4-((7-(3-(4-fluoropiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
105) N-(4-((7-(3-(4,4-difluoropiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
106) N-(3-fluoro-4-((6-methoxy-7-(3-(4-methylpiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
107) N-(4-((7-(3-(4,4-dimethylpiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
108) N-(3-fluoro-4-((7-(3-((3-hydroxycyclobutyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
109) N-(3-fluoro-4-((6-methoxy-7-(3-((3-methoxycyclobutyl)amino)propoxy)quinolin-4-yl)oxy)phenyl)-5-(4-fluorophenyl)-6-oxo-2,3,5,6-tetrahydrofuro[3,2-c]pyridine-7-carboxamide;
110) N-(3-fluoro-4-((6-methoxy-7-(3-(oxetan-3-ylamino)propoxy)quinolin-4-yl)oxy)phenyl)-5-(4-fluorophenyl)-6-oxo-2,3,5,6-tetrahydrofuro[3,2-c]pyridine-7-carboxamide;
111) N-(3-fluoro-4-((6-methoxy-7-(3-((oxetan-3-ylmethyl)amino)propoxy)quinolin-4-yl)oxy)phenyl)-5-(4-fluorophenyl)-6-oxo-2,3,5,6-tetrahydrofuro[3,2-c]pyridine-7-carboxamide;
112) N-(3-fluoro-4-((7-(3-((3-hydroxycyclopentyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
113) N-(3-fluoro-4-((7-(3-((3-hydroxycyclohexyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
114) N-(3-fluoro-4-((7-(3-(((3-hydroxycyclohexyl)methyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
115) N-(3-fluoro-4-((6-methoxy-7-(3-((tetrahydro-2H-pyran-4-yl)amino)propoxy)quinolin-4-yl)oxy)phenyl)-5-(4-fluorophenyl)-6-oxo-2,3,5,6-tetrahydrofuro[3,2-c]pyridine-7-carboxamide;
116) N-(3-fluoro-4-((6-methoxy-7-(3-(((tetrahydro-2H-pyran-4-yl)methyl)amino)propoxy)quinolin-4-yl)oxy)phenyl)-5-(4-fluorophenyl)-6-oxo-2,3,5,6-tetrahydrofuro[3,2-c]pyridine-7-carboxamide;
117) N-(3-fluoro-4-((7-(2-(3-hydroxyazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
118) N-(3-fluoro-4-((7-(2-(3-hydroxy-3-methylazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
119) N-(3-fluoro-4-((7-(2-(3-(hydroxymethyl)azetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
120) N-(3-fluoro-4-((6-methoxy-7-(2-(3-methoxyazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
121) N-(3-fluoro-4-((6-methoxy-7-(2-(3-methoxy-3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
122) N-(3-fluoro-4-((6-methoxy-7-(2-(3-(methoxymethyl)azetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
123) N-(3-fluoro-4-((7-(2-(3-fluoroazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
124) N-(4-((7-(2-(3,3-difluoroazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
125) N-(4-((7-(2-(3-ethynylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
126) N-(3-fluoro-4-((6-methoxy-7-(2-(3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
127) N-(4-((7-(2-(3,3-dimethylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
128) N-(4-((7-(2-(3-(dimethylamino)azetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
129) N-(3-fluoro-4-((7-(2-(3-hydroxypyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
130) N-(3-fluoro-4-((7-(2-(3-hydroxy-3-methylpyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
131) N-(3-fluoro-4-((6-methoxy-7-(2-(3-methoxypyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
132) N-(3-fluoro-4-((6-methoxy-7-(2-(pyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
133) N-(3-fluoro-4-((7-(2-(3-fluoropyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
134) N-(4-((7-(2-(3,3-difluoropyrrolidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
135) N-(3-fluoro-4-((7-(2-(4-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
136) N-(3-fluoro-4-((7-(2-(3-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
137) N-(3-fluoro-4-((7-(2-(4-hydroxy-4-methylpiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
138) N-(3-fluoro-4-((6-methoxy-7-(2-(4-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
139) N-(3-fluoro-4-((6-methoxy-7-(2-(3-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
140) N-(3-fluoro-4-((6-methoxy-7-(2-(4-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
141) N-(3-fluoro-4-((6-methoxy-7-(2-(3-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
142) N-(4-((7-(2-(1,1-dioxidothiomorpholino)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
143) N-(3-fluoro-4-((6-methoxy-7-(2-(piperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
144) N-(3-fluoro-4-((7-(2-(4-fluoropiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
145) N-(4-((7-(2-(4,4-difluoropiperidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
146) N-(3-fluoro-4-((6-methoxy-7-(2-(4-methylpiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
147) N-(4-((7-(2-(4,4-dimethylpiperidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
148) N-(3-fluoro-4-((7-(2-((3-hydroxycyclobutyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
149) N-(3-fluoro-4-((6-methoxy-7-(2-((3-methoxycyclobutyl)amino)ethoxy)qumolm-4-yl)oxy)phenyl)-5-(4-fluorophenyl)-6-oxo-2,3,5,6-tetrahydrofuro[3,2-c]pyridine-7-carboxamide;
150) N-(3-fluoro-4-((6-methoxy-7-(2-(oxetan-3-ylamino)ethoxy)quinolin-4-yl)oxy)phenyl)-5-(4-fluorophenyl)-6-oxo-2,3,5,6-tetrahydrofuro[3,2-c]pyridine-7-carboxamide;
151) N-(3-fluoro-4-((6-methoxy-7-(2-((oxetan-3-ylmethyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
152) N-(3-fluoro-4-((7-(2-((4-hydroxycyclohexyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
153) N-(3-fluoro-4-((7-(2-((3-hydroxycyclohexyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
154) N-(3-fluoro-4-((7-(2-(((3-hydroxycyclohexyl)methyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
155) N-(3-fluoro-4-((6-methoxy-7-(2-((tetrahydro-2H-pyran-4-yl)amino)ethoxy)quinolin-4-yl)oxy)phenyl)-5-(4-fluorophenyl)-6-oxo-2,3,5,6-tetrahydrofuro[3,2-c]pyridine-7-carboxamide;
156) N-(3-fluoro-4-((6-methoxy-7-(2-((4-methoxycyclohexyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
157) N-(3-fluoro-4-((7-(2-morpholinylethoxy)quinolin-4-yl)oxy)phenyl)-5-(4-fluorophenyl)-6-oxo-2,3,5,6-tetrahydrofuro[3,2-c]pyridine-7-carboxamide;
158) N-(3-fluoro-4-((7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
159) N-(3-fluoro-4-((7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide;
160) N-(3-fluoro-4-((7-(3-(4-methylpiperazin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
161) 7-(2-(3-fluoro-4-((7-(3-(piperidin-1-yl)propoxy)quinolin-4-yl)oxy)phenyl)acetyl)-5-(4-fluorophenyl)-3,5-dihydrofuro[3,2-c]pyridin-6(2H)-one;
162) 7-(2-(3-fluoro-4-((7-(3-(4-methylpiperidin-1-yl)propoxy)quinolin-4-yl)oxy)phenyl)acetyl)-5-(4-fluorophenyl)-3,5-dihydrofuro[3,2-c]pyridin-6(2H)-one;
163) N-(3-fluoro-4-((6-(methylcarbamoyl)-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
164) N-(3-fluoro-4-((6-(methylcarbamoyl)-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
165) N-(3-fluoro-4-((6-methoxy-7-(methylcarbamoyl)quinolin-4-yl)oxy)phenyl)-5-(4-fluorophenyl)-6-oxo-2,3,5,6-tetrahydrofuro[3,2-c]pyridine-7-carboxamide;
166) N-(3-fluoro-4-((7-(methylcarbamoyl)-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
167) N-(3-fluoro-4-((6-fluoro-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
168) N-(3-fluoro-4-((6-fluoro-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
169) N-(3-fluoro-4-((7-(2-morpholinoethoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide;
170) N-(3-fluoro-4-((7-(3-morpholinopropoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide;
171) N-(4-((6-chloro-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide;
172) N-(4-((6-chloro-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide;
173) N-(4-((6-cyano-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide;
174) N-(4-((6-cyano-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide;
175) N-(3-fluoro-4-((7-methoxy-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
176) N-(3-fluoro-4-((7-methoxy-6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
177) N-(3-fluoro-4-((6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
178) N-(3-fluoro-4-((6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
179) N-(4-((6-amino-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
180) N-(3-fluoro-4-((7-methoxy-6-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide;
181) N-(4-((7-amino-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
182) N-(3-fluoro-4-((6-methoxy-7-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide;
183) N-(3-fluoro-4-((6-methoxy-7-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide;
184) N-(4-((7-acetamido-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
185) N-(3-fluoro-4-((7-methoxy-6-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide;
186) N-(3-fluoro-4-((7-methoxy-6-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide;
187) N-(3-fluoro-4-((6-methoxy-7-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide; and
188) N-(3-fluoro-4-((6-methoxy-7-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide.

In addition, in another aspect of the present invention, there is provided a method for preparing the compound of Formula 1.

Preferably, the compound of Formula 1 can be prepared using a method as shown by the following schemes. However, the present invention is not limited to a case where the compound is prepared by this method. In particular, those skilled in the art will be able to fully understand that the compound of Formula 1 of the present invention can be prepared by various methods using techniques well known in the art.

The following schemes show, in respective preparation steps, methods for preparing representative compounds according to the present invention, and various compounds of the present invention may be prepared by changing reagents and solvents used in the following preparation steps or by changing the order of reactions. In addition, the compounds of the present invention in some examples were prepared according to procedures, which are not included in the scope of the following schemes, and detailed preparation procedures for these compounds are described in detail in each of these examples.

According to an embodiment, the compound of Formula 1 may be prepared according to the procedures in Schemes 1 and 2.

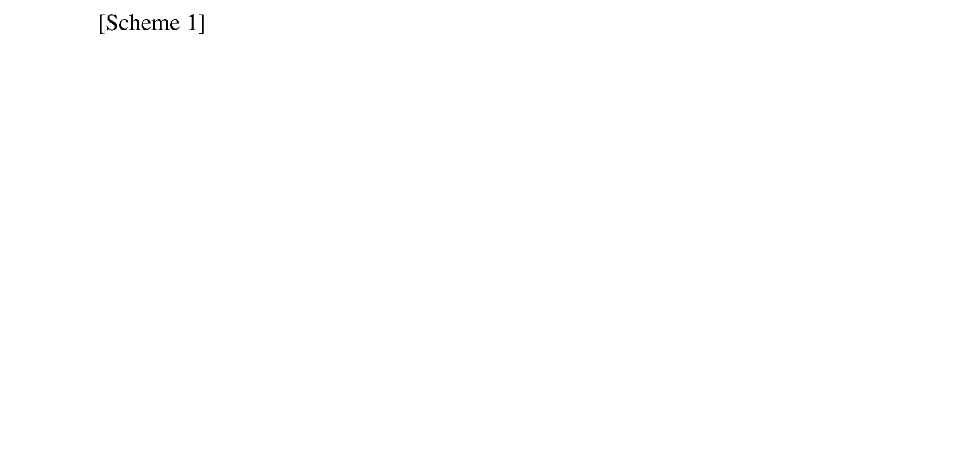

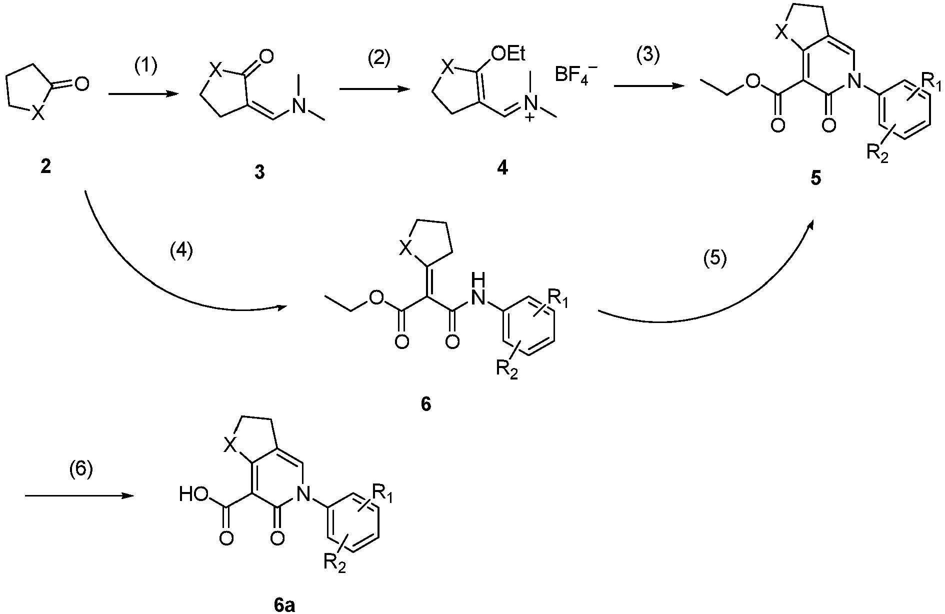

In Scheme 1, R₁, R₂, and X are as defined in Formula 1.

According to Scheme 1, a carboxylic acid compound (6a) is prepared using, as a starting material, a lactone-based compound (2) that can be easily obtained commercially or is prepared by a known method.

Regarding Scheme 1, detailed description of each step is as follows.

In step 1, a compound (2), which can be easily obtained commercially, is subjected to formylation reaction using dimethyldimethoxyacetal, to prepare a compound (3). The reaction may generally be carried out at a high temperature; however, this is disadvantageous in that a long reaction time is required. Thus, the reaction is carried out using a microwave reactor.

In step 2, a compound (4) is prepared using the formylated lactone compound (3) in step 1 and triethyloxonium tetrafluoroborate which can be easily obtained commercially. This reaction is carried out under anhydrous conditions and is preferably carried out using a solvent, which does not adversely affect the reaction, such as N,N-dichloromethane or chloroform. For the reaction temperature, the reaction is generally carried out at room temperature. In step 3, the compound (4) prepared in step 2 is reacted with an ethyl-3-amino-3-oxopropionate compound, which has been prepared by a known method, in the presence of sodium ethoxide, to prepare a cyclized compound (5). This reaction is preferably carried out using an ethanol solvent that does not adversely affect the reaction. The reaction temperature is not particularly limited. The reaction may generally be carried out at a cold to warm temperature, and is preferably carried out at room temperature.

Alternatively, to prepare the cyclized compound (5), as in step 4, the lactone-based compound (2), which can be easily obtained commercially and is used as a starting material, may be reacted with an ethyl-3-amino-3-oxopropionate compound, which has been prepared by a known method, in the presence of titanium tetrachloride and pyridine, to prepare compound (6). This reaction is preferably carried out using dichloromethane that does not adversely affect the reaction. The reaction temperature is not particularly limited; and the reaction may generally be carried out at a cold to room temperature, and preferably starting from a cold temperature up to room temperature.

Thereafter, in step 5, the compound (6) prepared in step 4 is subjected to formylation and cyclization using dimethyldimethoxyacetal, to prepare the compound (5). The reaction may be generally carried out at a warm or high temperature, and is preferably carried out at a warm temperature.

In step 6, the cyclized compound (5) prepared in steps 3 and 5 is subjected to hydrolysis, to prepare the carboxylic acid compound (6a). In general, the hydrolysis is carried out using a basic aqueous solution such as an aqueous sodium hydroxide solution or an aqueous lithium hydroxide solution. This reaction is carried out using a solvent, which does not adversely affect the reaction, such as ethanol, methanol, or tetrahydrofuran, in the presence of an aqueous lithium hydroxide solution that can be used in the hydrolysis. The reaction temperature is not particularly limited. The reaction may generally be carried out at room temperature or a warm temperature, preferably at a warm temperature, to prepare the carboxylic acid compound (6a).

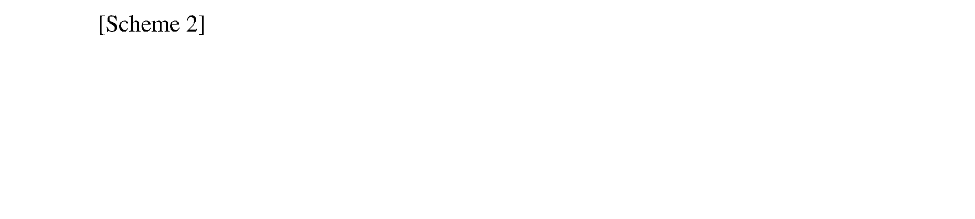

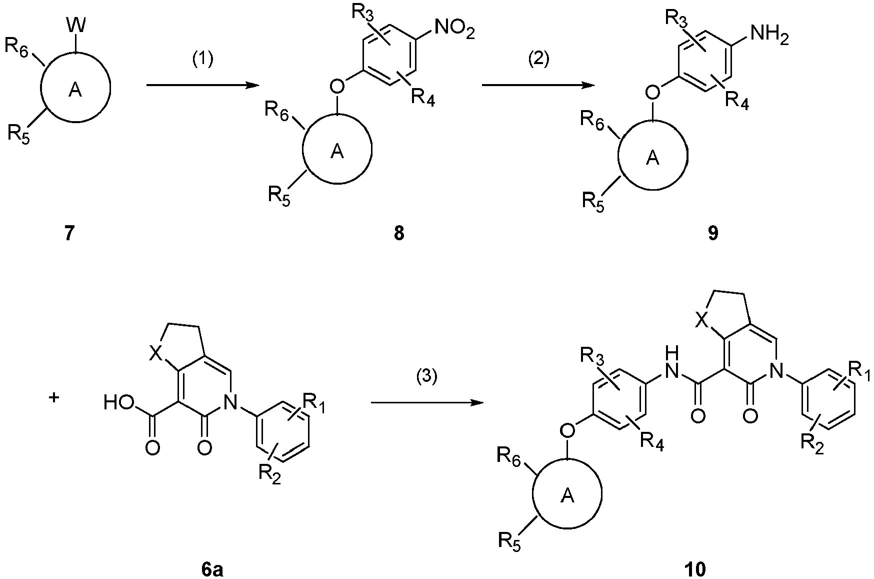

In Scheme 2, R₁ to R₆, A, and X are as defined in Formula 1, and W is a leaving group.

Scheme 2 specifically shows each step for preparing a desired compound (10) of the present invention.

In step 1, a monocyclic or bicyclic compound (7), which can be easily obtained commercially or prepared by a known method, is reacted with a nitrophenol compound, which can be easily obtained commercially, in the presence of a base such as potassium carbonate, to obtain a phenoxy compound (8). This reaction is generally etherification of a phenol compound and is carried out in the presence of a base that can be used for etherification. Examples of the base that can be used for this purpose include sodium hydrate (NaH), potassium carbonate, sodium carbonate, cesium carbonate, sodium or potassium alkoxide, and the like. In addition, the reaction is preferably carried out in the presence of a solvent that does not adversely affect the reaction. For example, the reaction is carried out using a solvent such as dichloromethane, chloroform, tetrahydrofuran, diethyl ether, toluene, N,N-dimethylformamide, acetonitrile, or diphenyl ether. The reaction temperature is not particularly limited. The reaction may generally be carried out at room temperature or a warm temperature, preferably at a warm temperature.

In step 2, the nitrophenol compound (8) prepared in step 1 is subjected to reduction in the presence of iron and ammonium chloride, to prepare an amine compound (9). This reaction is generally reduction of a nitro compound to an amine, and may be carried out using various reducing agents such as hydrogen, iron, tin(II) chloride, and zinc. In addition, for this reaction, a solvent, which does not adversely affect the reaction, is used, such as dichloromethane, ethyl acetate, methanol, ethanol, tetrahydrofuran, or N,N-dimethylformamide. As the case may be, the reaction is carried out using water as a cosolvent. The reaction temperature is not particularly limited. The reaction may generally be carried out at room temperature or a warm temperature, preferably at a warm temperature.

In step 3, a common amidation reaction is carried out, in which the amine compound (9) prepared in step 2 is reacted with the carboxylic acid compound (6a) prepared in Scheme 1 using a coupling reagent, to prepare the desired compound (10). In general, the reaction is carried out using a coupling reagent, which can be easily obtained commercially, such as 1-(3-dimethylaminopropyl)-3-ethylcarbodiimide (EDC), 1,3-dicyclohexyl carbodiimide (DCC), 1,1-carbonyl diimidazole (CDI), or 4-(3-((5-(2-fluoro-4-[5-(dimethylamino)methylene]-1H-1,2,3-triazolo[4,5-b]pyridinium 3-oxide hexafluorophosphate (HATU). This reaction may be carried out without using a base; however, the reaction is carried out using a solvent, which does not adversely affect the reaction, such as acetonitrile, dimethylformamide, or dichloromethane, in the presence of a common base, which can be used for an amidation reaction, such as 4-dimethylaminopyridine, pyridine, triethylamine, diethylisopropylamine, N-methylmorpholine, or dimethylphenylamine. The reaction temperature is not particularly limited. The reaction may generally be carried out at room temperature or a warm temperature, preferably at a warm temperature, to prepare the desired compound (10).

The desired compounds produced in the schemes can be separated and purified using conventional methods, for example, column chromatography, recrystallization, and the like.

The compound represented by Formula 1 or a pharmaceutically acceptable salt thereof has an excellent inhibitory effect on protein kinase activity. Thus, the compound or a pharmaceutical composition comprising the same can be useful for prevention or treatment of a disease related to protein kinase activity.

Here, the "prevention" refers to any act of inhibiting or delaying occurrence, spread, and recurrence of the disease; and the "treatment" refers to any act of ameliorating or beneficially altering symptoms of the disease by administration of the compound.

In yet another aspect of the present invention, there is provided a use of the compound represented by Formula 1 or a pharmaceutically acceptable salt thereof for preventing or treating a disease related to protein kinase activity.

In still yet another aspect of the present invention, there is provided a use of the compound represented by Formula 1 or a pharmaceutically acceptable salt thereof for inhibiting protein kinase activity.

In still yet another aspect of the present invention, there is provided a use of the compound represented by Formula 1 or a pharmaceutically acceptable salt thereof for manufacture of a medicament for preventing or treating a disease related to protein kinase activity.

In still yet another aspect of the present invention, there is provided a use of the compound represented by Formula 1 or a pharmaceutically acceptable salt thereof for manufacture of a medicament for inhibiting protein kinase activity.

In still yet another aspect of the present invention, there is provided a method for preventing or treating a disease related to protein kinase activity, comprising a step of administering, to a subject in need thereof, the compound represented by Formula 1 or a pharmaceutically acceptable salt thereof.

In still yet another aspect of the present invention, there is provided a method for inhibiting protein kinase activity, comprising a step of administering, to a subject in need thereof, the compound represented by Formula 1 or a pharmaceutically acceptable salt thereof.

Here, the term "subject in need thereof" refers to any animal, specifically a mammal, such as a monkey, a cow, a horse, a sheep, a pig, a chicken, a turkey, a quail, a cat, a dog, a mouse, a rat, a rabbit, and a guinea pig, including a human (patient), who has or is likely to develop the disease related to protein kinase activity. In addition, the subject in need thereof may mean a biological sample.

In addition, the "administration" means providing a predetermined substance to a subject in need thereof by any appropriate method, and administration of the compound of the present invention may be achieved via any common route as long as the route allows the substance to reach a target tissue.

In still yet another aspect of the present invention, there is provided a pharmaceutical composition for preventing or treating a disease related to protein kinase activity, comprising, as an active ingredient, the compound represented by Formula 1 or a pharmaceutically acceptable salt thereof.

The pharmaceutical composition may be intended for preventing or treating a disease related to protein kinase activity. Alternatively, the pharmaceutical composition may be intended for inhibiting protein kinase activity.

Examples of the protein kinase to be inhibited include ALK, Aurora B, AXL, DDR₁, FLT3, KIT, LCK, LTK, MEK, MER, c-MET, RET, VEGFR₂, TIE1, Tyro3, and the like (see Purnima Wagh et al., Adv Cancer Res. 2008, 100: 1-33). Preferably, the protein kinase to be inhibited by the compound or pharmaceutical composition of the present invention may be tyrosine kinase, which is a c-MET family member, and more specifically RON tyrosine kinase.

Meanwhile, generally, in about 60% of colorectal cancers, cancer cells proliferate through a signaling pathway created by binding between epidermal growth factor receptor (EGFR) and epidermal growth factor (EGF). Specifically, when EGFR and EGF are bound to each other, KRAS gene is activated, causing karyomitosis of cancer cells. Cetuximab is an antibody targeting EGFR and inhibits activation of the KRAS gene by blocking the binding between EGFR and EGF. However, in a case where due to a mutation in the KRAS gene, the KRAS gene is activated without the binding between EGFR and EGF, cancer cells proliferate even when cetuximab is administered. In such a case where the KRAS gene is mutated, treatment is achieved using other targeted therapeutic agents such as crizotinib that is targeted to anaplastic lymphoma kinase (ALK). In particular, RON is overexpressed or excessively activated in colorectal cancer or breast cancer. Thus, in this respect, the compound or pharmaceutical composition of the present invention can be useful as an alternative anticancer agent in a case where cetuximab cannot be used due to a mutation in the KRAS gene.

The disease related to protein kinase activity may be cancer, psoriasis, rheumatoid arthritis, inflammatory bowel disease, or chronic obstructive pulmonary disease. Specifically, the cancer may be selected from the group consisting of breast cancer, lung cancer, gastric cancer, prostate cancer, uterine cancer, ovarian cancer, kidney cancer, pancreatic cancer, liver cancer, colorectal cancer, skin cancer, head and neck cancer, and thyroid cancer.

The pharmaceutical composition may contain, as an active ingredient, the compound represented by Formula 1 or a pharmaceutically acceptable salt thereof in an amount of 0.1% to 90% by weight, specifically 0.1% to 75% by weight, and more specifically 1% to 50% by weight, with respect to a total weight of the composition.

The pharmaceutical composition may comprise conventional and non-toxic pharmaceutically acceptable additives which are blended into a preparation according to a conventional method. For example, the pharmaceutical composition may further comprise a pharmaceutically acceptable carrier, diluent, or excipient.

Examples of the additives used in the pharmaceutical composition may include sweeteners, binders, solvents, dissolution aids, wetting agents, emulsifiers, isotonic agents, absorbents, disintegrants, antioxidants, preservatives, lubricants, fillers, flavoring agents, and the like. For example, the additives may include lactose, dextrose, sucrose, mannitol, sorbitol, cellulose, glycine, silica, talc, stearic acid, stearin, magnesium stearate, magnesium aluminosilicate, starch, gelatin, gum tragacanth, alginic acid, sodium alginate, methylcellulose, sodium carboxymethylcellulose, agar, water, ethanol, polyethylene glycol, polyvinylpyrrolidone, sodium chloride, calcium chloride, orange essence, strawberry essence, vanilla flavor, and the like.

The pharmaceutical composition may be made into various preparation forms for oral administration (for example, tablets, pills, powders, capsules, syrups, or emulsions) or parenteral administration (for example, intramuscular, intravenous, or subcutaneous injection).

Preferably, the pharmaceutical composition may be made into a preparation for oral administration, in which additives used may include cellulose, calcium silicate, corn starch, lactose, sucrose, dextrose, calcium phosphate, stearic acid, magnesium stearate, calcium stearate, gelatin, talc, surfactants, suspending agents, emulsifying agents, diluents, and the like.

Specifically, solid preparations for oral administration include tablets, pills, powders, granules, capsules, and the like, and such solid preparations can be formulated by mixing the composition with at least one excipient, such as starch, calcium carbonate, sucrose, lactose, and gelatin. In addition, in addition to simple excipients, a lubricant such as magnesium stearate and talc may be used.

In addition, as liquid preparations for oral administration, suspensions, emulsions, syrups, and the like may be exemplified, in which in addition to water and liquid paraffin which are commonly used simple diluents, various excipients, such as wetting agents, sweeteners, fragrances, and preservatives, may be included therein.

In addition, preparations for parenteral administration include sterilized aqueous solutions, non-aqueous solutions, suspensions, emulsions, lyophilized preparations, and suppositories. As the non-aqueous solution or the suspension, propylene glycol, polyethylene glycol, vegetable oil such as olive oil, injectable ester such as ethyl oleate, or the like may be used. As a base for the suppository, Witepsol, Macrogol, and Tween 61, cocoa butter, laurin fat, glycerogelatin, or the like may be used. On the other hand, the injection may comprise conventional additives such as solubilizing agents, isotonic agents, suspending agents, emulsifying agents, stabilizing agents, and preservatives.

The compound or pharmaceutical composition of the present invention may be administered to a patient in a therapeutically effective amount or a pharmaceutically effective amount.

Here, the "therapeutically effective amount" or "pharmaceutically effective amount" refers to an amount of a compound or composition effective to prevent or treat a target disease, the amount being sufficient to treat the disease at a reasonable benefit/risk ratio, which is applicable to medical treatment, without causing adverse effects. A level of the effective amount may be determined depending on factors, including the patient's health status, disease type, disease severity, drug activity, drug sensitivity, method of administration, time of administration, route of administration and rate of excretion, duration of treatment, and drugs used simultaneously or in combination, and other factors well known in the medical field.

The compound or pharmaceutical composition of the present invention may be administered as an individual therapeutic agent or administered in combination with other therapeutic agents, may be administered sequentially or simultaneously with a conventional therapeutic agent, and may be administered once or multiple times. It is important to administer an amount such that a maximum effect can be obtained with a minimum amount without adverse effects by taking all of the above-described factors into consideration, and such an amount can be readily determined by those skilled in the art.

Specifically, an effective amount of the compound contained in the pharmaceutical composition may vary depending on the patient's age, sex, and body weight. Generally, the compound may be administered in an amount of 0.1 mg to 1,000 mg, or 5 mg to 200 mg, per kg body weight, on a daily or every-other-day basis, or once or three times a day. However, the effective amount may increase or decrease depending on route of administration, disease severity, the patient's sex, body weight, and age, and the like. Thus, the scope of the present invention is not limited thereto.

Preferably, the compound or pharmaceutical composition of the present invention may be administered for tumor therapy, in combination with chemotherapy, radiation therapy, immunotherapy, hormone therapy, bone marrow transplantation, stem cell replacement therapy, other biological therapies, surgical intervention, or a combination thereof. For example, the compound or composition of the present invention may be used as adjuvant therapy in combination with other long-term treatment strategies, or may be used to maintain the patient's condition after tumor regression is induced or chemoprophylactic therapy is applied in severely ill patients.

Preferably, the pharmaceutical composition may additionally comprise one or more active ingredients, and the additional active ingredient may be, but is not limited to, an anti-proliferative compound such as an aromatase inhibitor, anti-estrogen, a topoisomerase I inhibitor, a topoisomerase II inhibitor, a microtubule active compound, an alkylated compound, a histone deacetylase inhibitor, a compound that induces cell differentiation processes, a cyclooxygenase inhibitor, an MMP inhibitors, an mTOR inhibitor, an antineoplastic anti-metabolite, a platinum compound, a compound that targets or decreases protein activity or lipid kinase activity, an anti-angiogenic compound, a compound that targets, decreases, or inhibits protein activity or lipid phosphatase activity, a gonadorelin agonist, anti-androgen, a methionine aminopeptidase inhibitor, bisphosphonate, a biological response modifier, an anti-proliferative antibody, a heparanase inhibitor, an inhibitor of Ras oncogenic isoforms, a telomerase inhibitor, a proteasome inhibitor, a compound used for treatment of hematologic malignancies, a compound that targets, decreases, or inhibits Flt-3 activity, an HSP90 inhibitor, a kinesin spindle protein inhibitor, a MEK inhibitor, leucovorin, an EDG binder, an anti-leukemic compound, a ribonucleotide reductase inhibitor, an S-adenosylmethionine decarboxylase inhibitor, a hemostatic steroid, a corticosteroid, another chemotherapy compound, or a photosensitizing compound.

Preferably, the additional active ingredient may be a known anticancer agent. Nonlimiting examples of the anticancer agent include DNA alkylating agents, such as mechlorethamine, chlorambucil, phenylalanine, mustard, cyclophosphamide, ifosfamide, carmustine (BCNU), lomustine (CCNU), streptozotocin, busulfan, thiotepa, cisplatin, and carboplatin; anti-cancer antibiotics, such as dactinomycin (actinomycin D), doxorubicin (adriamycin), daunorubicin, idarubicin, mitoxantrone, plicamycin, mitomycin C, and bleomycin; and plant alkaloids, such as vincristine, vinblastine, paclitaxel, docetaxel, etoposide, teniposide, topotecan, and irinotecan; and the like.

The pharmaceutical composition may be administered in combination with a compound or antibody which targets protein kinases other than RON. Specifically, the pharmaceutical composition may be administered in combination with cetuximab that targets EGFR.

### Mode for the Invention

Hereinafter, the present invention will be described in more detail by way of the following examples. However, the following examples are for illustrative purposes only, and the scope of the present invention is not limited thereto.

Regarding starting materials for synthesizing the compound of the present invention, various synthetic methods therefor are known. In a case where the starting material is commercially available, such a material may be purchased from its supplier and used. Suppliers for reagents include, but are not limited to, Sigma-Aldrich, TCI, Wako, Kanto, Fluorchem, Acros, Alfa, Fluka, Dae-Jung, and the like.

First, compounds used for synthesis in the following examples were prepared as follows. Procedures in the following preparation examples may be appropriately modified depending on structures in examples in which such compounds are to be used.

### Preparation Example 1: 5-(4-Fluorophenyl)-6-oxo-2,3,5,6-tetrahydrofuro[3,2-c]pyridine-7-carboxylic acid

### Step 1: Synthesis of 3-((dimethylamino)methylene)-dihydrofuran-2-(3H)-one

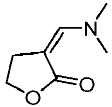

Gamma-butyrolactone (2.69 ml, 35.0 mmol) and dimethyldimethoxyacetal (11.62 ml, 87.5 mmol) were stirred, and reaction was allowed to proceed in a microwave reactor at a temperature of 230°C for 70 minutes or longer. The reaction mixture was concentrated to remove excess dimethyldimethoxyacetal. Solidification was performed with diethyl ether, and the precipitated solid was filtered while performing washing with diethyl ether. The filtered solid was concentrated under reduced pressure to obtain the title compound (2.9 g, yield: 59%, brownish solid).

¹H NMR (500 MHz, CDCl₃) δ 7.13 (s, 1H), 4.24 (t, J = 7.5 Hz, 2H), 3.11 (t, J = 7.5 Hz, 2H), 3.03 (s, 6H); ¹H NMR (500 MHz, DMSO-d₆) δ 7.00 (t, J = 1.5 Hz, 1H), 4.11 (t, J = 7.5 Hz, 2H), 3.06 (t, J = 7.5 Hz, 2H), 3.00 (s, 3H)

### Step 2: Synthesis of 3-((dimethylamino)methylene)-2-(3H)-dihydrofuranilidene ethyl oxonium tetrafluoroborate

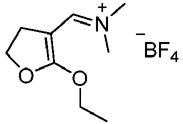

The compound (1.085 g, 7.68 mmol) obtained in step 1 was dissolved in 8 ml of chloroform. Then, triethyloxonium tetrafluoroborate (1.729 g, 7.68 mmol) was added thereto, and stirring was performed at room temperature for 1 day or longer under nitrogen. The reaction mixture was concentrated under reduced pressure, and a nuclear magnetic resonance device was used to check whether the starting material and the product were generated at a ratio of about 15:85. The next reaction was allowed to proceed without purification.

¹H NMR (500 MHz, DMSO-d6) δ 7.93 (s, 1H), 4.86-4.82 (m, 2H), 4.51 (q, J = 7.0 Hz, 2H), 3.33 (s, 3H), 3.30 (t, J = 8.5 Hz, 2H), 3.26 (s, 3H), 1.36 (t, J = 7.0 Hz, 3H)

### Step 3: Synthesis of ethyl 5-(4-fluorophenyl)-6-oxo-2,3,5,6,-tetrahydrofuro[3,2-c]pyridine-7-carboxylate

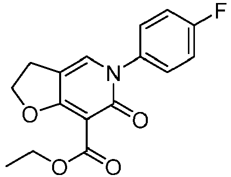

The crude mixture (1.96 g) obtained in step 2 was dissolved in 10 ml of ethanol. Then, sodium ethoxide (20 wt% ethanol solution, 2.56 ml, 6.53 mmol) was added thereto in a water bath at 0°C, and then stirring was slowly performed for 30 minutes up to room temperature. To the reaction mixture was added ethyl 3-((4-fluorophenyl)amino)-3-oxopropionate (1.47 g, 6.53 mmol), and stirring was performed at room temperature for 20 hours or longer. The reaction mixture was concentrated under reduced pressure and extracted with water and dichloromethane. The separated organic layer was dried over anhydrous magnesium sulfate, filtered, and concentrated under reduced pressure to obtain a residue. The residue was purified by column chromatography to obtain the title compound (900 mg, yield: 39% (based on an overall yield over steps 2 and 3)/46% (based on a yield over step 3), yellow solid).

¹H NMR (500 MHz, DMSO-d₆) δ 7.70 (t, J = 1.5 Hz, 1H), 7.42-7.40 (m, 2H), 7.34-7.31 (m, 2H), 4.74 (t, J = 8.0 Hz, 2H), 4.17 (q, J = 7.0 Hz, 2H), 3.05 (td, J = 8.0 Hz, 2H), 1.21 (t, J = 7.0 Hz, 3H)

### Step 4: Synthesis of 5-(4-fluorophenyl)-6-oxo-2,3,5,6-tetrahydrofuro[3,2-c]pyridine-7-carboxylic acid

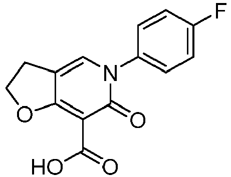

The compound (0.9 g, 2.97 mmol) obtained in step 3 was dissolved in 10 ml of ethanol and 5 ml of distilled water. Then, lithium hydroxide monohydrate (249 mg, 5.94 mmol) was added thereto, and the mixture was heated to 50°C and stirred for 4 hours or longer. The reaction mixture was concentrated under reduced pressure, and extracted with water and dichloromethane. To the separated water layer was added 1 N hydrochloric acid solution. Then, extraction was performed with water and dichloromethane. The separated organic layer was dried over anhydrous magnesium sulfate, filtered, and concentrated under reduced pressure. For the concentrated residue, a solid was precipitated with small amounts of dichloromethane and diethyl ether, and filtration was performed. Then, the filtered solid was dried to obtain the title compound (680 mg, yield: 84%, off-white solid).

¹H NMR (500 MHz, DMSO-d₆) δ 14.5 (bs, OH), 7.97 (s, 1H), 7.54-7.51 (m, 2H), 7.41-7.37 (m, 2H), 4.90 (t, J = 8.5 Hz, 2H), 3.11 (td, J = 8.5, 1.0 Hz, 2H)

### Preparation Example 2: 6-Oxo-5-phenyl-2,3,5,6-tetrahydrofuro[3,2-c]pyridine-7-carboxylic acid

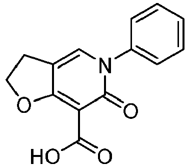

The title compound (yield: 23%, off-white solid) was prepared using the same synthetic route as in Preparation Example 1, except that in step 3, ethyl 3-oxo-3-(phenylamino)propionate was used in place of ethyl 3-((4-fluorophenyl)amino)-3-oxopropionate.

¹H NMR (500 MHz, DMSO-d₆) δ 14.6 (bs, OH), 7.98 (s, 1H), 7.56-7.53 (m, 2H), 7.51-7.48 (m, 1H), 7.46 (m, 2H), 4.92 (d, J =10.0 Hz, 2H), 3.14 (d, J =10.0 Hz, 2H)

### Preparation Example 3: 6-Oxo-5-(4-(trifluoromethyl)phenyl)-2,3,5,6-tetrahydrofuro[3,2-c]pyridine-7-carboxylic acid

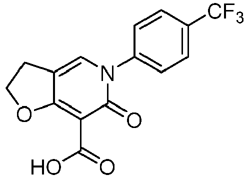

The title compound (yield: 2%, off-white solid) was prepared using the same synthetic route as in Preparation Example 1, except that in step 3, ethyl 3-oxo-3-(4-(trifluoromethyl)phenylamino)propionate was used in place of ethyl 3-((4-fluorophenyl)amino)- 3 -oxopropionate.

1H NMR (400 MHz, DMSO-d₆) δ 8.02 (s, 1H), 7.95 (d, J = 8.0 Hz, 2H), 7.73 (d, J = 8.0 Hz, 2H), 4.92 (t, J = 8.0 Hz, 2H), 3.13 (t, J = 8.0 Hz, 2H)

### Preparation Example 4: 5-(3-Fluorophenyl)-6-oxo-2,3,5,6-tetrahydrofuro[3,2-c]pyridine-7-carboxylic acid

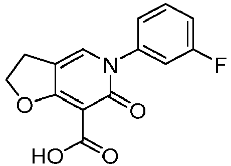

The title compound (yield: 15%, off-white solid) was prepared using the same synthetic route as in Preparation Example 1, except that in step 3, ethyl 3-oxo-3-(3-fluorophenylamino)propionate was used in place of ethyl 3-((4-fluorophenyl)amino)-3-oxopropionate.

1H NMR (400 MHz, DMSO-d₆) δ 7.97 (bs, 1H), 7.63-7.57 (m, 1H), 7.48-7.45 (m, 2H), 7.40-7.32 (m, 2H), 4.91 (t, J = 8.0 Hz, 2H), 3.12 (t, J = 8.0 Hz, 2H)

### Preparation Example 5: 2-(4-Fluorophenyl)-3-oxo-3,5,6,7-tetrahydro-2H-cyclopenta[c]pyridine-4-carboxylic acid

### Step 1: Synthesis of ethyl 2-cyclopentylidene-3-((4-fluorophenyl)amino)-3-oxopropionate

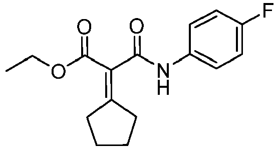

5.0 ml dichloromethane solution of ethyl 3-(4-fluorophenyl)amino-3-oxopropanoate (113 mg, 0.50 mmol) and 500 mg of 4Å molecular sieve was lowered to 0°C. Titanium chloride (1 M solution in dichloromethane, 0.55 ml, 0.55 mmol) was slowly added thereto for 10 minutes using a syringe. The reaction mixture was stirred at 0°C for 30 minutes, and then cyclopentanone (48 µl, 0.55 mmol) was added thereto. Stirring was further performed at 0°C for 10 minutes, and then anhydrous pyridine (81 µl, 1.00 mmol) was slowly added thereto. The reaction mixture was gradually warmed to room temperature, stirred, and then extracted with ethyl acetate and ice water. The organic layer was dried over anhydrous sodium sulfate and filtered. The resulting residue was purified by column chromatography to obtain the title compound (223 mg, yield: 77%, white solid).

1H NMR (500 MHz, CDCl₃) δ 8.92 (s, 1H), 7.55-7.52 (m, 2H), 7.04-7.00 (m, 2H), 4.30 (q, J = 7.0 Hz, 2H), 2.89 (t, J = 6.0 Hz, 2H), 2.79 (t, J = 6.0 Hz, 2H), 1.74-1.73 (m, 4H), 1.35 (t, J = 7.0 Hz, 3H)

### Step 2: Synthesis of ethyl 2-(4-fluorophenyl)-3-oxo-3,5,6,7-tetrahydro-2H-cyclopenta[c]pyridine-4-carboxylic acid

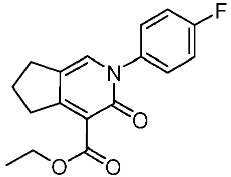

The compound (223 mg, 0.765 mmol) obtained in step 1 was dissolved in 0.8 mL of toluene, and dimethyldimethoxyacetal (0.20 mL, 1.530 mmol) was added thereto. The reaction mixture was heated to a temperature of 60°C and stirred for 12 hours or longer. The reaction mixture was concentrated under reduced pressure to remove residual dimethyldimethoxyacetal, and then purified by column chromatography to obtain the title compound (175 mg, yield: 90%, yellow liquid).

1H NMR (500 MHz, DMSO-d₆) δ 7.67 (br, 1H), 7.46-7.43 (m, 2H), 7.36-7.32 (m, 2H), 4.22 (q, J = 7.0 Hz, 2H), 2.89 (t, J = 7.5 Hz, 2H), 2.69 (t, J = 7.0 Hz, 2H), 2.02-1.96 (m, 2H), 1.25 (t, J = 7.5 Hz, 3H)

### Step 3: Synthesis of 2-(4-fluorophenyl)-3-oxo-3,5,6,7-tetrahydro-2H-cyclopenta[c]pyridine-4-carboxylic acid

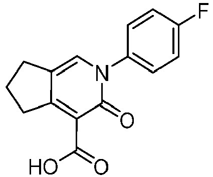

The compound (175 mg, 0.688 mmol) obtained in step 2 was dissolved in 3.0 ml of ethanol and 1.5 ml of distilled water. Then, lithium hydroxide monohydrate (29 mg, 0.688 mmol) was added thereto. The mixture was heated to 50°C and stirred for 3 hours or longer. The reaction mixture was concentrated under reduced pressure and extracted with water and dichloromethane. To the separated water layer was added 1 N hydrochloric acid solution. Then, extraction was performed with water and dichloromethane. The separated organic layer was dried over anhydrous magnesium sulfate, filtered, and concentrated under reduced pressure. For the concentrated residue, a solid was precipitated with small amounts of dichloromethane and diethyl ether, and filtration was performed. Then, the filtered solid was dried to obtain the title compound (158 mg, yield: 84%, pale orange solid).

1H NMR (500 MHz, DMSO-d₆) δ 8.03 (br, 1H), 7.59-7.56 (m, 2H), 7.43-7.39 (m, 2H), 2.22 (t, J = 7.5 Hz, 2H), 2.50 (t, J = 7.5 Hz, 2H, overlapped with DMSO), 2.09-2.03 (m, 2H)

### Preparation Example 6: 4-(4-Amino-2-fluorophenoxy)-7-methoxyquinoline-6-carboxamide

### Step 1: Synthesis of methyl 4-(2-fluoro-4-nitrophenoxy)-7-methoxyquinoline-6-carboxylate

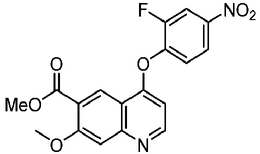

To 0.60 ml N-methyl-2-pyrrolidine solution of methyl 4-chloro-7-methoxyquinoline-6-carboxylate (151 mg, 0.60 mmol) and diisopropylamine (0.10 ml, 0.60 mmol) was added 2-fluoro-4-nitrophenol (104 mg, 0.066 mmol). Then, stirring was performed at 110°C for 24 hours or longer. The reaction mixture was concentrated and extracted with water and ethyl acetate. The separated organic layer was dried over anhydrous magnesium sulfate, filtered, and concentrated under reduced pressure. The resulting residue was purified by column chromatography to obtain the title compound (201 mg, yield: 89%, pale yellow solid).

¹H NMR (500 MHz, DMSO-d₆) δ 8.79 (d, J = 5.0 Hz, 1H), 8.56 (s, 1H), 8.50 (dd, J = 10.0, 5.0 Hz, 1H), 8.24 (dd, J = 10.0, 2.7 Hz, 1H), 7.76 (t, J = 10.0 Hz, 1H), 7.60 (s, 1H), 6.81 (d, J = 5.0 Hz, 1H), 4.00 (s, 3H), 3.86 (s, 3H)

### Step 2: Synthesis of methyl 4-(4-amino-2-fluorophenoxy)-7-methoxyquinoline-6-carboxylate

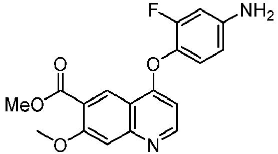

To a solution of the compound (201 mg, 0.55 mmol) obtained in step 1 in 5 ml of ethanol and 2.5 ml of distilled water were added iron (92 mg, 1.65 mmol) and ammonium chloride (294 mg, 5.50 mmol), and stirring was performed at a temperature of 100°C for 2 hours or longer. The reaction mixture was filtered using Celite, and the filtrate was concentrated. The concentrated filtrate was extracted with dichloromethane and water. The separated organic layer was dried over anhydrous magnesium sulfate, filtered, and concentrated under reduced pressure. To the resulting residue was added small amounts of dichloromethane and hexane, to precipitate a solid. The solid was filtered, washed with hexane, and dried under reduced pressure to obtain the title compound (180 mg, yield: 95%, yellow solid).

¹H NMR (500 MHz, DMSO-d₆) δ 8.68 (d, J = 5.5 Hz, 1H), 8.59 (s, 1H), 7.52 (s, 1H), 7.11 (t, J = 9.0 Hz, 1H), 6.55 (dd, J = 13.0, 2.5 Hz, 1H), 6.48-6.46 (m, 2H), 5.54 (s, 2H, - NH₂), 3.98 (s, 3H), 3.89 (s, 3H)

### Step 3: Synthesis of 4-(4-amino-2-fluorophenoxy)-7-methoxyquinoline-6-carboxamide

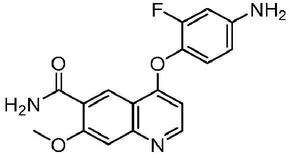

The compound (110.0 mg, 0.32 mmol) obtained in step 2, aqueous ammonia (1 ml), methanol (1 ml), and dimethyl sulfoxide (0.3 ml) were placed in a sealed tube and stirred at a temperature of 90°C for 12 hours or longer. The reaction mixture was cooled to room temperature, and extraction was performed with water and dichloromethane. The separated organic layer was dried over anhydrous sodium sulfate, filtered, and concentrated under reduced pressure to obtain the title compound (72.0 mg, yield: 68%, brown solid).

¹H NMR (500 MHz, DMSO-d₆) δ 8.66 (s, 1H), 8.64 (d, J = 5.0 Hz, 1H), 7.87 (s, 1H), 7.74 (s, 1H), 7.50 (s, 1H), 7.10 (t, J = 5.0 Hz, 1H), 6.55 (d, J = 15.0 Hz, 1H), 6.48-6.44 (m, 2H), 5.53 (s, 2H), 4.02 (s, 3H)

### Preparation Example 7: 4-(4-Amino-2-fluorophenoxy)-7-methoxy-N-methylquinoline-6-carboxamide

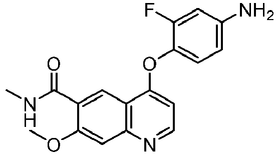

To 0.35 ml N-methyl-2-pyrrolidine solution of the compound (50 mg, 0.146 mmol) obtained in step 2 of Preparation Example 3 was added an excess amount of 33 wt% methylamine (0.4 ml, 26.6 mmol), and stirring was performed at a temperature of 40°C for 16 hours or longer. The reaction mixture was concentrated and extracted with water and ethyl acetate. The separated organic layer was dried over anhydrous magnesium sulfate, filtered, and concentrated under reduced pressure. The resulting residue was purified by column chromatography to obtain the title compound (34 mg, yield: 68%, brownish solid).

¹H NMR (500 MHz, DMSO-d₆) δ 8.65 (d, J = 5.0 Hz, 1H), 8.59 (s, 1H), 8.38-8.37 (m, 1H), 7.50 (s, 1H), 7.09 (t, J = 9.0 Hz, 1H), 6.55 (dd, J = 13.5, 2.0 Hz, 1H), 6.47 (dd, J = 8.5, 2.0 Hz, 1H), 6.44 (d, J = 5.5 Hz, 1H), 5.53 (s, 2H, -NH₂), 4.01 (s, 3H), 2.84 (d, J = 4.5 Hz, 3H)

### Preparation Example 8: 4-(4-Amino-2-fluorophenoxy)-7-methoxy-N,N-dimethylquinoline-6-carboxamide

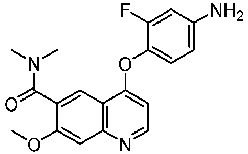

The compound (100 mg, 0.29 mmol) obtained in step 2 of Preparation Example 3 was dissolved in 1 ml of ethanol and 0.5 ml of distilled water. Then, lithium hydroxide monohydrate (49 mg, 1.17 mmol) was added thereto. The mixture was heated to a temperature of 50°C and stirred for 4 hours or longer. The reaction mixture was concentrated under reduced pressure, and extracted with 1 N hydrochloric acid solution and dichloromethane. The separated organic layer was dried over anhydrous magnesium sulfate, filtered, and concentrated under reduced pressure. To 1.5 ml dimethylformamide solution of the concentrated residue, 1-(3-dimethylaminopropyl)-3-ethylcarbodiimide (EDC, 84 mg, 0.44 mmol), diisopropylamine (76 µl, 0.44 mmol), and HOBt (59 mg, 044 mmol) was added dimethylamine (40 wt% in water (H₂O), 74 µl, 0.58 mmol), and stirring was performed at room temperature for 15 hours or longer. The reaction mixture was concentrated under reduced pressure, and extracted with ethyl acetate and aqueous ammonium chloride solution. The separated organic layer was dried over anhydrous magnesium sulfate, filtered, and concentrated under reduced pressure. The resulting residue was purified by column chromatography to obtain the title compound (14 mg, yield: 13%, brownish solid).

¹H NMR (500 MHz, DMSO-d₆) δ 8.63 (d, J = 5.5 Hz, 1H), 8.04 (s, 1H), 7.49 (s, 1H), 7.09 (t, J = 9.0 Hz, 1H), 6.55 (dd, J = 13.0, 2.5 Hz, 1H), 6.47-6.44 (m, 2H), 5.52 (s, 2H, - NH₂), 3.97 (s, 3H), 3.02 (s, 3H), 2.80 (s, 3H)

### Preparation Example 9: 4-Chloro-7-methoxy-N-(2-morpholinoethyl)quinoline-6-carboxamide

### Step 1: Synthesis of 4-chloro-7-methoxyquinoline-6-carboxylic acid

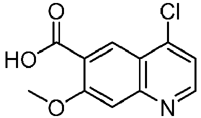

Methyl 4-chloro-7-methoxyquinoline-6-carboxylate (200 mg, 0.79 mmol) and lithium hydroxide monohydrate (66.7 mg, 1.59 mmol) were added to 6 ml tetrahydrofuran/methanol/water (3:2:1) solution, and stirring was performed at room temperature for 3 hours. To the reaction mixture was added an aqueous hydrochloric acid solution (1 M) so that the PH was adjusted to 5. Filtration was performed with water to obtain the title compound (157 mg, yield: 83%, ivory solid).

¹H NMR (500 MHz, DMSO-d₆) δ 13.34 (s, 1H), 8.83 (d, J = 5.0 Hz, 1H), 8.39 (s, 1H), 7.66 (d, J = 5.0 Hz, 1H), 7.59 (s, 1H), 3.99 (s, 3H)

### Step 2: Synthesis of 4-chloro-7-methoxy-N-(2-morpholinoethyl)quinoline-6-carboxamide

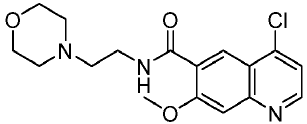

The compound obtained in step 1 (100 mg, 1.03 mmol) was dissolved in 4 ml of dichloromethane solution. To the mixture was then added oxalyl chloride (0.05 ml, 0.63 mmol) and added DMF as much as a catalytic amount. Stirring was performed at room temperature for 30 minutes. Subsequently, 4-(2-aminoethyl)morpholine (0.22 ml, 1.68 mmol) was added thereto at a temperature of 0°C and stirring was performed at room temperature for 16 hours. Then, the reaction mixture was extracted with water and dichloromethane. The separated organic layer was dried over anhydrous magnesium sulfate, filtered, and concentrated under reduced pressure. The resulting residue was purified by column chromatography to obtain the title compound (125 mg, yield: 85%, white solid).

¹H NMR (500 MHz, DMSO-d₆) δ 8.83 (d, J = 4.5 Hz, 1H), 8.53 (s, 1H), 8.52 (d, J = 5.5 Hz, 1H), 7.67 (d, J = 5.0 Hz, 1H), 7.62 (s, 1H), 4.06 (s, 3H), 3.61 (t, J = 4.5 Hz, 4H), 3.45 (dd, J = 12.0, 6.5 Hz, 2H), 2.50 (m, 2H, overlapped with DMSO), 2.45 (s, 4H)

### Preparation Example 10: Synthesis of 4-chloro-7-methoxy-N-(tetrahydro-2H-pyran-4- yl)quinoline-6-carboxamide

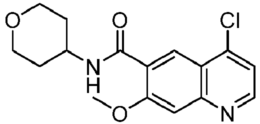

The title compound (39 mg, yield: 76%, white solid) was obtained using the same synthetic route as in Preparation Example 9, except that 4-aminotetrahydropyran was used as a starting material in step 2.

¹H NMR (500 MHz, DMSO-d₆) δ 8.82 (d, J = 5.0 Hz, 1H), 8.37 (d, J = 7.5 Hz, 1H), 8.28 (s, 1H), 7.66 (d, J = 4.5 Hz, 1H), 7.59 (s, 1H), 4.01 (s, 3H), 3.89-3.85 (m, 2H), 3.45-3.40 (m, 2H), 1.84-1.81 (m, 2H), 1.60-1.52 (m, 2H)

### Preparation Example 11: Synthesis of 4-chloro-N-ethyl-7-methoxyquinoline-6-carboxamide

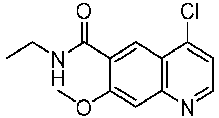

The title compound (158 mg, yield: 95%, ivory solid) was obtained using the same synthetic route as in Preparation Example 9, except that ethylamine (2 M) was used as a starting material in step 2.

¹H NMR (500 MHz, DMSO-d₆) δ 8.82 (d, J = 5.0 Hz, 1H), 8.45 (t, J = 5.5 Hz, 1H), 8.38 (s, 1H), 7.65 (d, J = 5.0 Hz, 1H), 7.59 (s, 1H), 4.02 (s, 3H), 3.31 (m, 2H, overlapped with H₂O), 1.147 (t, J = 7.0 Hz, 3H)

### Preparation Example 12: N-(4-chloro-7-methoxyquinolin-6-yl)acetamide

### Step 1: Synthesis of 4-chloro-7-methoxyquinolin-6-amine

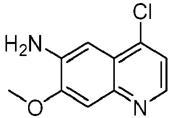

4-Chloro-7-methoxyquinoline-6-carboxylic acid (400 mg, 1.68 mmol) was dissolved in dimethylformamide (3 ml) and triethylamine (0.35 ml, 2.52 mmol). To the mixture was then added diphenylphosphoryl azide (0.54 mL, 2.52 mmol), and stirring was performed at room temperature for 2 hours. After 2 hours, water (1.3 ml) was added thereto, and stirring was performed at a temperature of 100°C for 1 hour. The reaction mixture was extracted with water and ethyl acetate. The separated organic layer was dried over anhydrous sodium sulfate, filtered, and concentrated under reduced pressure. The resulting residue was purified by column chromatography to obtain the title compound (66 mg, yield: 18%, red solid).

¹H NMR (500 MHz, DMSO-d₆) δ 8.36 (d, J = 5.0 Hz, 1H), 7.38 (d, J = 5.0 Hz, 1H), 7.28 (s, 1H), 7.11 (s, 1H), 5.77 (s, 2H), 3.96 (s, 3H)

### Step 2: Synthesis of N-(4-chloro-7-methoxyquinolin-6-yl)acetamide

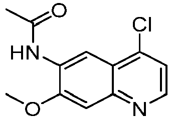

The compound (64 mg, 0.3 mmol) obtained in step 1 was dissolved in dichloromethane (1 ml) and triethylamine (0.12 ml, 0.92 mmol). Then, acetic anhydride (0.05 ml, 0.61 mmol) was added thereto, and stirring was performed at a temperature of 40°C for 4 hours and 20 minutes. The reaction mixture was extracted with water and dichloromethane. The separated organic layer was dried over anhydrous sodium sulfate, filtered, and concentrated under reduced pressure. The resulting residue was purified by column chromatography to obtain the title compound (74 mg, yield: 98%, yellow solid).

¹H NMR (500 MHz, CDCl₃-d₆) δ 9.21 (s, 1H), 8.61 (d, J = 5.0 Hz, 1H), 8.06 (bs, 1H), 7.43 (s, 1H), 7.37 (d, J = 5.0 Hz, 1H), 4.06 (s, 3H), 2.29 (s, 3H)

### Preparation Example 13: N-(4-Chloro-7-methoxyquinolin-6-yl)-2-morpholinoacetamide

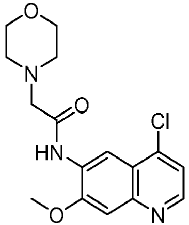

Morpholine-4-yl-acetic acid (173.9 mg, 1.19 mmol) and 1-[bis(dimethylamino)methylene]-1H-1,2,3-triazolo[4,5-b]pyridinium-3-oxide hexafluorophosphate (HATU, 455.5 mg, 1.19 mmol) were dissolved in dimethylformamide (1.6 ml). Then, N,N-diisopropyl ethylamine (0.47 ml, 2.87 mmol) and the compound (100 mg, 0.47 mmol) prepared in step 1 of Preparation Example 9 were sequentially added thereto, and stirring was performed at room temperature for 24 hours. The reaction was quenched with saturated aqueous sodium bicarbonate solution, and extraction was performed with ethyl acetate. Then, the separated organic layer was dried over anhydrous magnesium sulfate and concentrated under reduced pressure. The resulting residue was purified by column chromatography to obtain the title compound (160.0 mg, yield: 82%, white solid).

¹H NMR (400 MHz, DMSO-d₆) δ 11.90 (s, 1H), 9.07 (s, 1H), 8.67 (d, J = 4.4 Hz, 1H), 7.60 (d, J = 4.4 Hz, 1H), 7.57 (s, 1H), 4.11 (s, 3H), 3.69 (t, J = 4.4 Hz, 4H), 3.24 (s, 2H), 2.50 (t, J = 2.0 Hz, 4H)

### Preparation Example 14: 7-Chloro-2-methyl-3H-imidazo[4,5-b]pyridine

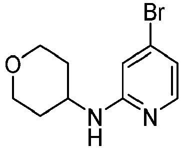

4-Bromo-2-fluoropyridine (200 mg, 1.11 mmol) was dissolved in dimethyl sulfoxide (3.78 ml). Then, 4-aminotetrahydropyran (0.135 ml, 1.31 mmol) and cesium carbonate (747 mg, 2.27 mmol) were added thereto, and stirring was performed at a temperature of 90°C for 2 hours and 30 minutes. The reaction mixture was concentrated, and extracted with brine, water, and ethyl acetate. The separated organic layer was dried over anhydrous magnesium sulfate, filtered, and concentrated under reduced pressure. The resulting residue was purified by column chromatography to obtain the title compound (107 mg, yield: 36.6%, white solid).

¹H NMR (500 MHz, DMSO-d₆) δ 7.84 (d, J = 5.5 Hz, 1H), 6.77 (d, J = 7.5 Hz, 1H), 6.68 (d, J = 1.5 Hz, 1H), 6.64-6.63 (dd, J = 2.0 Hz, J = 5.5 Hz, 1H), 3.86-3.82 (m, 2H), 3.40-3.35 (m, 2H), 1.85-1.82 (m, 2H), 1.42-1.34 (m, 2H)

### Preparation Example 15: 6-(2-Fluoro-4-nitrophenoxy)-N-(1-methylpiperidin-4-yl)pyrimidine-4-amine

### Step 1: Synthesis of 4-chloro-6-(2-fluoro-4-nitrophenoxy)pyrimidine

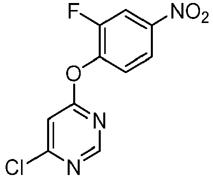

4,6-dichloropyrimidine (200 mg, 1.34 mmol), 2-fluoro-4-nitrophenol (211 mg, 1.34 mmol), and potassium carbonate (278 mg, 2.01 mmol) were added to 3 ml dimethylformamide solution. Stirring was performed at a temperature of 80°C for 3 hours. The reaction mixture was extracted with water and ethyl acetate. Then, the separated organic layer was dried over anhydrous magnesium sulfate, filtered, and concentrated under reduced pressure. The resulting residue was purified by column chromatography to obtain the title compound (277 mg, yield: 77%, white solid).

¹H NMR (500 MHz, DMSO-d₆) δ 8.71 (d, J = 0.5 Hz, 1H), 8.42 (dd, J = 10.5, 3.0 Hz, 1H), 8.24-8.21 (m, 1H), 7.78 (t, J = 8.0 Hz, 1H), 7.75 (d, J = 1.0 Hz, 1H)

### Step 2: Synthesis of 6-(2-fluoro-4-nitrophenoxy)-N-(1-methylpiperidin-4-yl)pyrimidine-4-amine

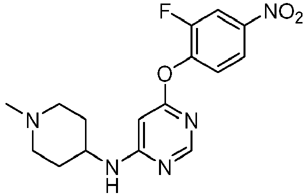

4-Amino-1-methylpiperidine (176 mg, 1.541 mmol) and potassium carbonate (284 mg, 2.05 mmol) were added to 10 ml of acetonitrile, and stirring was performed at room temperature for 10 minutes. Thereafter, the compound (277 mg, 1.027 mmol) obtained in step 1 was added thereto, and stirring was performed at a temperature of 90°C for 16 hours. The reaction mixture was concentrated and extracted with water and ethyl acetate. The separated organic layer was dried over anhydrous magnesium sulfate, filtered, and concentrated under reduced pressure. The resulting residue was purified by column chromatography to obtain the title compound (83 mg, yield: 23%, yellow solid).

¹H NMR (500 MHz, DMSO-d₆) δ 8.33 (dd, J = 10.0, 2.5 Hz, 1H), 8.16-8.14 (m, 1H), 8.12 (s, 1H), 7.62 (t, J = 8.0 Hz, 1H), 7.58 (d, J = 7.5 Hz, 1H), 6.07 (s, 1H), 2.85 (s, 2H), 2.27 (s, 3H), 2.19 (s, 2H), 1.88 (d, J = 11.5 Hz, 2H), 1.50 (d, J = 8.5 Hz, 2H)

### Preparation Example 16: 3-Chloro-4-(2-fluoro-4-nitrophenoxy)-N-(3-morpholinopropyl)pyridine-2-amine

The title compound (43 mg, yield: 11%, brown solid) was obtained using the same synthetic route as in Preparation Example 16, except that 3,4-dichloropyridine-2-amine was used as a starting material in step 1 and 4-(3-chloropropyl)morpholine was used as a starting material in step 2.

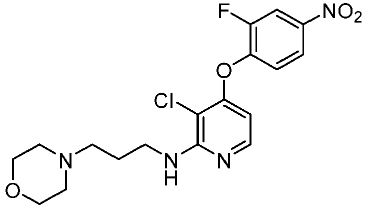

¹H NMR (400 MHz,CDCl₃) δ 8.12 (dd, J = 9.6, 2.8 Hz, 1H), 8.05 (d, J = 9.2 Hz, 1H), 7.98 (d, J = 5.6 Hz, 1H), 7.11 (t, J = 8.0 Hz, 1H), 6.68 (s, 1H), 6.15 (d, J = 5.6 Hz, 1H), 3.77 (t, J = 4.4 Hz, 4H), 3.62-3.57 (m, 2H), 2.55 (t, J = 6.0 Hz, 2H), 2.51 (s, 4H), 1.87-1.84 (m, 2H)

### Preparation Example 17: 4-(4-Amino-2-fluorophenoxy)-N-(1-methylpiperidin-4-yl)pyridine-2-amine

### Step 1: Synthesis of 4-((2-chloropyridin-4-yl)oxy)-3-fluoroaniline

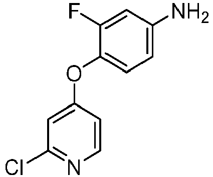

4-Amino-2-fluorophenol (301 mg, 2.37 mmol) was dissolved in dimethylacetamide (7.0 ml). Then, potassium t-butoxide (350 mg, 2.86 mmol) was added thereto, and stirring was performed at room temperature for 30 minutes. After 30 minutes, 2,4-dichloropyridine (300 mg, 2.02 mmol) was added thereto, and stirring was performed at a temperature of 80°C for 3 hours. The reaction mixture was extracted with water and ethyl acetate. The separated organic layer was dried over anhydrous sodium sulfate, filtered, and concentrated under reduced pressure. The resulting residue was purified by column chromatography to obtain the title compound (350 mg, yield: 72%, yellow solid).

¹H NMR (500 MHz, DMSO-d₆) δ 8.27 (d, J = 6.0 Hz, 1H), 7.03 (t, J = 9.0 Hz, 1H), 6.94 (d, J = 2.0 Hz, 1H), 6.91-6.89 (dd, J = 2.0 Hz, J = 5.5 Hz, 1H), 6.53-6.49 (dd, J = 3.0 Hz, J = 13.5 Hz, 1H), 6.43-6.41 (dd, J = 2.5 Hz, J = 9.0 Hz, 1H), 5.52 (s, 2H)

### Step 2: Synthesis of 4-(4-amino-2-fluorophenoxy)-N-(1-methylpiperidin-4-yl)pyridine-2-amine

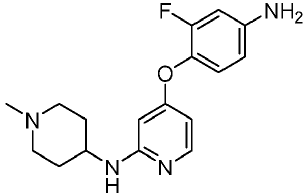

The compound obtained in step 1 (215 mg, 0.90 mmol), 4-amino-1-methylpiperidine (0.22 mL, 1.80 mmol), and potassium carbonate (249 mg, 1.80 mmol) were dissolved in dimethyl sulfoxide (3.0 mL), and then stirred at a temperature of 200°C for 2 days. The reaction mixture was extracted with water and ethyl acetate. The separated organic layer was dried over anhydrous sodium sulfate, filtered, and concentrated under reduced pressure. The resulting residue was purified by column chromatography to obtain the title compound (20 mg, yield: 7%, red solid).

¹H NMR (500 MHz, DMSO-d₆) δ 7.79 (d, J = 6.0 Hz, 1H), 6.92 (t, J = 9.0 Hz, 1H), 6.94-6.45 (dd, J = 2.5 Hz, J = 13.5 Hz, 1H), 6.40-6.36 (m, 2H), 6.08-6.07 (dd, J = 2.0 Hz, J = 5.5 Hz, 1H), 5.74 (d, J = 2.0 Hz, 1H), 5.39 (s, 2H), 2.67 (d, J = 11.0 Hz, 2H), 2.12 (s, 3H), 1.94 (t, J = 11.0 Hz, 2H), 1.80-1.77 (dd, J = 2.0 Hz, J = 12.5 Hz, 2H), 1.36-1.29 (m, 2H)

### Preparation Example 18: tert-Butyl 4-(5-(4-amino-2-fluorophenoxy)-1-methyl-¹H -indazol-6-yl)-1H - pyrazole-1-carboxylate

### Step 1: Synthesis of tert-butyl 4-(5-(2-fluoro-4-nitrophenoxy)-1-methyl-1H-indazol-6-yl)-1H-pyrazole-1-carboxylate

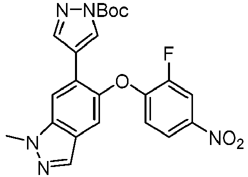

1-Tert-butoxycarbonyl-4-(4,4,5,5-tetramethyl[1,3,2]dioxaborolan-2-yl)pyrazole (300 mg, 0.82 mmol), 6-bromo-5-(2-fluoro-4-nitrophenoxy)-1-methyl-1H-indazole (361 mg, 1.23 mmol), di-t-butyl dicarbonate (44 mg, 0.20 mmol), and anhydrous potassium carbonate (226 mg, 1.64 mmol) were dissolved in tetrahydrofuran. Then, dichloro[1,1'-bis(di-t-butylphosphino)ferrocene]palladium (26 mg, 0.04 mmol) was added thereto, and stirring was performed at a temperature of 60°C for 24 hours or longer. The reaction was quenched with saturated aqueous sodium hydrogen carbonate solution, and extraction was performed with ethyl acetate. The separated organic layer was dried over anhydrous magnesium sulfate and concentrated under reduced pressure. The resulting residue was purified by column chromatography to obtain the title compound (130 mg, yield: 35%, yellow solid).

¹H NMR (500 MHz, CDCl₃) δ 8.56 (s, 1H), 8.38 (dd, J = 5.0 and 5.0 Hz, 1H), 8.32 (s, 1H), 8.29 (s, 1H), 8.09 (d, J = 5.0 Hz, 1H), 7.97-7.94 (m, 1H), 7.75 (s, 1H), 6.87 (t, J = 10.0 Hz, 1H), 4.14 (s, 3H), 1.57 (s, 9H)

### Step 2: Synthesis of tert-butyl 4-(5-(4-amino-2-fluorophenoxy)-1-methyl-1H-indazol-6-yl)-1H-pyrazole-1-carboxylate

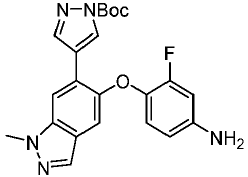

The compound (130 mg, 0.29 mmol) prepared in step 1 was dissolved in ethanol and water. Then, iron (48 mg, 0.86 mmol) and ammonium chloride (153 mg, 2.87 mmol) were sequentially added thereto at room temperature. The temperature was raised to 100°C, and stirring was performed for 3 hours. After completion of the reaction, the reaction mixture was filtered using a Celite pad and concentrated under reduced pressure. The resulting residue was extracted with dichloromethane. The separated organic layer was dried over anhydrous sodium sulfate and concentrated under reduced pressure. Then, filtration was performed with hexane to obtain the title compound (90 mg, yield: 74%, yellow solid).

¹H NMR (500 MHz, DMSO-d₃) δ 12.98 (s, 1H), 8.19 (brs, 1H), 8.08 (brs, 1H), 7.93 (s, 1H), 7.84 (s, 1H), 6.94 (s, 1H), 6.85 (t, J = 7.5 Hz, 1H), 6.52 (dd, J = 5.0 and 5.0 Hz, 1H), 6.39 (dd, J = 5.0 and 5.0 Hz, 1H), 5.30 (s, 2H), 4.04 (s, 3H)

### Preparation Example 19: 7-Chloro-2-methyl-3H-imidazo[4,5-b]pyridine

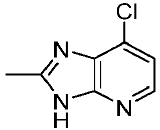

4-Chloropyridine-2,3-diamine (300 mg, 2.08 mmol), polyphosphoric acid (1.04 ml, 21.9 mmol), and acetic acid (0.178 ml, 3.12 mmol) were mixed, and stirring was performed at a temperature of 150°C for 1 hour and 30 minutes. The reaction mixture was concentrated, and extracted with water and ethyl acetate. The separated organic layer was dried over anhydrous magnesium sulfate, filtered, and concentrated under reduced pressure. The resulting residue was purified by column chromatography to obtain the title compound (313 mg, yield: 89%, ivory solid).

¹H NMR (500 MHz, CDCl₃-d₆) δ 12.04 (bs, 1H), 8.20 (d, J = 5.0 Hz, 1H), 7.27 (d, J = 5.5 Hz, 1H), 2.75 (s,3H)

### Preparation Example 20: Synthesis of 4-chloro-7-hydroxy-N-methylquinoline-6-carboxamide

### Step 1: Synthesis of 4-chloro-7-methoxyquinoline-6-carboxylic acid

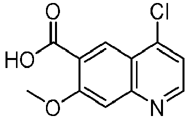

Methyl 4-chloro-7-methoxyquinoline-6-carboxylate (7 g, 28 mmol) and lithium hydroxide monohydrate (2.34 g, 55.63 mmol) were added to 200 ml tetrahydrofuran/methanol/water (3:2:1) solution, and stirred at room temperature for 3 hours or longer. An aqueous hydrochloric acid solution (1 M) was added thereto so that the pH was adjusted to 5. Then, filtration was performed with water to obtain the title compound (5.968 g, yield: 90%, ivory solid).

¹H NMR (500 MHz, DMSO-d₆) δ 13.34 (s, 1H), 8.83 (d, J = 5.0 Hz, 1H), 8.39 (s, 1H), 7.66 (d, J = 5.0 Hz, 1H), 7.59 (s, 1H), 3.99 (s, 3H)

### Step 2: Synthesis of 4-chloro-7-methoxy-N-methylquinoline-6-carboxamide

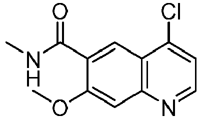

The compound obtained in step 1 (500 mg, 2.1 mmol) was dissolved in 20 ml of dichloromethane solution. To the mixture was then added oxalyl chloride (0.27 ml, 3.16 mmol) and added DMF as much as a catalytic amount. Stirring was performed at room temperature for 30 minutes. Subsequently, methylamine hydrochloride (568 mg, 8.42 mmol) was added thereto at a temperature of 0°C, and stirring was performed at room temperature for 16 hours. Then, the reaction mixture was extracted with water and dichloromethane. The separated organic layer was dried over anhydrous magnesium sulfate, filtered, and concentrated under reduced pressure. The resulting residue was purified by column chromatography to obtain the title compound (527 mg, yield: 100%, yellow solid).

¹H NMR (400 MHz, DMSO-d₆) δ 8.82 (d, J = 4.8 Hz, 1H), 8.43 (s, 2H), 7.66 (d, J = 4.4 Hz, 1H), 7.60 (s, 1H), 4.02 (s, 3H), 2.84 (d, J = 4.4 Hz, 3H)

### Step 3: Synthesis of 4-chloro-7-hydroxy-N-methylquinoline-6-carboxamide

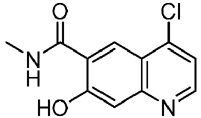

The compound (200 mg, 0.8 mmol) obtained in step 2 was dissolved in 4 ml of dichloroethane solution. Then, 1 M boron tribromide solution was added thereto at a temperature of 0°C, and stirring was performed at a temperature of 50°C for 4 hours or longer. The reaction mixture was filtered with dichloromethane and methanol, and the filtrate was concentrated under reduced pressure. The resulting residue was purified by column chromatography to obtain the title compound (155 mg, yield: 82%, yellow solid).

¹H NMR (400 MHz, DMSO-d₆) δ 10.02 (s, 1H), 9.09 (s, 1H), 8.88 (s, 1H), 8.77 (s, 1H), 8.03 (d, J = 6.4 Hz, 1H), 2.90 (d, J = 5.2 Hz, 3H)

### Preparation Example 21: 4-Chloro-6-methoxy-N-methylquinoline-7-carboxamide

The title compound (yellow solid) was obtained using the same synthetic route as in Preparation Example 20 up to step 2, except that methyl 5-amino-2-methoxybenzoate was used as a starting material in step 1.

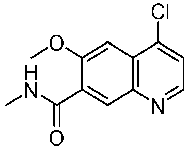

¹H NMR (400 MHz, CDCl₃) δ 8.59 (s, 1H), 8.48 (d, J = 5.2 Hz, 1H), 7.71 (d, J = 4.0 Hz, 1H), 7.25 (d, J = 5.2 Hz, 1H), 7.10 (s, 1H), 3.88 (s, 3H), 2.91 (d, J = 4.8 Hz, 3H)

### Preparation Example 22: 4-Chloro-6-hydroxy-N-methylquinoline-7-carboxamide

The title compound (brown solid) was obtained using the same synthetic route as in step 3 of Preparation Example 20, except that 4-chloro-6-methoxy-N-methylquinoline-7-carboxamide of Preparation Example 21 was used as a starting material.

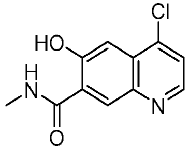

¹H NMR (400 MHz, CDCl₃) δ 12.51 (s, 1H), 9.17 (s, 1H), 8.69 (d, J = 4.8 Hz, 1H), 8.61 (s, 1H), 7.74 (d, J = 4.8 Hz, 1H), 7.48 (s, 1H), 2.89 (d, J = 4.4 Hz, 3H)

### Preparation Example 23: 4-Chloro-6-fluoroquinolin-7-ol

### Step 1: Synthesis of 5-(((4-fluoro-3-methoxyphenyl)amino)methylene)-2,2-dimethyl-1,3-dioxane-4,6-dione

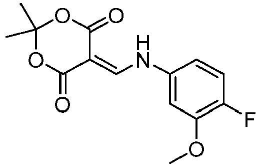

4-Fluoro-3-methoxyaniline (1.0 g, 7.08 mmol) was dissolved in acetonitrile (10.0 ml). Then, Meldrum's acid (1.12 g, 7.79 mmol) and trimethylorthoformate (0.92 ml, 8.49 mmol) were added thereto, and stirring was performed at a temperature of 80°C for 4 hours and 40 minutes. The temperature was lowered. The resulting solid was washed with acetonitrile solvent to obtain the title compound (1.78 g, yield: 85%, yellow solid).

¹H NMR (500 MHz, CDCl₃) δ 11.24 (d, J = 14.0 Hz, 1H), 8.56 (d, J = 14.5 Hz, 1H), 7.14-7.11 (dd, J = 9.0 Hz, J = 10.5 Hz, 1H), 6.82-6.80 (dd, J = 2.5 Hz, J = 7.0 Hz, 1H), 6.79-6.76 (m, 1H), 3.93 (s, 3H), 1.76 (s, 6H)

### Step 2: Synthesis of 4-chloro-6-fluoro-7-methoxyquinoline

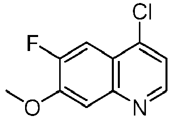

The compound (1.57 g, 4.86 mmol) obtained in step 1 was dissolved in diphenyl ether (6.0 ml), and then stirred at a temperature of 230°C for 1 hour and 10 minutes. The temperature was lowered. Then, hexane was added thereto and solidification was performed. Then, the compound (940 mg, 5.31 mmol) obtained by filtration was dissolved in phosphoryl chloride (2.6 ml) and then stirred at a temperature of 110°C for 40 minutes. To the reaction was added sodium carbonate so that the PH was adjusted to 7. Then, extraction was performed with water and ethyl acetate. The separated organic layer was dried over anhydrous sodium sulfate, filtered, and concentrated under reduced pressure. The resulting residue was purified by column chromatography to obtain the title compound (570 mg, yield: 50%, yellow solid).

¹H NMR (500 MHz, DMSO-d₆) δ 8.77 (d, J = 5.0 Hz, 1H), 7.94 (d, J = 11.5 Hz, 1H), 7.69 (d, J = 8.5 Hz, 1H), 7.67 (d, J = 4.5 Hz, 1H), 4.04 (s, 3H)

### Step 3: Synthesis of 4-chloro-6-fluoroquinolin-7-ol

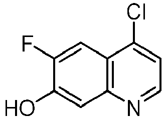

The compound (525 mg, 2.48 mmol) obtained in step 2 was dissolved in 1,2-dichloroethane (2.4 mL). Then, 1 M BBr₃ (7.44 mL, 7.44 mmol) was added thereto at a temperature of 0°C, and stirring was performed at a temperature of 100°C for 8 hours. To the reaction mixture was added methanol to remove BBr₃. Then, extraction was performed with water and ethyl acetate. The separated organic layer was dried over anhydrous sodium sulfate, filtered, and concentrated under reduced pressure. The resulting residue was purified by column chromatography to obtain the title compound (490 mg, yield: 100%, brown solid).

¹H NMR (500 MHz, DMSO-d₆) δ 11.51 (bs, 1H), 8.79 (d, J = 5.0 Hz, 1H), 7.96 (d, J = 11.5 Hz, 1H), 7.70 (d, J = 5.0, 1H), 7.55 (d, J = 8.5, 1H)

### Preparation Example 24: 4-Chloro-6-(trifluoromethyl) quinolin-7-ol

The title compound (yellow solid) was obtained using the same synthetic route as in Preparation Example 21, except that 3-methoxy-4-(trifluoromethyl)phenylamine was used as a starting material in step 1.

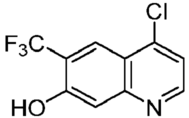

¹H NMR (500 MHz, DMSO-d₆) δ 11.61 (s, 1H), 8.82 (d, J = 4.5 Hz, 1H), 8.34 (s,1H), 7.64 (d, J = 5.0 Hz, 1H), 7.54 (s, 1H)

### Preparation Example 25: 4,6-Dichloroquinoline-7-ol

The title compound (brown solid) was obtained using the same synthetic route as in Preparation Example 21 up to step 3, except that 4-chloro-3-methoxyaniline was used as a starting material in step 1.

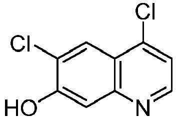

¹H NMR (500 MHz, DMSO-d₆) δ 11.41 (s, 1H), 8.72 (d, J = 3.6 Hz, 1H), 8.16 (s, 1H), 7.58 (d, J = 4.0 Hz, 1H), 7.52 (s, 1H)

### Preparation Example 26: Synthesis of 4-chloro-7-hydroxyquinoline-6-carbonitrile

The title compound (yellow solid) was obtained using the same synthetic route as in Preparation Example 21, except that 4-amino-2-methoxybenzonitrile was used as a starting material in step 1.

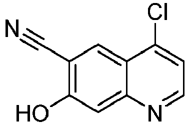

¹H NMR (400 MHz, DMSO-d₆) δ 11.90 (s, 1H), 8.92 (d, J = 4.4 Hz, 1H), 8.59 (s, 1H), 7.63 (d, J = 4.4 Hz, 1H), 7.49 (s, 1H)

### Preparation Example 27: 4-Chloro-7-methoxyquinolin-6-ol

### Step 1: Synthesis of 2-methoxy-4-nitrophenyl acetate

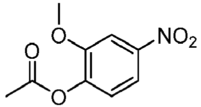

2-Methoxy-4-nitrophenol (1.0 g, 5.91 mmol) and pyridine (0.52 ml, 6.50 mmol) were dissolved in 3 ml of dichloromethane solution. Then, acetic anhydride (0.62 ml, 6.50 mmol) was added thereto at a temperature of 0°C for 15 minutes, and stirring was performed at room temperature for 4 hours. The reaction mixture was concentrated and extracted with water and ethyl acetate. The separated organic layer was dried over anhydrous magnesium sulfate, filtered, and concentrated under reduced pressure to obtain the title compound (1.25 g, yield: 100%, white solid).

¹H NMR (500 MHz, DMSO-d₆) δ 7.92 (d, J = 2.5 Hz, 1H), 7.90 (dd, J = 8.5, 2.5 Hz, 1H), 7.42 (d, J = 8.5 Hz, 1H), 3.92 (s, 3H), 2.32 (s, 3H)

### Step 2: Synthesis of 4-amino-2-methoxyphenyl acetate

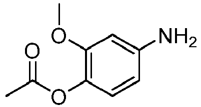

The compound (1.25 g, 5.91 mmol) obtained in step 1 was dissolved in 10 ml of ethanol solution. Then, palladium/carbon (43 mg) was added thereto, and stirring was performed at room temperature under hydrogen gas for 17 hours. The reaction mixture was filtered using Celite, and the filtrate was concentrated to obtain the title compound (1.07 g, yield: 100%, brown liquid).

¹H NMR (500 MHz, DMSO-d₆) δ 6.65 (d, J = 8.5 Hz, 1H), 6.29 (d, J = 2.5 Hz, 1H), 6.07 (dd, J = 8.5, 2.5 Hz, 1H), 5.05 (s, 2H), 3.65 (s, 3H), 2.16 (s, 3H)

### Step 3: Synthesis of 4-chloro-7-methoxyquinolin-6-yl acetate

The title compound (450 mg, yield: 32%, ivory solid) was obtained using the same procedure as in Preparation Example 21 up to step 2, except that the compound obtained in step 2 (1.07 g, 5.92 mmol) was used as a starting material.

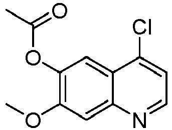

¹H NMR (400 MHz, DMSO-d₆) δ 8.78 (d, J = 4.8 Hz, 1H), 7.90 (s, 1H), 7.66-7.64 (m, 2H), 3.97 (s, 3H), 2.35 (s, 3H)

### Step 4: Synthesis of 4-chloro-7-methoxyquinolin-6-ol

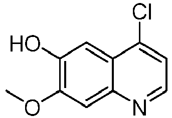

The compound obtained in step 3 (450 mg, 1.79 mmol) and lithium hydroxide monohydrate (83 mg, 1.97 mmol) were added to 15 ml methanol/water (4:1) solution, and stirred at room temperature for 2 hours or longer. Neutralization was achieved by addition of an aqueous hydrochloric acid solution (1 M). Then, the reaction mixture was concentrated under reduced pressure and filtered with water to obtain the title compound (370 mg, yield: 99%, ivory solid).

¹H NMR (400 MHz, DMSO-d₆) δ 10.4 (s, 1H), 8.54 (d, J = 4.4 Hz, 1H), 7.49 (d, J = 4.8 Hz, 1H), 7.42 (s, 1H), 7.38 (s, 1H), 3.96 (s, 3H)

### Preparation Example 28: 4-Chloro-7-methoxy-6-nitroquinoline

The title compound (yellow solid) was obtained using the same synthetic route as in Preparation Example 23 up to step 2, except that 3-methoxy-4-nitroaniline was used as a starting material in step 1.

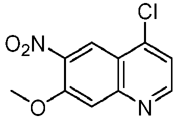

¹H NMR (400 MHz, CDCl₃) δ 8.84 (d, J = 4.4 Hz, 1H), 8.65 (s,1 H), 7.68 (s, 1H), 7.50 (d, J = 4.8 Hz, 1H), 4.10 (s, 3H)

### Preparation Example 29: Synthesis of 4-chloro-6-methoxy-7-nitroquinoline

The title compound (yellow solid) was obtained using the same synthetic route as in Preparation Example 23 up to step 2, except that 4-methoxy-3-nitroaniline was used as a starting material in step 1.

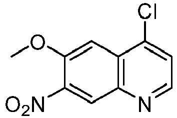

¹H NMR (400 MHz, DMSO-d₆) δ 8.77 (d, J =4.4 Hz, 1H), 8.47 (s, 1H), 7.64 (s, 1H), 7.60 (d, J =5.2 Hz, 1H), 4.11 (s, 3H)

### Preparation Example 30: Synthesis of 6-bromo-4-chloro-7-methoxyquinoline

The title compound (white solid) was obtained using the same synthetic route as in Preparation Example 23 up to step 2, except that 4-bromo-3-methoxyaniline was used as a starting material in step 1.

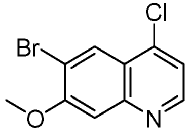

¹H NMR (400 MHz, DMSO-d₆) δ 8.82 (d, J = 4.8 Hz, 1H), 8.38 (s,1H),7.66 (d, J = 4.8 Hz, 1H), 7.61 (s,1H), 4.04 (s,3H)

### Example 1: N-(3-Fluoro-4-((2-(5-(((2-methoxyethyl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide hydrochloride

### Step 1: Synthesis of N-((6-bromopyridin-3-yl)methyl)-2-methoxyethan-1-amine

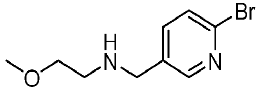

6-Bromopyridine-3-carboxaldehyde (5 g, 26.88 mmol) was dissolved in 1,2-dichloroethane. Then, 2-methoxyethylamine (3.5 ml, 40.32 mmol) and acetic acid (1.6 ml, 28.76) mmol) were sequentially added thereto, and stirring was performed for 20 minutes. Then, sodium triacetoxyborohydride (8.5 g, 40.32 mmol) was added thereto, and stirring was performed at room temperature for 2 hours. The reaction was quenched with 1 N aqueous hydrochloric acid solution, and the pH was adjusted to 9 with 2 N aqueous sodium hydroxide solution. Then, extraction was performed with dichloromethane. The separated organic layer was dried over anhydrous sodium sulfate, filtered, and concentrated under reduced pressure. The resulting residue was purified by column chromatography to obtain the title compound (4.05 g, yield: 70%, off-white solid).

¹H NMR (500MHz, DMSO-d₆) δ 8.31 (d, J = 5.0 Hz, 1H), 7.70 (dd, J = 10.0 and 5.0 Hz, 1H), 7.58 (d, J = 10.0 Hz, 1H), 3.69 (s, 2H), 3.37 (t, J = 5.0 Hz, 1H), 3.22 (s, 3H), 2.60 (d, J = 5.0 Hz, 2H)

### Step 2: Synthesis of tert-butyl ((6-bromopyridin-3-yl)methyl)(2-methoxyethyl)carbamate

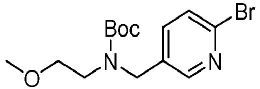

The compound (4.05 g, 16.52 mmol) prepared in step 1 was dissolved in tetrahydrofuran. Then, di-t-butyl dicarbonate (3.9 mL, 17.02 mmol) was added thereto, and stirring was performed at room temperature for 30 minutes. The reaction mixture was extracted with ethyl acetate. The separated organic layer was dried over anhydrous sodium sulfate, filtered, and concentrated under reduced pressure. The resulting residue was purified by column chromatography to obtain the title compound (5.52 g, yield: 97%, off-white solid).

¹H NMR (500MHz, DMSO-d₆) δ 8.27 (s, 1H), 7.64-7.59 (m, 2H), 4.40 (s, 2H), 3.40 (m, 4H), 3.21 (s, 3H), 1.42-1.31 (m, 9H)

### Step 3: Synthesis of tert-butyl ((6-(7-chlorothieno[3,2-b]pyridin-2-yl)pyridin-3-yl)methyl) (2-methoxyethyl)carbamate

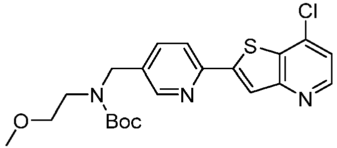

7-Chlorothieno[3,2-b]pyridine (5.4 g, 31.86 mmol) was dissolved in tetrahydrofuran. Then, 2.5 M n-butyllithium hexane solution (2.7 mL, 31.86 mmol) was slowly added thereto at a temperature of -78°C, and stirring was performed for 30 minutes. 1 M zinc chloride diethyl ether solution (31.9 mL, 31.86 mmol) was slowly added thereto. After 10 minutes, the temperature was gradually raised to room temperature, and stirring was performed for 1 hour. Subsequently, tetrakis(triphenylphosphine)palladium (920 mg, 0.79 mmol) and the compound (5.5 g, 15.93 mmol) prepared in step 2 were added thereto, and the mixture was refluxed for 2 hours. The refluxed mixture was cooled to room temperature and concentrated under reduced pressure. The resulting residue was filtered with acetonitrile and dried to obtain the title compound (5.2 g, yield: 75%, off-white solid).

¹H NMR (500MHz, DMSO-d₆) δ 8.66 (d, J = 5.0 Hz, 1H), 8.54 (s, 1H), 8.42 (s, 1H), 8.29 (d, J = 5.0 Hz, 1H), 7.82 (dd, J = 10.0 and 5.0 Hz, 1H), 7.59 (d, J = 5.0 Hz, 1H), 4.50 (s, 2H), 3.45-3.36 (m, 4H), 3.23 (s, 3H), 1.45-1.34 (m, 9H)

### Step 4: Synthesis of tert-butyl ((6-(7-(2-fluoro-4-nitrophenoxy)thieno[3,2-b]pyridin-2-yl)pyridin-3-yl)methyl)(2-methoxyethyl)carbamate

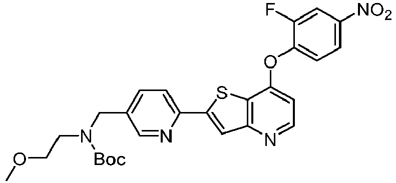

The compound (2.0 g, 4.61 mmol) prepared in step 3 was dissolved in diphenyl ether. Then, anhydrous potassium carbonate (765 mg, 5.53 mmol) and 2-fluoro-4-nitrophenol (1.4 g, 9.22 mmol) were sequentially added thereto, and stirring was performed at a temperature of 160°C for 5 hours. The reaction mixture was cooled to room temperature and extracted with ethyl acetate. The separated organic layer was dried over anhydrous sodium sulfate, filtered, and concentrated under reduced pressure. The resulting residue was dissolved in tetrahydrofuran. Then, di-t-butyl dicarbonate (1.06 ml, 4.61 mmol) was added thereto, and stirring was performed at room temperature for 30 minutes. The reaction mixture was concentrated under reduced pressure. The resulting residue was purified by column chromatography to obtain the title compound (1.2 g, yield: 46%, off-white solid).

¹H NMR (500MHz, DMSO-d₆) δ 8.62 (d, J = 5.0 Hz, 1H), 8.50-8.48 (m, 2H), 8.39 (s, 1H), 8.28 (d, J = 5.0 Hz, 1H), 8.21 (d, J = 10.0 and 5.0 Hz, 1H), 7.80 (d, J = 10.0 Hz, 1H), 7.72 (t, J = 10.0 Hz, 1H), 6.98 (d, J = 5.0 Hz, 1H), 4.48 (s, 2H), 3.43-3.35 (m, 4H), 3.23 (s, 3H), 1.44-1.33 (m, 9H)

### Step 5: Synthesis of tert-butyl ((6-(7-(4-amino-2-fluorophenoxy)thieno[3,2-b]pyridin-2-yl)pyridin-3-yl)methyl)(2-methoxyethyl)carbamate

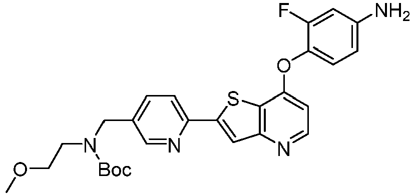

The compound (1.2 g, 2.16 mmol) prepared in step 4 was dissolved in ethanol and water. Then, iron (363 mg, 6.49 mmol) and ammonium chloride (1.16 g, 21.64 mmol) were sequentially added thereto at room temperature. The temperature was raised to 100°C, and stirring was performed for 3 hours. After completion of the reaction, the reaction mixture was filtered using a Celite pad in a warm state, and concentrated under reduced pressure. The resulting residue was extracted with dichloromethane. The separated organic layer was dried over anhydrous sodium sulfate, concentrated under reduced pressure, and filtered with diethyl ether to obtain the title compound (833 mg, yield: 73%, off-white solid).

¹H NMR (500MHz, DMSO-d₆) δ 8.51-8.49 (m, 2H), 8.30 (s, 1H), 8.24 (d, J = 5.0 Hz, 1H), 7.78 (dd, J = 10.0 and 5.0 Hz, 1H), 7.13 (t, J = 10.0 Hz, 1H), 6.60 (d, J = 5.0 Hz, 1H), 6.53 (dd, J = 15.0 and 5.0 Hz, 1H), 6.45 (dd, J = 10.0 and 5.0 Hz, 1H), 5.55 (s, 2H), 4.48 (s, 2H), 3.43-3.33 (m, 4H), 3.23 (s, 3H), 1.44-1.34 (m, 9H)

### Step 6: Synthesis of tert-butyl ((6-(7-(2-fluoro-4-(5-(4-fluorophenyl))-6-oxo-2,3,5,6-tetrahydrofuro[3,2-c]phenoxy)thieno[3,2-b]pyridin-2-yl)pyridin-3-yl)methyl)(2-methoxyethyl)carbamate

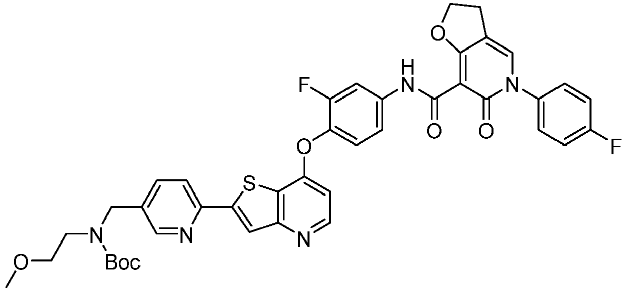

The carboxylic acid (60 mg, 0.22 mmol) prepared in Preparation Example 1 and 1-[bis(dimethylamino)methylene]-1H-1,2,3-triazolo[4,5-b]pyridinium-3-oxide hexafluorophosphate (HATU, 82 mg, 0.12 mmol) were dissolved in dimethylformamide. Then, N,N-diisopropyl ethylamine (21 mg, 0.21 mmol) and the compound (57 mg, 0.11 mmol) prepared in step 5 were sequentially added thereto, and stirring was performed at room temperature for 24 hours or longer. The reaction was quenched with saturated aqueous sodium bicarbonate solution, and extraction was performed with ethyl acetate. Then, the separated organic layer was dried over anhydrous magnesium sulfate and concentrated under reduced pressure. The resulting residue was purified by column chromatography to obtain the title compound (21 mg, yield: 27%, yellow solid).

¹H NMR (500 MHz, DMSO-d₆) δ 11.91 (s, 1H), 8.53 (d, J = 5.0 Hz, 2H), 8.34 (s, 1H), 8.28 (s, 1H), 8.01 (d, J = 15.0 Hz, 1H), 7.89 (s, 1H), 7.79 (d, J = 10.0 Hz, 1H), 7.53-7.50 (m, 2H), 7.46 (d, J = 5.0 Hz, 2H), 7.39 (t, J = 7.5 Hz, 2H), 6.70 (d, J = 5.0 Hz, 1H), 4.85 (s, 2H), 3.44 (s, 2H), 3.23 (s, 3H), 3.11 (t, J = 7.5 Hz, 2H), 1.44-1.34 (m, 9H)

### Step 7: Synthesis of N-(3-fluoro-4-((2-(5-(((2-methoxyethyl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide hydrochloride

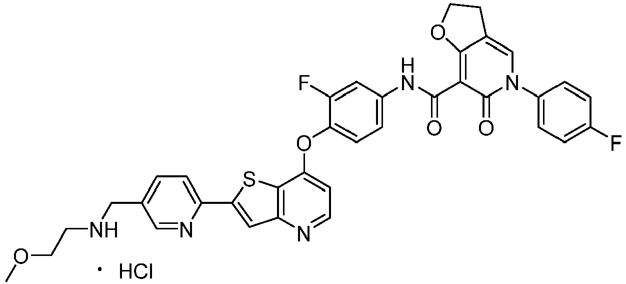

To the compound (20 mg, 0.02 mmol) prepared in step 6 was added 25 µl of 4 M hydrochloric acid solution in 1,4-dioxane, and stirring was performed at room temperature for 1 hour or longer. The reaction mixture was concentrated under reduced pressure and dissolved in a small amount of ethanol. Then, diethyl ether was added thereto. The resulting solid was filtered and dried to obtain the title compound (15 mg, yield: 83%, yellow solid).

¹H NMR (500 MHz, DMSO-d₆) δ 11.93 (s, 1H), 8.73 (s, 1H), 8.55 (d, J = 5.0 Hz, 1H), 8.44 (d, J = 5.0 Hz, 1H), 8.37 (d, J = 10.0 Hz, 1H), 8.09 (d, J = 10.0 Hz, 1H), 8.01 (d, J = 10.0 Hz, 1H), 7.89 (s, 1H), 7.53-7.50 (m, 2H), 7.46 (d, J = 10.0 Hz, 1H), 7.39 (t, J = 10.0 Hz, 2H), 6.71 (d, J = 5.0 Hz, 1 H), 4.85 (t, J = 10.0 Hz, 2H), 4.25 (s, 2H), 3.60 (t, J = 5.0 Hz, 2H), 3.32 (s, 3H), 3.14-3.09 (m, 4H)

### Example 2: N-(3-Fluoro-4-((2-(5-(((2-methoxyethyl)amino)methyl)pyridin-2-yl)thieno[3,2-b]pyridin-7-yl)oxy)phenyl)-6-oxo-5-phenyl-2,3,5,6-tetrahydrofuro[3,2-c]pyridine-7-carboxamide hydrochloride

The title compound (yellow solid) was obtained using the same synthetic route as in Example 1, except that the carboxylic acid compound of Preparation Example 2 was used as a starting material in step 6.

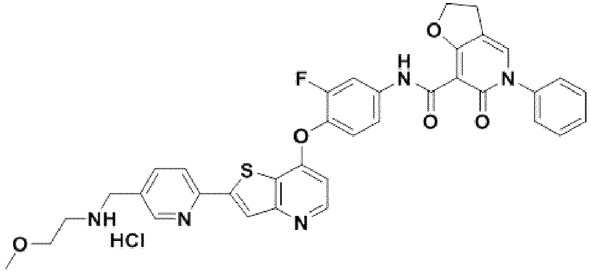

¹H NMR (500 MHz, DMSO-d₆) δ 11.93 (s, 1H), 8.73 (s, 1H), 8.55 (d, J = 5.0 Hz, 1H), 8.44 (d, J = 5.0 Hz, 1H), 8.37 (d, J = 10.0 Hz, 1H), 8.09 (d, J = 10.0 Hz, 1H), 8.01 (d, J = 10.0 Hz, 1H), 7.89 (s, 1H), 7.53-7.50 (m, 2H), 7.46 (d, J = 10.0 Hz, 1H), 7.39 (t, J = 10.0 Hz, 2H), 6.71 (d, J = 5.0 Hz, 1 H), 4.85 (t, J = 10.0 Hz, 2H), 4.25 (s, 2H), 3.60 (t, J = 5.0 Hz, 2H), 3.32 (s, 3H), 3.14-3.09 (m, 4H)

### Example 3: N-(3-Fluoro-4-((2-(pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (off-white solid) was obtained using the same procedure as in step 6 of Example 1, except that 3-fluoro-4-((2-(pyridin-2-yl)thieno[3,2-b]pyridine-7-yl)oxy)aniline, which can be easily prepared using steps 3, 4, and 5 of Example 1, was used as a starting material.

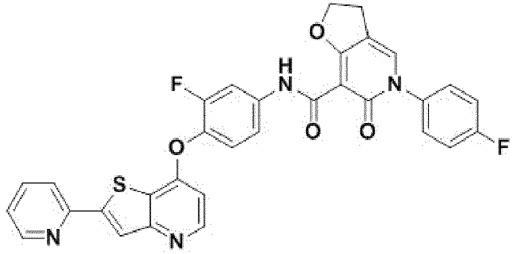

¹H NMR (500 MHz, DMSO-d₆) δ 11.93 (s, 1H), 8.66 (d, J = 5.0 Hz, 1H), 8.54 (d, J = 5.0 Hz, 1H), 8.38 (s, 1H), 8.29 (d, J = 10.0 Hz, 1H), 8.00 (d, J = 15.0 Hz, 1H), 7.96 (d, J = 15.0 Hz, 1H), 7.88 (s, 1H), 7.53-7.50 (m, 2H), 7.46-7.43 (m, 3H), 7.40-7.37 (m, 2H), 6.70 (d, J = 10 Hz, 1H) 4.85 (t, J = 7.5 Hz, 2H), 3.11 (t, J = 10.0 Hz, 2H)

### Example 4: N-(3-Fluoro-4-((2-pyridin-2-yl)thieno[3,2-b]pyridin-7-yl)oxy)phenyl)-6-oxo-5-phenyl-2,3,5,6-tetrahydrofuro[3,2-c]pyridine-7-carboxamide

The title compound (off-white solid) was obtained using the same procedure as in step 6 of Example 1, except that 3-fluoro-4-((2-(pyridin-2-yl)thieno[3,2-b]pyridine-7-yl}oxy)aniline, which can be easily prepared using steps 3, 4, and 5 of Example 1, and the carboxylic acid compound of Preparation Example 2 were used as starting materials.

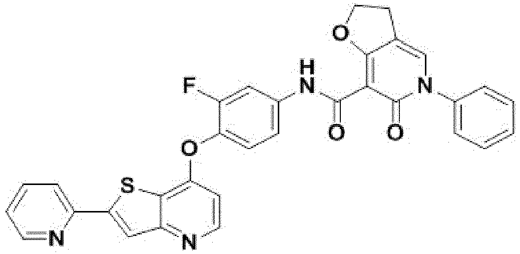

¹H NMR (500 MHz, DMSO-d₆) δ 11.98 (s, 1H), 8.66 (d, J = 5.0 Hz, 1H), 8.55 (d, J = 5.0 Hz, 1H), 8.40 (s, 1H), 8.30 (d, J = 10.0 Hz, 1H), 7.96 (d, J = 5.0 Hz, 2H), 7.89 (d, J = 5.0 Hz, 1H), 7.55-7.53 (m, 2H), 7.48-7.44 (m, 6H), 6.70 (d, J = 10 Hz, 1H) 4.85 (t, J = 7.5 Hz, 2H), 3.11 (t, J = 7.5 Hz, 2H)

### Example 5: N-(3-Fluoro-4-((2-(5-(morpholinomethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide hydrochloride

The title compound (off-white solid) was obtained using the same procedure as in steps 6 and 7 of Example 1, except that 3-fluoro-4-((2-(5-morpholinomethylpyridin-2-yl)-3a,7a-dihydrothieno[3,2-b]pyridin-7-yl)oxy)aniline, which can be easily prepared using steps 3, 4, and 5 of Example 1, was used as a starting material.

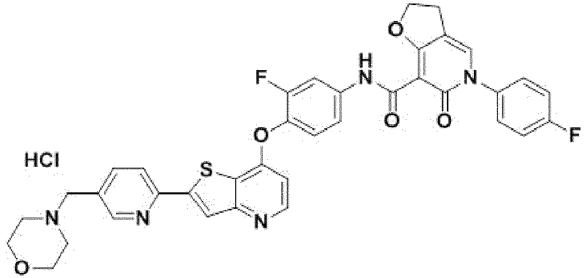

¹H NMR (500 MHz, DMSO-d₆) δ 11.93 (s, 1H), 10.66 (brs, 1H), 8.78 (d, J = 5.0 Hz, 1H), 8.58 (t, J = 5.0 Hz, 1H), 8.48 (d, J = 5.0 Hz, 1H), 8.42 (d, J = 10.0 Hz, 1H), 8.17 (d, J = 5.0 Hz, 1H), 8.04-7.95 (m, 2H), 7.88 (s, 1H), 7.56-7.37 (m, 5H), 6.77 (dd, J = 5.0 and 10.0 Hz, 1H), 4.85 (t, J = 10.0 Hz, 2H), 4.45 (brs, 2H), 3.97 (t, J = 15.0 Hz, 2H), 3.83 (t, J = 7.5 Hz, 1H), 3.70 (t, J = 12.5 Hz, 2H), 3.11 (t, J = 10.0 Hz, 2H), 2.93 (t, J = 7.5 Hz, 1H)

### Example 6: N-(3-Fluoro-4-((2-(5-(morpholinomethyl)pyridin-2-yl)thieno[3,2-b]pyridin-7-yl)oxy)phenyl)-6-oxo-5-phenyl-2,3,5,6-tetrahydrofuro[3,2-c]pyridine-7-carboxamide hydrochloride

The title compound (brown solid) was obtained using the same procedure as in steps 6 and 7 of Example 1, except that 3-fluoro-4-((2-(5-morpholinomethylpyridin-2-yl)-3a,7a-dihydrothieno[3,2-b]pyridin-7-yl)oxy)aniline, which can be easily prepared using steps 3, 4, and 5 of Example 1, and the carboxylic acid compound of Preparation Example 2 were used as starting materials.

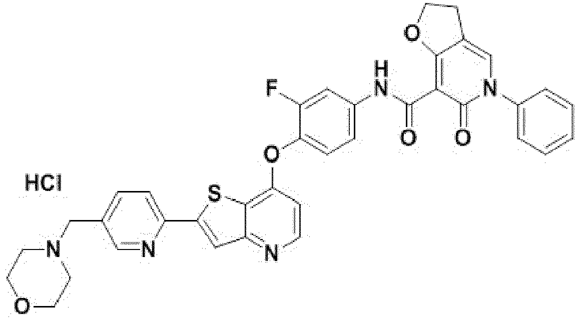

¹H NMR (500 MHz, DMSO-d₆) δ 11.97 (s, 1H), 10.25 (brs, 1H), 8.57-8.53 (m, 1H), 8.45 (d, J = 5.0 Hz, 1H), 8.42 (d, J = 5.0 Hz, 1H), 8.11 (s, 1H), 8.02 (d, J = 10.0 Hz, 1H), 7.97 (d, J = 15.0 Hz, 1H), 7.89 (s, 1H), 7.59-7.55 (m, 2H), 7.53-7.4.3 (m, 4H), 7.13 (t, J = 10.0 Hz, 1H), 6.76 (t, J = 5.0 Hz, 1H), 4.85 (t, J = 7.5 Hz, 1H), 4.45 (brs, 3H), 3.97 (d, J = 10.0 Hz, 3H), 3.84 (t, J = 7.5 Hz, 2H), 3.11 (t, J = 7.5 Hz, 2H), 2.94 (t, J = 7.5 Hz, 2H)

### Example 7: N-(3-Fluoro-4-((2-(5-morpholinomethyl)pyridin-2-yl)thieno[3,2-b]pyridin-7-yl)phenyl)-2-(4-fluorophenyl)-3-oxo-3,5,6,7-tetrahydro-2H-cyclopenta[c]pyridine-4-carboxamide

The title compound (off-white solid) was obtained using the same procedure as in step 6 of Example 1, except that 3-fluoro-4-((2-(5-morpholinomethylpyridin-2-yl)-3a,7a-dihydrothieno[3,2-b]pyridin-7-yl)oxy)aniline, which can be easily prepared using steps 3, 4, and 5 of Example 1, and the carboxylic acid compound of Preparation Example 5 were used as starting materials.

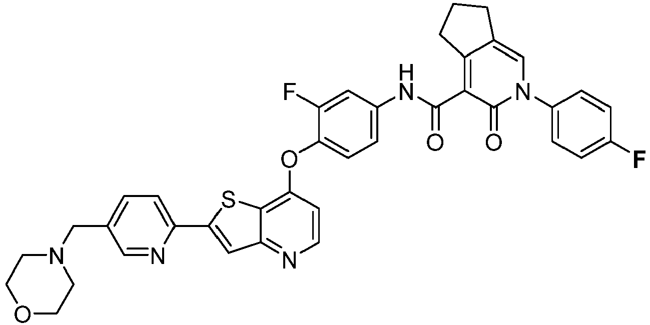

¹H NMR (500 MHz, DMSO-d₆) δ 8.55 (d, J = 1.5 Hz, 1H), 8.53 (d, J = 5.5 Hz, 1H), 8.33 (s, 1H), 8.24 (d, J = 8.0 Hz, 1H), 8.05-8.03 (m, 1H), 7.92 (br, 1H), 7.87 (dd, J = 8.5, 2.0 Hz, 1H), 7.57-7.55 (m, 2H), 7.50-7.45 (m, 2H), 7.42-7.39 (m, 2H), 6.70 (d, J = 5.0 Hz, 1H), 3.58 (t, J = 4.5 Hz, 4H), 3.55 (s, 2H), 2.75 (t, J = 7.5 Hz, 2H), 2.39 (brs, 4H), 2.07-2.01 (m, 2H)

### Example 8: N-(3-Fluoro-4-((2-(1-methyl-1H-imidazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (yellow solid) was obtained using the same procedures as in step 6 of Example 1, except that 3-fluoro-4-((2-(1-methyl-1H-imidazol-4-yl)thieno[3,2-b]pyridin-7-yl)oxy)aniline, which can be easily prepared using steps 3, 4, and 5 of Example 1, was used as a starting material.

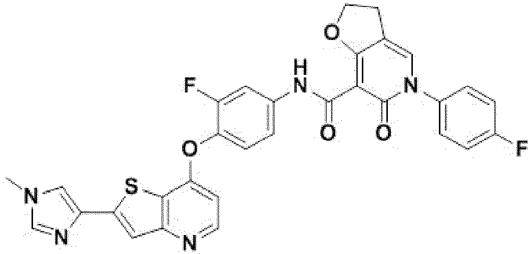

¹H NMR (500 MHz, DMSO-d₆) δ 11.92 (s, 1H), 8.43 (d, J = 5.0 Hz, 1H), 7.99 (d, J = 10.0 Hz, 1H), 7.87 (d, J = 5.0 Hz, 2H), 7.72 (s, 1H), 7.68 (s, 1H), 7.52-7.50 (m, 2H), 7.44-7.36 (m, 4H), 6.59 (d, J = 5.0 Hz, 1H), 4.85 (t, J = 10.0 Hz, 2H), 3.72 (s, 3H), 3.10 (t, J = 10.0 Hz, 2H)

### Example 9: N-(3-Fluoro-4-((2-(1-methyl-1H-imidazol-4-yl)thieno[3,2-b]pyridin-7-yl)oxy)phenyl)-6-oxo-5-phenyl-2,3,5,6-tetrahydrofuro[3,2-c]pyridine-7-carboxamide

The title compound (white solid) was obtained using the same procedure as in step 6 of Example 1, except that 3-fluoro-4-((2-(1-methyl-1H-imidazol-4-yl)thieno[3,2-b]pyridin-7-yl)oxy)aniline, which can be easily prepared using steps 3, 4, and 5 of Example 1, and the carboxylic acid compound of Preparation Example 2 were used as starting materials.

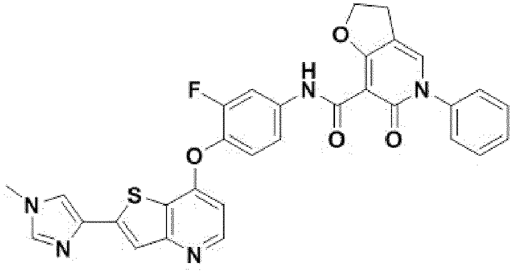

¹H NMR (500 MHz, DMSO-d₆) δ 12.10 (s, 1H), 8.43 (d, J = 5.0 Hz, 1H), 8.00 (dd, J = 10.0, 5.0 Hz, 1H), 7.64 (s, 1H), 7.56 (t, J = 10.0 Hz, 2H), 7.51-7.48 (m, 2H), 7.39-7.31 (m, 5H), 7.16 (t, J = 10.0 Hz, 1H), 6.46 (d, J = 5.0 Hz, 1H), 4.98 (t, J = 10.0 Hz, 2H), 3.76 (s, 3H), 3.18 (t, J = 10.0 Hz, 2H)

### Example 10: N-(3-Fluoro-4-((2-(1-methyl-1H-pyrazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same procedures as in step 6 of Example 1, except that 3-fluoro-4-((2-(1-methyl-1H-pyrazol-4-yl)thieno[3,2-b]pyridin-7-yl)oxy)aniline, which can be easily prepared using steps 3, 4, and 5 of Example 1, was used as a starting material.

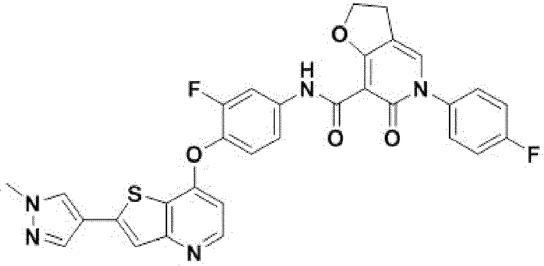

¹H NMR (500 MHz, DMSO-d₆) δ 11.92 (s, 1H), 8.45 (d, J = 5.5 Hz, 1H), 8.32 (s, 1H), 8.01-7.98 (m, 2H), 7.88 (t, J = 1.5 Hz, 1H), 7.69 (s, 1H), 7.52-7.50 (m, 2H), 7.44-7.37 (m, 4H), 6.60 (d, J = 5.5 Hz, 1H), 4.87 (t, J = 8.0 Hz, 2H), 3.90 (s, 3H), 3.12 (t, J = 8.0 Hz, 2H)

### Example 11: N-(3-Fluoro-4-((2-(5-((3-hydroxyazetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (off-white solid) was obtained using the same synthetic route as in steps 1, 3, 4, 5, and 6 of Example 1, except that azetidin-3-ol was used as a starting material in step 1.

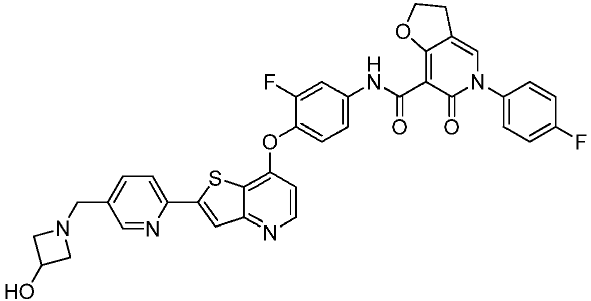

¹H NMR (400 MHz, CDCl₃-d₆) δ 12.04 (s, 1H), 8.53 (s, 1H), 8.48-8.45 (m, 1H), 8.02-7.95 (m, 2H), 7.84-7.82 (m, 1H), 7.73 (d, J = 8.4 Hz, 1H), 7.37-7.33 (m, 4H), 7.26-7.15 (m, 3H, overlapped), 6.52 (d, J = 5.2 Hz, 1H), 4.98 (t, J = 8.4 Hz, 2H), 4.51-4.48 (m, 1H), 3.7-3.68 (m, 4H), 3.49 (d, J = 2.8 Hz, 1H), 3.17 (t, J = 8.0 Hz, 2H), 3.01 (t, J = 5.2 Hz, 2H)

### Example 12: N-(3-Fluoro-4-((2-(5-((3-(hydroxymethyl)azetidin-1-yl)methyl)pyridin-2-yl)thieno[3,2-b]pyridin-7-yl)oxy)phenyl)-5-(4-fluorophenyl)-6-oxo-2,3,5,6- tetrahydrofuro[3,2-c]pyridine-7-carboxamide

The title compound (white solid) was obtained using the same synthetic route as in steps 1, 3, 4, 5, and 6 of Example 1, except that azetidin-3-ylmethanol hydrochloride was used as a starting material in step 1.

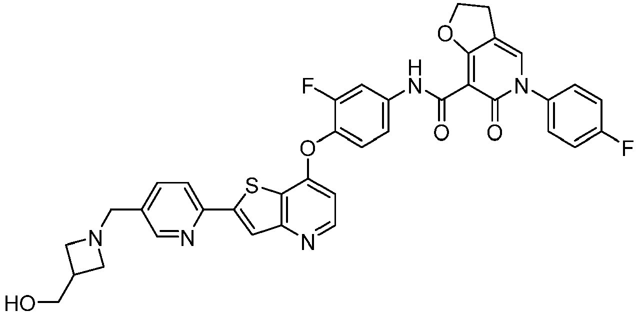

¹H NMR (400 MHz, DMSO-d₆) δ 11.93 (s, 1H), 8.53 (m, 2H), 8.32 (s, 1H), 8.23 (d, J = 7.6 Hz, 1H), 8.02 (m, 1H), 7.88 (s, 1H), 7.82 (dd, J = 8.0 1,6 Hz, 1H), 7.53-7.48 (m, 2H), 7.46-7.36 (m, 3H), 6.69 (d, J = 5.6 Hz, 1H), 4.87 (t, J = 8.4 Hz, 2H), 4.61 (t, J = 5.6 Hz, 1H), 3.60 (s, 2H), 3.52 (t, J = 6.0 Hz, 2H), 3.25 (q, J = 6.8 Hz, 2H), 3.13 (d, J = 7.6 Hz, 2H), 2.94 (d, J = 6.4 Hz, 2H)

### Example 13: N-(3-Fluoro-4-((2-(5-((3-hydroxy-3-methylazetidin-1-yl)methyl)pyridin-2-yl)thieno[3,2-b]pyridin-7-yl)oxy)phenyl)-5-(4-fluorophenyl)-6-oxo-2,3,5,6- tetrahydrofuro[3,2-c]pyridine-7-carboxamide

The title compound (white solid) was obtained using the same synthetic route as in steps 1, 3, 4, 5, and 6 of Example 1, except that 3-methyl-3-hydroxyazetidine hydrochloride was used as a starting material in step 1.

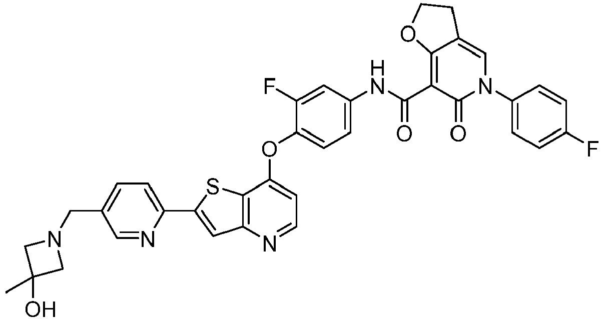

¹H NMR (400 MHz, DMSO-d₆) δ 11.93 (s, 1H), 8.53 (m, 2H), 8.32 (s, 1H), 8.23 (d, J = 8.0 Hz, 1H), 8.02 (d, J = 14.4 Hz, 1H), 7.88 (s, 1H), 7.83 (d, J = 8.8 Hz, 1H), 7.56-7.36 (m, 7H), 7.17 (d, J = 8.8 Hz, 1H), 6.70 (d, J = 5.2 Hz, 1H), 5.19 (s, 1H), 4.87 (t, J = 8.4 Hz, 2H), 3.64 (s, 2H), 3.21-3.17 (m, 3H), 3.13 (t, J = 7.2 Hz, 2H), 2.94 (d, J = 6.8 Hz, 1H), 1.37 (s, 3H)

### Example 14: N-(3-Fluoro-4-((2-(5-((3-methoxyazetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 3-methoxyazetidine hydrochloride was used as a starting material in step 1.

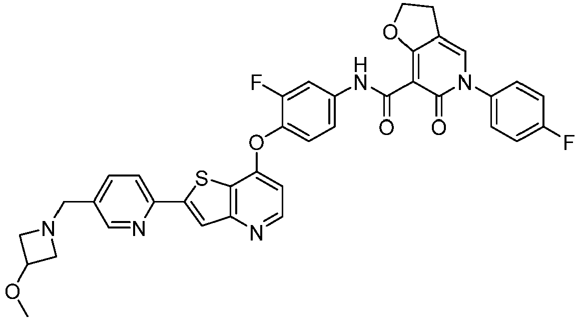

¹H NMR (400 MHz, CDCl₃) δ 12.04 (S, 1H), 8.54 (s, 1H), 8.48 (d, J = 5.6 Hz, 1H), 8.02 (dd, J = 12.4, 2.4 Hz, 1H), 7.97 (s, 1H), 7.84 (d, J = 6.8 Hz, 1H), 7.73 (dd, J = 8.4, 2.4 Hz, 1H), 7.39-7.33 (m, 4H), 7.26-7.16 (m, 3H), 6.52 (d, J = 5.6 Hz, 1H), 5.01 (t, J = 8.8 Hz, 2H), 4.10 (q, J = 6.0 Hz, 1H), 3.68 (s, 2H), 3.65-3.62 (m, 2H), 3.26 (s, 3H), 3.20 (J = 8.8 Hz, 1H), 3.01-2.98 (m, 2H)

### Example 15: N-(3-Fluoro-4-((2-(5-((3-(methoxymethyl)azetidin-1-yl)methyl)pyridin-2-yl)thieno[3,2-b]pyridin-7-yl)oxy)phenyl)-5-(4-fluorophenyl)-6-oxo-2,3,5,6- tetrahydrofuro[3,2-c]pyridine-7-carboxamide

The title compound (white solid) was obtained using the same synthetic route as in steps 1, 3, 4, 5, and 6 of Example 1, except that 3-(methoxymethyl)azetidine hydrochloride was used as a starting material in step 1.

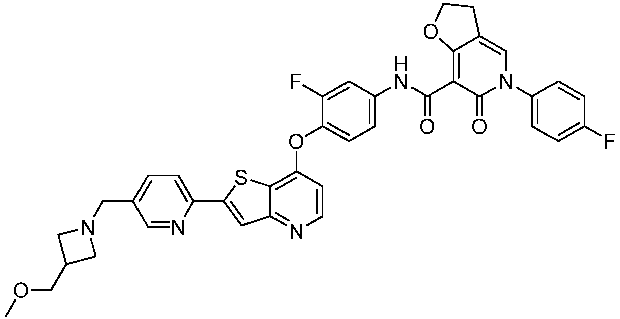

¹H NMR (400 MHz, CDCl₃) δ 12.03 (s, 1H), 8.53 (d, J = 1.6 z, 1H), 8.47 (d, J = 5.2 Hz, 1H), 8.01-7.98 (dd, J = 2.8 Hz, J = 12.8 Hz, 1H), 7.95 (s, 1H), 7.82 (d, J = 8.4 Hz, 1H), 7.72-7.69 (m, 1H), 7.37-7.32 (m, 4H), 7.26-7.15 (m, 3H, overlapped), 6.51 (d, J = 5.6 Hz, 1H), 4.98 (t, J = 8.4 Hz, 2H), 3.63 (s, 2H), 3.52 (d, J = 6.4 Hz, 2H), 3.39 (t, J = 7.2 Hz, 2H), 3.35 (s, 3H), 3.17 (t, J = 7.2 Hz, 2H), 3.02 (t, J = 6.4 Hz, 2H), 2.77-2.71 (m, 1H)

### Example 16: N-(3-Fluoro-4-((2-(5-((3-methoxy-3-methylazetidin-1-yl)methyl)pyridin-2-yl)thieno[3,2-b]pyridin-7-yl)oxy)phenyl)-5-(4-fluorophenyl)-6-oxo-2,3,5,6- tetrahydrofuro[3,2-c]pyridine-7-carboxamide

The title compound (white solid) was obtained using the same synthetic route as in steps 1, 3, 4, 5, and 6 of Example 1, except that 3-methoxy-3-methylazetidine hydrochloride was used as a starting material in step 1.

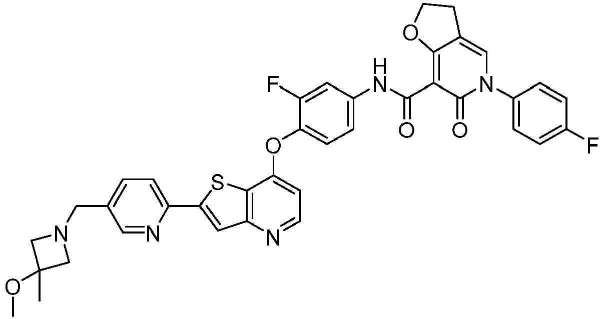

¹H NMR (400 MHz, CDCl₃) δ 12.04 (S, 1H), 8.55 (s, 1H), 8.48 (dd, J = 5.6, 1.6 Hz, 1H), 8.02 (d, J = 12.4 Hz, 1H), 7.96 (s, 1H), 7.83 (d, J = 8.4 Hz, 1H), 7.74 (d, J = 7.6 Hz, 1H), 7.38-7.33 (m, 4H), 7.26-7.18 (m, 3H), 6.52 (d, J = 5.6 Hz, 1H), 5.01 (t, J = 8.8 Hz, 2H), 3.71 (s, 2H), 3.26 (d, J = 7.6 Hz, 2H), 3.20-3.16 (m, 5H), 3.10 (d, J = 7.2 Hz, 2H), 1.49 (s, 3H)

### Example 17: N-(3-Fluoro-4-((2-(5-((3-fluoroazetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 3-fluoroazetidine hydrochloride was used as a starting material in step 1.

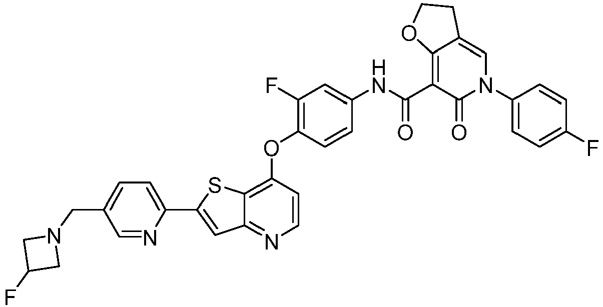

¹H NMR (500 MHz, DMSO-d₆) δ 11.92 (s, 1H), 8.53 (d, J = 1.0 Hz, 1H), 8.52 (d, J = 5.5 Hz, 1H), 8.33 (s, 1H), 8.23 (d, J = 8.0 Hz, 1H), 8.00 (dd, J = 14.0, 2.0 Hz, 1H), 7.87 (s, 1H), 7.84 (dd, J = 8.5, 2.0 Hz, 2H), 7.52-7.50 (m, 2H), 7.45-7.43 (m, 2H), 7.40-7.36 (m, 2H), 6.69 (d, J = 5.6 Hz, 1H), 5.19 (dp, doublet of pentets, J = 58.0, 5.0 Hz, 1H), 4.85 (t, J = 8.4 Hz, 2H), 3.70 (s, 2H), 3.62-3.56 (m, 2H), 3.23-3.16 (m, 2H), 3.11 (t, J = 7.5 Hz, 2H)

### Example 18: N-(4-((2-(5-((3,3-Difluoroazetidin-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide

The title compound (yellow solid) was obtained using the same synthetic route as in steps 1, 3, 4, 5, and 6 of Example 1, except that 3,3-difluoroazetidine hydrochloride was used as a starting material in step 1.

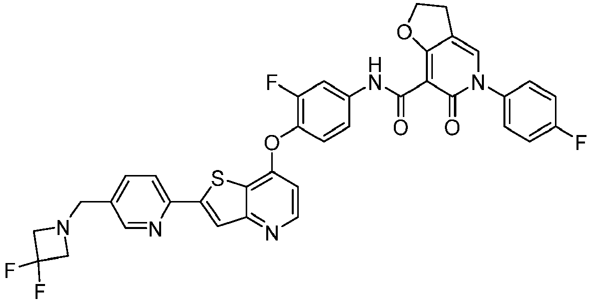

¹H NMR (400 MHz, DMSO-d₆) δ 11.91 (s, 1H), 8.56 (d, J = 1.2 Hz, 1H), 8.53 (d, J = 5.2 Hz, 1H), 8.33 (s, 1H), 8.23 (d, J = 8.4 Hz, 1H), 7.99 (dd, J = 13.2,1.6 Hz, 1H), 7.89-7.86 (m, 2H_2 peaks overlapped), 7.52-7.48 (m, 2H), 7.47-7.44 (m, 2H), 7.42-7.36 (m, 2H), 6.69 (d, J = 5.6 Hz, 1H), 4.85 (t, J = 8.4 Hz, 2H), 3.81 (s, 2H), 3.68 (d, J = 12.8 Hz, 2H), 3.65 (d, J = 12.4 Hz, 2H), 3.11 (t, J = 8.0 Hz, 2H)

### Example 19: N-(4-((2-(5-((3-Cyanoazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that azetidine-3-carbonitrile hydrochloride was used as a starting material in step 1.

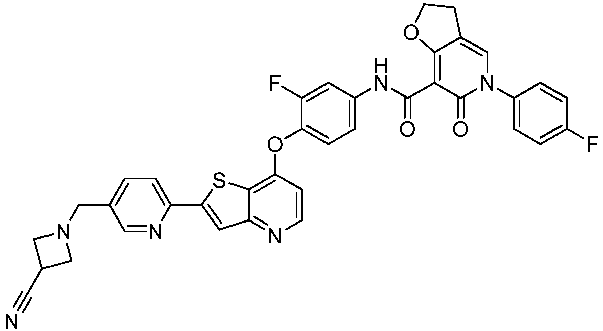

¹H NMR (400 MHz, DMSO-d₆) δ 11.93 (s, 1H), 8.52 (d, J = 5.2 Hz, 2H), 8.34 (s, 1H), 8.25 (d, J = 8.0 Hz, 1H), 8.02-7.98 (m, 1H), 7.88 (s, 1H) 7.84-7.82 (m, 1H), 7.53-7.48 (m, 2H), 7.46-7.36 (m, 4H), 6.69 (d, J = 5.2 Hz, 1H), 4.85 (t, J = 8.4 Hz, 2H), 3.65 (s, 2H), 3.52-3.46 (m, 3H), 3.37-3.31 (m, 2H, overlapped), 3.10 (t, J = 8.0 Hz, 2H)

### Example 20: N-(4-((2-(5-((3-(Dimethylamino)azetidin-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 3-(dimethylamino)azetidine dihydrochloride was used as a starting material in step 1.

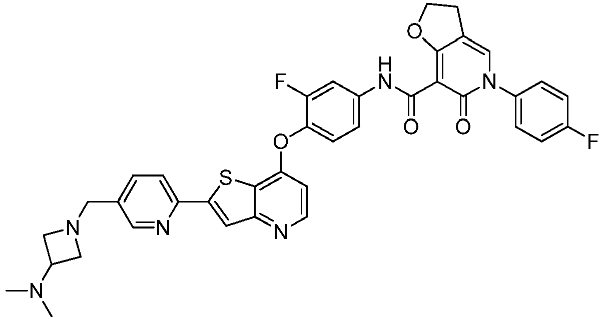

¹H NMR (400 MHz, DMSO-d₆) δ 11.90 (s, 1H), 8.52 (d, J = 4.8 Hz, 2H), 8.30 (s, 1H), 8.21 (d, J = 8.0 Hz, 1H), 8.01-7.97 (m, 1H), 7.86 (t, J = 1.2 Hz, 1H), 7.83-7.80 (dd, J = 2.4 Hz, J = 8.4 Hz, 1H), 7.52-7.49 (m, 2H), 7.45-7.35 (m, 4H), 6.69 (d, J = 5.2 Hz, 1H), 4.85 (t, J = 8.4 Hz, 2H), 3.62 (s, 2H), 3.37 (t, J = 7.6 Hz, 2H), 3.11 (t, J = 7.6 Hz, 2H), 2.87-2.77 (m, 3H), 2.01 (s, 6H)

### Example 21: N-(3-Fluoro-4-((2-(5-((3-methylazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 3-methylazetidine hydrochloride was used as a starting material in step 1.

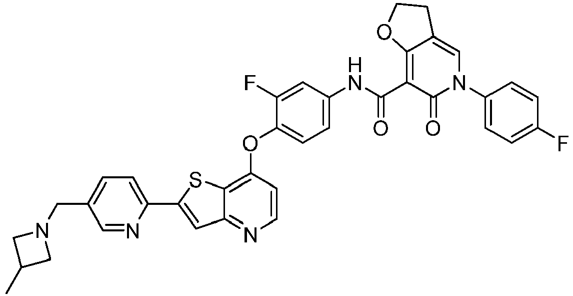

¹H NMR (400 MHz, DMSO-d₆) δ 11.90 (s, 1H), 8.52 (d, J = 5.6 Hz, 2H), 8.30 (s, 1H), 8.21 (d, J = 8.4 Hz, 1H), 8.01-7.97 (m, 1H), 7.86 (s, 1H) 7.82-7.80 (m, 1H), 7.52-7.49 (m, 2H), 7.44-7.35 (m, 4H), 6.69 (d, J = 5.2 z, 1H), 4.85 (t, J = 8.4 Hz, 2H), 3.61 (s, 2H), 3.37 (t, J = 7.6 Hz, 2H), 3.11 (t, J = 7.2 Hz, 2H), 2.76 (t, J = 6.4 Hz, 2H), 2.50-2.42 (m, 1H, overlapped), 1.13 (d, J = 6.8 Hz, 3H)

### Example 22: N-(4-((2-(5-((3,3-Dimethylazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 3,3-dimethylazetidine hydrochloride was used as a starting material in step 1.

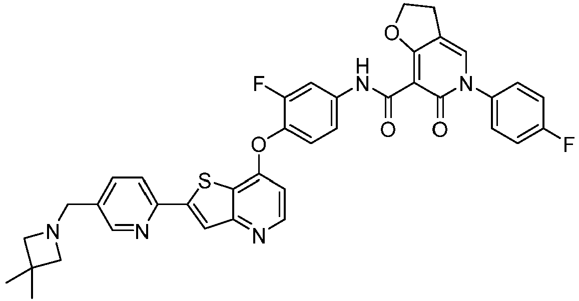

¹H NMR (400 MHz, CDCl₃) δ 12.04 (s, 1H), 8.53 (s, 1H), 8.47 (d, J = 5.2 Hz, 1H), 8.02-7.98 (dd, J = 2.4 Hz, J = 12.8 Hz, 1H), 7.95 (s, 1H), 7.82 (d, J = 8.0 Hz, 1H) 7.73-7.71 (m, 1H), 7.37-7.34 (m, 4H), 7.26-7.17 (m, 3H,overlapped), 6.51 (d, J = 5.2 Hz, 1H), 4.98 (t, J = 8.4 Hz, 2H), 3.65 (s, 2H), 3.17 (t, J = 7.6 Hz, 2H), 3.00 (s, 4H), 1.24 (s, 6H)

### Example 23: N-(3-Fluoro-4-((2-(5-((3-hydroxypyrrolidin-1-yl)methyl)pyridin-2-yl)thieno[3,2-b]pyridin-7-yl)oxy)phenyl)-5-(4- fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 3-hydroxypyrrolidine was used as a starting material in step 1.

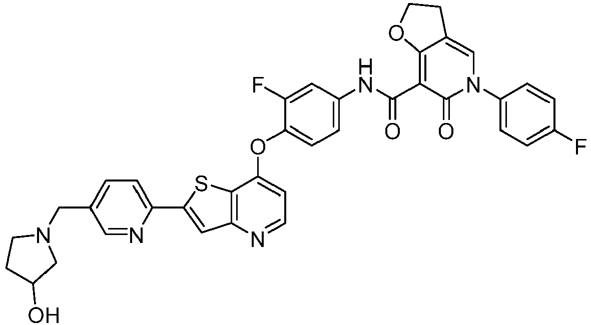

¹H NMR (400 MHz, DMSO-d₆) δ 12.04 (s, 1H), 8.58 (s, 1H), 8.49 (d, J = 5.2 Hz, 1H), 8.02 (dd, J = 12.8, 2.4 Hz, 1H), 7.98 (s, 1H), 7.85 (d, J = 7.6 Hz, 1H), 7.80 (d, J = 9.6 Hz, 1H), 7.38 (m, 4H), 7.26-7.18 (m, 3H), 6.52 (d, J = 4.0 Hz, 1H), 5.01 (t, J = 8.8 Hz, 2H), 4.37 (s, 1H), 3.70 (s, 2H), 3.20 (t, J = 8.8 Hz, 2H), 2.92 (q, J = 4.0 Hz, 1H), 2.73 (d, J = 10.0 Hz, 1H), 2.62-2.59 (m, 1H), 2.37 (d, J = 6.4 Hz, 1H), 2,24-2.17 (m, 1H) 1.81 (q, J = 7.2 Hz, 1H).

### Example 24: N-(3-Fluoro-4-((2-(5-((3-hydroxy-3-methylpyrrolidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 3-methylpyrrolidin-3-ol was used as a starting material in step 1.

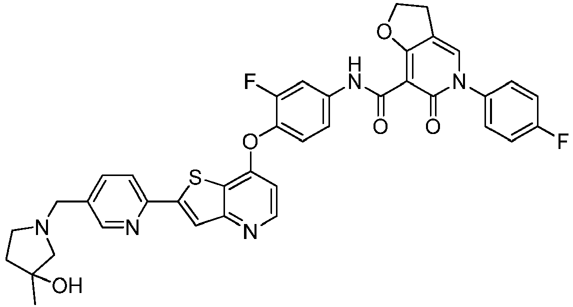

¹H NMR (400 MHz, DMSO-d₆) δ 11.90 (s, 1H), 8.56 (d, J = 1.6 Hz, 1H), 8.52 (d, J = 4.8 Hz, 1H), 8.31 (s, 1H), 8.23 (d, J = 8.0 Hz, 1H), 8.01-7.97 (m, 1H) 7.87-7.84 (m, 2H), 7.52-7.49 (m, 2H), 7.45-7.33 (m, 4H), 6.69 (d, J = 5.2 Hz, 1H), 4.85 (t, J = 8.8 Hz, 2H), 4.52 (s, 1H), 3.65 (s, 2H), 3.11 (t, J = 7.2 Hz, 2H), 2.69-2.43 (m, 4H, overlapped), 1.74-1.64 (m, 2H), 1.24 (s, 3H)

### Example 25: N-(3-Fluoro-4-((2-(5-((3-methoxypyrrolidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 3-methoxypyrrolidine hydrochloride was used as a starting material in step 1.

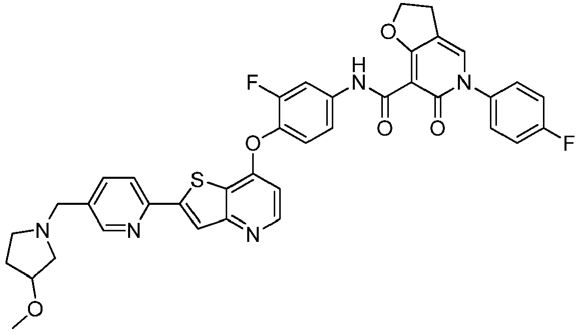

¹H NMR (400 MHz, CDCl₃) δ 12.01 (s, 1H), 8.56 (d, J = 1.2 Hz, 1H), 8.48 (d, J = 5.2 Hz, 1H), 8.02-7.98 (dd, J = 2.8 Hz, J = 12.8 Hz, 1H), 7.96 (s, 1H), 7.84 (d, J = 8.4 Hz, 1H), 7.80-7.77 (m, 1H), 7.37-7.34 (m, 4H), 7.26-7.15 (m, 3H, overlapped), 6.52 (d, J = 5.6 Hz, 1H), 4.98 (t, J = 8.4 Hz, 2H), 3.96-3.91 (m, 1H), 3.67 (s, 2H), 3.27 (s, 3H), 3.17 (t, J = 8.4 Hz, 2H), 2.77-2.68 (m, 2H), 2.61-2.58 (m, 1H), 2.53-2.48 (m, 1H), 2.12-2.05 (m, 1H), 1.86-1.79 (m, 1H)

### Example 26: N-(3-Fluoro-4-((2-(5-(pyrrolidin-1-ylmethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that pyrrolidine was used as a starting material in step 1.

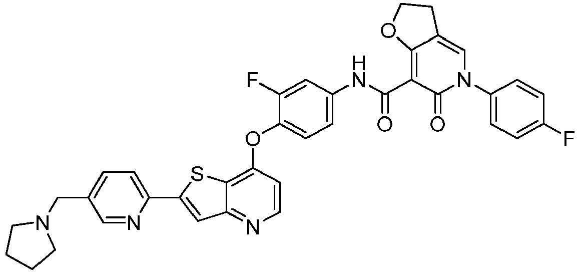

¹H NMR (400 MHz, CDCl₃) δ 12.22 (s, 1H), 8.96 (d, J = 1.6 Hz, 1H), 8.56-8.54 (m, 2H), 8.45-8.43 (dd, J = 2.4 Hz, J = 8.4 Hz, 1H), 8.13-8.09 (m, 1H), 8.05 (d, J = 8.4 Hz, 1H), 7.45-7.34 (m, 4H), 7.31-7.22 (m, 3H), 6.92 (d, J = 6.4 Hz, 1H), 4.99 (t, J = 8.4 Hz, 2H), 4.37-4.36 (m, 2H), 3.73-3.71 (m, 2H), 3.19 (t, J = 8.4 Hz, 2H), 2.98-2.92 (m, 2H), 2.29-2.26 (m, 1H), 2.14-2.08 (m, 1H), 1.69 (bs, 2H)

### Example 27: N-(3-Fluoro-4-((2-(5-((3-fluoropyrrolidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 3-fluoropyrrolidine hydrochloride was used as a starting material in step 1.

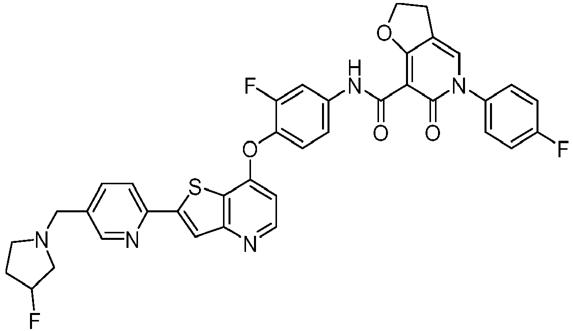

¹H NMR (400 MHz, DMSO-d₆) δ 11.90 (s, 1H), 8.56 (d, J = 1.6 Hz, 1H), 8.52 (d, J = 5.6 Hz, 1H), 8.31 (s, 1H), 8.23 (d, J = 8.4 Hz, 1H), 8.01-7.97 (m, 1H), 7.88-7.86 (m, 2H), 7.50-7.37 (m, 6H), 6.69 (d, J = 5.6 Hz, 1H), 4.85 (t, J = 8.4 Hz, 2H), 3.69 (s, 2H), 3.11 (m, 2H), 2.85-2.76 (m, 2H), 2.72-2.60 (m, 1H), 2.44-2.34 (m, 1H), 2.21-2.08 (m, 1H), 1.98-1.85 (m, 1H), 1.17-1.14 (m, 1H)

### Example 28: N-(4-((2-(5-((3,3-Difluoropyrrolidin-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 3,3-difluoropyrrolidine hydrochloride was used as a starting material in step 1.

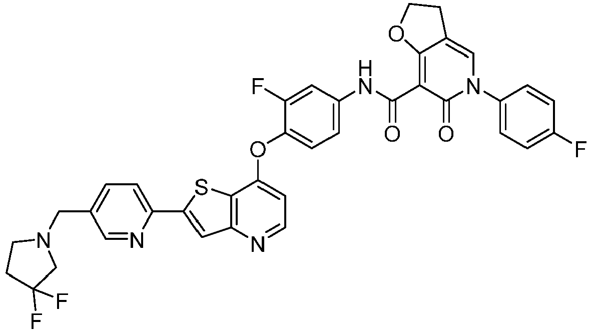

¹H NMR (400 MHz, DMSO-d₆) δ 11.90 (s, 1H), 8.56 (d, J = 1.2 Hz, 1H), 8.52 (d, J = 5.6 Hz, 1H), 8.32 (s, 1H), 8.24 (d, J = 8 Hz, 1H), 8.01-7.95 (m, 1H), 7.89-7.85 (m, 2H), 7.52-7.35 (m, 6H), 6.69 (d, J = 5.2 Hz, 1H), 4.85 (t, J = 8.4 Hz, 2H), 3.71 (s, 2H), 3.11 (t, J = 8 Hz, 2H), 2.91 (t, J = 13.6 Hz, 2H), 2.75-2.71 (m, 2H), 2.32-2.23 (m, 2H)

### Example 29: N-(3-Fluoro-4-((2-(5-((4-hydroxypiperidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 4-hydroxypiperidine was used as a starting material in step 1.

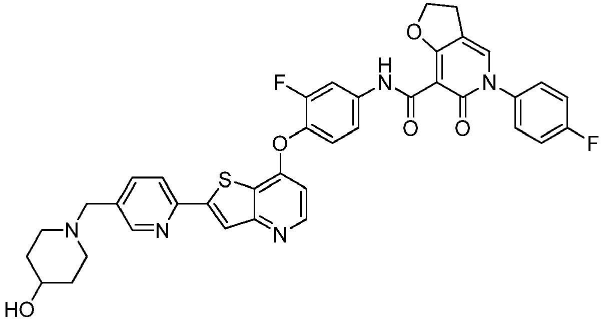

¹H NMR (400 MHz, DMSO-d₆) δ 11.91 (s, 1H), 8.52 (d, J = 5.2 Hz, 2H), 8.31 (s, 1H), 8.23 (d, J = 8.0 Hz, 1H), 8.01-7.98 (m, 1H), 7.87 (s, 1H), 7.85-7.83 (m, 1H), 7.52-7.47 (m, 2H), 7.45-7.35 (m, 4H), 6.69 (d, J = 5.2 Hz, 1H), 4.85 (t, J = 8.4 Hz, 2H), 4.55 (d, J = 4.0 Hz, 1H), 3.52 (s, 2H), 3.17 (d, J = 5.6 Hz, 1H), 3.10 (t, J = 7.6 Hz, 2H), 2.68-3.66 (m, 2H), 2.08 (t, J = 8.0 Hz, 2H), 1.72-1.69 (m, 2H), 1.44-1.36 (m, 2H)

### Example 30: N-(3-Fluoro-4-((2-(5-((3-hydroxypiperidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in steps 1, 3, 4, 5, and 6 of Example 1, except that 3-hydroxypiperidine was used as a starting material in step 1.

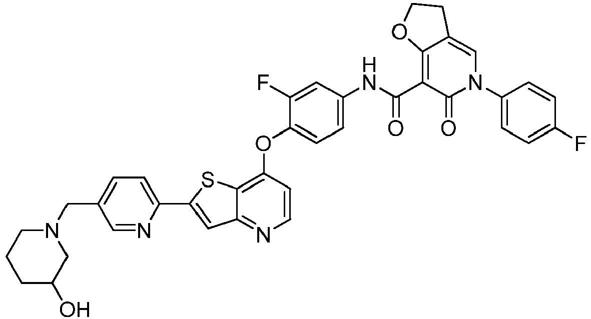

¹H NMR (400 MHz, DMSO-d₆) δ 11.93 (s, 1H), 8.54-8.51 (m, 2H), 8.33 (s, 1H), 8.25 (d, J = 8.4 Hz, 1H), 8.02-7.98 (m, 1H), 7.88 (s, 1H) 7.86-7.83 (m, 1H), 7.53-7.46 (m, 2H), 7.44-7.34 (m, 4H), 6.69 (d, J = 5.2 Hz, 1H), 4.85 (t, J = 8.4 Hz, 2H), 4.61 (d, J = 5.2 Hz, 1H), 3.60-3.44 (m, 3H), 3.10 (t, J = 7.6 Hz, 2H), 2.79 (d, J = 7.6 Hz, 1H), 2.66 (d, J = 10.8 Hz, 1H), 1.90 (t, J = 7.6 Hz, 1H), 1.79-1.73 (m, 2H), 1.64-1.60 (m, 1H), 1.47-1.38 (m, 1H), 1.11-1.03 (m, 1H)

### Example 31: N-(3-Fluoro-4-((2-(5-((4-methoxypiperidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 4-methoxypiperidine hydrochloride was used as a starting material in step 1.

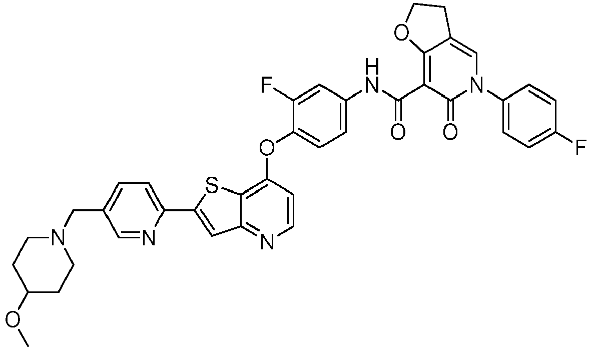

¹H NMR (400 MHz, CDCl₃) δ 12.04 (s, 1H), 8.55 (d, J = 1.2 Hz, 1H), 8.48 (d, J = 5.6 Hz, 1H), 8.02-7.96 (m, 2H), 7.84-7.82 (d, J = 8.0 Hz, 1H), 7.77-7.75 (m, 1H), 7.36-7.15 (m, 4H), 7.26-7.15 (m, 3H, overlapped), 6.52 (d, J = 5.6 z, 1H), 4.98 (t, J = 8.8 Hz, 2H), 3.54 (s, 2H), 3.33 (s, 3H), 3.25-3.15 (m, 3H), 2.73 (t, J = 6.0 Hz, 2H), 2.19 (t, J = 9.2 Hz, 2H), 1.91-1.88 (m, 2H), 1.62-1.56 (m, 2H, overlapped)

### Example 32: N-(3-Fluoro-4-((2-(5-((3-methoxypiperidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 3-methoxypiperidine hydrochloride was used as a starting material in step 1.

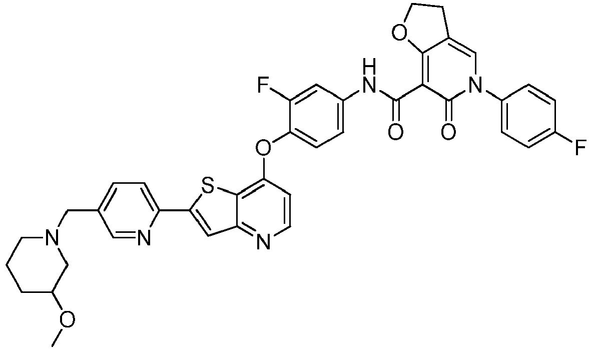

¹H NMR (400 MHz, CDCl₃) δ 12.04 (s, 1H), 8.53 (s, 1H), 8.44 (d, J = 4.8 Hz, 1H), 7.99-7.96 (m, 2H), 7.85 (d, J = 8.4 Hz, 1H), 7.77-7.75 (m, 1H), 7.35-7.31 (m, 4H), 7.26-7.15 (m, 3H, overlapped), 6.51 (d, J = 5.2 Hz, 1H), 4.96 (t, J = 8.4 Hz, 2H), 3.56 (s, 2H), 3.33 (s, 3H), 3.16 (t, J = 8.4 Hz, 2H), 2.90-2.88 (m, 1H), 2.64-2.61 (m, 1H), 2.12-1.92 (m, 4H), 1.75-1.56 (m, 1H, overlapped), 0.86-0.80 (m, 2H)

### Example 33: N-(3-Fluoro-4-((2-(5-(piperidin-1-ylmethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that piperidine was used as a starting material in step 1.

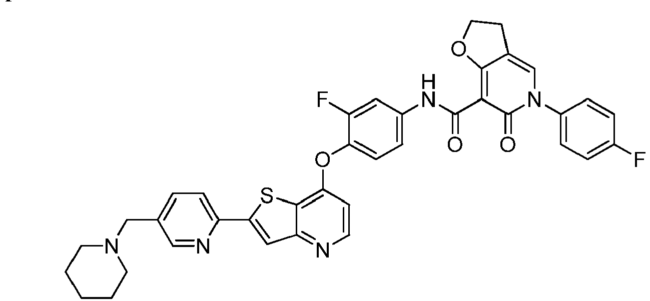

¹H NMR (400 MHz, CDCl₃) δ 12.03 (s, 1H), 8.55 (d, J = 1.2 Hz, 1H), 8.48 (d, J = 2.4 Hz, 1H), 8.02-7.98 (dd, J = 2.4 Hz, J = 12.8 Hz, 1H), 7.96 (s, 1H), 7.83 (d, J = 8.0 Hz, 1H) 7.78 (m, 1H), 7.37-7.34 (m, 4H), 7.26-7.18 (m, 3H,overlapped), 6.51 (d, J = 5.2 Hz, 1H), 4.98 (t, J = 8.4 Hz, 2H), 3.52 (s, 2H), 3.17 (t, J = 8.8 Hz, 2H), 2.40 (bs 4H), 1.61-1.56 (m, 4H, overlapped), 1.45-1.44 (m, 2H)

### Example 34: N-(3-Fluoro-4-((2-(5-((4-fluoropiperidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 4-fluoropiperidine hydrochloride was used as a starting material in step 1.

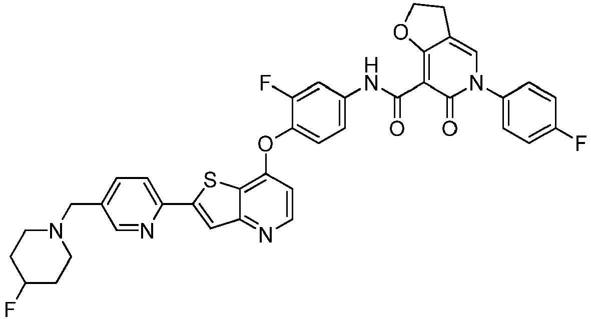

¹H NMR (400 MHz, CDCl₃) δ 12.01 (s, 1H), 8.54 (s, 1H), 8.46-8.44 (d, J = 5.2 Hz, 1H), 7.99-7.94 (m, 2H), 7.81 (d, J = 8.0 Hz, 1H), 7.74-7.72 (m, 1H) 7.35-7.31 (m, 4H), 7.26-7.13 (m, 3H, overlapped), 6.50 (d, J = 4.8 Hz, 1H), 4.95 (t, J = 8.4 Hz, 2H), 4.76-4.60 (m, 1H), 3.53 (s, 2H), 3.15 (t, J = 8.0 Hz, 2H), 2.58 (t, J = 6.4 Hz, 2H), 2.39 (t, J = 5.6 Hz, 2H), 1.94-1.82 (m, 4H)

### Example 35: N-(4-((2-(5-((4,4-Difluoropiperidin-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 4,4-difluoropiperidine hydrochloride was used as a starting material in step 1.

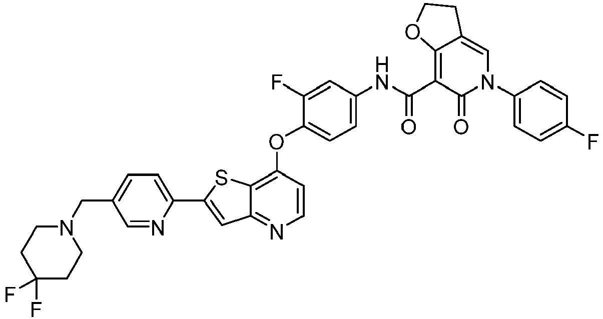

¹H NMR (400 MHz, DMSO-d₆) δ 11.90 (s, 1H), 8.57 (d, J = 1.6 Hz, 1H), 8.52 (d, J = 5.2 Hz, 1H), 8.32 (s, 1H), 8.24 (d, J = 8.4 Hz, 1H), 8.01-7.97 (m, 1H), 7.89-7.86 (m, 2H), 7.52-7.35 (m, 6H), 6.69 (d, J = 5.2 Hz, 1H), 4.85 (t, J = 8.4 Hz, 2H), 3.64 (s, 2H), 3.11 (t, J = 7.2 Hz, 2H), 2.67-5.65 (m, 2H), 2.54-2.52 (m,2H), 1.99-1.97 (m, 4H)

### Example 36: N-(3-Fluoro-4-((2-(5-((4-methylpiperidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 4-methylpiperidine was used as a starting material in step 1.

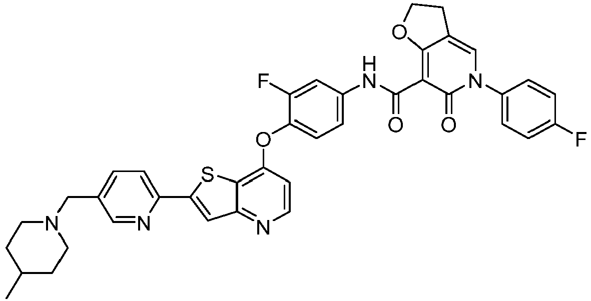

¹H NMR (400 MHz, DMSO-d₆) δ 11.88 (s, 1H), 8.49-8.48 (m, 2H), 8.28 (s, 1H), 8.20-8.18 (d, J = 8.0 Hz, 1H), 7.98-7.94 (m, 1H), 7.83-7.79 (m, 2H), 7.49-7.46 (m, 2H), 7.42-7.40 (m, 2H), 7.37-7.32 (t, J = 8.8 Hz, 2H), 6.66-6.64 (d, J = 5.6 Hz, 1H), 4.83-4.79 (t, J = 8.0 Hz, 2H), 3.48-3.45 (d, J = 13.6 Hz, 2H), 3.09-3.05 (d, J = 8.4 Hz, 2H), 2.75-2.72 (d, J = 11.2 Hz, 2H), 1.95-1.89 (m, 2H), 1.55-1.52 (d, J = 10.8 Hz, 2H), 1.15-1.09 (m, 3H), 0.86-0.84 (d, J = 6.4 Hz, 3H)

### Example 37: N-(4-((2-(5-((4,4-Dimethylpiperidin-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 4,4-dimethylpiperidine hydrochloride was used as a starting material in step 1.

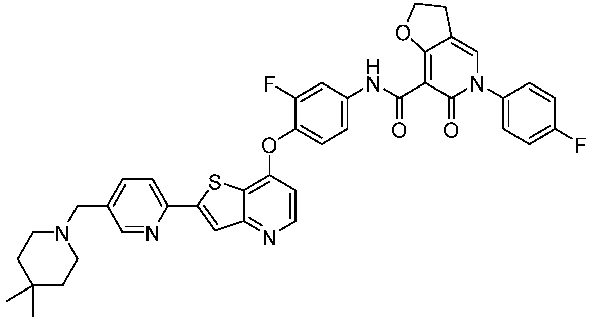

¹H NMR (400 MHz, DMSO-d₆) δ 11.92 (s, 1H), 8.53-8.52 (m, 2H), 8.32 (s, 1H), 8.24-8.22 (d, J = 8.0 Hz, 1H), 8.02-7.98 (m, 1H), 7.87-7.83 (m, 2H), 7.53-7.36 (m, 6H), 6.69-6.68 (d, J = 5.2 Hz, 1H), 4.87-4.83 (t, J = 8.4 Hz, 2H), 3.54 (s, 2H), 3.13-3.09 (t, J = 7.6 Hz, 2H), 2.38-2.35 (t, J = 4.4 Hz, 4H), 1.35-1.32 (t, J = 11.2 Hz, 4H), 0.90 (s, 6H)

### Example 38: N-(3-Fluoro-4-((2-(5-((((3-hydroxycyclohexyl)methyl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in steps 1, 3, 4, 5, and 6 of Example 1, except that 3-(aminomethyl)cyclohexanol was used as a starting material in step 1.

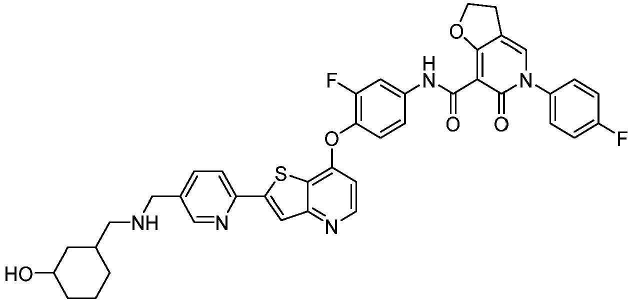

¹H NMR (400 MHz, DMSO-d₆) δ 11.91 (s, 1H), 8.58 (s, 1H), 8.52 (d, J = 5.2 Hz, 1H), 8.53 (s, 1H), 8.22 (d, J = 8.4 Hz, 1H), 8.00 (dd, J = 12.4, 2.0 Hz, 1H), 7.89 (d, J = 8.0 Hz, 1H), 7.86 (s, 1H), 7.53-7.47 (m, 2H), 7.46-7.42 (m, 2H), 7.41-7.36 (m, 2H), 6.69 (d, J = 5.6 Hz, 1H), 4.85 (t, J = 8.4 Hz, 2H), 4.44 (d, J = 4.8 Hz, 1H), 3.77 (bs, 2H), 3.11 (t, J = 8.0 Hz, 2H), 2.42-2.32 (m, 2H), 1.98-1.90 (m, 1H), 1.85-1.76 (m, 1H), 1.72-1.62 (m, 2H), 1.54-1.42 (m, 1H), 1.22-1.12 (m, 1H), 1.08-0.94 (m, 1H), 0.80-0.68 (m, 2H)

### Example 39: N-(3-Fluoro-4-((2-(5-(((tetrahydro-2H-pyran-4-yl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

The title compound (pale yellow solid) was obtained using the same synthetic route as in steps 1, 3, 4, 5, and 6 of Example 1, except that 4-aminotetrahydropyran was used as a starting material in step 1.

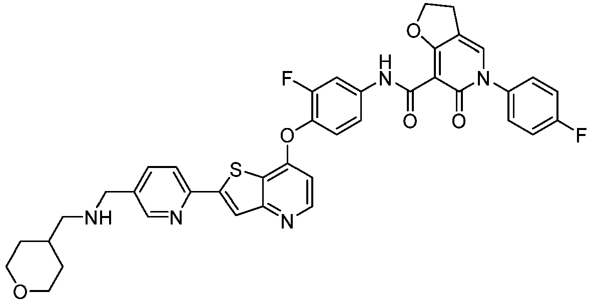

¹H NMR (400 MHz, DMSO-d₆) δ 11.91 (s, 1H), 8.59 (d, J = 1.6 Hz, 1H), 8.52 (d, J = 5.6 Hz, 1H), 8.30 (s, 1H), 8.21 (d, J = 8.4 Hz, 1H), 7.99 (dd, J = 14.0, 1.6 Hz, 1H), 7.91 (dd, J = 8.4, 1.6 Hz, 1H), 7.86 (t, J = 1.6 Hz, 1H), 7.53-7.47 (m, 2H), 7.45-7.42 (m, 2H), 7.40-7.36 (m, 2H), 6.69 (d, J = 4.4 Hz, 1H), 4.85 (t, J = 8.4 Hz, 2H), 3.85-3.80 (m, 4H), 3.50-3.30 (2H_overlapped with H₂O), 3.11 (t, J = 8.0 Hz, 2H), 2.63-2.56 (m, 1H), 1.84-1.74 (m, 2H), 1.35-1.24 (m, 2H)

### Example 40: N-(4-((6,7-Dimethoxyquinolin-4-yl)oxy)-3-fluorophenyl)-5-(4-fluorophenyl)-6-oxo-2,3,5,6-tetrahydrofuro[3,2-c]pyridine-7-carboxamide

### Step 1: Synthesis of 4-(2-fluoro-4-nitrophenoxy)-6,7-dimethoxyquinoline

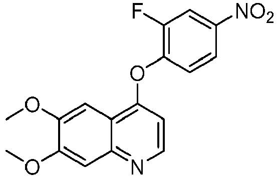

4-Chloro-6,7-dimethoxyquinoline (200 mg, 0.89 mmol) was dissolved in diphenyl ether. Then, anhydrous potassium carbonate (147 mg, 1.07 mmol) and 2-fluoro-4-nitrophenol (280 mg, 1.78 mmol) were sequentially added thereto, and stirring was performed at a temperature of 160°C for 5 hours. The reaction mixture was cooled to room temperature, and extracted with ethyl acetate. The separated organic layer was dried over anhydrous sodium sulfate, filtered, and concentrated under reduced pressure. The residue obtained by the concentration under reduced pressure was purified by column chromatography to obtain the title compound (240 mg, yield: 78%, yellow solid).

¹H NMR (500 MHz, DMSO-d₆) δ 8.82 (d, J = 5.0 Hz, 1H), 8.56 (d, J = 15.0 Hz, 1H), 8.29 (d, J = 10.0 Hz, 1H), 7.82 (t, J = 7.5 Hz, 1H), 7.67 (s, 1H), 7.56 (s, 1H), 7.08 (d, J = 5.0 Hz, 1H), 4.03 (s, 3H), 4.00 (s, 3H)

### Step 2: Synthesis of 4-((6,7-dimethoxyquinolin-4-yl)oxy)-3-fluoroaniline

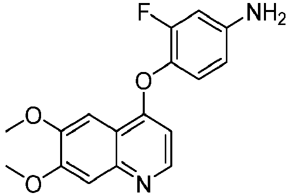

The compound (240 mg, 0.69 mmol) prepared in step 1 was dissolved in ethanol and water. Then, iron (116 mg, 2.09 mmol) and ammonium chloride (372 mg, 6.97 mmol) were sequentially added thereto at room temperature. The temperature was raised to 100°C, and stirring was performed for 2 hours. After completion of the reaction, the reaction mixture was filtered using a Celite pad in a warm state and concentrated under reduced pressure. The resulting residue was extracted with dichloromethane. The separated organic layer was dried over anhydrous sodium sulfate, concentrated under reduced pressure, and filtered with diethyl ether to obtain the title compound (170 mg, yield: 77%, yellow solid).

¹H NMR (500MHz, DMSO-d₆) δ 8.46 (d, J = 5.0 Hz, 1H), 7.50 (s, 1H), 7.38 (s, 1H), 7.07 (t, J = 10.0 Hz, 1H), 6.55 (d, J = 15.0 Hz, 1H), 6.46 (d, J = 10.0 Hz, 1H), 6.39 (d, J = 5.0 Hz, 1H), 5.50 (brs, 2H), 3.94 (s, 6H)

### Step 3: Synthesis of N-(4-((6,7-Dimethoxyquinolin-4-yl)oxy)-3-fluorophenyl)-5-(4-fluorophenyl)-6-oxo-2,3,5,6-tetrahydrofuro[3,2-c]pyridine-7-carboxamide

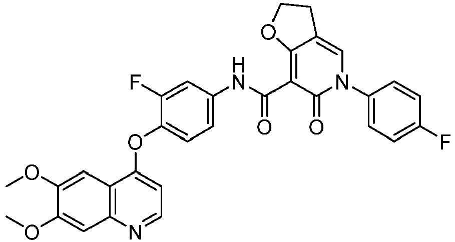

The carboxylic acid (18 mg, 0.07 mmol) prepared in Preparation Example 1 and 1-[bis(dimethylamino)methylene]-1H-1,2,3-triazolo[4,5-b]pyridinium-3-oxide hexafluorophosphate (HATU, 27 mg, 0.07 mmol) were dissolved in dimethylformamide. Then, N,N-diisopropylethylamine (21 mg, 0.07 mmol) and 4-((6,7-dimethoxyquinolin-4-yl)oxy)-3-fluoroaniline (15 mg, 0.05 mmol) prepared in step 2 were sequentially added thereto, and stirring was performed at room temperature for 24 hours or longer. The reaction was quenched with saturated aqueous sodium bicarbonate solution and extracted with ethyl acetate. The separated organic layer was dried over anhydrous magnesium sulfate and concentrated under reduced pressure. The resulting residue was purified by column chromatography to obtain the title compound (21 mg, yield: 26%, brown solid).

¹H NMR (500 MHz, DMSO-d₆) δ 11.89 (s, 1H), 8.48 (d, J = 5.0 Hz, 1H), 7.99 (d, J = 15.0 Hz, 1H), 7.88 (brs, 1H), 7.52 (s, 1H), 7.51-7.50 (m, 2H), 7.43-7.37 (m, 5H), 6.47 (d, J = 5.0 Hz, 1H), 4.85 (t, J = 10.0 Hz, 2H), 3.95 (s, 6H), 3.11 (t, J = 7.5 Hz, 2H)

### Example 41: N-(4-((6,7-Dimethoxyquinolin-4-yl)oxy)-3-fluorophenyl)-6-oxo-5-phenyl-2,3,5,6-tetrahydrofuro[3,2-c]pyridine-7-carboxamide

The title compound (yellow solid) was obtained using the same synthetic route as in Example 40, except that the carboxylic acid compound of Preparation Example 2 was used as a starting material in step 3.

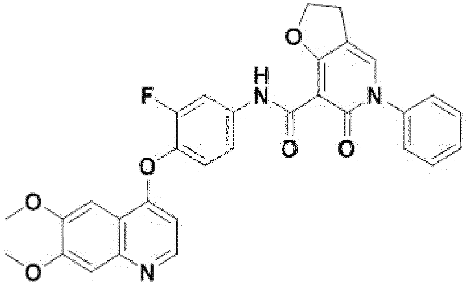

¹H NMR (500 MHz, DMSO-d₆) δ 11.95 (s, 1H), 8.53 (s, 1H), 8.01 (d, J = 10.0 Hz, 1H), 7.88 (s, 1H), 7.56-7.53 (m, 3H), 7.50-7.42 (m, 6H), 6.55 (brs, 1H), 4.85 (t, J = 10.0 Hz, 2H), 3.96 (s, 6H), 3.11 (t, J = 10.0 Hz, 2H)

### Example 42: N-(4-((6,7-Dimethoxyquinolin-4-yl)oxy)-3-fluorophenyl)-6-oxo-5-(4-(trifluoromethyl)phenyl)-2,3,5,6-tetrahydrofuro[3,2-c]pyridine-7-carboxamide

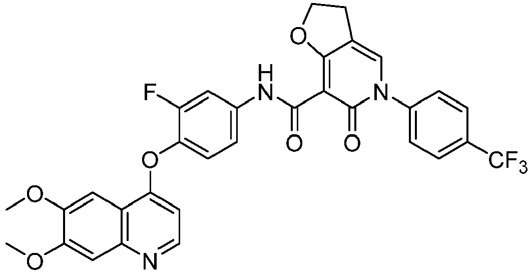

The title compound (yellow solid) was obtained using the same synthetic route as in Example 40, except that the carboxylic acid compound of Preparation Example 3 was used as a starting material in step 3.

¹H NMR (400 MHz, DMSO-d₆) δ 11.77 (bs, 1H), 8.49 (d, J = 5.2 Hz, 1H), 7.99 (dd, J = 11.6, 2.4 Hz, 1H), 7.95-7.93 (m, 3H), 7.72 (d, J = 8.4 Hz, 1H), 7.54 (s, 1H), 7.42 (d, J = 7.6 Hz, 2H), 6.50 (d, J = 5.2 Hz, 1H), 4.86 (t, J = 8.4 Hz, 2H), 3.952 (s, 3H), 3.950 (s, 3H), 3.13 (t, J = 7.2 Hz, 2H)

### Example 43: N-(4-((6,7-Dimethoxyquinolin-4-yl)oxy)-3-fluorophenyl)-6-oxo-5-(3-fluorophenyl)-2,3,5,6-tetrahydrofuro[3,2-c]pyridine-7-carboxamide

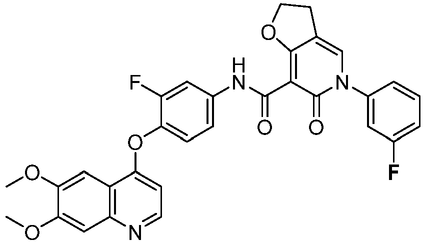

The title compound (yellow solid) was obtained using the same synthetic route as in Example 40, except that the carboxylic acid compound of Preparation Example 4 was used as a starting material in step 3.

¹H NMR (400 MHz, DMSO-d₆) δ 11.83 (bs, 1H), 8.49 (d, J = 5.6 Hz, 1H), 7.99 (dd, J = 12.8, 2.0 Hz, 1H), 7.91 (br, 1H), 7.62-7.57 (m, 1H), 7.53 (s, 1H), 7.46-7.43 (m, 2H), 7.42 (s, 1H), 7.40-7.31 (m, 3H), 6.47 (d, J = 4.8 Hz, 1H), 4.85 (t, J = 8.4 Hz, 2H), 3.95 (s, 6H), 3.11 (t, J = 8.4 Hz, 2H)

### Example 44: N-(3-Fluoro-4-((6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 40, except that 4-chloro-6-methoxyquinoline was used as a starting material in step 1.

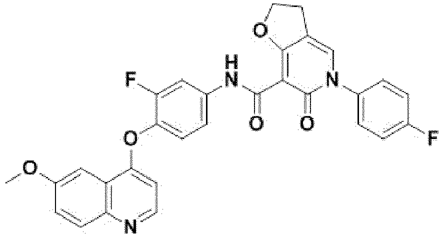

¹H NMR (500MHz, DMSO-d₆) δ 11.90 (s, 1H), 8.53 (d, J = 5.0 Hz, 1H), 8.00 (d, J = 10.0 Hz, 1H), 7.95 (d, J = 10.0 Hz, 1H), 7.88 (s, 1H), 7.58 (d, J = 5.0 Hz, 1H), 7.53-7.37 (m, 7H), 6.59 (d, J = 5.0 Hz, 1H), 4.85 (t, J = 5.0 Hz, 1H), 3.94 (s, 3H), 3.11 (t, J = 5.0 Hz, 3H).

### Example 45: N-(3-Fluoro-4-((7-methoxyquinolin-4-yl)oxy)phenyl)-5-(4-fluorophenyl)-6-oxo-2,3,5,6-tetrahydrofuro[3,2-c]pyridine-7-carboxamide

The title compound (off-white solid) was obtained using the same synthetic route as in Example 40, except that 4-chloro-7-methoxyquinoline was used as a starting material in step 1.

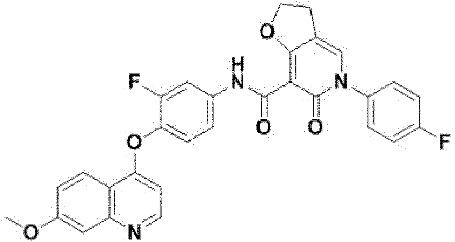

¹H NMR (500 MHz, CDCl₃) δ 11.91 (s, 1H), 8.61 (d, J = 5.0 Hz, 1H), 8.23 (d, J = 10.0 Hz, 1H), 8.00 (d, J = 15.0 Hz, 1H), 7.88 (s, 1H), 7.54-7.50 (m, 3H), 7.43-7.39 (m, 5H), 7.31 (dd, J = 5.0 and 5.0 Hz, 1H), 4.86 (t, J = 7.5 Hz, 2H), 3.95 (s, 3H), 3.12 (t, J = 7.5 Hz, 2H)

### Example 46: N-(4-((6,7-Dimethoxyquinazolin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 40, except that 4-chloro-6,7-dimethoxyquinazoline was used as a starting material in step 1.

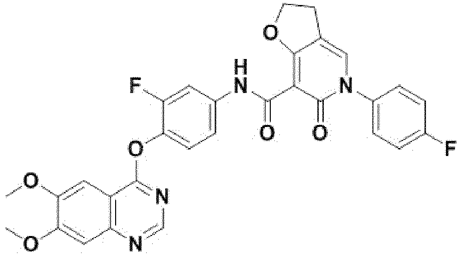

¹H NMR (500 MHz, CDCl₃) δ 12.08 (s, 1H), 8.06 (dd, J = 12.5, 2.0 Hz, 1H), 7.73 (s, 1H), 7.59 (s, 1H), 7.40-7.35 (m, 4H), 7.26-7.19 (m, 3H), 5.00 (t, J = 8.5 Hz, 2H), 3.19 (t, J = 8.0 Hz, 2H)

### Example 47: N-(4-((6-Carbamoyl-7-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (off-white solid) was obtained using the same synthetic route as in Example 40, except that the 4-(4-amino-2-fluorophenoxy)-7-methoxyquinoline-6-carboxamide compound of Preparation Example 6 was used as a starting material in step 3.

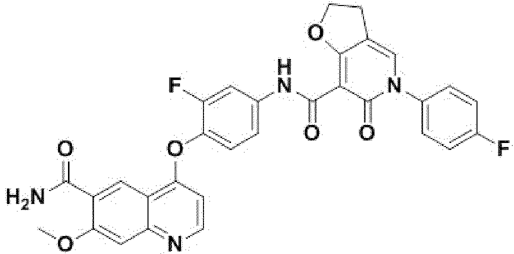

¹H NMR (500 MHz, DMSO-d₆) δ 11.9 (s, 1H), 8.67-8.66 (m, 2H), 8.01 (d, J = 15.0 Hz, 1H), 7.88 (s, 2H), 7.76 (s, 1H), 7.53-7.50 (m, 3H), 7.45-7.37 (m, 4H), 6.51 (d, J = 5.0 Hz, 1H), 4.85 (t, J = 10.0 Hz, 2H), 4.03 (s, 3H), 3.11 (t, J = 10.0 Hz, 3H)

### Example 48: N-(3-Fluoro-4-((7-methoxy-6-(methylcarbamoyl)quinolin-4-yl)oxy)phenyl)-5-(4-fluorophenyl)-6-oxo-2,3,5,6-tetrahydrofuro[3,2-c]pyridine-7-carboxamide

The title compound (orange solid) was obtained using the same synthetic route as in Example 40, except that the 4-(4-amino-2-fluorophenoxy)-7-methoxy-N-methylquinoline-6-carboxamide compound of Preparation Example 7 was used as a starting material in step 3.

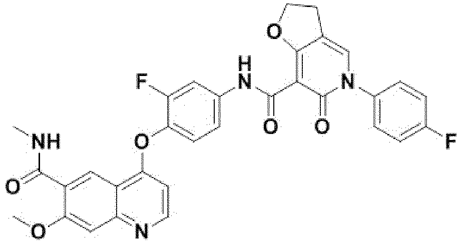

¹H NMR (500 MHz, DMSO-d₆) δ 8.66 (d, J = 5.0 Hz, 1H), 8.60 (s, 1H), 8.40-8.38 (m, 1H), 8.02-7.99 (m, 1H), 7.88 (s, 1H), 7.53-7.50 (m, 3H), 7.44-7.43 (m, 1H), 7.40-7.37 (m, 2H), 6.52 (d, J = 5.0 Hz, 1H), 4.85 (t, J = 8.5 Hz, 2H), 4.02 (s, 3H), 3.11 (t, J = 8.5 Hz, 2H), 2.84 (d, J = 4.5 Hz, 3H)

### Example 49: N-(4-((6-(Dimethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (orange solid) was obtained using the same synthetic route as in Example 40, except that the 4-(4-amino-2-fluorophenoxy)-7-methoxy-N,N-dimethylquinoline-6-carboxamide compound of Preparation Example 8 was used as a starting material in step 3.

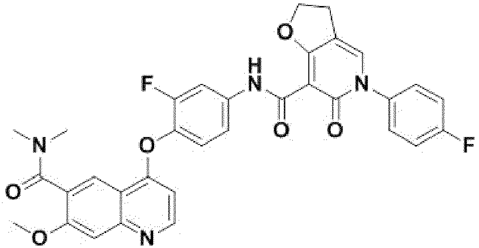

¹H NMR (500 MHz, DMSO-d₆) δ 8.65 (d, J = 5.0 Hz, 1H), 8.07 (s, 1H), 8.02-7.99 (m, 1H), 7.88 (s, 1H), 7.53-7.50 (m, 3H), 7.45-7.42 (m, 2H), 7.40-7.37 (m, 2H), 6.52 (d, J = 5.0 Hz, 1H), 4.85 (t, J = 8.5 Hz, 2H), 3.98 (s, 3H), 3.11 (t, J = 7.5 Hz, 2H), 3.02 (s, 3H), 2.80 (s, 3H)

### Example 50: N-(3-Fluoro-4-((7-methoxy-6-((2-morpholinoethyl)carbamoyl)quinolin-4-yl)oxy)phenyl)-5-(4-fluorophenyl)-6-oxo-2,3,5,6-tetrahydrofuro[3,2-c]pyridine-7-carboxamide

The title compound (ivory solid) was obtained using the same synthetic route as in Example 40, except that the 4-chloro-7-methoxy-N-(2-morpholinoethyl)quinoline-6-carboxamide compound of Preparation Example 10 was used as a starting material in step 1.

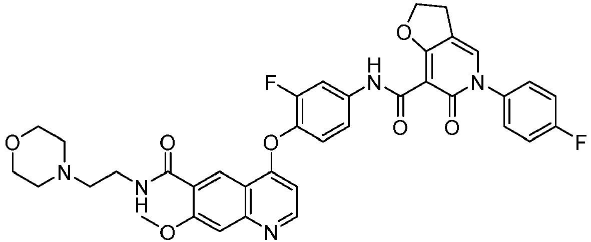

¹H NMR (500 MHz, DMSO-d₆) δ 11.93 (s, 1H), 8.70 (s, 1H), 8.68 (d, J = 5.0 Hz, 1H), 8.52 (t, J = 5.5 Hz, 1H), 8.02-8.00 (m, 1H), 7.88 (s, 1H), 7.56 (s, 1H), 7.53-7.50 (m, 2H), 7.44 (d, J = 5.5 Hz, 2H), 7.40-7.37 (m, 2H), 6.53 (d, J = 5.0 Hz, 1H), 4.85 (t, J = 8.5 Hz, 2H), 4.06 (s, 3H), 3.61 (t, J = 4.5 Hz, 4H), 3.45 (dd, J = 12.0, 6.5 Hz, 2H), 3.11 (t, J = 7.5 Hz, 2H), 2.50 (m, 2H, overlapped with DMSO), 2.45 (s, 4H)

### Example 51: N-(4-((6-(Ethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (ivory solid) was obtained using the same synthetic route as in Example 40, except that the 4-chloro-N-ethyl-7-methoxyquinoline-6-carboxamide compound of Preparation Example 11 was used as a starting material in step 1.

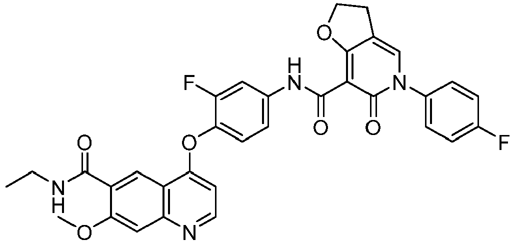

¹H NMR (400 MHz, DMSO-d₆) δ 11.92 (s, 1H), 8.66 (d, J = 5.6 Hz, 1H), 8.54 (s, 1H), 8.43 (t, J = 5.2 Hz, 1H), 8.03-7.99 (m, 1H), 7.88 (s, 1H), 7.54-7.50 (m, 3H), 7.44-7.36 (m, 4H), 6.51 (d, J = 4.4 Hz, 1H), 4.85 (t, J = 8.4 Hz, 2H), 4.02 (s, 3H), 3.31 (m, 2H, overlapped with H₂O), 3.11 (t, J = 8.0 Hz, 2H), 1.145 (t, J = 7.2 Hz, 3H)

### Example 52: N-(4-((6-Acetamido-7-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (ivory solid) was obtained using the same synthetic route as in Example 40, except that the N-(4-chloro-7-methoxyquinolin-6-yl)acetamide compound of Preparation Example 12 was used as a starting material in step 1.

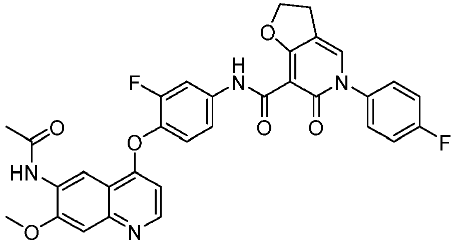

¹H NMR (500 MHz, DMSO-d₆) δ 11.90 (s, 1H), 9.49 (s, 1H), 9.01 (s, 1H), 8.52 (d, J = 5.0 Hz, 1H), 8.00-7.97 (dd, J = 2.0 Hz, J = 15.5 Hz, 1H), 7.87 (d, J = 1.5 Hz, 1H), 7.52-7.49 (m, 2H), 7.46 (s, 1H), 7.44-7.36 (m, 4H), 6.46 (d, J = 5.5 Hz, 1H), 4.85 (t, J = 8.5 Hz, 2H), 4.03 (s, 3H), 3.10 (t, J = 7.5 Hz, 2H), 2.18 (s, 3H)

### Example 53: N-(3-Fluoro-4-((7-methoxy-6-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 40, except that the N-(4-chloro-7-methoxyquinolin-6-yl)-2-morpholinoacetamide compound of Preparation Example 13 was used as a starting material in step 1.

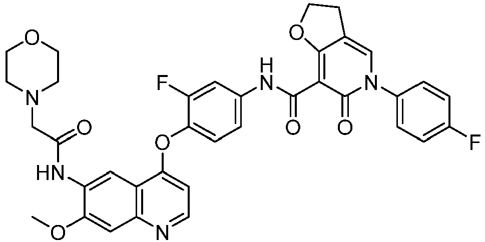

¹H NMR (400 MHz, DMSO-d₆) δ 11.91 (s, 1H), 10.13 (s, 1H), 9.12 (s, 1H), 8.53 (d, J = 5.2 Hz, 1H), 8.01-7.97 (dd, J = 2.0 Hz, J = 13.2 Hz, 1H), 7.87 (s, 1H), 7.53-7.49 (m, 3H), 7.43-7.36 (m,4H), 6.49 (d, J = 5.2 Hz, 1H), 4.85 (t, J = 8.4 Hz, 2H), 4.10 (s, 3H), 3.69 (t, J = 4.4 Hz, 4H), 3.22 (s, 2H), 3.10 (t, J = 7.6 Hz, 2H), 2.59 (bs, 4H)

### Example 54: N-(4-((2-Carbamoyl-3-chloropyridin-4-yl)oxy)-3-fluorophenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 40, except that 3,4-dichloropicolinamide was used as a starting material in step 1.

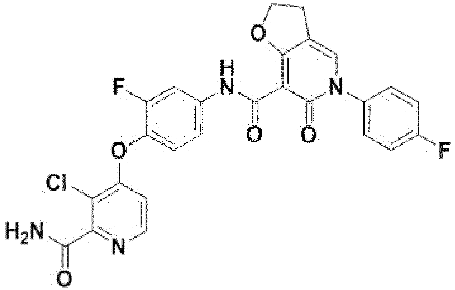

¹H NMR (500 MHz, DMSO-d₆) δ 11.98 (br s, 1H), 8.33-8.32 (m, 1H), 8.08 (m, 1H), 7.96-7.93 (m, 1H), 7.88-7.87 (m, 1H), 7.78 (m, 1H), 7.48-7.38 (m, 5H), 6.87-6.86 (m, 1H), 6.53-6.52 (m, 1H), 4.87-4.85 (m, 2H), 3.14-3.11 (m, 2H)

### Example 55: N-(4-((2-Amino-3-chloropyridin-4-yl)oxy)-3-fluorophenyl)-5-(4-fluorophenyl)-6-oxo-2,3,5,6-tetrahydrofuro[3,2-c]pyridine-7-carboxamide

The N-(4-((2-carbamoyl-3-chloropyridin-4-yl)oxy)-3-fluorophenyl)-5-(4-fluorophenyl)-6-oxo-2,3,5,6-tetrahydrofuro[3,2-c]pyridine-7-carboxamide compound (42mg, 0.08 mmol) of Example 54 and iodosobenzene diacetate (30mg, 0.09 mmol) were dissolved in a mixed solution of ethyl acetate, acetonitrile, and water, and then stirred at room temperature for 3 hours or longer. Thereafter, dichloromethane and water were added thereto for exraction. The separated organic layer was dried over anhydrous magnesium sulfate, and concentrated under reduced pressure to obtain a residue. The residue was stirred in acetone diethyl ether, and then filtered to obtain the title compound (42 mg, yield: 78%, yellow solid).

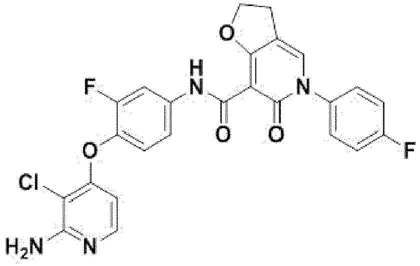

¹H NMR (500 MHz, DMSO-d₆) δ 11.93 (br s, 1H), 8.32 (d, J = 5.0 Hz, 1H), 7.88 (d, J = 5.0 Hz, 1H), 7.74 (d, J = 5. 0Hz, 1H), 7.51 (d, J = 5.0 Hz, 1H), 7.45-7.39 (m, 4H), 6.81 (d, J = 5.0 Hz, 1H), 6.42 (br s, 2H), 5.71 (d, J = 5.0 Hz, 1H), 4.87-4.83 (m, 2H), 3.12-3.09 (m, 2H)

### Example 56: N-(4-((2-Aminopyridin-4-yl)oxy)-3-fluorophenyl)-5-(4-fluorophenyl)-6-oxo-2,3,5,6-tetrahydrofuro[3,2-c]pyridine-7-carboxamide

The title compound (brown solid) was obtained using the same synthetic route as in Example 40, except that 2-amino-4-chloropyridine was used as a starting material in step 1.

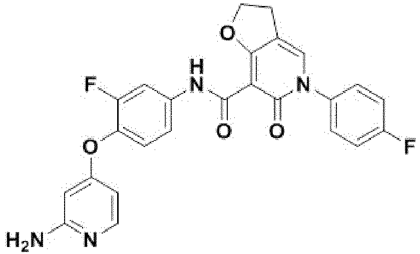

¹H NMR (500 MHz, DMSO-d₆) δ 11.91 (s, 1H), 8.02 (d, J = 15.0 Hz, 1H), 7.95 (d, J = 10.0 Hz, 1H), 7.89 (s, 1H), 7.51-7.49 (m, 2H), 7.45 (d, J = 10.0 Hz, 1H), 7.41-7.37 (m, 2H), 6.68 (d, J = 10.0 Hz, 2H), 6.11 (d, J = 5.0 Hz, 1H), 4.84 (t, J = 7.5 Hz, 2H), 3.10 (t, J = 10.0 Hz, 2H)

### Example 57: N-(4-((2-Aminopyrimidin-4-yl)oxy)-3-fluorophenyl)-5-(4-fluorophenyl)-6-oxo-2,3,5,6-tetrahydrofuro[3,2-c]pyridine-7-carboxamide

The title compound (brown solid) was obtained using the same synthetic route as in Example 40, except that 2-amino-4-chloropyrimidine was used as a starting material in step 1.

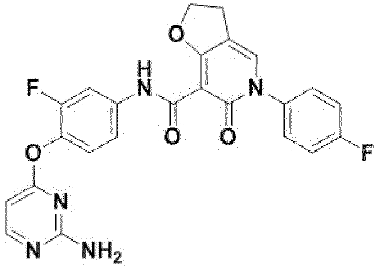

¹H NMR (500 MHz, DMSO-d₆) δ 11.90 (s, 1H), 8.71 (d, J = 5.0 Hz, 1H), 8.68 (d, J = 5.0 Hz, 1H), 7.90 (s, 1H), 7.51-7.48 (m, 3H), 7.45-7.42 (m, 2H) 7.37 (d, J = 10.0 Hz, 1H), 6.11 (d, J = 5.0 Hz, 1H), 4.84 (t, J = 7.5 Hz, 2H), 3.10 (t, J = 10.0 Hz, 2H)

### Example 58: N-(4-((6-Aminopyrimidin-4-yl)oxy)-3-fluorophenyl)-5-(4-fluorophenyl)-6-oxo-2,3,5,6-tetrahydrofuro[3,2-c]pyridine-7-carboxamide

The title compound (off-white solid) was obtained using the same synthetic route as in Example 40, except that 4-amino-6-chloropyrimidine was used as a starting material in step 1.

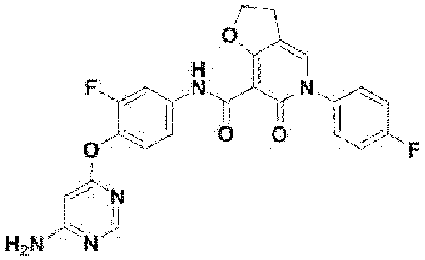

¹H NMR (500 MHz, DMSO-d₆) δ 11.93 (s, 1H), 8.32 (d, J = 5.0 Hz, 1H), 7.74 (d, J = 5.0 Hz, 1H), 7.51-7.43 (m, 4H), 7.40 (d, J = 5.0 Hz, 2H), 6.81 (d, J = 5.0 Hz, 1 H), 6.42 (brs, 2H), 4.85 (t, J = 10.0 Hz, 2H), 3.11 (t, J = 7.5 Hz, 2H)

### Example 59: N-(3-Fluoro-4-((4-((tetrahydro-2H-pyran-4-yl)amino)pyridin-2-yl)oxy)phenyl)-5-(4-fluorophenyl)-6-oxo-2,3,2,6-tetrahydrofuro[3,2-c]pyridine-7-carboxamide

The title compound (yellow solid) was obtained using the same synthetic route as in Example 40, except that 6-chloro-N-(tetrahydro-2H-pyran-4-yl)pyrimidine-4-amine, which can be easily prepared using the method in WO2013/130976, was used as a starting material in step 1.

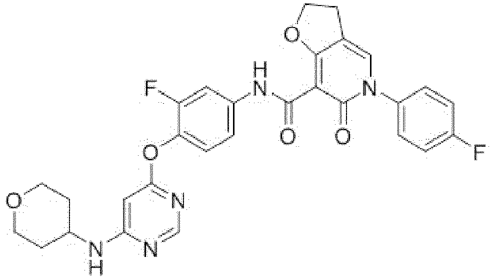

¹H NMR (500 MHz, DMSO-d₆) δ 11.83 (s, 1H), 8.10 (bs, 1H), 7.86 (d, J = 1.5 Hz, 1H), 7.84 (d, J = 2.0 Hz, 1H), 7.51-7.48 (m, 2H), 7.42-7.36 (m, 3H), 7.31 (d, J = 10.0 Hz, 1H), 7.23 (t, J = 9.0 Hz, 1H), 5.83 (bs, 1H), 4.85 (t, J = 8.0 Hz, 2H), 3.84 (dd, J = 3.0 Hz, J = 8.5 Hz, 2H), 3.40-3.38 (m, 2H), 3.11 (t, J = 7.5 Hz, 2H), 1.82 (d, J = 12.5 Hz, 2H), 1.41 (d, J = 19.0 Hz, 2H)

### Example 60: N-(3-Fluoro-4-((2-((tetrahydro-2H-pyran-4-yl)amino)pyridin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 40, except that the 4-bromo-N-(tetrahydro-2H-pyran-4-yl)pyridine-2-amine compound of Preparation Example 14 was used as a starting material in step 1.

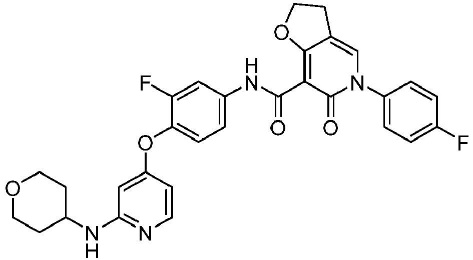

¹H NMR (500 MHz, DMSO-d₆) δ 11.82 (s, 1H), 7.93-7.90 (dd, J = 2.5 Hz, J = 13.0 Hz, 1H), 7.87 (t, J = 1.5 Hz, 1H), 7.85 (d, J = 6.0 Hz, 1H), 7.51-7.48 (m, 2H), 7.39-7.35 (m, 3H), 7.26 (t, J = 9.0 Hz, 1H), 6.53 (d, J = 5.5 Hz, 1H), 6.17-6.16 (dd, J = 1.5 Hz, J = 5.5 Hz, 1H), 5.78 (d, J = 1.5 Hz, 1H), 4.85 (t, J = 8.5 Hz, 2H), 3.82-3.78 (m, 2H), 3.38-3.35 (m, 2H, overlapped with DMSO), 3.11-3.08 (m, 2H), 1.82-1.79 (m, 2H), 1.36-1.28 (m, 2H)

### Example 61: N-(3-Fluoro-4-((6-((1-methylpiperidin-4-yl)amino)pyrimidin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 40, except that the 6-(2-fluoro-4-nitrophenoxy)-N-(1-methylpiperidin-4-yl)pyrimidine-4-amine compound of Preparation Example 15 was used as a starting material in step 2.

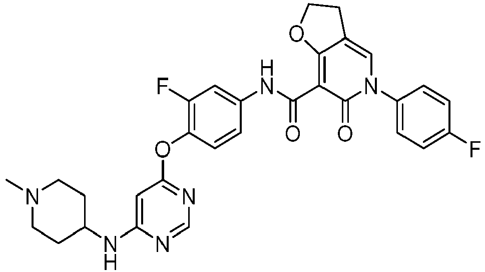

¹H NMR (500 MHz, DMSO-d₆) δ 11.83 (s, 1H), 8.09 (s, 1H), 7.86 (d, J = 1.5 Hz, 1H), 7.84 (d, J = 2.0 Hz, 1H), 7.52-7.49 (m, 2H), 7.38 (t, J = 9.0 Hz, 2H), 7.33 (d, J = 8.0 Hz, 1H), 7.30 (d, J = 8.5 Hz, 1H), 7.21 (t, J = 8.5 Hz, 1H), 5.82 (s, 1H), 4.84 (t, J = 8.0 Hz, 2H), 3.10 (t, J = 8.0 Hz, 2H), 2.70 (d, J = 11.0 Hz, 2H), 2.15 (s, 3H), 2.02-1.94 (m, 4H), 1.81 (d, J = 11.0 Hz, 2H)

### Example 62: N-(4-((3-Chloro-2-((3-morpholinopropyl)amino)pyridin-4-yl)oxy)-3-fluorophenyl)-5-(4-fluorophenyl)-6-oxo-2,3,5,6-tetrahydrofuro[3,2-c]pyridine-7-carboxamide

The title compound (ivory solid) was obtained using the same synthetic route as in Example 40, except that the 3-chloro-4-(2-fluoro-4-nitrophenoxy)-N-(3-morpholinopropyl)pyridine-2-amine compound of Preparation Example 16 was used as a starting material in step 2.

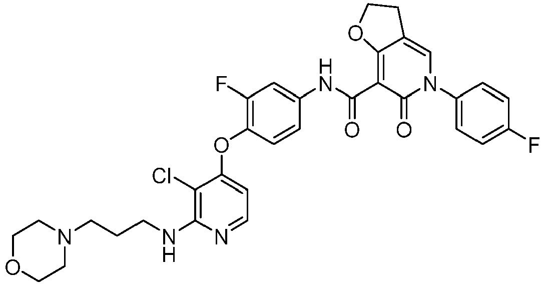

¹H NMR (400 MHz, DMSO-d₆) δ 11.87 (s, 1H), 7.93 (dd, J = 13.6, 2.4 Hz, 1H), 7.87 (s, 1H), 7.83 (d, J = 5.6 Hz, 1H), 7.52-7.48 (m, 2H), 7.40-7.35 (m, 3H), 7.26 (t, J = 8.8 Hz, 1H), 6.84 (s, 1H), 5.94 (d, J = 5.6 Hz, 1H), 4.84 (t, J = 8.0 Hz, 2H), 3.61 (s, 4H), 3.44-3.39 (m, 2H), 3.10 (t, J = 8.4 Hz, 2H), 2.5 (m, 2H, overlapped with DMSO), 2.45 (s, 4H), 1.75 (t, J = 6.8 Hz, 2H)

### Example 63: N-(3-Fluoro-4-((2-((1-methylpiperidin-4-yl)amino)pyridin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 40, except that the 4-(4-amino-2-fluorophenoxy)-N-(1-methylpiperidin-4-yl)pyridine-2-amine compound of Preparation Example 17 was used as a starting material in step 3.

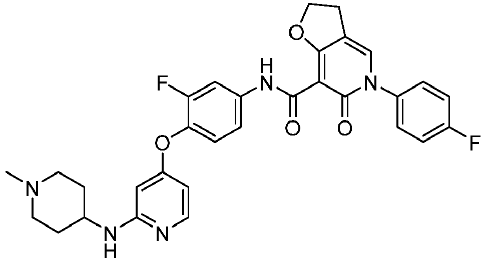

¹H NMR (500 MHz, DMSO-d₆) δ 11.81 (s, 1H), 7.93-7.90 (dd, J = 2.5 Hz, J = 13.0 Hz, 1H), 7.86 (s, 1H), 7.83 (d, J = 5.5 Hz, 1H), 7.51-7.48 (m, 2H), 7.39-7.36 (m, 3H), 7.25 (t, J = 9.0 Hz, 1H), 6.42 (d, J = 7.5 Hz, 1H), 6.14-6.13 (dd, J = 2.5 Hz, J = 6.0 Hz, 1H), 5.77 (d, J = 2.0 Hz, 1H), 4.85 (t, J = 8.0 Hz, 2H), 3.11 (t, J = 8.5 Hz, 2H), 2.66 (d, J = 11.0 Hz, 2H), 2.12 (s, 3H), 1.95 (t, J = 10.5 Hz, 2H), 1.80 (d, J = 10.0 Hz, 2H), 1.36-1.29 (m, 2H)

### Example 64: N-(3-Fluoro-4-((1-methyl-6-(1H-pyrazol-4-yl)-1H-indazol-5-yl)oxy)phenyl)-5-(4-fluorophenyl)-2-oxo-2,3,5,6-tetrahydrofuro[3,2-c]pyridine-7-carboxamide

The title compound (off-white solid) was obtained using the same synthetic route as in Example 40, except that the t-butyl 4-(5-(4-amino-2-fluorophenoxy)-1-methyl-1H-indazol-6-yl)-1H-pyrazole-1-carboxylate compound of Preparation Example 18 was used as a starting material in step 3.

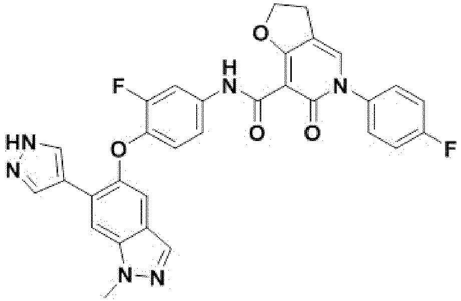

¹H NMR (500 MHz, CDCl₃) δ 12.99 (s, 1H), 11.77 (s, 1H), 8.16 (s, 1H), 8.03 (s, 1H), 8.00 (s, 1H), 7.93 (d, J = 5.0 Hz, 1H), 7.85 (d, J = 5.0 Hz, 1H), 7.50-7.48 (m, 2H), 7.37 (t, J = 7.5 Hz, 2H), 7.24 (s, 1H), 7.18 (dd, J = 5.0 and 5.0 Hz, 1H), 7.12-7.07 (qt, J = 10.0 Hz, 1H), 6.86 (t, J = 10.0 Hz, 1H), 4.83 (t, J = 10.0 Hz, 2H), 4.07 (s, 3H), 3.09 (t, J = 7.5 Hz, 2H)

### Example 65: N-(4-((1H-pyrrolo[2,3-b]pyridin-4-yl)oxy)-3-fluorophenyl)-5-(4-fluorophenyl)-6-oxo-2,3,5,6-tetrahydrofuro[3,2-c]pyridine-7-carboxamide

The title compound (brown solid) was obtained using the same synthetic route as in Example 40, except that 4-chloro-7-azaindole was used as a starting material in step 1.

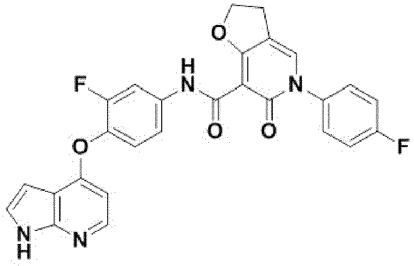

¹H NMR (500 MHz, DMSO-d₆) δ 11.86 (s, 1H), 11.77 (brs, 1H), 8.07 (d, J = 5.0 Hz, 1H), 7.95 (d, J = 5.0 Hz, 1H), 7.87 (s, 1H), 7.52-7.49 (m, 2H), 7.40-7.36 (m, 4H), 7.32 (t, J = 10.0 Hz, 2H), 6.37 (d, J = 5.0 Hz, 1H), 6.24-6.23 (m, 1H), 4.85 (t, J = 7.5 Hz, 2H), 3.10 (t, J = 7.5 Hz, 2H)

### Example 66: N-(4-((3H-imidazo[4,5-b]pyridin-7-yl)oxy)-3-fluorophenyl)-5-(4-fluorophenyl)-6-oxo-2,3,5,6-tetrahydrofuro[3,2-c]pyridine-7-carboxamide

The title compound (brown solid) was obtained using the same synthetic route as in Example 40, except that 7-chloro-3H-imidazo[4,5-b]pyridine was used as a starting material in step 1.

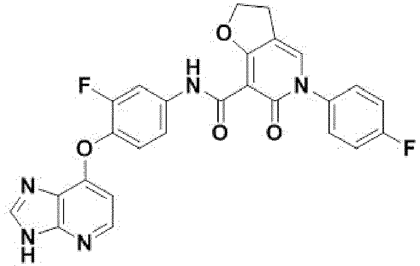

¹H NMR (500 MHz, DMSO-d₆) δ 14.21 (s, 1H), 11.91 (s, 1H), 8.51 (s, 1H), 8.33 (s, 1H), 7.95-7.92 (dd, J = 2.0, 1.5 Hz, 1H), 7.87 (s, 1H), 7.52-7.49 (m, 2H), 7.43-7.36 (m, 4H), 4.85 (t, J = 8.5 Hz, 2H), 3.10 (t, J = 8.0 Hz, 2H)

### Example 67: N-(3-Fluoro-4-((2-methyl-3H-imidazo[4,5-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 40, except that 7-chloro-2-methyl-3H-imidazo[4,5-b]pyridine of Preparation Example 19 was used as a starting material in step 1.

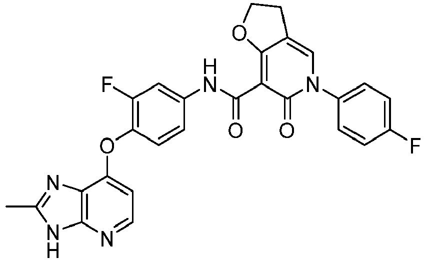

¹H NMR (500 MHz, DMSO-d₆) δ 12.87 (bs, 1H), 14.86 (s, 1H), 8.05 (d, J = 5.5 Hz, 1H), 7.95 (d, J = 13.0 Hz, 1H), 7.87 (s,1H), 7.52-7.49 (m, 2H), 7.40 (t, J = 9.0 Hz, 3H), 7.29 (t, J = 8.5 Hz, 1H), 6.44 (d, J = 5.5 Hz, 1H), 4.86 (t, J = 8.5 Hz, 2H), 3.11 (t, J = 7.5 Hz, 2H), 2.48 (s, 3H, overlapped with DMSO)

### Example 68: N-(4-((1H-pyrazolo[3,4-b]pyridin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 40, except that 4-chloro-1H-pyrazolo[3,4-d]pyridine was used as a starting material in step 1.

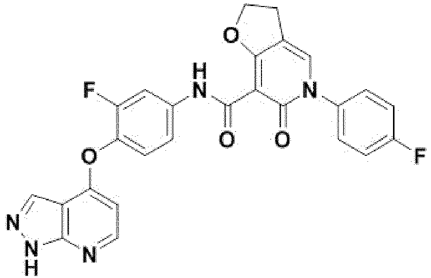

¹H NMR (500 MHz, DMSO-d₆) δ 13.77 (s, 1H), 11.92 (s, 1H), 8.35 (d, J = 5.5 Hz, 1H), 8.01-7.98 (dd, J = 1.5 Hz, J = 13.5 Hz, 1H), 7.88 (s, 1H), 7.82 (s, 1H), 7.52-7.49 (m, 2H), 7.43 (t, J = 2.5 Hz, 2H), 7.40-7.35 (m, 2H), 6.46 (d, J = 5.5 Hz, 1H), 4.85 (t, J = 8.0 Hz, 2H), 3.10 (t, J = 7.0 Hz, 2H)

### Example 69: N-(3-Fluoro-4-((furo[3,2-b]pyridin-7-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 40, except that 7-chlorofuro[3,2-b]pyridine was used as a starting material in step 1.

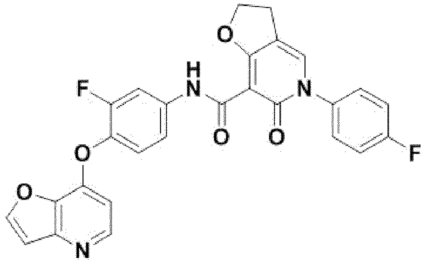

¹H NMR (500 MHz, DMSO-d₆) δ 8.36 (d, J = 5.5 Hz, 1H), 8.31 (d, J = 2.5 Hz 1H), 8.00-7.97 (m, 1H), 7.87 (s, 1H), 7.52-7.49 (m, 2H), 7.41-7.36 (m, 4H), 7.17 (d, J = 2.5 Hz, 1H), 6.70 (d, J = 5.5 Hz, 1H), 4.84 (t, J = 8.5 Hz, 2H), 3.11 (t, J = 8.0 Hz, 2H)

### Example 70: N-(3-Fluoro-4-((2-methyl-1H-pyrrolo[2,3-b]pyridin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 40, except that 4-chloro-2-methyl-1H-pyrrolo[2,3-b]pyridine was used as a starting material in step 1.

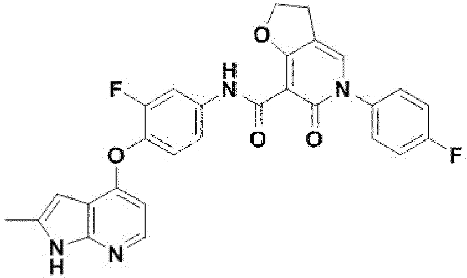

¹H NMR (500 MHz, DMSO-d₆) δ 11.85 (s, 1H), 11.59 (s, 1H), 7.96 (m, 2H), 7.87 (s, 1H), 7.52 (m, 2H), 7.40 (m, 3H), 7.28 (t, J = 9.0 Hz, 2H), 6.36 (d, J = 5.5 Hz, 1H), 4.86 (t, J = 8.0 Hz, 2H), 3.12 (t, J = 8.0 Hz, 2H), 2.36 (s, 3H)

### Example 71: N-(4-((7H-pyrrolo[2,3-d]pyrimidin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 40, except that 4-chloro-7H-pyrrolo[2,3-b]pyrimidine was used as a starting material in step 1.

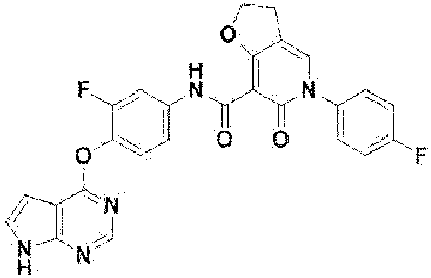

¹H NMR (500 MHz, DMSO-d₆) δ 12.28 (s, 1H), 11.87 (s, 1H), 8.29 (s, 1H), 7.91 (d, J = 12.0 Hz, 1H), 7.53-7.50 (m, 3H), 7.40-7.34 (m, 5H), 6.58 (d, J = 2.5 Hz, 1H), 4.87 (t, J = 8.5 Hz, 2H), 3.12 (t, J = 8.0 Hz, 2H)

### Example 72: N-(4-((9H-purin-6-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 40, except that 6-chloro-9H-purine was used as a starting material in step 1.

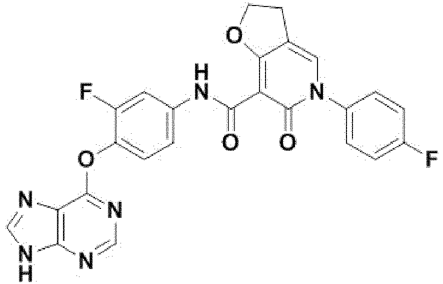

¹H NMR (500 MHz, DMSO-d₆) δ 13.67 (s, 1H), 11.90 (s, 1H), 8.51 (brs, 1H), 8.43 (s, 1H), 7.93 (d, J = 13.5 Hz, 1H), 7.88 (s, 1H), 7.53-7.50 (m, 2H), 7.40-7.37 (m, 4H), 4.87 (t, J = 8.0 Hz, 2H), 3.12 (t, J = 8.0 Hz, 2H)

### Example 73: N-(4-((1H-pyrazolo[3,4-d]pyrimidin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 40, except that 4-chloro-1H-pyrazolo[3,4-d]pyrimidine was used as a starting material in step 1.

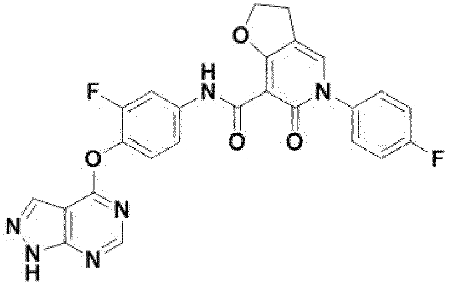

¹H NMR (500 MHz, DMSO-d₆) δ 14.21 (s, 1H), 11.91 (s, 1H), 8.51 (s, 1H), 8.33 (s, 1H), 7.95-7.92 (dd, J = 2.0 Hz, J = 13.5 Hz, 1H), 7.87 (s, 1H), 7.52-7.49 (m, 2H), 7.43-7.35 (m, 4H), 4.85 (t, J = 8.5 Hz, 2H), 3.10 (t, J = 8.0 Hz, 2H)

### Example 74: N-(4-((6,7-Dihydro-5H-pyrrolo[2,3-d]pyrimidin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 40, except that 4-chloro-6,7-dihydro-5H-pyrrolo[2,3-d]pyrimidine was used as a starting material in step 1.

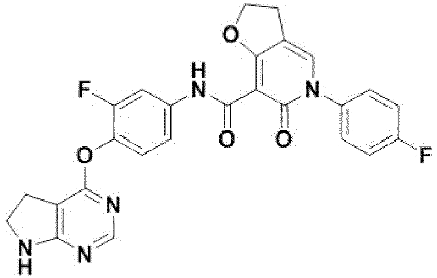

¹H NMR (500 MHz, DMSO-d₆) δ 11.82 (s, 1H), 7.89 (s, 1H), 7.86 (t, J = 1.5 Hz, 1H), 7.83 (dd, J = 13.0, 2.5 Hz 1H), 7.52-7.49 (m, 2H), 7.40-7.36 (m, 3H), 7.28 (dd, J = 9.0, 1.5 Hz 1H), 7.20 (t, J = 9 Hz, 1H), 4.84 (t, J = 8.5 Hz, 2H), 3.56 (t, J = 9.0 Hz, 2H), 3.10 (t, J = 7.5 Hz, 2H), 2.99 (t, J = 9.0 Hz, 2H)

### Example 75: N-(3-Fluoro-4-(pyrazolo[1,5-a]pyrimidin-7-yloxy)phenyl)-5-(4-fluorophenyl)-6-oxo-2,3,5,6-tetrahydro[3,2-c]pyridine-7-carboxamide

The title compound (white solid) was obtained using the same synthetic route as in Example 40, except that 7-chloro-pyrazolo[1,5-a]pyrimidine was used as a starting material in step 1.

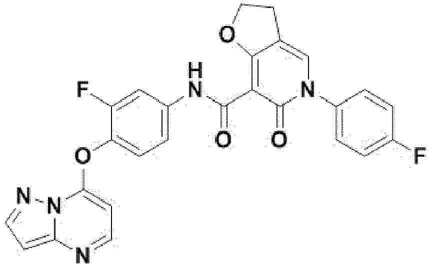

¹H NMR (500 MHz, DMSO-d₆) δ 10.16 (s, 1H), 8.23 (d, J = 5.0 Hz, 2H), 7.88 (s, 1H), 7.53 (dd, J = 11.5, 2.0 Hz 1H), 7.49-7.46 (m, 2H), 7.39-7.32 (m, 4H), 6.57 (d, J = 2.5 Hz, 1H), 6.46-6.45 (d, J = 5.5 Hz, 1H), 4.87 (t, J = 8 Hz, 2H), 3.11 (t, J = 7.5 Hz, 2H)

### Example 76: N-(3-Fluoro-4-((6-methoxy-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide

### Step 1: Synthesis of 4-chloro-6-methoxy-7-((4-methoxybenzyl)oxy)quinoline

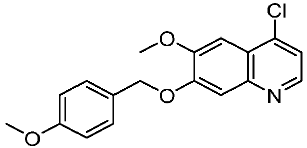

4-Chloro-6-methoxyquinolin-7-ol (5.0 g, 23.85 mmol) and potassium carbonate (9.9 g, 71.55 mmol) were dissolved in dimethylformamide. Then, 4-methoxybenzyl chloride (3.9 ml, 28.62) mmol) was added thereto, and stirring was performed at a temperature of 70°C for 5 hours. The reaction mixture was filtered, and then the obtained filtrate was extracted with ethyl acetate and water. The separated organic layer was dried over anhydrous sodium sulfate, filtered, and concentrated under reduced pressure. The resulting residue was solidified with methanol to obtain the title compound (4.9 g, yield: 62%, yellow solid).

¹H NMR (400 MHz, DMSO-d₆) δ 8.61 (d, J = 4.8 Hz, 1H), 7.56 (d, J = 4.0 Hz, 2H), 7.45 (d, J = 8.8 Hz, 2H), 7.38 (s, 1H), 6.98 (d, J = 6.8 Hz, 2H), 5.22 (s, 2H), 3.95 (s, 3H), 3.76 (s, 3H)

### Step 2: Synthesis of 4-(2-fluoro-4-nitrophenoxy)-6-methoxy-7-((4-methoxybenzyl)oxy)quinoline

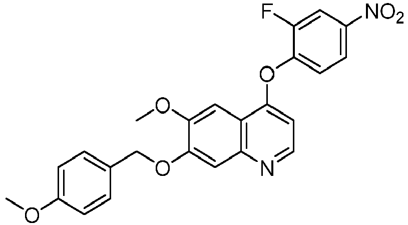

The compound (4.9 g, 14.90 mmol) prepared in step 1 and 2-fluoro-4-nitrophenol (3.5 g, 22.3 mmol) were dissolved in chlorobenzene. Then, diisopropylethylamine (5.2 ml, 29.80 mmol) was added thereto, and stirring was performed overnight at a temperature of 145°C. The reaction mixture was extracted with water and ethyl acetate. Then, the separated organic layer was dried over anhydrous sodium sulfate, filtered, and concentrated under reduced pressure. The resulting residue was solidified with acetone to obtain the title compound (5.1 g, yield: 76%, ivory solid).

¹H NMR (400 MHz, DMSO-d₆) δ 8.56 (d, J = 4.8 Hz, 1H), 8.48-8.44 (d, J = 2.4 Hz, J = 10.4 Hz, 1H), 8.21 (d, J = 8.4 Hz, 1H), 7.61 (t, J = 8.8 Hz, 1H), 7.55 (s, 1H), 7.46 (s, 2H), 7.44 (s, 1H), 6.99 (d, J = 8.4 Hz, 2H), 6.78 (d, J = 5.2 Hz, 1H), 5.23 (s, 2H), 3.91 (s, 3H), 3.77 (s, 3H)

### Step 3: Synthesis of 3-fluoro-4-((6-methoxy-7-((4-methoxybenzyl)oxy)quinolin-4-yl)oxy)aniline

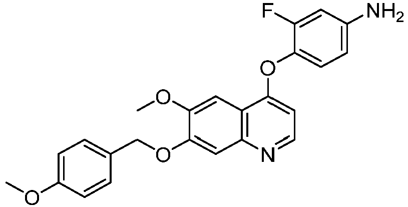

The compound (5.1 g, 11.30 mmol) prepared in step 2, iron (1.9 g, 33.90 mmol), and ammonium chloride (6.05 g, 113.10 mmol) were dissolved in ethanol and water, and then stirred overnight at a temperature of 80°C. The reaction mixture was extracted with an aqueous sodium hydrogen carbonate solution and dichloromethane. The separated organic layer was dried over anhydrous sodium sulfate, filtered, and concentrated under reduced pressure. The resulting residue was solidified with hexane to obtain the title compound (4.1 g, yield: 86%, pale yellow solid).

¹H NMR (400 MHz, DMSO-d₆) δ 8.44 (d, J = 4.8 Hz, 1H), 7.51 (d, J = 12.4 Hz, 2H), 7.45 (d, J = 8.0 Hz, 2H), 7.06 (t, J = 8.8 Hz, 1H), 6.99 (d, J = 8.8 Hz, 2H), 6.55 (d, J = 13.2 Hz, 1H), 6.47 (d, J = 6.8 Hz, 1H), 6.39 (d, J = 4.8 Hz, 1H), 5.49 (s, 2H, NH₂), 5.20 (s, 2H), 3.93 (s, 3H), 3.76 (s, 3H)

### Step 4: Synthesis of N-(3-fluoro-4-((6-methoxy-7-((4-methoxybenzyl)oxy)quinolin-4-yl)oxy)phenyl)-5-(4-fluorophenyl)-6-oxo-2,3,5,6-tetrahydrofuro[3,2-c]pyridine-7-carboxamide

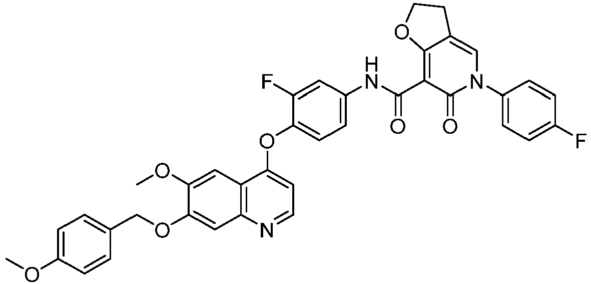

The carboxylic acid compound (3.2 g, 11.70 mmol) of Preparation Example 1 was dissolved in dichloromethane. Then, 1-[bis(dimethylamino)methylene]-1H-1,2,3-triazolo[4,5-b]pyridinium 3-oxide hexafluorophosphate (HATU, 4.5 g, 11.7 mmol), N,N-diisopropylethylamine (8.5 ml, 48.80 mmol), and the compound (4.1 g, 9.750 mmol) obtained in step 3 were added thereto, and stirring was performed at room temperature for 1 hour and 30 minutes. The reaction mixture was extracted with water and dichloromethane. The separated organic layer was dried over anhydrous sodium sulfate, filtered, and concentrated under reduced pressure. The resulting residue was solidified with methanol to obtain the title compound (6.0 g, ivory solid).

¹H NMR (400 MHz, DMSO-d₆) δ 11.88 (s, 1H, NH), 8.46 (d, J = 5.2 Hz, 1H), 8.01-7.97 (d, J = 12.8, 2.0 Hz, 1H), 7.87 (s, 1H), 7.53-7.49 (m, 4H), 7.46-7.36 (m, 6H), 6.99 (d, J = 8.8 Hz, 2H), 6.46 (d, J = 5.2 Hz, 1H), 5.21 (s, 2H), 4.84 (t, J = 8.0 Hz, 2H), 3.93 (s, 3H), 3.76 (s, 3H), 3.10 (t, J = 8.0 Hz, 2H)

### Step 5: Synthesis of N-(3-fluoro-4-((7-hydroxy-6-methoxyquinolin-4-yl)oxy)phenyl)-5-(4-fluorophenyl)-6-oxo-2,3,5,6-tetrahydrofuro[3,2-c]pyridine-7-carboxamide

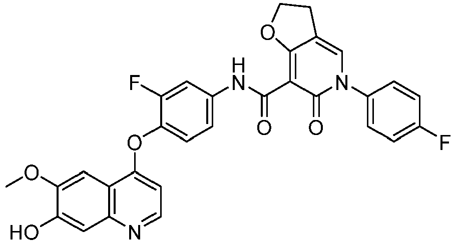

The compound (6.0 g, 8.860 mmol) prepared in step 4 was dissolved in dichloromethane. Then, trifluoroacetic acid (8.5 ml, 110.70 mmol) was added thereto, and stirring was performed at room temperature for 2 hours. The reaction mixture was concentrated under reduced pressure, and then an aqueous sodium hydroxide solution (2N) was added thereto. The resulting solid was filtered with water and acetone, and dried to obtain the title compound (4.6 g, yield: 55% (over steps 4 and 5), ivory solid).

¹H NMR (400 MHz, DMSO-d₆) δ 11.93 (s, 1H, NH), 11.13 (bs, 1H, OH), 8.62 (d, J = 6.4 Hz, 1H), 8.06 (d, J = 14.4 Hz, 1H), 7.88 (s, 1H), 7.64 (s, 1H), 7.54-7.35 (m, 8H), 4.85 (t, J = 8.8 Hz, 2H), 4.00 (s, 3H), 3.11 (t, J = 6.8 Hz, 2H)

### Step 6: Synthesis of N-(4-((7-(3-bromopropoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

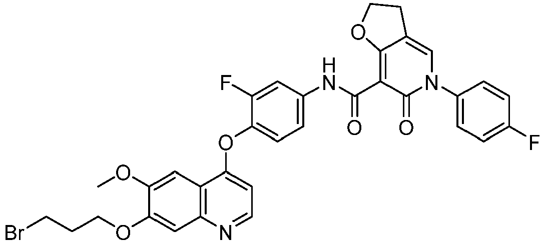

The compound (2.6g, 4.66 mmol) prepared in step 5, 1,3-dibromopropane (1.42 ml, 14.00 mmol), and potassium carbonate (1.93 g, 14.00 mmol) were dissolved in dimethylformamide, and then stirred overnight at room temperature. The reaction mixture was extracted with water and ethyl acetate. Then, the separated organic layer was dried over anhydrous sodium sulfate, filtered, and concentrated under reduced pressure. The resulting residue was purified by column chromatography to obtain the title compound (1.28 g, yield: 40%, pink solid).

¹H NMR (400 MHz, DMSO-d₆) δ 11.89 (s, 1H, NH), 8.48 (d, J = 5.6 Hz, 1H), 8.01-7.97 (d, J = 12.8, 2.0 Hz, 1H), 7.87 (s, 1H), 7.54-7.38 (m, 8H), 6.48 (d, J = 5.6 Hz, 1H), 4.84 (t, J = 8.0 Hz, 2H), 4.27 (t, J = 6.0 Hz, 2H), 3.95 (s, 3H), 3.71 (t, J = 6.4 Hz, 2H), 3.10 (t, J = 8.0 Hz, 2H), 2.35 (t, J = 6.0 Hz, 2H)

### Step 7: Synthesis of N-(3-fluoro-4-((6-methoxy-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide

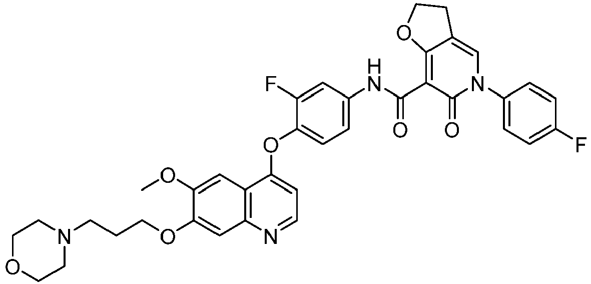

The compound (50 mg, 0.07 mmol) prepared in step 6, morpholine (19 µl, 0.22 mmol), and potassium carbonate (31 mg, 0.22 mmol) were dissolved in dimethylformamide, and then stirred at a temperature of 80°C for 3 hours. The reaction mixture was extracted with water and ethyl acetate. Then, the separated layer was dried over anhydrous sodium sulfate, filtered, and concentrated under reduced pressure. The resulting residue was purified by column chromatography to obtain the title compound (22 mg, yield: 43%, white solid).

¹H NMR (500 MHz, DMSO-d₆) δ 11.89 (s, 1H), 8.47 (d, J = 5.0 Hz, 1H), 7.99 (d, J = 10.0 Hz, 1H), 7.88 (s, 1H), 7.53-7.50 (m, 3H), 7.45-7.37 (m, 5H), 6.46 (d, J = 5.0 Hz, 1H), 4.85 (t, J = 10.0 Hz, 2H), 4.20 (t, J = 5.0 Hz, 2H), 3.95 (s, 3H), 3.61 (m, 4H), 3.11 (t, J = 10.0 Hz, 2H), 2.51 (m, 4H, partially overlapped with DMSO), 2.50 (m, 2H, overlapped with DMSO), 2.01 (m, 2H)

### Example 77: N-(3-Fluoro-4-((6-methoxy-7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide

The title compound (pale orange solid) was obtained using the same synthetic route as in Example 76, except that the carboxylic acid compound of Preparation Example 5 was used as a starting material in step 4.

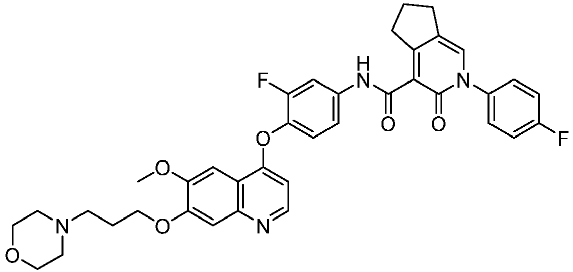

¹H NMR (500 MHz, CDCl₃) δ 12.54 (s, 1H), 8.48 (d, J = 6.0 Hz, 1H), 8.09 (s, 1H), 8.06 (dd, J = 12.0 Hz, 2.0 Hz, 1H), 7.62 (s, 1H), 7.42 (s, 1H) 7.40-7.36 (m, 3H), 7.27-7.20 (m, 3H), 6.68 (d, J = 6.0 Hz, 1H), 4.42 (t, J = 6.0 Hz, 2H), 4.11 (brs, 4H), 4.06 (s, 3H), 3.62 (t, J = 6.0, 2H), 3.26 (brs, 4H), 2.84 (t, J = 7.0, 2H), 2.59 (brs, 4H), 2.19 (q, J = 7.5 Hz, 2H)

### Example 78: N-(3-Fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6.

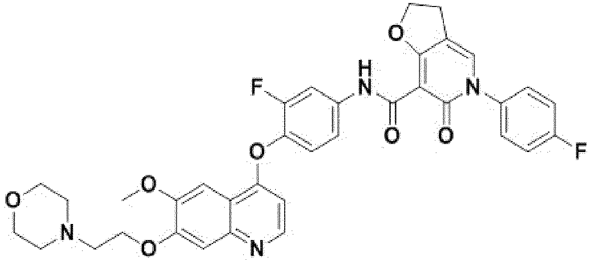

¹H NMR (500 MHz, DMSO-d₆) δ 11.89 (s, 1H), 8.48 (d, J = 5.0 Hz, 1H), 8.00 (d, J = 10.0 Hz, 1H), 7.88 (s, 1H), 7.54-7.49 (m, 3H), 7.45-7.37 (m, 5H), 6.48 (d, J = 5.0 Hz, 1H), 4.85 (t, J = 10.0 Hz, 2H), 4.32 (brs, 2H), 3.95 (s, 3H), 3.63 (brs, 4H), 3.11 (t, J = 10.0 Hz, 2H), 2.52 (m, 2H, partially overlapped with DMSO), 2.50 (m, 4H, overlapped with DMSO)

### Example 79: N-(3-Fluoro-4-((6-methoxy-7-(2-morpholinoethoxy)quinolin-4-yl)oxy)phenyl)-6-oxo-5-phenyl -2,3,5,6-tetrahydrofuro[3,2-c]pyridine-7-carboxamide

The title compound (yellow solid) was obtained using the same synthetic route as in Example 76, except that the carboxylic acid of Preparation Example 2 was used as a starting material in step 4 and 1,2-dibromoethane was used as a starting material in step 6.

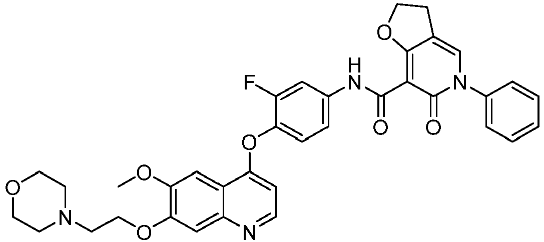

¹H NMR (400 MHz, CDCl₃) δ 11.90 (s,1H), 8.47 (d, J = 5.2 Hz, 1H), 8.00-7.97 (dd, J = 1.6 Hz, J = 11.6Hz, 1H), 7.88 (s, 1H), 7.56-7.37 (m, 9H), 6.46 (d, J = 5.2 Hz, 1H), 4.84 (t, J = 8.0 Hz, 2H), 4.27 (t, J = 5.2 Hz, 2H), 3.94 (s, 3H), 3.59 (t, J = 4.8 Hz, 4H), 3.11 (t, J = 8.0 Hz, 2H), 2.79 (t, J = 6.0 Hz, 2H), 2.52-2.50 (m, 4H, overlapped with DMSO)

### Example 80: N-(3-Fluoro-4-((6-methoxy-7-(3-(4-methylpiperazin-1-yl)propoxy)quinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 4-methylpiperazine was used as a starting material in step 7.

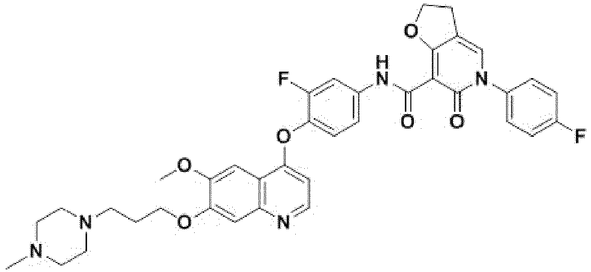

¹H NMR (500 MHz, CDCl₃) δ 12.13 (s, 1H), 8.48 (d, J = 6.0 Hz, 1H), 8.07 (d, J = 13.0 Hz, 1H), 7.81 (d, J = 7.5 Hz, 1H), 7.60 (s, 1H), 7.39-7.34 (m, 4H), 7.26 (m, 2H), 7.20 (t, J = 8.5 Hz, 1H), 6.59 (d, J = 5.0 Hz, 1H), 5.01 (t, J = 8.5 Hz, 2H), 4.40 (t, J = 5.5 Hz, 2H), 4.05 (s, 3H), 3.34 (m, 2H) 3.21 (t, J = 8.0 Hz, 2H), 2.92-2.58 (m, 11H), 2.11 (m, 2H)

### Example 81: N-(3-Fluoro-4-((7-(3-(3-hydroxyazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that azetidin-3-ol was used as a starting material in step 7.

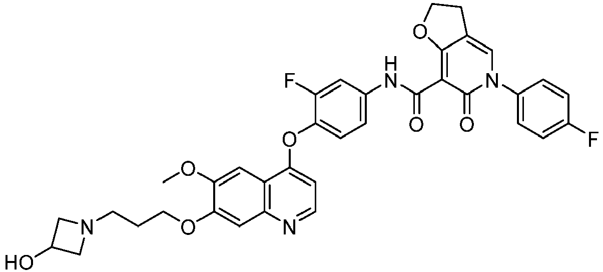

¹H NMR (400 MHz, CDCl₃) δ 12.03 (S, 1H), 8.47 (d, J = 5.2 Hz, 1H), 8.02 (dd, J = 12.8, 2.4 Hz, 1H), 7.56 (s, 1H), 7.48 (s, 1H) 7.38-7.34 (m, 4H), 7.26-7.22 (m, 2H), 7.17 (t, J = 9.2 Hz, 1H), 6.43 (d, J = 5.2 Hz, 1H), 5.01 (t, J = 8.4 Hz, 2H), 4.47 (q, J = 5.6 Hz, 1H), 4.26 (t, J = 6.8 Hz, 2H), 4.03 (s, 3H), 3.68 (t, J = 6.4 Hz, 2H), 3.20 (t, J = 7.2 Hz, 2H), 2.97 (t, J = 5.6 Hz, 2H), 2.69 (t, J = 6.8 Hz, 2H), 2.02 (q, J = 7.2 Hz, 2H)

### Example 82: N-(3-Fluoro-4-((7-(3-(3-hydroxy-3-methylazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 3-methyl-3-hydroxyazetidine hydrochloride was used as a starting material in step 7.

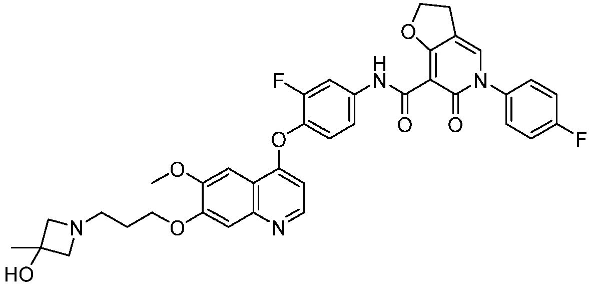

¹H NMR (400 MHz, CDCl₃) δ 12.03 (S, 1H), 8.48 (d, J = 5.6 Hz, 1H), 8.02 (dd, J = 12.8, 2.8 Hz, 1H), 7.56 (s, 1H), 7.48 (s, 1H) 7.38-7.34 (m, 4H), 7.26-7.22 (m, 2H), 7.17 (d, J = 8.8 Hz, 1H), 6.43 (d, J = 5.6 Hz, 1H), 5.01 (t, J = 8.4 Hz, 2H), 4.26 (t, J = 6.8 Hz, 2H), 4.03 (s, 3H), 3.34 (d, J = 8.0 Hz, 2H), 3.20 (t, J = 6.8 Hz, 2H), 3.09 (d, J = 7.6 Hz, 2H), 2.71 (t, J = 6.4 Hz, 2H), 2.03 (q, J = 7.2 Hz, 2H), 1.49 (s, 3H)

### Example 83: N-(3-Fluoro-4-((7-(3-(3-(hydroxymethyl)azetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that azetidin-3-ylmethanol hydrochloride was used as a starting material in step 7.

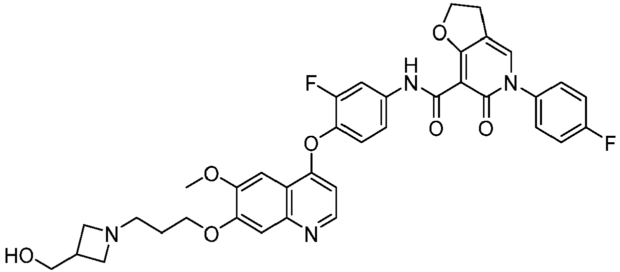

¹H NMR (400 MHz, DMSO-d₆) δ 11.87 (S, 1H), 8.49 (d, J = 5.2 Hz, 1H), 8.01 (dd, J = 13.2,2.4 Hz, 1H), 7.87 (s, 1H), 7.59 (s, 1H), 7.52-7.49 (m, 2H), 7.46-7.36 (m, 5H), 6.49 (d, J = 5.2 Hz, 1H), 5.06 (bs, 1H), 4.87 (t, J = 8.0 Hz, 2H), 4.25 (t, J = 6.0 Hz, 2H), 4.10 (m, 5H), 3.53 (bs, 2H), 3.13 (t, J = 8.4 Hz, 2H), 2.83 (bs, 1H), 2.09 (m, 5H)

### Example 84: N-(3-Fluoro-4-((6-methoxy-7-(3-(3-methoxyazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 3-methoxyazetidine hydrochloride was used as a starting material in step 7.

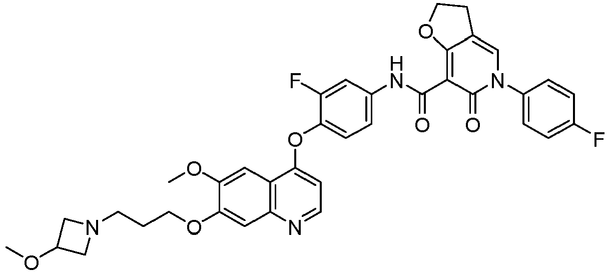

¹H NMR (400 MHz, CDCl₃) δ 12.03 (S, 1H), 8.48 (d, J = 5.6 Hz, 1H), 8.02 (dd, J = 12.8, 2.4 Hz, 1H), 7.60 (s, 1H), 7.39-7.34 (m, 5H), 7.26-7.22 (m, 2H), 7.17 (t, J = 8.8 Hz, 1H), 6.43 (d, J = 5.6 Hz, 1H), 5.01 (t, J = 8.0 Hz, 2H), 4.24 (t, J = 6.4 Hz, 2H), 4.07-4.02 (m, 4H), 3.70 (bs, 1H), 3.26 (s, 3H), 3.20 (t, J = 8.8 Hz, 2H), 2.96 (bs, 2H), 2.74 (t, J = 6.8 Hz, 2H), 2.03 (t, J = 6.8 Hz, 2H)

### Example 85: N-(3-Fluoro-4-((6-methoxy-7-(3-(3-methoxy-3-methylazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 75, except that 3-methoxy-3-methylazetidine hydrochloride was used as a starting material in step 7.

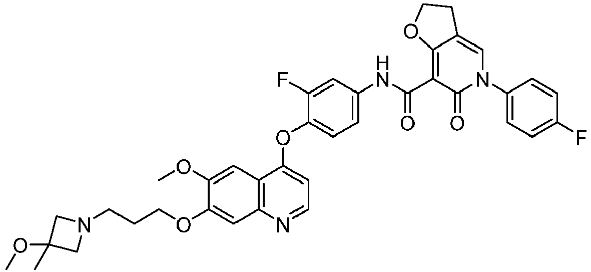

¹H NMR (400 MHz, CDCl₃) δ 12.03 (S, 1H), 8.48 (d, J = 5.2 Hz, 1H), 8.02 (dd, J = 12.4, 2.4 Hz, 1H), 7.56 (s, 1H), 7.39-7.34 (m, 5H), 7.34-7.22 (m, 2H), 7.17 (t, J = 9.2 Hz, 1H), 6.43 (d, J = 5.2 Hz, 1H), 5.00 (t, J = 8.4 Hz, 2H), 4.25 (t, J = 6.8 Hz, 2H), 4.02 (s, 3H), 3.29 (bs, 1H), 3.20-3.16 (m, 5H), 3.06 (bs, 2H), 2.73 (bs, 2H), 2.04 (t, J = 6.8 Hz, 2H)

### Example 86: N-(3-Fluoro-4-((7-(3-(3-fluoroazetidin-1-yl)propoxy)-6-methoxyquinolin-4-yl)oxy)phenyl)-5-(4-fluorophenyl)-6-oxo-2,3,5,6-tetrahydrofuro[3,2-cpyridine-7-carboxamide

The title compound (white solid) was obtained using the same synthetic route as in Example 75, except that 3-fluoroazetidine hydrochloride was used as a starting material in step 7.

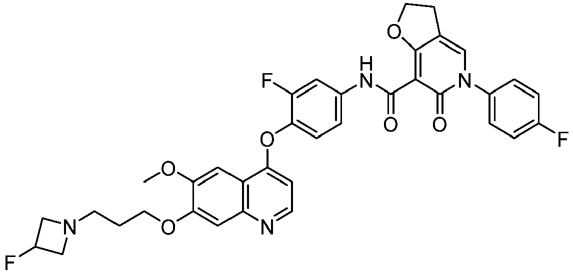

¹H NMR (400 MHz, DMSO-d₆) δ 11.89 (s, 1H), 8.47-8.46 (d, J = 5.6 Hz, 1H), 8.01-7.97 (dd, J = 13.3, 1.6 Hz, 1H), 7.88 (s, 1H), 7.53-7.49 (m, 3H), 7.45-7.33 (m, 5H), 6.46-6.45 (d, J = 5.2 Hz, 1H), 5.25-5.06 (m, 1H), 4.87-4.83 (t, J = 8.0 Hz, 2H), 4.18-4.14 (t, J = 6.4 Hz, 2H), 3.94 (s, 3H), 3.63-3.55 (m, 2H), 3.13-3.04 (m, 4H), 2.64-2.60 (t, J = 6.8 Hz, 2H), 1.86-1.80 (m, 2H)

### Example 87: N-(4-((7-(3-(3,3-Difluoroazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 3,3-difluoroazetidine hydrochloride was used as a starting material in step 7.

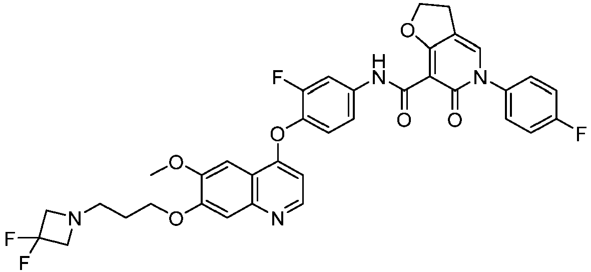

¹H NMR (400 MHz, CDCl₃) δ 12.04 (S, 1H), 8.48 (d, J = 5.6 Hz, 1H), 8.03 (dd, J = 12.4, 2.4 Hz, 1H), 7.57 (s, 1H), 7.40-7.37 (m, 5H), 7.26-7.22 (m, 2H), 7.17 (t, J = 8.8 Hz, 1H), 6.44 (d, J = 5.6 Hz, 1H), 5.01 (t, J = 8.4 Hz, 2H), 4.26 (t, J = 6.4 Hz, 2H), 4.03 (s, 3H), 3.64 (t, J = 11.6 Hz, 4H), 3.20 (t, J = 8.0 Hz, 2H), 2.81 (t, J = 6.8 Hz, 2H), 2.05 (q, J = 6.8 Hz, 3H)

### Example 88: N-(4-((7-(3-(3-Cyanoazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that azetidine-3-carbonitrile was used as a starting material in step 7.

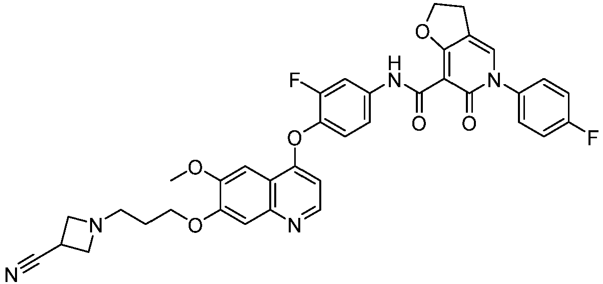

¹H NMR (400 MHz, CDCl₃) δ 12.04 (S, 1H), 8.48 (d, J = 5.2 Hz, 1H), 8.03 (dd, J = 12.4, 2.4 Hz, 1H), 7.57 (s, 1H), 7.40-7.37 (m, 5H), 7.26-7.22 (m, 2H), 7.17 (t, J = 8.8 Hz, 1H), 6.44 (d, J = 5.6 Hz, 1H), 5.01 (t, J = 8.4 Hz, 2H), 4.26 (t, J = 6.4 Hz, 2H), 4.03 (s, 3H), 3.64 (t, J = 11.6 Hz, 4H), 3.20 (t, J = 8.0 Hz, 2H), 2.81 (t, J = 6.8 Hz, 2H), 2.05 (q, J = 6.8 Hz, 3H)

### Example 89: N-(3-Fluoro-4-((6-methoxy-7-(3-(3-methylazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 3-methylazetidine hydrochloride was used as a starting material in step 7.

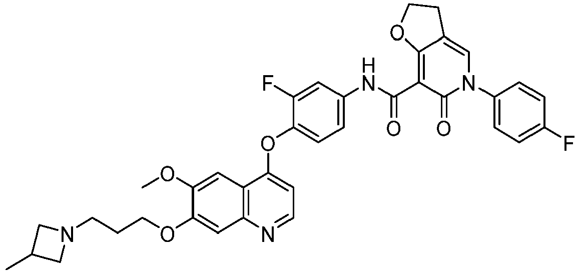

¹H NMR (400 MHz, DMSO-d₆) δ 11.87 (S, 1H), 8.49 (d, J = 5.2 Hz, 1H), 8.01 (dd, J = 12.8, 2.4 Hz, 1H), 7.87 (s, 1H), 7.56 (s, 1H), 7.52-7.49 (m, 2H), 7.46-7.36 (m, 5H), 6.49 (d, J = 5.2 Hz, 1H), 4.87 (t, J = 8.0 Hz, 2H), 4.25 (t, J = 6.0 Hz, 2H), 4.09 (q, J = 5.6 Hz, 2H), 3.97 (s, 3H), 3.18 (d, J = 5.6 Hz, 5H), 3.13 (t, J = 8.4 Hz, 2H), 2.09 (bs, 2H), 1.18 (d, J = 6.0 Hz, 3H).

### Example 90: N-(3-Fluoro-4-((7-(3-(3-hydroxypyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (brown solid) was obtained using the same synthetic route as in Example 76, except that DL-3-pyrrolidinol was used as a starting material in step 7.

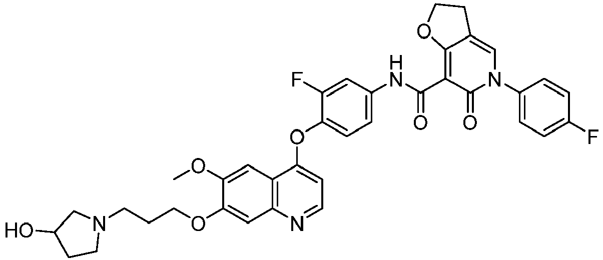

¹H NMR (400 MHz, MeOH-d₄) δ 8.41 (d, J = 5.2 Hz, 1H), 7.97 (dd, J = 12.8, 2.4 Hz, 1H), 7.71 (t, J = 1.6 Hz, 1H), 7.64 (s, 1H), 7.48-7.45 (m, 2H), 7.37-7.27 (m, 5H), 6.49 (dd, J = 5.6, 1.2 Hz, 1H), 4.93 (t, J = 8.8 Hz, 2H), 4.38-4.36 (m, 1H), 4.25 (t, J = 6.4 Hz, 2H), 4.01 (s, 3H), 3.20 (td, J = 8.8, 1.6Hz, 2H), 2.93-2.89 (m, 2H), 2.83-2.75 (m, 2H), 2.68-2.56 (m, 2H), 2.18-2.11 (m, 2H), 1.77-1.73 (m, 2H)

### Example 91: N-(3-Fluoro-4-((7-(3-(3-hydroxy-3-methylpyrrolidin-1-yl)propoxy)-6-methoxyquinoline-4-yl)oxy)phenyl)-5-(4-fluorophenyl)-6-oxo-2,3,5,6-tetrahydrofuro[3,2-c]pyridine-7-carboxamide

The title compound (pale yellow solid) was obtained using the same synthetic route as in Example 76, except that 3-methylpyrrolidin-3-ol was used as a starting material in step 7.

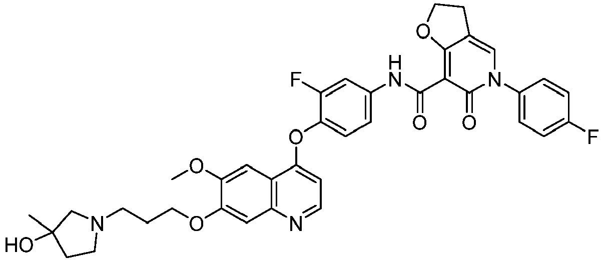

¹H NMR (400 MHz, MeOH-d₄) δ 8.38 (d, J = 5.2 Hz, 1H), 7.96 (m, 1H), 7.65-7.58 (m, 2H), 7.45-7.21 (m, 7H), 6.47 (d, J = 5.2 Hz, 1H), 4.90 (t, J = 8.8 Hz, 2H), 4.87 (2H, overlapped H₂O) 4.29 (t, J = 6.4 H, 2H), 4.00 (s, 3H), 3.22 (t, J = 6.4 Hz, 2H), 3.13-3.02 (m, 2H), 2.93-2.77 (m, 2H), 2.34-2.15 (m, 2H), 1.98 (t, J = 7.6 Hz, 2H), 1.39 (s, 3H)

### Example 92: N-(3-Fluoro-4-((6-methoxy-7-(3-(3-methoxypyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 3-methoxypyrrolidine hydrochloride was used as a starting material in step 7.

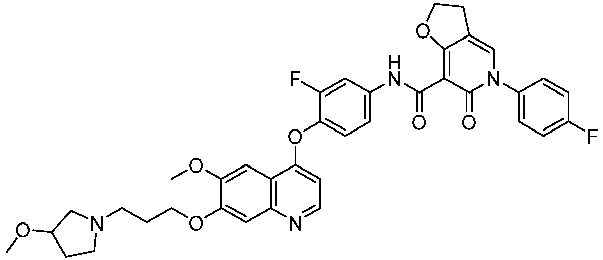

¹H NMR (400 MHz, MeOH-d₄) δ 8.41 (d, J = 5.6 Hz, 1H), 7.97 (dd, J = 12.8, 2.4 Hz, 1H), 7.71 (t, J = 1.6 Hz, 1H), 7.64 (s, 1H), 7.48-7.45 (m, 2H), 7.39-7.28 (m, 5H), 6.50 (dd, J = 5.2,0.8 Hz, 1H), 4.93 (t, J = 8.4 Hz, 2H), 4.24 (t, J = 6 Hz, 2H), 4.01 (s, 3H), 3.99-3.97 (m, 1H), 3.29 (s, 3H), 3.20 (td, J = 8.4, 1.6 Hz, 2H), 2.82-2.73 (m, 4H), 2.17-2.06 (m, 4H)

### Example 93: N-(3-Fluoro-4-((6-methoxy-7-(3-(pyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that pyrrolidine was used as a starting material in step 7.

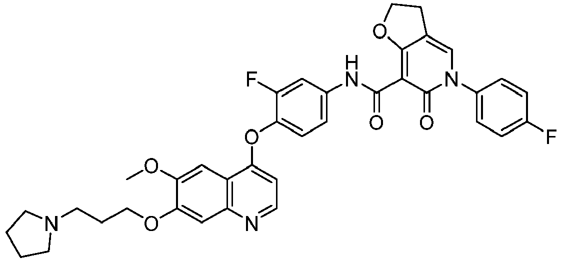

¹H NMR (400 MHz, DMSO-d₆) δ 11.86 (s, 1H), 8.46 (d, J = 5.2 Hz, 1H), 7.98 (dd, J = 12.8, 2 Hz, 1H), 7.86 (s, 1H), 7.53-7.48 (m, 3H), 7.44-7.35 (m, 5H), 6.46 (d, J = 5.2 Hz, 1H), 4.84 (t, J = 8.4 Hz, 2H), 4.20 (t, J = 6.4 Hz, 2H), 3.94 (s, 3H), 3.11 (t, J = 8.4 Hz, 2H), 2.67-2.65 (m, 2H), 2.58-2.56 (m, 2H), 2.32 (t, J = 1.6 Hz, 2H), 2.02-1.96 (m, 2H), 1.70-1.68 (m, 4H)

### Example 94: N-(3-Fluoro-4-((7-(3-(3-fluoropyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 3-fluoropyrrolidine hydrochloride was used as a starting material in step 7.

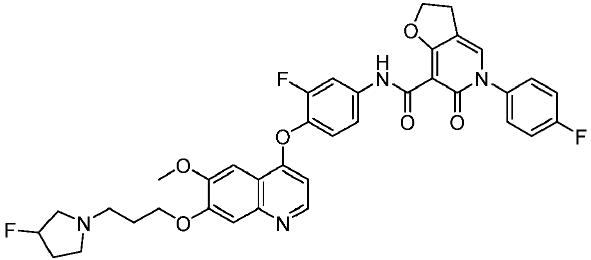

¹H NMR (400 MHz, MeOH-d₄) δ 8.4 (d, J = 5.6 Hz, 1H), 7.97 (dd, J = 12.8, 2.4 Hz, 1H), 7.71 (t, J = 1.6 Hz, 1H), 7.64 (s, 1H), 7.48-7.45 (m, 2H), 7.37-7.27 (m, 5H), 6.50 (dd, J = 5.6, 1.2 Hz), 4.93 (t, J = 8.8 Hz, 2H), 4.25 (t, J = 6 Hz, 2H), 4.01 (s, 3H), 3.20 (td, J = 8.8, 1.6 Hz, 2H), 3.10-2.97 (m, 1H), 2.84-2.73 (m, 4H), 2.59-2.53 (m, 2H), 2.29-2.01 (m, 4H)

### Example 95: N-(4-((7-(3-(3,3-Difluoropyrrolidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (pale yellow solid) was obtained using the same synthetic route as in Example 76, except that 3,3-difluoropyrrolidine hydrochloride was used as a starting material in step 7.

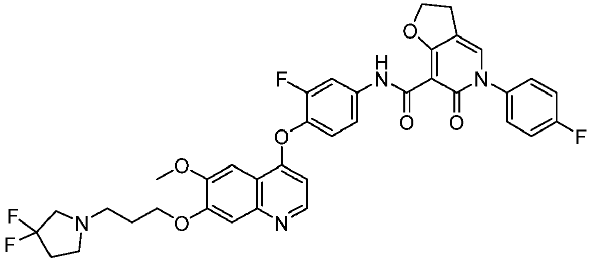

¹H NMR (400 MHz, DMSO-d₆) δ 11.86 (s, 1H), 8.46 (d, J = 5.6 Hz, 1H), 7.98 (dd, J = 12.8, 2 Hz, 1H), 7.86 (t, J = 1.6 Hz, 1H), 7.52-7.48 (m, 3H), 7.44-7.35 (m, 5H), 6.46 (d, J = 4.4 Hz, 1H), 4.84 (t, J = 8.4 Hz, 2H), 4.19 (t, J = 6.4 Hz, 2H), 3.94 (s, 3H), 3.11 (t, J = 9.2 Hz, 2H), 2.92 (t, J = 13.6 Hz, 2H), 2.72 (t, J = 6.8 Hz, 2H), 2.62 (t, J = 7.6 Hz, 2H), 2.30-2.19 (m, 2H), 2.01-1.94 (m, 2H)

### Example 96: N-(3-Fluoro-4-((7-(3-(4-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (pale yellow solid) was obtained using the same synthetic route as in Example 76, except that 4-hydroxypiperidine was used as a starting material in step 7.

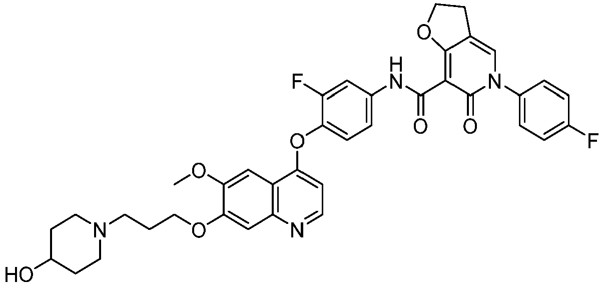

¹H NMR (400 MHz, DMSO-d₆) δ 11.87 (s, 1H), 8.47 (d, J = 5.6 Hz, 1H), 7.98 (dd, J = 12.8, 2.0 Hz, 1H), 7.86 (t, J = 1.6 Hz, 1H), 7.52-7.49 (m, 3H), 7.45-7.36 (m, 5H), 6.47 (d, J = 5.2 Hz, 1H), 4.85 (t, J = 8.4 Hz, 2H), 4.49 (d, J = 4.4 Hz, 1H), 4.18 (t, J = 6.4 Hz, 2H), 3.11 (t, J = 8.4 Hz, 2H), 2.74-2.68 (m, 2H), 2.33 (t, J = 2.8 Hz, 2H), 2.09-1.91 (m, 4H), 1.75-1.67 (m, 2H), 1.45-1.33 (m, 2H)

### Example 97: N-(3-Fluoro-4-((7-(3-(3-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 3-hydroxypiperidine was used as a starting material in step 7.

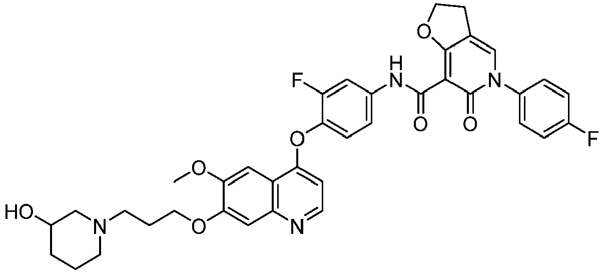

¹H NMR (400 MHz, DMSO-d₆) δ 11.87 (s, 1H), 8.46 (d, J = 5.2 Hz, 1H), 7.98 (dd, J = 12.8, 2.0 Hz, 1H), 7.86 (t, J = 1.6 Hz, 1H), 7.52-7.49 (m, 3H), 7.45-7.36 (m, 5H), 6.46 (dd, J = 5.2,0.8 Hz, 1H), 4.85 (t, J = 8.4 Hz, 2H), 4.56 (d, J = 4.8 Hz, 1H), 4.18 (t, J = 6.4 Hz, 2H), 3.95 (s, 3H), 3.50-3.46 (m, 1H), 3.11 (t, J = 8.0 Hz, 2H), 2.88-2.80 (m, 1H), 2.72-2.63 (m, 1H), 2.49-2.42 (m, 2H_ overlapped w/ DMSO), 2.00-1.91 (m, 2H), 1.90-1.82 (m, 1H), 1.82-1.71 (m, 2H), 1.68-1.56 (m, 1H), 1.48-1.35 (m, 1H), 1.14-1.13 (m, 1H)

### Example 98: N-(3-Fluoro-4-((7-(3-(4-hydroxy-4-methylpiperidin-1-yl)propoxy)-6-methoxyquinoline-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 4-methylpiperidin-4-ol was used as a starting material in step 7.

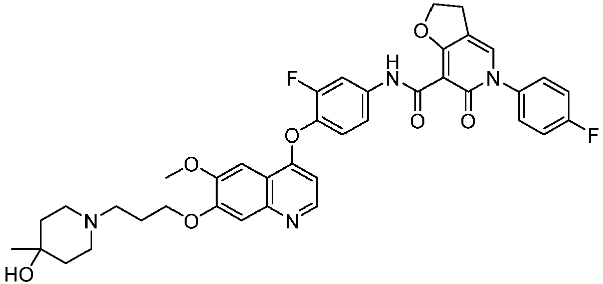

¹H NMR (400 MHz, DMSO-d₆) δ 11.87 (s, 1H), 8.46 (d, J = 5.6 Hz, 1H), 7.98 (dd, J = 12.8, 2.0 Hz, 1H), 7.86 (t, J = 1.6 Hz, 1H), 7.53-7.49 (m, 3H), 7.45-7.35 (m, 5H), 6.46 (dd, J = 5.2,1.2 Hz, 1H), 4.85 (t, J = 8.4 Hz, 2H), 4.18 (t, J = 6.4 Hz, 2H), 3.94 (s, 3H), 3.11 (t, J = 8.4 Hz, 2H), 2.46 (t, J = 7.2 Hz, 3H), 2.42-2.36 (m, 4H), 1.99-1.92 (m, 2H), 1.47 (t, J = 5.6 Hz, 4H), 1.10 (s, 3H)

### Example 99: N-(3-Fluoro-4-((6-methoxy-7-(3-(4-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 4-methoxypiperidine was used as a starting material in step 7.

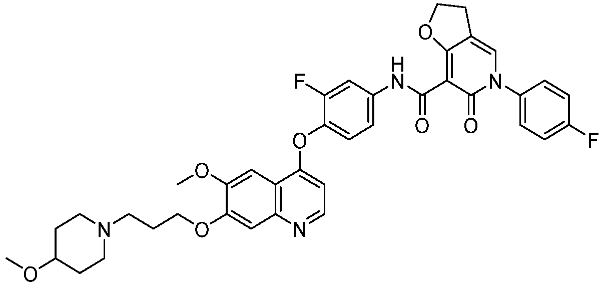

¹H NMR (400 MHz, DMSO-d₆) δ 11.87 (s, 1H), 8.46 (d, J = 5.6 Hz, 1H), 7.98 (dd, J = 13.2,2.4 Hz, 1H), 7.86 (t, J = 1.2 Hz, 1H), 7.52-7.48 (m, 3H), 7.44-7.36 (m, 5H), 6.46 (dd, J = 4.8, 0.8 Hz, 1H), 4.85 (t, J = 8.4 Hz, 2H), 4.19 (t, J = 6.4 Hz, 2H), 3.94 (s, 3H), 3.30 (1H_overlapped with H₂O), 3.22 (s, 3H), 3.11 (t, J = 8.4 Hz, 2H), 2.78-2.68 (m, 2H), 2.50-2.44 (bs, 2H_ overlapped with DMSO), 2.18 (bs, 2H), 2.04-1.19 (m, 2H), 1.88-1.78 (m, 2H), 1.52-1.36 (m, 2H)

### Example 100: N-(3-Fluoro-4-((6-methoxy-7-(3-(3-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 3-methoxypiperidine was used as a starting material in step 7.

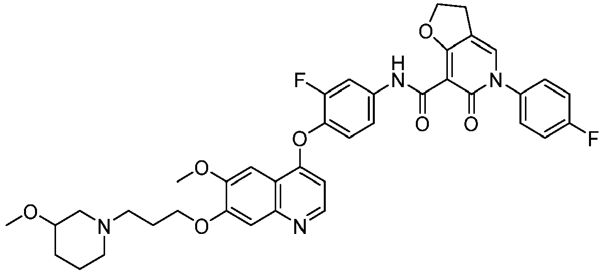

¹H NMR (400 MHz, MeOH-d₄) δ 8.41 (d, J = 5.2 Hz, 1H), 7.97 (dd, J = 12.8, 2.4 Hz, 1H), 7.71 (t, J = 1.6 Hz, 1H), 7.64 (s, 1H), 7.48-7.45 (m, 2H), 7.37-7.27 (m, 5H), 6.50 (dd, J = 5.6, 1.2 Hz, 1H), 4.93 (t, J = 8.8 Hz, 2H), 4.24 (t, J = 6 Hz, 2H), 4.01 (s, 3H), 3.38 (s, 3H), 3.20 (td, J = 8.8, 1.6 Hz, 2H), 3.00-2.97 (m, 1H), 2.78-2.68 (m, 4H), 2.30-2.25 (m, 2H), 2.19-2.12 (m, 2H), 1.96-1.79 (m, 2H), 1.62-1.52 (m, 1H)

### Example 101: N-(3-Fluoro-4-((6-methoxy-7-(3-(4-oxopiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that piperidin-4-one hydrochloride was used as a starting material in step 7.

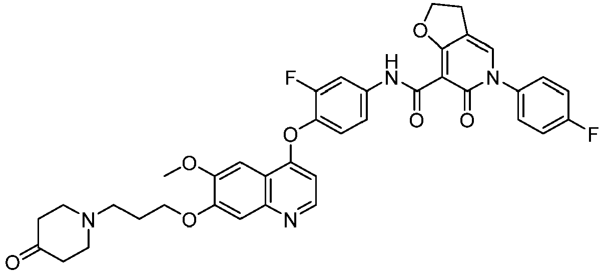

¹H NMR (400 MHz, DMSO-d₆) δ 11.87 (s, 1H), 8.47 (d, J = 5.2 Hz, 1H), 7.98 (dd, J = 12.8, 2.4 Hz, 1H), 7.86 (t, J = 1.6 Hz, 1H), 7.53-7.49 (m, 3H), 7.45-7.36 (m, 5H), 6.46 (d, J = 5.2 Hz, 1H), 4.85 (t, J = 8.0 Hz, 2H), 4.25 (t, J = 6.4 Hz, 2H), 3.95 (s, 3H), 3.11 (t, J = 8.4 Hz, 2H), 2.73 (t, J = 6.0 Hz, 2H), 2.63 (t, J = 7.2 Hz, 2H), 2.37 (t, J = 6.0 Hz, 2H), 2.06-1.99 (m, 2H)

### Example 102: N-(4-((7-(3-(1,1-Dioxidothiomorpholino)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (pale yellow solid) was obtained using the same synthetic route as in Example 76, except that thiomorpholine 1,1-dioxide was used as a starting material in step 7.

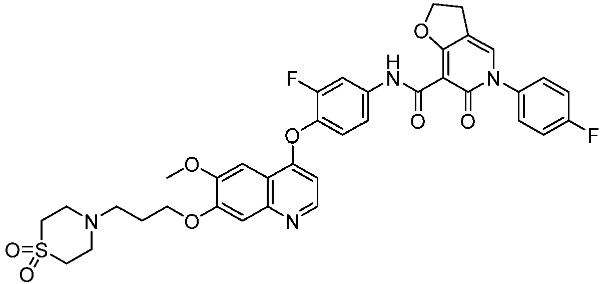

¹H NMR (400 MHz, DMSO-d₆) δ 11.87 (s, 1H), 8.47 (d, J = 4.8 Hz, 1H), 7.98 (dd, J = 13.2,2.4 Hz, 1H), 7.86 (t, J = 1.6 Hz, 1H), 7.53-7.49 (m, 3H), 7.45-7.36 (m, 5H), 6.46 (d, J = 5.2 Hz, 1H), 4.85 (t, J = 8.4 Hz, 2H), 4.22 (t, J = 6.4 Hz, 2H), 3.95 (s, 3H), 3.11-3.04 (m, 6H), 2.96-2.91 (m, 4H), 2.67 (t, J = 8.4 Hz, 2H), 2.01-1.95 (m, 2H)

### Example 103: N-(3-Fluoro-4-((6-methoxy-7-(3-(piperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that piperidine was used as a starting material in step 7.

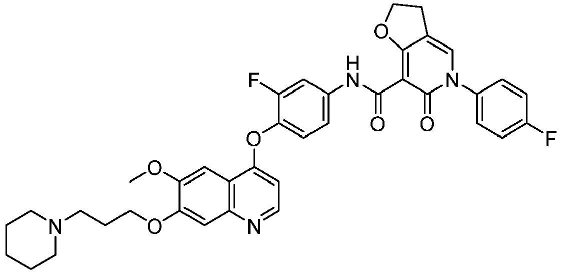

¹H NMR (400 MHz, DMSO-d₆) δ 11.87 (s, 1H), 8.47 (d, J = 4.8 Hz, 1H), 7.98 (dd, J = 12.8, 1.6 Hz, 1H), 7.86 (t, J = 1.6 Hz, 1H), 7.53-7.49 (m, 3H), 7.43-7.36 (m, 5H), 6.46 (dd, J = 4.8, 1.2 Hz, 1H), 4.85 (t, J = 8.4 Hz, 2H), 4.20 (t, J = 6.4 Hz, 2H), 3.95 (s, 3H), 3.11 (t, J = 7.2 Hz, 2H), 2.64-2.36 (m, 6H_overlapped with DMSO), 2.08-1.94 (m, 2H), 1.64-1.48 (m, 4H), 1.48-1.36 (m, 2H)

### Example 104: N-(3-Fluoro-4-((7-(3-(4-fluoropiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 4-fluoropiperidine was used as a starting material in step 7.

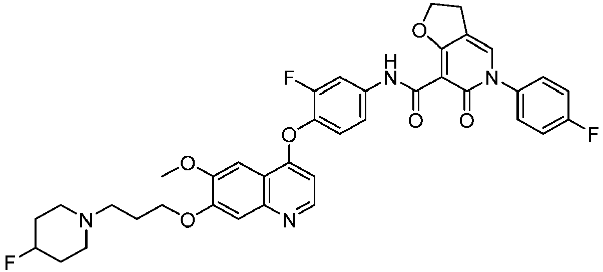

¹H NMR (400 MHz, DMSO-d₆) δ 11.83 (s, 1H), 8.43 (d, J = 4.8 Hz, 1H), 7.94 (dd, J = 12.8, 1.6 Hz, 1H), 7.82 (bs, 1H), 7.49-7.45 (m, 3H), 7.42-7.32 (m, 4H), 6.42 (dd, J = 5.6, 1.2 Hz, 1H), 4.93-4.66 (m, 3H), 4.16 (t, J = 6.8 Hz, 2H), 3.07 (t, J = 8.8 Hz, 2H), 2.65-2.50 (m, 4H), 2.44-2.32 (m, 2H), 1.98-1.80 (m, 4H), 1.80-1.64 (m, 2H)

### Example 105: N-(4-((7-(3-(4,4-Difluoropiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 4,4-difluoropiperidine hydrochloride was used as a starting material in step 7.

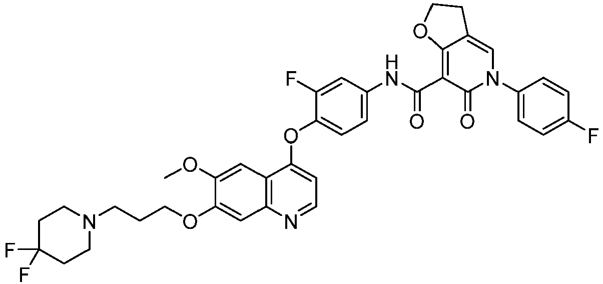

¹H NMR (400 MHz, DMSO-d₆) δ 11.87 (s, 1H), 8.47 (d, J = 4.8 Hz, 1H), 7.97 (dd, J = 13.2,2.0 Hz, 1H), 7.86 (s, 1H), 7.54-7.49 (m, 3H), 7.45-7.36 (m, 5H), 6.47 (d, J = 4.8 Hz, 1H), 4.85 (t, J = 8.4 Hz, 2H), 4.23 (bs, 2H), 3.95 (s, 3H), 3.11 (t, J = 8.4 Hz, 2H), 2.78-2.50 (m, 6H), 2.16-1.87 (m, 6H)

### Example 106: N-(3-Fluoro-4-((6-methoxy-7-(3-(4-methylpiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 4-methylpiperidine was used as a starting material in step 7.

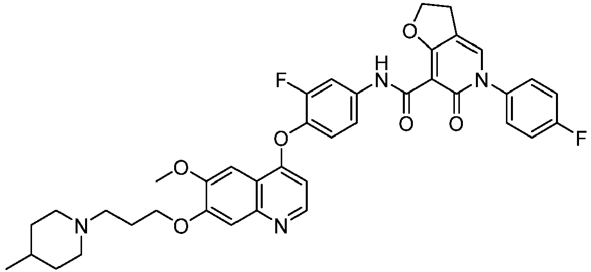

¹H NMR (400 MHz, DMSO-d₆) δ 11.83 (s, 1H), 8.43 (d, J = 4.8 Hz, 1H), 7.95 (dd, J = 13.6, 2.0 Hz, 1H), 7.83 (s, 1H), 7.49-7.45 (m, 3H), 7.44-7.32 (m, 5H), 6.42 (d, J = 4.8 Hz, 1H), 4.81 (t, J = 8.4 Hz, 2H), 4.15 (t, J = 6.4 Hz, 2H), 3.91 (s, 3H), 3.07 (t, J = 8.0 Hz, 2H), 2.92-2.78 (m, 2H), 2.56-2.40 (m, 2H_overlapped with DMSO), 2.10-1.84 (m, 4H), 1.62-1.48 (m, 2H), 1.38-1.26 (m, 1H), 1.18-1.14 (m, 2H), 0.85 (d, J = 7.2 Hz, 3H)

### Example 107: N-(4-((7-(3-(4,4-Dimethylpiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4,4-dimethylpiperidine hydrochloride was used as a starting material in step 7.

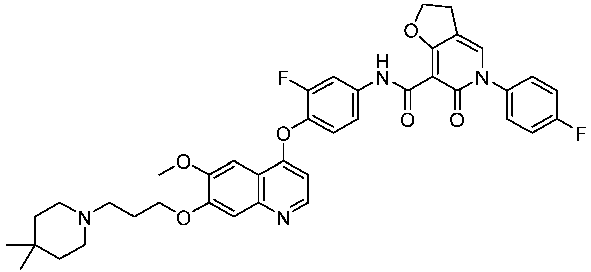

¹H NMR (400 MHz, MeOH-d₄) δ 8.43 (d, J = 5.6 Hz, 1H), 7.97 (dd, J = 12.8, 2.4 Hz, 1H), 7.71 (t, J = 1.6 Hz, 1H), 7.67 (s, 1H), 7.48-7.45 (m, 2H), 7.39-7.27 (m, 5H), 6.51 (d, J = 5.2 Hz, 1H), 4.95 (t, J = 8.4 Hz, 2H), 4.29 (t, J = 6 Hz, 2H), 4.02 (s, 3H), 3.20 (td, J = 8.4, 1.6 Hz, 2H), 3.13-2.94 (m, 4H), 2.31-2.24 (m, 2H), 2.21-2.01 (m, 2H) 1.61 (t, J = 6 Hz, 4H), 1.05 (s, 6H)

### Example 108: N-(3-Fluoro-4-((7-(3-((3-hydroxycyclobutyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 3-aminocyclobutanol hydrochloride was used as a starting material in step 7.

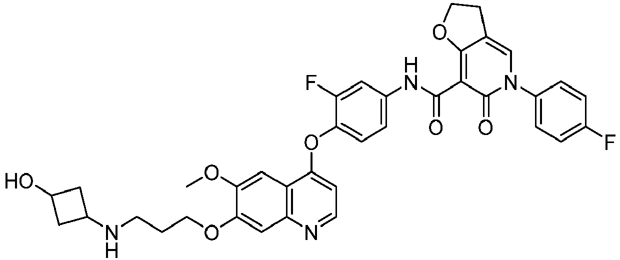

¹H NMR (400 MHz, MeOH-d₄) δ 8.43 (d, J = 5.2 Hz, 1H), 7.97 (dd, J = 12.8, 2.4 Hz, 1H), 7.71 (t, J = 1.2 Hz, 1H), 7.68 (s, 1H), 7.49-7.45 (m, 2H), 7.39-7.20 (m, 5H), 6.52 (d, J = 5.2 Hz, 1H), 4.93 (t, J = 8.4 Hz, 2H), 4.32 (t, J = 5.6 Hz, 2H), 4.04-4.01 (m, 4H), 3.20 (td, J = 8.4, 1.2 Hz, 2H), 3.04 (t, J = 6.8 Hz, 2H), 2.73-2.68 (m, 2H), 2.22-2.17 (m, 2H), 2.05-2.03 (m, 1H), 1.95-1.91 (m, 2H)

### Example 109: N-(3-Fluoro-4-((6-methoxy-7-(3-((3-methoxycyclobutyl)amino)propoxy)quinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 3-methoxycyclobutanamine hydrochloride was used as a starting material in step 7.

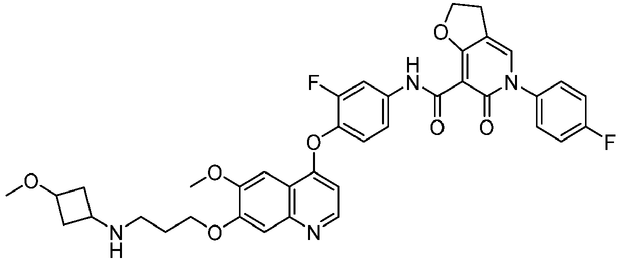

¹H NMR (400 MHz, MeOH-d₄) δ 8.38 (d, J = 5.6 Hz, 1H), 7.97 (dd, J = 12.8, 2.4 Hz, 1H), 7.71 (t, J = 1.6 Hz, 1H), 7.67 (s, 1H), 7.49-7.45 (m, 2H), 7.40-7.27 (m, 5H), 6.51 (dd, J = 5.6, 1.2 Hz, 1H), 4.93 (t, J = 8.4 Hz, 2H), 4.52 (bs, 1H), 4.30 (t, J = 5.6 Hz, 2H), 4.06-4.01 (m, 4H), 3.24 (s, 3H), 3.20 (td, J = 8.8, 1.2 Hz, 2H), 2.91 (q, J = 7.2 Hz, 2H), 2.29-2.13 (m, 4H)

### Example 110: N-(3-Fluoro-4-((6-methoxy-7-(3-(oxetan-3-ylamino)propoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 3-oxetanamine was used as a starting material in step 7.

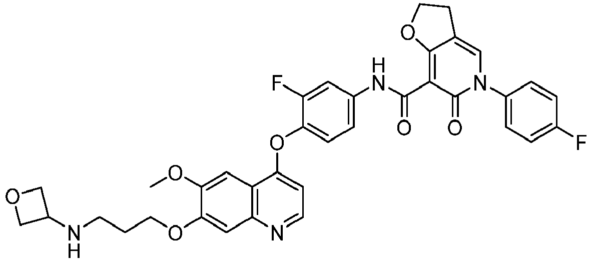

¹H NMR (400 MHz, MeOH-d₄) δ 8.42 (d, J = 5.2 Hz, 1H), 7.97 (dd, J = 12.4, 2.0 Hz, 1H), 7.71 (t, J =1.6 Hz, 1H), 7.66 (s, 1H), 7.48-7.45 (m, 2H), 7.37-7.27 (m, 5H), 6.50 (dd, J = 5.2,0.8 Hz, 1H), 4.93 (t, J = 8.4 Hz, 2H), 4.84 (t, J = 6.8 Hz, 2H), 4.54 (t, J = 8.4 Hz, 2H), 4.28 (t, J = 6 Hz, 2H), 4.04-3.97 (m, 4H), 3.20 (td, J = 8.4, 1.6 Hz, 2H), 2.80 (t, J = 6.8 Hz, 2H), 2.09 (p, J = 6.4 Hz, 2H)

### Example 111: N-(3-Fluoro-4-((6-methoxy-7-(3-((oxetan-3-ylmethyl)amino)propoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 3-oxetanemethanamine was used as a starting material in step 7.

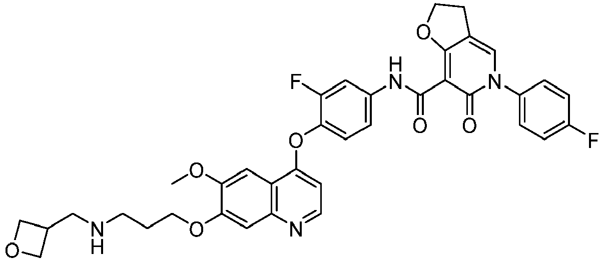

¹H NMR (400 MHz, MeOH-d₄) δ 8.43 (d, J = 5.2 Hz, 1H), 7.97 (dd, J = 12.8, 2.4 Hz, 1H), 7.71 (t, J = 1.6 Hz, 1H), 7.66 (s, 1H), 7.48-7.45 (m, 2H), 7.39-7.27 (m, 5H), 6.51 (dd, J = 5.2,1.2 Hz, 1H), 4.93 (t, J = 8.4 Hz, 2H), 4.85-4.82 (m, 2H), 4.46 (t, J = 6 Hz, 2H), 4.29 (t, J = 5.6 Hz, 2H), 4.02-3.98 (m, 4H), 3.20 (td, J = 8, 1.2 Hz, 2H), 3.09 (d, J = 7.6 Hz, 2H), 2.97 (t, J = 6.8 Hz, 2H), 2.16 (p, J = 6 Hz, 2H)

### Example 112: N-(3-Fluoro-4-((7-(3-((3-hydroxycyclopentyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 3-aminocyclopentanol hydrochloride was used as a starting material in step 7.

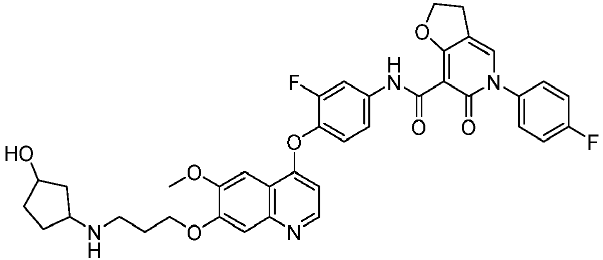

¹H NMR (400 MHz, MeOH-d₄) δ 8.43 (d, J = 5.6 Hz, 1H), 7.97 (dd, J = 12.8, 2.4 Hz, 1H), 7.71 (t, J = 1.6 Hz, 1H), 7.49-7.45 (m, 2H), 7.38-7.27 (m, 5H), 6.51 (dd, J = 5.6, 1.2 Hz, 1H), 4.93 (t, J = 8.4 Hz, 2H), 4.37-4.35 (m, 1H), 4.31 (t, J = 5.6 Hz, 2H), 4.03 (s, 3H), 3.59-3.45 (m, 2H), 3.20 (td, J = 8.4, 1.6 Hz, 2H), 3.02 (t, J = 8 Hz, 2H), 2.23-2.17 (m, 2H), 2.07-2.02 (m, 2H), 1.73-1.49 (m, 4H)

### Example 113: N-(3-Fluoro-4-((7-(3-((3-hydroxycyclohexyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4-aminocyclohexanol was used as a starting material in step 7.

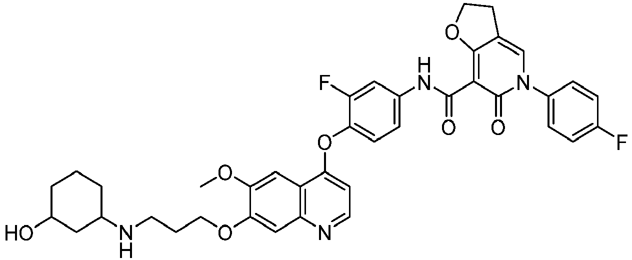

¹H NMR (400 MHz, MeOH-d₄) δ 8.43 (d, J = 1.2 Hz, 1H), 7.98 (dd, J = 12.8, 2.4 Hz, 1H), 7.71 (t, J = 1.6 Hz, 1H), 7.68 (s, 1H), 7.49-7.45 (m, 2H), 7.40-7.27 (m, 6H), 6.52 (d, J = 4.4 Hz, 1H), 4.93 (t, J = 7.2 Hz, 2H), 4.32 (t, J = 5.6 Hz, 2H), 4.03 (s, 3H), 3.63-3.59 (m, 1H), 3.49-3.45 (m, 2H), 3.20 (td, J = 8.4, 1.6 Hz, 2H), 3.14-3.12 (m, 2H), 2.22-2.19 (m, 2H), 2.15 (t, J =8 Hz, 2H), 2.03-2.02 (m, 1H), 1.62-1.52 (m, 2H)

### Example 114: N-(3-Fluoro-4-((7-(3-(((3-hydroxycyclohexyl)methyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 3-aminomethylcyclohexanol was used as a starting material in step 7.

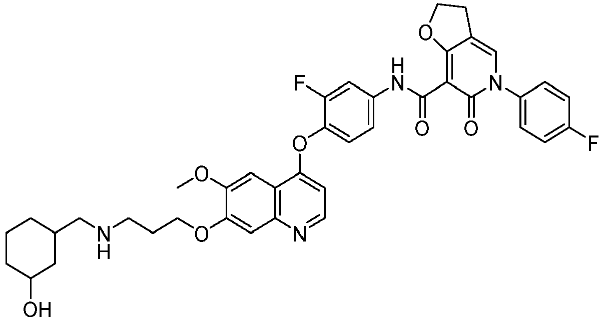

¹H NMR (400 MHz, MeOH-d₄) δ 8.42 (d, J = 5.2 Hz, 1H), 7.97 (dd, J = 12.8, 2.4 Hz, 1H), 7.71 (t, J = 1.6 Hz, 1H), 7.66 (s, 1H), 7.48-7.45 (m, 2H), 7.40-7.27 (m, 5H), 6.51 (d, J = 5.2 Hz, 1H), 4.93 (t, J = 8.8 Hz, 2H), 4.30 (t, J = 5.6 Hz, 2H), 4.03 (s, 3H), 3.57-3.46 (m, 1H), 3.20 (td, J = 8.4, 1.6 Hz, 2H), 2.95 (t, J = 6.8 Hz, 2H), 2.62 (d, J =7.2 Hz, 2H), 2.20-2.15 (m, 2H), 2.05-2.02 (m, 1H), 1.97-1.94 (m, 1H), 1.80-1.75 (m, 2H), 1.69-1.63 (m, 2H), 1.35-1.15 (m, 2H), 0.94-0.85 (m, 2H)

### Example 115: N-(3-Fluoro-4-((6-methoxy-7-(3-((tetrahydro-2H-pyran-4-yl)amino)propoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 3-aminotetrahydropyran was used as a starting material in step 7.

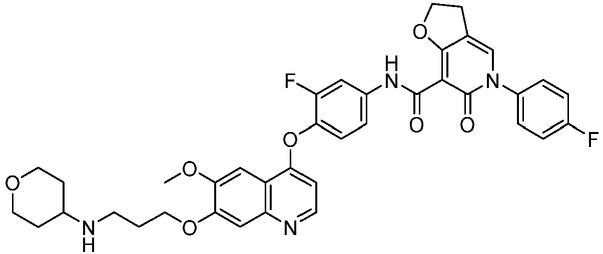

¹H NMR (400 MHz, MeOH-d₄) δ 8.42 (d, J = 5.6 Hz, 1H), 7.97 (dd, J = 12.4, 2 Hz, 1H), 7.71 (t, J = 1.6 Hz, 1H), 7.66 (s, 1H), 7.48-7.45 (m, 2H), 7.39-7.23 (m, 5H), 6.51 (dd, J = 5.6, 1.2 Hz, 1H), 4.93 (t, J = 8.4 Hz, 2H), 4.31 (t, J = 5.6 Hz, 2H), 4.02-3.93 (m, 5H), 3.44 (td, J = 12,2 Hz, 2H), 3.20 (td, J = 8.4, 1.6 Hz, 2H), 3.02 (t, J = 6.8 Hz, 2H), 2.94-2.89 (m, 1H), 2.17 (p, J = 6.4 Hz, 2H), 1.97-1.93 (m, 2H), 1.49 (qd, J = 12.4, 4.8 Hz, 2H)

### Example 116: N-(3-Fluoro-4-((6-methoxy-7-(3-(((tetrahydro-2H-pyran-4-yl)methyl)amino)propoxy)quinoline-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 3-aminomethyltetrahydropyran was used as a starting material in step 7.

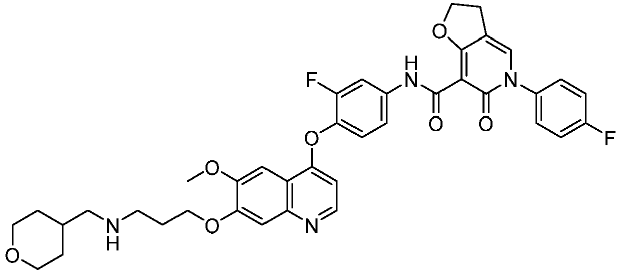

¹H NMR (400 MHz, MeOH-d₄) δ 8.42 (d, J = 5.6 Hz, 1H), 7.97 (dd, J = 12.8, 2.4 Hz, 1H), 7.71 (t, J = 1.6 Hz, 1H), 7.66 (s, 1H), 7.48-7.45 (m, 2H), 7.40-7.27 (m, 5H), 6.52 (dd, J = 5.6 Hz, 1.2 Hz, 1H), 4.93 (t, J = 8.4 Hz, 2H), 4.30 (t, J = 5.6 Hz, 2H), 4.01 (s, 3H), 3.97-3.93 (m, 2H), 3.43 (td, J = 12,1.6 Hz, 2H), 3.20 (td, J = 8.4, 1.2 Hz, 2H), 2.97 (t, J = 7.2 Hz, 2H), 2.64 (d, J = 6.8 Hz, 2H), 2.18 (p, J = 6.4 Hz, 2H), 1.89-1.82 (m, 1H), 1.72-1.68 (m, 2H), 1.37-1.26 (m, 4H)

### Example 117: N-(3-Fluoro-4-((7-(2-(3-hydroxyazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 1,2-dibromoethane was used as a starting material in step 6 and azetidin-3-ol was used as a starting material in step 7.

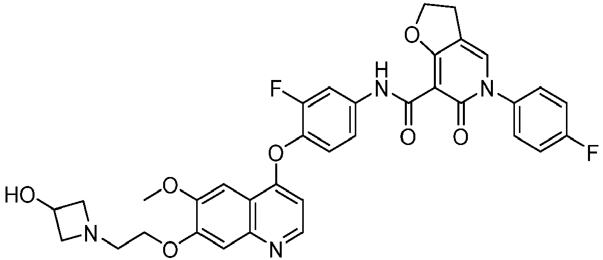

¹H NMR (400 MHz, MeOD-d₄) δ 8.44 (d, J = 5.2 Hz, 1H), 8.01-7.97 (dd, J = 2.0 Hz, J = 12.8Hz, 1H), 7.73 (s,1H), 7.67 (s,1H), 7.49-7.46 (m, 2H), 7.40-7.27 (m, 5H), 6.53 (d, J = 5.2 Hz, 1H), 4.93 (t, J = 8.4 Hz, 2H, overlapped with H₂O), 4.50 (t, J = 6.4 Hz, 1H), 4.30 (t, J = 4.8 Hz, 2H), 4.11 (t, J = 8.8 Hz, 2H), 4.03 (s,3H), 3.56-3.52 (m, 2H), 3.34-3.27 (m, 2H, overlapped with MeOH), 3.20 (t, J = 8.0 Hz, 2H).

### Example 118: N-(3-Fluoro-4-((7-(2-(3-hydroxy-3-methylazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 1,2-dibromoethane was used as a starting material in step 6 and 3-methyl-3-hydroxyazetidine hydrochloride was used as a starting material in step 7.

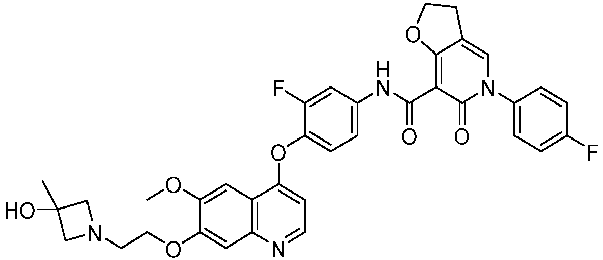

¹H NMR (400 MHz, MeOD-d₄) δ 8.44 (d, J = 5.6 Hz, 1H), 8.01-7.97 (dd, J = 12.8, 2.0 Hz, 1H), 7.73 (s,1H), 7.67 (s,1H), 7.49-7.46 (m, 2H), 7.37-7.27 (m, 5H), 6.53 (d, J = 4.4 Hz, 1H), 4.93 (t, J = 8.4 Hz, 2H, overlapped with H₂O), 4.33 (t, J = 4.8 Hz, 2H), 4.03 (s, 3H), 3.85 (d, J = 9.2 Hz, 2H), 3.66 (d, J = 10.8 Hz, 2H), 3.36-3.30 (m, 2H, overlapped with MeOH), 3.20 (t, J = 6.8 Hz, 2H), 1.51 (s, 3H)

### Example 119: N-(3-Fluoro-4-((7-(2-(3-(hydroxymethyl)azetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 1,2-dibromoethane was used as a starting material in step 6 and azetidin-3-ylmethanol hydrochloride was used as a starting material in step 7.

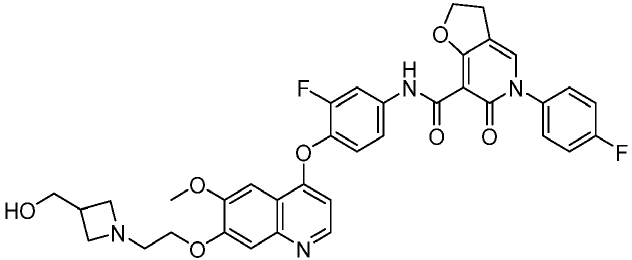

¹H NMR (400 MHz, MeOD-d₄) δ 8.45 (d, J = 5.6 Hz, 1H), 8.01-7.97 (dd, J = 12.8, 2.0 Hz, 1H), 7.73 (s, 1H), 7.68 (s, 1H), 7.49-7.46 (m, 2H), 7.41-7.28 (m, 5H), 6.54 (d, J = 5.6 Hz, 1H), 4.93 (t, J = 8.4 Hz, 2H, overlapped with H₂O), 4.36 (t, J = 4.4 Hz, 2H), 4.12 (t, J = 9.6 Hz, 2H), 4.03 (s, 3H), 3.90 (t, J = 6.8 Hz, 2H), 3.70 (d, J = 4.4 Hz, 2H), 3.53-3.49 (m, 2H), 3.20 (t, J = 8.0 Hz, 2H), 2.96-2.92 (m, 1H)

### Example 120: N-(3-Fluoro-4-((6-methoxy-7-(2-(3-methoxyazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-methoxyazetidine hydrochloride was used as a starting material in step 7.

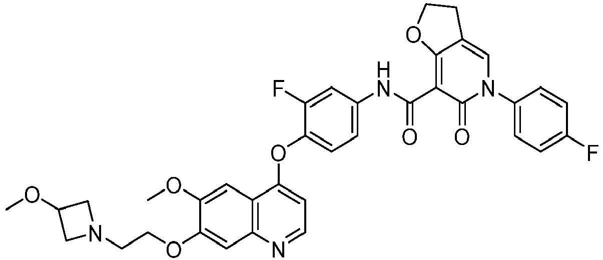

¹H NMR (400 MHz, DMSO-d₆) δ 11.88 (s, 1H), 8.47-8.46 (d, J = 4.8 Hz, 1H), 8.01-7.97 (dd, J = 13.2,2.4 Hz, 1H), 7.87 (s, 1H), 7.53-7.49 (m, 3H), 7.43-7.36 (m, 5H), 6.47-6.46 (d, J = 4.8 Hz, 1H), 4.87 (t, J = 8.0 Hz, 2H), 4.21 (t, J = 5.6 Hz, 2H), 3.95 (s, 3H), 3.85-3.83 (m, 2H), 3.13 (t, J = 7.6 Hz, 2H), 3.02 (t, J = 5.6 Hz, 2H), 2.73-2.65 (m, 1H), 1.82 (d, J = 12.0 Hz, 2H)

### Example 121: N-(3-Fluoro-4-((6-methoxy-7-(2-(3-methoxy-3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-methoxy-3-methylazetidine hydrochloride was used as a starting material in step 7.

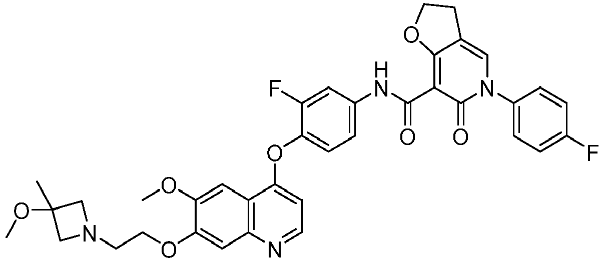

¹H NMR (400 MHz, DMSO-d₆) δ 11.88 (s, 1H), 8.46 (d, J = 5.6 Hz, 1H), 7.99-7.96 (m, 1H), 7.83 (s, 1H), 7.52-7.47 (m, 3H), 7.41-7.35 (m, 5H), 6.46 (d, J = 4.4 Hz, 1H), 4.86 (t, J = 8.4 Hz, 2H), 4.13 (t, J = 4.8 Hz, 2H), 3.93 (s, 3H), 3.23 (d, J = 8.0 Hz, 1H), 3.17 (d, J = 5.6 Hz, 2H), 3.13-3.05 (m, 6H), 2.89 (t, J = 4.8 Hz, 2H), 1.37 (s, 3H)

### Example 122: N-(3-Fluoro-4-((6-methoxy-7-(2-(3-(methoxymethyl)azetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-methoxymethylazetidine hydrochloride was used as a starting material in step 7.

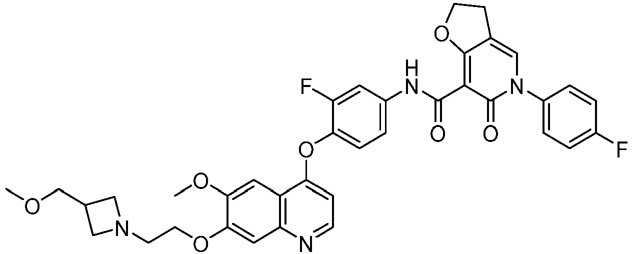

¹H NMR (400 MHz, DMSO-d₆) δ 11.88 (s, 1H), 8.47 (d, J = 5.2 Hz, 1H), 8.01 (dd, J = 12.8, 2.0 Hz, 1H), 7.87 (s, 1H), 7.53-7.49 (m, 3H), 7.45-7.36 (m, 5H), 6.46 (d, J = 5.2 Hz, 1H), 4.87 (t, J = 8.0 Hz, 2H), 4.10 (t, J = 5.2 Hz, 2H), 3.94 (s, 3H), 3.45 (d, J = 6.8 Hz, 2H), 3.34 (m, 2H, overlapped with H₂O), 3.24 (s, 3H), 3.13 (t, J = 7.6 Hz, 2H), 2.97 (t, J = 6.4 Hz, 2H), 2.82 (t, J = 5.6 Hz, 2H), 2.63-2.56 (m, 1H)

### Example 123: N-(3-Fluoro-4-((7-(2-(3-fluoroazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-fluoroazetidine hydrochloride was used as a starting material in step 7.

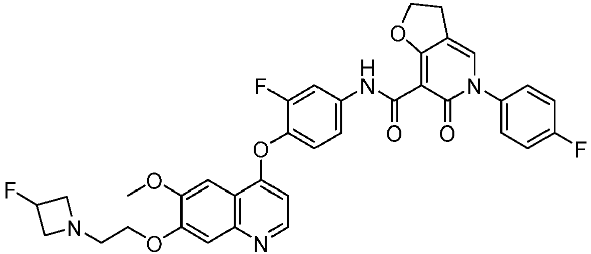

¹H NMR (400 MHz, DMSO-d₆) δ 11.89 (s, 1H), 8.47 (d, J = 5.2 Hz, 1H), 8.01 (dd, J = 2.0 Hz, J = 12.8 Hz, 1H), 7.88 (t, J = 1.2 Hz, 1H), 7.52-7.48 (m, 3H), 7.45-7.36 (m, 5H), 4.15 (t, J = 5.2 Hz, 2H), 3.94 (s, 3H), 3.71-3.64 (m, 2H), 3.28-3.24 (m, 1H), 3.22-3.18 (m, 1H), 3.13 (t, J = 8.4 Hz, 2H), 2.92 (t, J = 4.8 Hz, 2H)

### Example 124: N-(4-((7-(2-(3,3-difluoroazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 1,2-dibromoethane was used as a starting material in step 6 and 3,3-difluoroazetidine hydrochloride was used as a starting material in step 7.

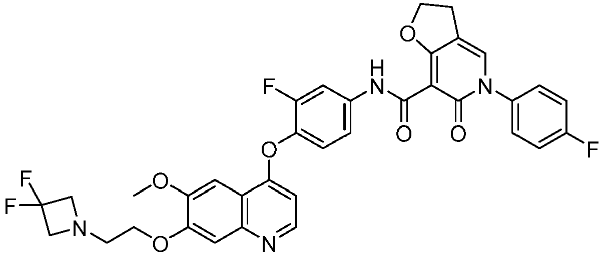

¹H NMR (400 MHz, MeOD-d₄) δ 8.42 (d, J = 5.6 Hz, 1H), 8.01-7.97 (dd, J = 12.8, 2.0 Hz, 1H), 7.72 (s,1H), 7.65 (s,1H), 7.49-7.46 (m, 2H), 7.37-7.27 (m, 5H), 6.51 (d, J = 5.6 Hz, 1H), 4.93 (t, J = 8.0 Hz, 2H, overlapped with H₂O), 4.26 (t, J = 4.4 Hz, 2H), 4.02 (s, 3H), 3.84 (t, J = 12.4 Hz, 4H), 3.20 (t, J = 8.0 Hz, 2H), 3.14 (t, J = 4.4 Hz, 2H)

### Example 125: N-(4-((7-(2-(3-ethynylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 1,2-dibromoethane was used as a starting material in step 6 and azetidine-3-carbonitrile was used as a starting material in step 7.

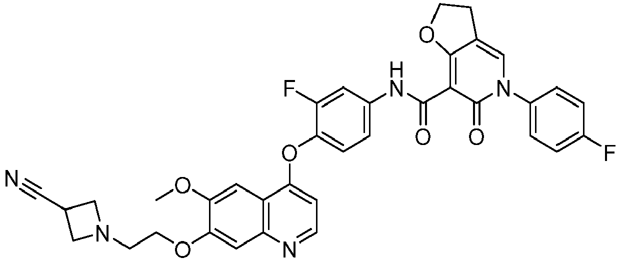

¹H NMR (400 MHz, MeOD-d₆) δ 8.42 (d, J = 5.2 Hz, 1H), 8.01-7.97 (dd, J = 2.0 Hz, J = 12.8Hz, 1H), 7.72 (s,1H), 7.64 (s,1H), 7.49-7.46 (m, 2H), 7.39-7.27 (m, 5H), 6.51 (d, J = 5.6 Hz, 1H), 4.93 (t, J = 8.4 Hz, 2H, overlapped with H₂O), 4.21 (t, J = 5.2Hz, 2H), 4.01 (s,3H), 3.77 (t, J = 8.4 Hz, 2H), 3.58 (t, J = 6.8 Hz, 2H), 3.48-3.46 (m, 1H), 3.22 (t, J = 7.2 Hz, 2H), 3.01 (t, J = 4.8 Hz, 2H)

### Example 126: N-(3-Fluoro-4-((6-methoxy-7-(2-(3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 1,2-dibromoethane was used as a starting material in step 6 and 3-methylazetidine hydrochloride was used as a starting material in step 7.

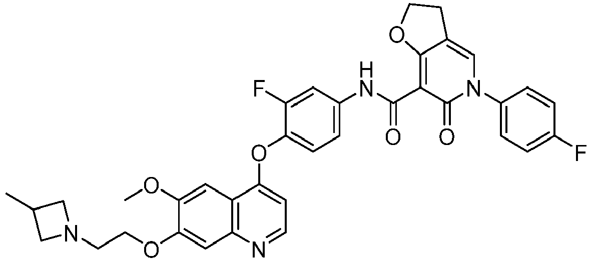

¹H NMR (400 MHz, MeOD-d₄) δ 8.42 (d, J = 5.2 Hz, 1H), 8.00-7.97 (dd, J = 12.8, 2.0 Hz, 1H), 7.72 (s,1H), 7.64 (s,1H), 7.49-7.46 (m, 2H), 7.37-7.27 (m, 5H), 6.50 (d, J = 5.6 Hz, 1H), 4.93 (t, J = 8.0 Hz, 2H, overlapped with H₂O), 4.33-4.30 (m, 1H), 4.18 (t, J = 5.2 Hz, 2H), 4.00 (s, 3H), 3.66 (t, J = 7.2 Hz, 2H), 3.34-3.29 (m, 2H, overlapped with MeOH), 3.22 (t, J = 8.8 Hz, 2H), 3.01-2.97 (m, 2H), 1.19 (d, J = 6.4 Hz, 3H)

### Example 127: N-(4-((7-(2-(3,3-Dimethylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 1,2-dibromoethane was used as a starting material in step 6 and 3,3-dimethylazetidine hydrochloride was used as a starting material in step 7.

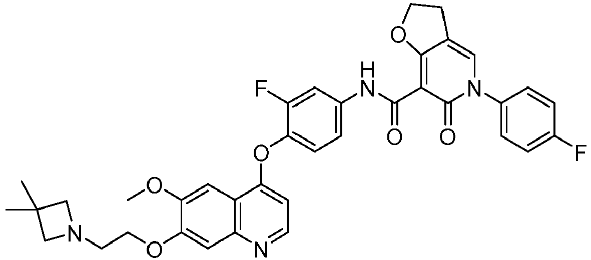

¹H NMR (500 MHz, MeOD-d₄) δ 8.42 (d, J = 5.5 Hz, 1H), 8.00-7.97 (dd, J = 13.0, 2.5 Hz, 1H), 7.72 (s, 1H), 7.64 (s, 1H), 7.48-7.46 (m, 2H), 7.39-7.28 (m, 5H), 6.51 (d, J = 5.0 Hz, 1H), 4.93 (t, J = 8.5 Hz, 2H), 4.22 (t, J = 5.0 Hz, 2H), 4.00 (s, 3H), 3.25 (s, 4H), 3.20 (t, J = 8.5 Hz, 2H), 3.04 (t, J = 5.0 Hz, 2H), 1.26 (s, 6H)

### Example 128: N-(4-((7-(2-(3-(dimethylamino)azetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-dimethylaminoazetidine dihydrochloride was used as a starting material in step 7.

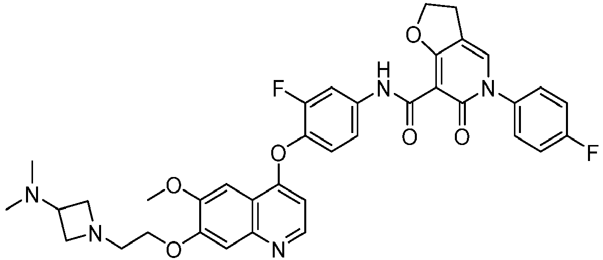

¹H NMR (400 MHz, DMSO-d₆) δ 11.88 (s, 1H), 8.47 (d, J = 5.6 Hz, 1H), 8.01 (dd, J = 12.8, 2.0 Hz, 1H), 7.87 (s, 1H), 7.53-7.49 (m, 3H), 7.45-7.36 (m, 5H), 6.47 (d, J = 5.2 Hz, 1H), 4.87 (t, J = 8.0 Hz, 2H), 4.12 (t, J = 5.2 Hz, 2H), 3.94 (s, 3H), 3.48-3.44 (m, 2H), 3.13 (t, J = 7.6 Hz, 2H), 2.88-2.82 (m, 4H), 2.78-2.72 (m, 1H)

### Example 129: N-(3-Fluoro-4-((7-(2-(3-hydroxypyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and DL-3-pyrrolidinol was used as a starting material in step 7.

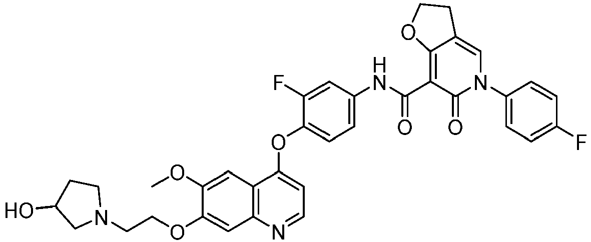

¹H NMR (400 MHz, DMSO-d₆) δ 11.88 (s, 1H), 8.47 (d, J = 4.8 Hz, 1H), 8.01 (dd, J = 13.2,2.0 Hz, 1H), 7.87 (s, 1H), 7.53-7.49 (m, 3H), 7.45-7.36 (m, 5H), 6.47 (d, J = 5.2 Hz, 1H), 4.87 (t, J = 8.0 Hz, 2H), 4.70 (d, J = 4.8 Hz, 1H), 4.24-4.19 (m, 3H), 3.95 (s, 3H), 3.13 (t, J = 8.0 Hz, 2H), 2.88 (t, J = 6.0 Hz, 2H), 2.83-2.79 (m, 1H), 2.73-2.67 (m, 1H), 2.55-2.54 (m, 2H, overlapped with DMSO), 2.46 (dd, J = 9.6, 3.6 Hz, 1H), 2.03-1.94 (m, 1H), 1.58-1.51 (m, 1H)

### Example 130: N-(3-Fluoro-4-((7-(2-(3-hydroxy-3-methylpyrrolidin-1-yl)ethoxy)-6-methoxyquinoline-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-methylpyrrolidin-3-ol was used as a starting material in step 7.

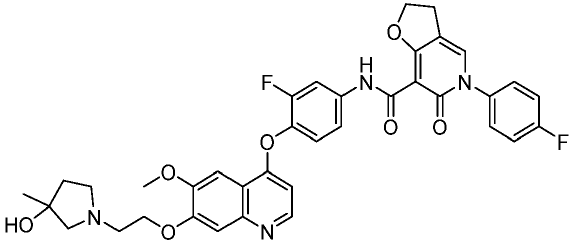

¹H NMR (400 MHz, DMSO-d₆) δ 11.88 (s, 1H), 8.47 (d, J = 4.8 Hz, 1H), 8.01 (dd, J = 13.2,2.4 Hz, 1H), 7.87 (s, 1H), 7.53-7.49 (m, 3H), 7.45-7.36 (m, 5H), 6.47 (d, J = 5.2 Hz, 1H), 4.87 (t, J = 8.0 Hz, 2H), 4.53 (s, 1H), 4.23 (t, J = 5.6 Hz, 2H), 3.94 (s, 3H), 3.13 (t, J = 8.4 Hz, 2H), 2.88 (t, J = 6.0 Hz, 2H), 2.76-2.72 (m, 1H), 2.66-2.56 (m, 3H), 1.78-1.65 (m, 2H), 1.25 (s, 3H)

### Example 131: N-(3-Fluoro-4-((6-methoxy-7-(2-(3-methoxypyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 1,2-dibromoethane was used as a starting material in step 6 and 3-methoxypyrrolidine hydrochloride was used as a starting material in step 7.

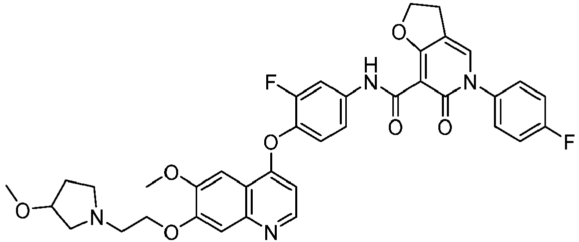

¹H NMR (400 MHz, CDCl₃) δ 12.02 (s, 1H, NH), 8.48 (d, J = 5.2 Hz, 1H), 8.01 (dd, J = 2.4 Hz, J = 12.8 Hz, 1H), 7.57 (s, 1H), 7.41 (s, 1H), 7.37-7.34 (m, 4H), 7.26-7.24 (m, 2H, overlapped with CDCl₃), 7.15 (t, J = 8.8 Hz, 1H), 6.44 (d, J = 4.4 Hz, 1H), 5.29 (s, 2H), 4.98 (t, J = 8.4 Hz, 2H), 4.40 (bs, 2H), 4.02 (s, 3H), 3.99 (bs, 1H), 3.31 (s, 3H), 3.17 (t, J = 8.8 Hz, 4H), 2.97-2.88 (m, 2H), 2.16-2.10 (m, 1H), 1.94 (bs, 1H)

### Example 132: N-(3-Fluoro-4-((6-methoxy-7-(2-(pyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and pyrrolidine was used as a starting material in step 7.

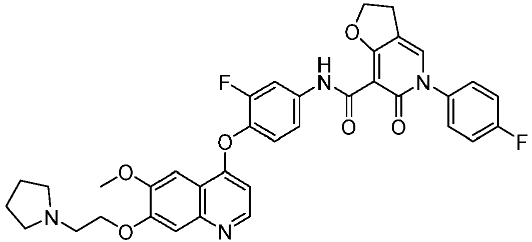

¹H NMR (400 MHz, MeOD-d₄) δ 8.42 (d, J = 5.6 Hz, 1H), 8.00 (dd, J = 12.8, 2.4 Hz, 1H), 7.72 (s, 1H), 7.65 (s, 1H), 7.49-7.46 (m, 2H), 7.38-7.27 (m, 5H), 6.51 (d, J = 5.6 Hz, 1H), 4.93 (t, J = 8.4 Hz, 2H, overlapped with H₂O), 4.34 (t, J = 5.6 Hz, 2H), 4.01 (s, 3H), 3.20 (t, J = 6.8 Hz, 2H), 3.08 (t, J = 5.6 Hz, 2H), 2.78 (s, 4H), 1.88-1.84 (m, 4H)

### Example 133: N-(3-Fluoro-4-((7-(2-(3-fluoropyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 1,2-dibromoethane was used as a starting material in step 6 and 3-fluoropyrrolidine hydrochloride was used as a starting material in step 7.

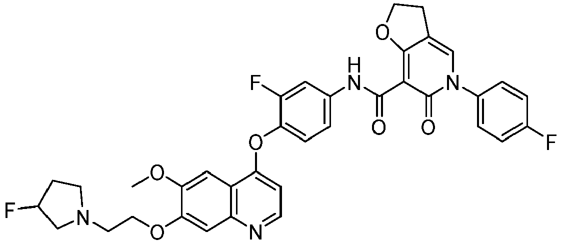

¹H NMR (400 MHz, MeOD-d₄) δ 8.42 (d, J = 5.6 Hz, 1H), 8.00 (dd, J = 12.8, 2.4 Hz, 1H), 7.71 (s, 1H), 7.65 (s, 1H), 7.49-7.45 (m, 2H), 7.38-7.27 (m, 5H), 6.51 (d, J = 5.6 Hz, 1H), 5.30 (t, J = 5.6 Hz, 1H), 5.16 (t, J = 5.6 Hz, 1H), 4.92 (t, J = 8.4 Hz, 2H, overlapped with H₂O), 4.35 (t, J = 5.6 Hz, 2H), 4.01 (s, 3H), 3.19-3.13 (m, 5H), 2.99-2.88 (m, 1H), 2.75 (dd, J = 15.6 8.8 Hz, 1H), 2.29-2.01 (m, 2H)

### Example 134: N-(4-((7-(2-(3,3-Difluoropyrrolidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3,3-difluoropyrrolidine hydrochloride was used as a starting material in step 7.

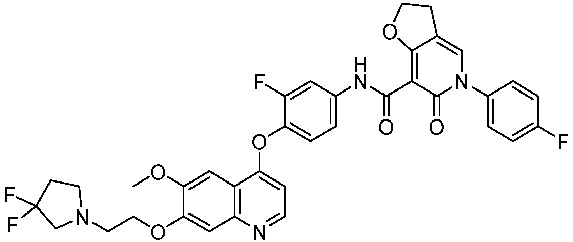

¹H NMR (400 MHz, DMSO-d₆) δ 11.88 (s, 1H), 8.48 (d, J = 5.6 Hz, 1H), 8.01 (dd, J = 13.2,2.0 Hz, 1H), 7.87 (s, 1H), 7.53-7.49 (m, 3H), 7.43-7.36 (m, 5H), 6.47 (dd, J = 4.8, 0.8 Hz, 1H), 4.87 (t, J = 8.4 Hz, 2H), 4.28 (t, J = 6.0 Hz, 2H), 3.95 (s, 3H), 3.11-3.02 (m, 4H), 2.95 (t, J = 5.6 Hz, 2H), 2.87 (t, J = 6.8 Hz, 2H), 2.29-2.21 (m, 2H)

### Example 135: N-(3-Fluoro-4-((7-(2-(4-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 4-hydroxypiperidine was used as a starting material in step 7.

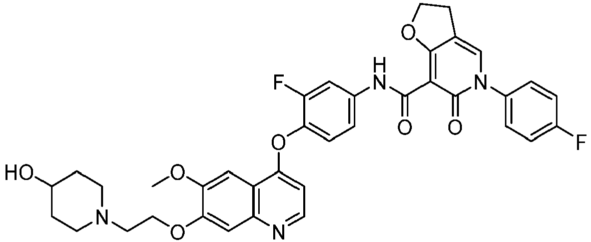

¹H NMR (400 MHz, CDCl₃) δ 12.19 (s, 1H), 8.55 (d, J = 6.4 Hz, 1H), 8.23 (s, 1H), 8.11 (d, J = 12.8 Hz, 1H), 7.64 (s, 1H), 7.41-7.33 (m, 5H), 7.26-7.19 (m, 2H, overlapped with CDCl₃), 6.78 (d, J = 6.0 Hz, 1H), 5.01 (t, J = 8.8 Hz, 1H), 4.87 (s, 2H), 4.21 (s, 1H), 4.11-4.05 (m, 4H), 3.82-3.79 (m, 1H), 3.69-3.62 (m, 2H), 3.56-3.51 (m, 3H), 3.02 (t, J = 7.6 Hz, 2H), 2.97 (dd, J = 10.4, 3.6 Hz,, 1H), 2.82-2.78 (m, 3H), 2.01-2.96 (m, 1H), 1.91 (t, J = 10.0 Hz, 1H), 1.81-1.77 (m, 1H), 1.64-1.60 (m, 1H), 1.47-1.38 (m, 1H), 1.12-1.02 (m, 1H)

### Example 136: N-(3-Fluoro-4-((7-(2-(3-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-hydroxypiperidine was used as a starting material in step 7.

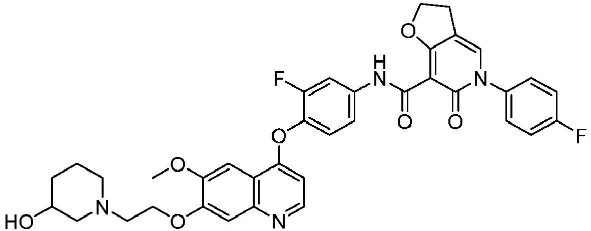

¹H NMR (400 MHz, DMSO-d₆) δ 11.88 (s, 1H), 8.47 (d, J = 5.2 Hz, 1H), 8.01 (dd, J = 13.6, 2.0 Hz, 1H), 7.87 (s, 1H), 7.52-7.49 (m, 3H), 7.45-7.36 (m, 5H), 6.47 (d, J = 5.2 Hz, 1H), 4.87 (t, J = 8.0 Hz, 2H), 4.61 (d, J = 4.8 Hz, 1H), 4.26 (t, J = 5.6 Hz, 2H), 3.94 (s, 3H), 3.50-3.43 (m, 1H), 3.13 (t, J = 7.6 Hz, 2H), 2.97 (dd, J = 10.4, 3.6 Hz, 1H), 2.82-2.78 (m, 3H), 2.01-1.96 (m, 1H), 1.91 (t, J = 10.0 Hz, 1H), 1.81-1.77 (m, 1H), 1.64-1.60 (m, 1H), 1.47-1.38 (m, 1H), 1.12-1.02 (m, 1H)

### Example 137: N-(3-Fluoro-4-((7-(2-(4-hydroxy-4-methylpiperidin-1-yl)ethoxy)-6-methoxyquinoline-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 4-methylpiperidin-4-ol was used as a starting material in step 7.

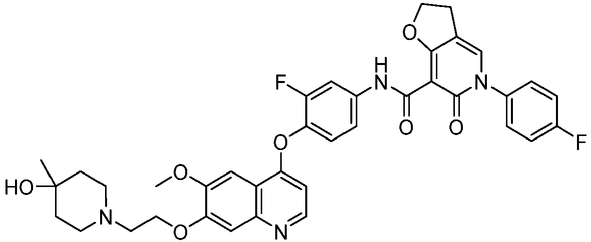

¹H NMR (400 MHz, DMSO-d₆) δ 11.88 (s, 1H), 8.47 (d, J = 5.2 Hz, 1H), 8.01 (dd, J = 12.8, 1.6 Hz, 1H), 7.87 (s, 1H), 7.52-7.49 (m, 3H), 7.42-7.36 (m, 5H), 6.46 (d, J = 5.2 Hz, 1H), 4.87 (t, J = 8.4 Hz, 2H), 4.26 (t, J = 6.0 Hz, 2H), 4.11 (s, 1H), 3.94 (s, 3H), 3.13 (t, J = 7.6 Hz, 1H), 2.50 (m, 4H), 2.79 (t, J = 6.4 Hz, 2H), 1.49 (t, J = 5.6 Hz, 4H), 1.10 (s, 3H)

### Example 138: N-(3-Fluoro-4-((6-methoxy-7-(2-(4-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 1,2-dibromoethane was used as a starting material in step 6 and 4-methoxypiperidine was used as a starting material in step 7.

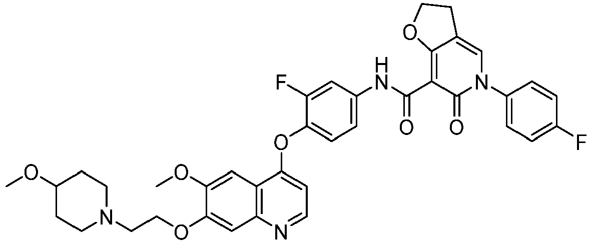

¹H NMR (400 MHz, CDCl₃) δ 12.02 (s, 1H, NH), 8.49 (d, J = 5.2 Hz, 1H), 8.01 (dd, J = 12.8, 2.8 Hz, 1H), 7.57 (s, 1H), 7.41 (s, 1H), 7.37-7.34 (m, 4H), 7.26-7.21 (m, 2H, overlapped with CDCl₃), 7.15 (t, J = 8.8 Hz, 1H), 6.44 (d, J = 4.4 Hz, 1H), 4.98 (t, J = 8.4 Hz, 2H), 4.39 (bs, 2H), 4.02 (s, 3H), 3.34 (s, 3H), 3.30 (bs, 1H), 3.17 (t, J = 8.8 Hz, 2H), 3.03-2.95 (m, 4H), 2.04 (bs, 2H), 1.72 (bs, 2H), 1.58 (bs, 2H, overlapped with H₂O)

### Example 139: N-(3-Fluoro-4-((6-methoxy-7-(2-(3-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 1,2-dibromoethane was used as a starting material in step 6 and 3-methoxypiperidine was used as a starting material in step 7.

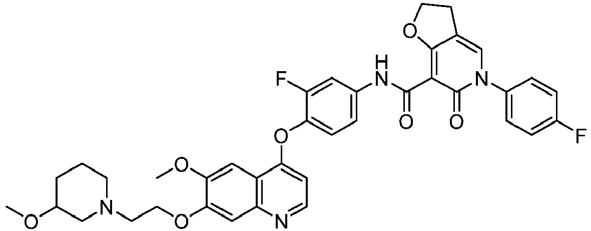

¹H NMR (400 MHz, CDCl₃) δ 12.06 (s, 1H, NH), 8.49 (d, J = 5.2 Hz, 1H), 8.03 (dd, J = 12.8, 2.0 Hz, 1H), 7.58 (s,1H), 7.45 (s,1H), 7.37-7.34 (m, 4H), 7.24-7.22 (m, 2H, overlapped with CDCl₃), 7.15 (t, J = 8.8 Hz, 1H), 6.46 (d, J = 5.2 Hz, 1H), 4.99 (t, J = 8.4 Hz, 2H), 4.41 (bs, 2H), 4.03 (s, 3H), 3.48 (bs, 1H), 3.39 (s, 3H), 3.23-3.08 (m, 4H), 2.51 (bs,2H), 1.86 (bs,4H), 1.70 (bs,2H)

### Example 140: N-(3-Fluoro-4-((6-methoxy-7-(2-(4-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and piperidin-4-one hydrochloride was used as a starting material in step 7.

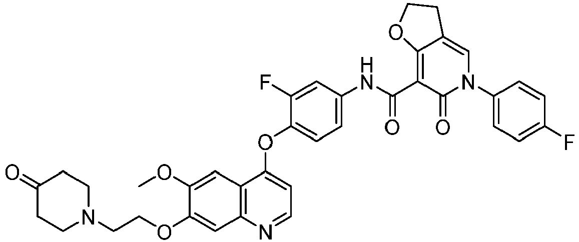

¹H NMR (500 MHz, MeOD-d₄) δ 8.43 (d, J = 5.5 Hz, 1H), 8.00 (dd, J = 12.5, 2.0 Hz, 1H), 7.72 (s, 1H), 7.65 (s, 1H), 7.48-7.46 (m, 2H), 7.40-7.28 (m, 5H), 6.51 (d, J = 4.0 Hz, 1H), 4.93 (t, J = 8.5 Hz, 2H), 4.38 (t, J = 5.5 Hz, 1H), 4.34 (t, J = 5.5 Hz, 1H), 4.01 (s, 3H), 3.20 (t, J = 8.5 Hz, 2H), 3.10 (t, J = 5.5 Hz, 1H), 3.03-2.99 (m, 3H), 2.73 (bs, 2H), 2.49 (t, J = 6.5 Hz, 2H), 1.84 (dd, J = 11.0, 5.5 Hz, 2H)

### Example 141: N-(3-Fluoro-4-((6-methoxy-7-(2-(3-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and piperidin-3-one hydrochloride was used as a starting material in step 7.

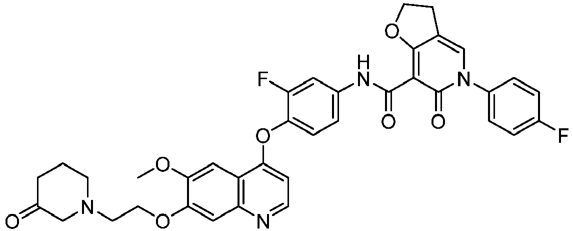

¹H NMR (400 MHz, DMSO-d₆) δ 11.88 (s, 1H), 8.47 (d, J = 4.8 Hz, 1H), 8.01 (d, J = 15.2 Hz, 1H), 7.86 (s, 1H), 7.54-7.49 (m, 3H), 7.44-7.36 (m, 5H), 6.47 (d, J = 5.2 Hz, 1H), 4.87 (t, J = 8.4 Hz, 2H), 4.29 (t, J = 5.6 Hz, 2H), 3.94 (s, 3H), 3.13-3.09 (m, 2H), 2.93 (t, J = 5.2 Hz, 1H), 2.80 (t, J = 5.2 Hz, 1H), 2.69-2.65 (m, 2H), 2.33-2.30 (m, 2H), 1.88-1.85 (m, 1H)

### Example 142: N-(4-((7-(2-(1,1-Dioxidothiomorpholino)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and thiomorpholine 1,1-dioxide was used as a starting material in step 7.

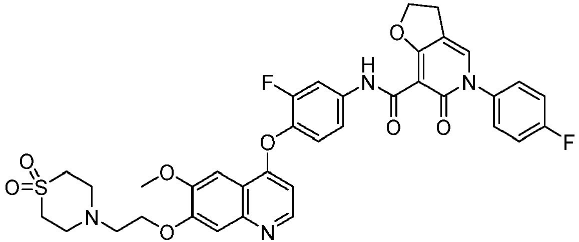

¹H NMR (400 MHz, CDCl₃) δ 12.20 (s, 1H), 8.49 (d, J = 6.4 Hz, 1H), 8.31 (s, 1H), 8.20 (dd, J = 12.8, 2.4 Hz, 1H), 7.65 (s, 1H), 7.41-7.34 (m, 4H), 7.27-7.22 (m, 3H, overlapped with CDCl₃), 6.77 (d, J = 6.4 Hz, 1H), 5.02 (t, J = 8.4 Hz, 2H), 4.61 (bs, 2H), 4.07 (s, 3H), 3.71 (t, J = 5.6 Hz, 1H), 3.52 (bs, 4H), 3.37 (bs, 4H), 3.25 (bs, 1H), 3.21 (t, J = 7.2 Hz, 2H)

### Example 143: N-(3-Fluoro-4-((6-methoxy-7-(2-(piperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and piperidine was used as a starting material in step 7.

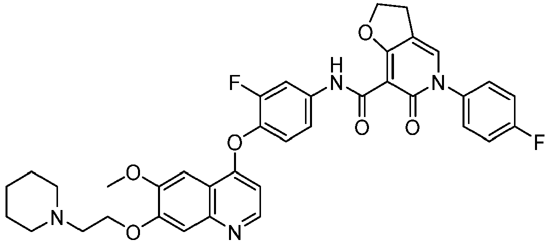

¹H NMR (400 MHz, MeOD-d₄) δ 8.43 (d, J = 5.6 Hz, 1H), 8.01 (dd, J = 12.8, 2.4 Hz, 1H), 7.72 (s, 1H), 7.66 (s, 1H), 7.49-7.46 (m, 2H), 7.38-7.27 (m, 5H), 6.52 (d, J = 5.2 Hz, 1H), 4.93 (t, J = 8.8 Hz, 2H, overlapped with H₂O), 4.38 (t, J = 5.6 Hz, 2H), 4.02 (s, 3H), 3.20 (t, J = 7.2 Hz, 2H), 3.07 (t, J = 4.8 Hz, 2H), 2.80 (bs, 4H), 1.72-1.68 (m, 4H), 1.55-1.54 (m, 2H)

### Example 144: N-(3-Fluoro-4-((7-(2-(4-fluoropiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 1,2-dibromoethane was used as a starting material in step 6 and 4-fluoropiperidine was used as a starting material in step 7.

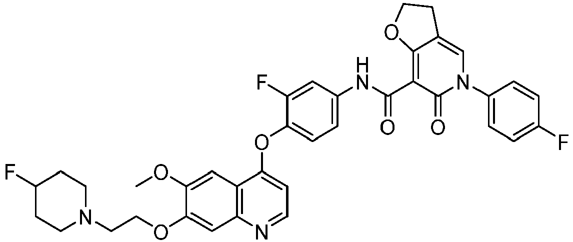

¹H NMR (400 MHz, MeOD-d₄) δ 8.43 (d, J = 5.2 Hz, 1H), 8.01 (dd, J = 12.4, 1.6 Hz, 1H), 7.72 (s, 1H), 7.65 (s, 1H), 7.49-7.46 (m, 2H), 7.39-7.27 (m, 5H), 6.51 (d, J = 5.6 Hz, 1H), 4.91 (t, J = 8.4 Hz, 2H, overlapped with H₂O), 4.78-4.72 (m, 1H), 4.66-4.60 (m, 1H), 4.34 (t, J = 5.6 Hz, 2H), 4.01 (s, 3H), 3.20 (t, J = 8.0 Hz, 2H), 2.99-2.96 (m, 2H), 2.86-2.81 (m, 2H), 2.70-2.62 (m, 2H), 2.40-1.96 (m, 1H), 1.96-1.84 (m, 2H)

### Example 145: N-(4-((7-(2-(4,4-Difluoropiperidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 1,2-dibromoethane was used as a starting material in step 6 and 4,4-difluoropiperidine hydrochloride was used as a starting material in step 7.

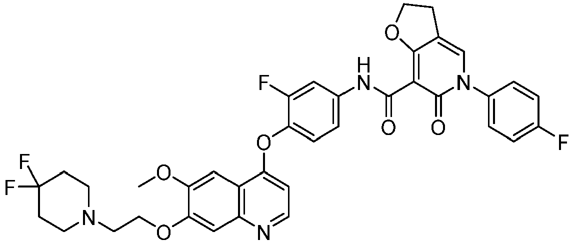

¹H NMR (400 MHz, DMSO-d₄) δ 11.88 (s, 1H), 8.48 (d, J = 5.2 Hz, 1H), 8.01 (dd, J = 12.8, 2.0 Hz, 1H), 7.87 (s, 1H), 7.53-7.49 (m, 3H), 7.44-7.36 (m, 5H), 6.47 (d, J = 5.2 Hz, 1H), 4.87 (t, J = 8.0 Hz, 2H), 4.29 (t, J = 5.2 Hz, 2H), 3.94 (s, 3H), 3.13 (t, J = 7.2 Hz, 2H), 2.90 (t, J = 6.0 Hz, 2H), 2.69 (t, J = 5.2 Hz, 4H), 2.02-1.90 (m, 4H)

### Example 146: N-(3-Fluoro-4-((6-methoxy-7-(2-(4-methylpiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide

The title compound (clear oil) was obtained using the same synthetic route as in Example 76, except that 1,2-dibromoethane was used as a starting material in step 6 and 4-methylpiperidine was used as a starting material in step 7.

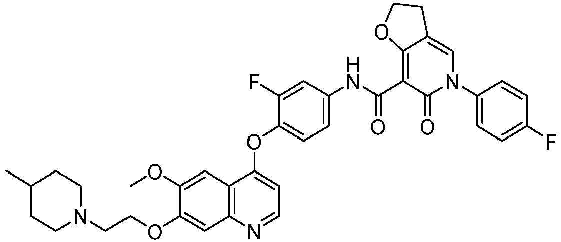

¹H NMR (400 MHz, MeOD-d₄) δ 8.42 (d, J = 6.5 Hz, 1H), 8.00 (dd, J = 12.8, 2.4 Hz, 1H), 7.71 (s, 1H), 7.64 (s, 1H), 7.49-7.45 (m, 2H), 7.36-7.27 (m, 5H), 6.50 (d, J = 5.2 Hz, 1H), 4.92 (t, J = 8.4 Hz, 2H, overlapped with H₂O), 4.35 (t, J = 5.6 Hz, 2H), 4.01 (s, 3H), 3.19-3.13 (m, 4H), 2.99 (t, J = 5.2 Hz, 2H), 2.30 (t, J = 11.6 Hz, 2H), 1.72 (d, J = 13.2 Hz, 2H), 1.33-1.28 (m, 3H), 0.97 (d, J = 6.4 Hz, 3H)

### Example 147: N-(4-((7-(2-(4,4-Dimethylpiperidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 4,4-dimethylpiperidine hydrochloride was used as a starting material in step 7.

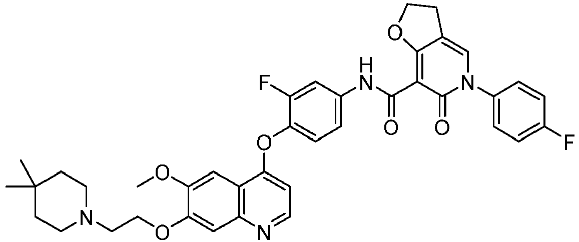

¹H NMR (400 MHz, DMSO-d₆) δ 11.88 (s, 1H), 8.47 (d, J = 5.2 Hz, 1H), 8.01 (dd, J = 12.8, 1.6 Hz, 1H), 7.86 (s, 1H), 7.53-7.49 (m, 3H), 7.44-7.36 (m, 5H), 6.47 (d, J = 5.2 Hz, 1H), 4.87 (t, J = 8.4 Hz, 2H), 4.27 (bs, 2H), 3.94 (s, 3H), 3.13 (t, J = 8.4 Hz, 2H), 2.85 (bs, 1H), 2.54-2.50 (m, 5H, overlapped with DMSO), 1.36 (bs, 4H), 0.91 (s, 6H)

### Example 148: N-(3-Fluoro-4-((7-(2-((3-hydroxycyclobutyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-aminocyclobutanol hydrochloride was used as a starting material in step 7.

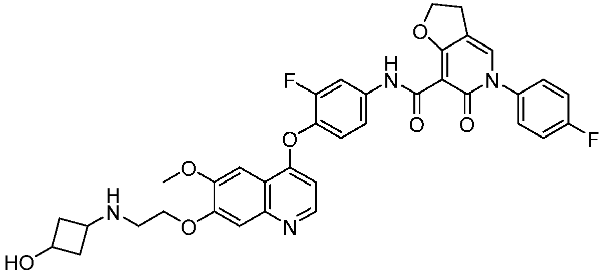

¹H NMR (400 MHz, DMSO-d₆) δ 11.88 (s, 1H), 8.48-8.46 (d, J = 5.2 Hz, 1H), 8.01 (dd, J = 12.8, 2.0 Hz, 1H), 7.88 (t, J = 2.0 Hz, 1H), 7.54-7.49 (m, 3H), 7.43-7.36 (m, 5H), 6.47-6.46 (m, 1H), 4.96 (d, J = 6.0 Hz, 1H), 4.87 (t, J = 8.0 Hz, 2H), 4.20 (t, J = 6.0 Hz, 2H), 3.95 (s, 3H), 3.80-3.75 (m, 1H), 3.13 (t, J = 8.0 Hz, 2H), 2.95 (t, J = 6.0 Hz, 2H), 2.82-2.78 (m, 1H), 2.03-1.99 (m, 1H), 1.62-1.55 (m, 2H)

### Example 149: N-(3-Fluoro-4-((6-methoxy-7-(2-((3-methoxycyclobutyl)amino)ethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-methoxycyclobutanamine hydrochloride was used as a starting material in step 7.

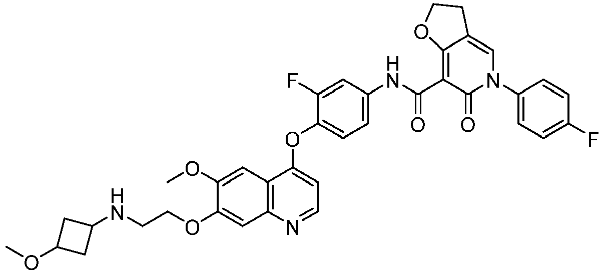

¹H NMR (400 MHz, DMSO-d₆) δ 11.88 (s, 1H), 8.47 (d, J = 5.2 Hz, 1H), 8.01 (dd, J = 13.2,2.4 Hz, 1H), 7.87 (t, J = 1.6 Hz, 1H), 7.53-7.49 (m, 3H), 7.43-7.36 (m, 5H), 6.47-6.46 (d, J = 4.8 Hz, 1H), 4.87 (t, J = 8.0 Hz, 1H), 4.19 (q, J = 6.0 Hz, 2H), 3.95-3.01 (m, 4H), 3.54-3.49 (m, 1H), 3.13-3.09 (m, 5H), 2.92-2.84 (m, 3H), 2.08-1.91 (m, 2H), 1.58-1.51 (m, 1H)

### Example 150: N-(3-Fluoro-4-((6-methoxy-7-(2-(oxetan-3-ylamino)ethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-oxetanamine was used as a starting material in step 7.

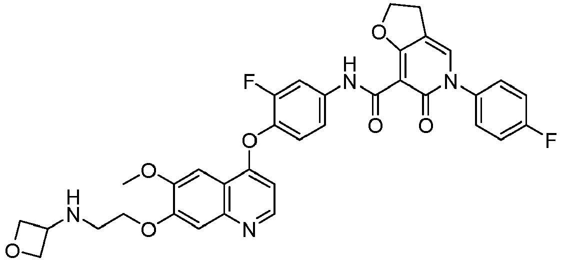

¹H NMR (400 MHz, DMSO-d₆) δ 11.88 (s, 1H), 8.47 (d, J = 4.8 Hz, 1H), 8.01 (dd, J = 13.2,2.4 Hz, 1H), 7.87 (s, 1H), 7.53-7.49 (m, 3H), 7.45-7.36 (m, 5H), 6.47 (d, J = 5.6 Hz, 1H), 4.87 (t, J = 8.0 Hz, 2H), 4.67 (t, J = 6.4 Hz, 2H), 4.35 (t, J = 6.4 Hz, 2H), 4.15 (t, J = 6.0 Hz, 2H), 3.99-3.96 (m, 4H), 3.13 (t, J = 8.0 Hz, 2H), 2.96 (t, J = 5.2 Hz, 2H)

### Example 151: N-(3-Fluoro-4-((6-methoxy-7-(2-((oxetan-3-ylmethyl)amino)ethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-oxetanemethanamine was used as a starting material in step 7.

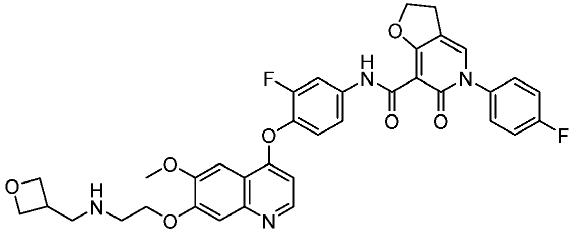

¹H NMR (400 MHz, DMSO-d₆) δ 11.88 (s, 1H), 8.47 (d, J = 4.8 Hz, 1H), 8.01 (dd, J = 13.2,2.0 Hz, 1H), 7.87 (s, 1H), 7.53-7.49 (m, 3H), 7.45-7.36 (m, 5H), 6.47 (d, J = 5.2 Hz, 1H), 4.87 (t, J = 8.0 Hz, 2H), 4.65-4.61 (m, 2H), 4.30 (t, J = 5.6 Hz, 2H), 4.21 (t, J = 6.0 Hz, 2H), 3.95 (s, 3H), 3.13-3.02 (m, 3H), 2.98 (t, J = 6.0 Hz, 2H), 2.91 (d, J = 7.2 Hz, 1H)

### Example 152: N-(3-Fluoro-4-((7-(2-((4-hydroxycyclohexyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 4-aminocyclohexanol was used as a starting material in step 7.

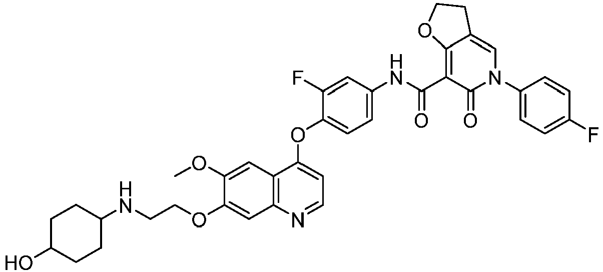

¹H NMR (400 MHz, MeOD-d₄) δ 8.43 (d, J = 5.6 Hz, 1H), 8.01 (dd, J = 12.8, 2.0 Hz, 1H), 7.72 (s, 1H), 7.67 (s, 1H), 7.49-7.46 (m, 2H), 7.38-7.27 (m, 5H), 6.52 (d, J = 5.6 Hz, 1H), 4.93 (t, J = 8.0 Hz, 2H, overlapped with H₂O), 4.29 (t, J = 5.2 Hz, 2H), 4.03 (s, 3H), 3.57-3.52 (m, 1H),3.20 (t, J = 8.4 Hz, 2H), 3.14 (t, J = 5.2 Hz, 2H), 2.64-2.59 (m, 1H), 2.05-1.96 (m, 4H), 1.37-1.21 (m, 4H)

### Example 153: N-(3-Fluoro-4-((7-(2-((3-hydroxycyclohexyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-aminocyclohexanol was used as a starting material in step 7.

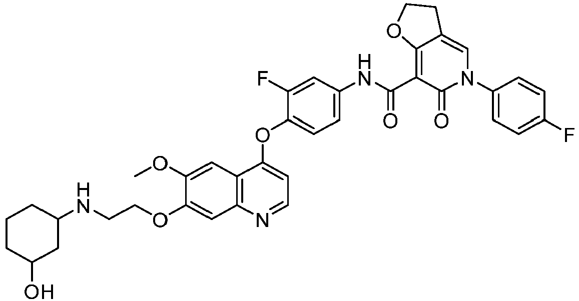

¹H NMR (400 MHz, DMSO-d₆) δ 11.88 (s, 1H), 8.47 (d, J = 4.8 Hz, 1H), 8.01 (dd, J = 12.8, 2.0 Hz, 1H), 7.87 (t, J = 2.0 Hz, 1H), 7.53-7.49 (m, 3H), 7.43-7.36 (m, 5H), 6.47 (dd, J = 5.2,0.8 Hz, 1H), 4.87 (t, J = 8.0 Hz, 2H), 4.55 (bs, 1H), 4.19 (t, J = 6.0 Hz, 2H), 3.95 (s, 3H), 3.43-3.39 (m, 1H), 3.13-3.09 (m, 2H), 3.00 (t, J = 11.2 Hz, 2H), 2.08-2.04 (m, 1H), 1.83-1.75 (m, 2H), 1.68-1.64 (m, 1H), 1.51-1.39 (m, 1H), 1.20-1.13 (m, 1H), 1.08-0.83 (m, 3H)

### Example 154: N-(3-Fluoro-4-((7-(2-(((3-hydroxycyclohexyl)methyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-aminomethylcyclohexanol was used as a starting material in step 7.

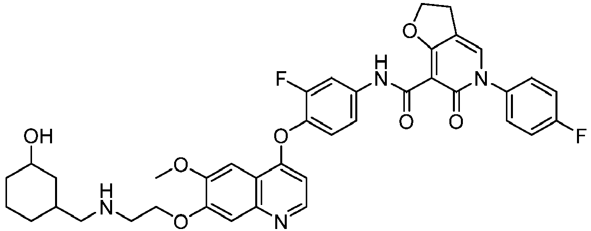

¹H NMR (400 MHz, MeOD-d₄) δ 8.43 (d, J = 5.6 Hz, 1H), 7.99 (dd, J = 12.8, 2.8 Hz, 1H), 7.71 (s, 1H), 7.67 (s, 1H), 7.48-7.45 (m, 2H), 7.39-7.27 (m, 5H), 6.52 (d, J = 5.6 Hz, 1H), 4.93 (t, J = 8.8 Hz, 2H), 4.31 (t, J = 5.2 Hz, 2H), 4.03 (s, 3H), 3.56-3.51 (m, 1H), 3.20 (t, J = 7.2 Hz, 2H), 3.10 (t, J = 5.2 Hz, 2H), 2.63 (dd, J = 6.8, 1.6 Hz, 2H), 2.06 (d, J = 11.6 Hz, 1H), 1.96 (d, J = 13.6 Hz, 1H), 1.83-1.75 (m, 2H), 1.70-1.58 (m, 1H), 1.35-1.28 (m, 1H), 1.21-1.14 (m, 1H), 0.96-0.84 (m, 2H)

### Example 155: N-(3-Fluoro-4-((6-methoxy-7-(2-((tetrahydro-2H-pyran-4-yl)amino)ethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 3-aminotetrahydropyran was used as a starting material in step 7.

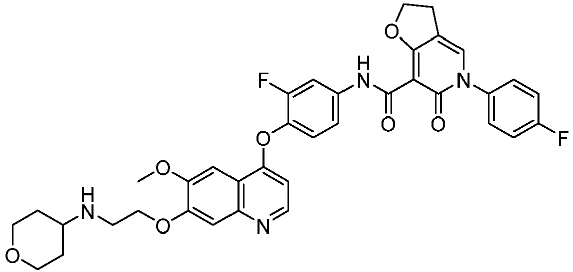

¹H NMR (400 MHz, DMSO-d₆) δ 11.88 (s, 1H), 8.47 (d, J = 4.8 Hz, 1H), 8.01 (dd, J = 13.2,2.4 Hz, 1H), 7.87 (s, 1H), 7.53-7.49 (m, 3H), 7.45-7.36 (m, 5H), 6.47 (d, J = 4.8 Hz, 1H), 4.87 (t, J = 8.0 Hz, 2H), 4.21 (t, J = 5.6 Hz, 2H), 3.95 (s, 1H), 3.85-3.83 (m, 2H), 3.13 (t, J = 7.6 Hz, 2H), 3.02 (t, J = 5.6 Hz, 2H), 2.73-2.67 (m, 1H), 2.50 (m, 2H, overlapped with DMSO), 1.82 (d, J = 12.0 Hz, 2H), 1.30 (d, J = 12.0 Hz, 2H)

### Example 156: N-(3-Fluoro-4-((6-methoxy-7-(2-((4-methoxycyclohexyl)amino)ethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 1,2-dibromoethane was used as a starting material in step 6 and 4-methoxycyclohexanamine was used as a starting material in step 7.

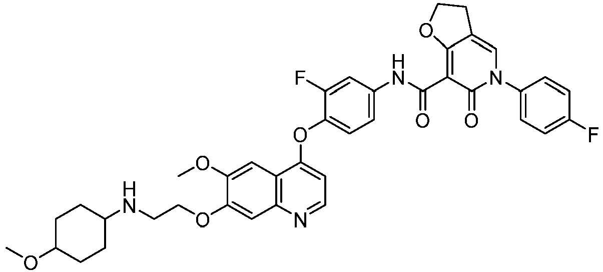

¹H NMR (400 MHz, CDCl₃) δ 12.01 (s, 1H, NH), 8.48 (d, J = 5.6 Hz, 1H), 8.01 (dd, J = 12.4, 2.4 Hz, 1H), 7.56 (s, 1H), 7.41 (s, 1H), 7.37-7.34 (m, 4H), 7.26-7.21 (m, 2H), 7.15 (t, J = 8.8 Hz, 1H), 6.43 (d, J = 4.0 Hz, 1H), 4.98 (t, J = 8.4 Hz, 2H), 4.28 (t, J = 5.6 Hz, 2H), 4.01 (s, 3H), 3.38-3.35 (m, 1H), 3.30 (s, 3H), 3.19-3.14 (m, 4H), 2.64-2.60 (m, 1H), 2.09-1.99 (m, 1H), 1.90-1.88 (m, 2H), 1.70-1.66 (m, 2H), 1.55-1.47 (m, 2H), 1.28-1.16 (m, 1H)

### Example 157: N-(3-Fluoro-4-((7-(2-morpholinylethoxy)quinolin-4-yl)oxy)phenyl)-5-(4-fluorophenyl)-6-oxo-2,3,5,6-tetrahydrofuro[3,2-c]pyridine-7-carboxamide

The title compound (off-white solid) was obtained using the same synthetic route as in Example 76, except that 4-chloro-7-hydroxyquinoline was used as a starting material in step 1 and 1,2-dibromoethane was used as a starting material in step 6.

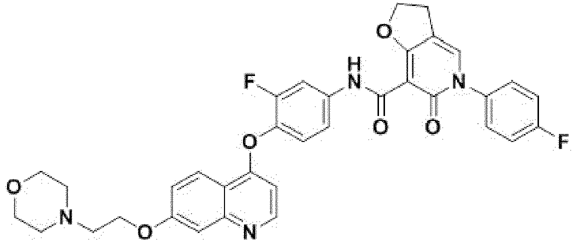

¹H NMR (500 MHz, DMSO-d₆) δ 11.91 (s, 1H), 8.61 (d, J = 5.0 Hz, 1H), 8.22 (d, J = 10.0 Hz, 1H), 8.01 (dd, J = 5.0 and 5.0 Hz, 1H), 7.88 (s, 1H), 7.53-7.50 (m, 2H), 7.44-7.37 (m, 5H), 7.31 (dd, J = 5.0 and 5.0 Hz, 1H), 6.48 (d, J = 5.0 Hz, 1H), 4.85 (t, J = 10.0 Hz, 2H), 4.28 (t, J = 5.0 Hz, 2H), 3.60 (t, J = 5.0 Hz, 4H), 3.17 (d, J = 5.0 Hz, 2H), 3.11 (t, J = 7.5 Hz, 2H), 2.78 (t, J = 5.0 Hz, 2H)

### Example 158: N-(3-Fluoro-4-((7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide

The title compound (off-white solid) was obtained using the same synthetic route as in Example 76, except that 4-chloro-7-hydroxyquinoline was used as a starting material in step 1.

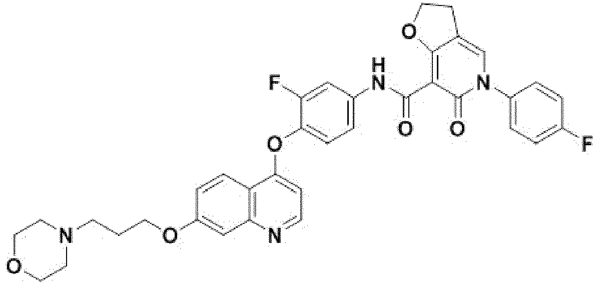

¹H NMR (500 MHz, CDCl₃) δ 12.06 (s, 1H), 8.57 (d, J = 5.0 Hz, 1H), 8.25 (d, J = 5.0 Hz, 1H), 8.03 (dd, J = 5.0 and 5.0 Hz, 1H), 7.88 (d, J = 10.0 Hz, 1H), 7.66 (d, J = 5.0 Hz, 1H), 7.38-7.35 (m, 5H), 7.29-7.23 (m, 2H), 7.21 (dd, J = 5.0 and 5.0 Hz, 1H), 7.14 (t, J = 10.0 Hz, 1H), 4.99 (t, J = 7.5 Hz, 2H), 4.14 (t, J = 7.5 Hz, 2H), 3.77 (t, J = 5.0 Hz, 4H), 3.18 (t, J = 10.0 Hz, 2H), 2.68 (t, J = 7.5 Hz, 2H), 2.61 (brs, 4H), 2.12-2.06 (m, 2H)

### Example 159: N-(3-Fluoro-4-((7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide

The title compound (pale orange solid) was obtained using the same synthetic route as in Example 76, except that 4-chloro-7-hydroxyquinoline was used as a starting material in step 1 and the carboxylic acid compound of Preparation Example 5 was used as a starting material in step 4.

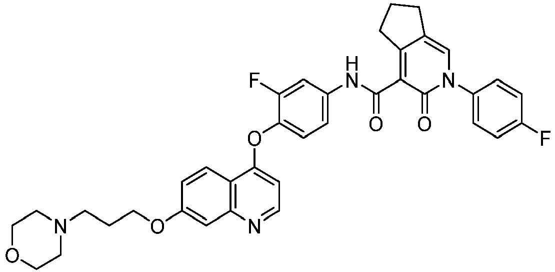

¹H NMR (500 MHz, DMSO-d₆) δ 12.33 (bs, 1H), 8.60 (d, J = 5.0 Hz, 1H), 8.22 (d, J = 9.5 Hz, 1H), 8.03 (dd, J = 13.0, 2.5 Hz, 1H), 7.91 (s, 1H), 7.57-7.54 (m, 2H), 7.48-7.46 (m, 1H), 7.44-7.38 (m, 4H), 7.30 (dd, J = 9.0, 2.5 Hz, 1H), 6.47 (d, J = 5.0 Hz, 1H), 4.20 (t, J = 6.5 Hz, 2H), 3.58 (t, J = 4.5 Hz, 4H), app 3.40 (overlapped, 2H), 2.75 (t, J = 7.5 Hz, 2H), 2.47 (t, J = 7.5 Hz, 2H), 2.39 (bs, 4H), 2.07-2.01 (m, 2H), 1.99-1.94 (m, 2H)

### Example 160: N-(3-Fluoro-4-((7-(3-(4-methylpiperazin-1-yl)propoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4-chloro-7-hydroxyquinoline was used as a starting material in step 1 and 4-methylpiperazine was used as a starting material in step 7.

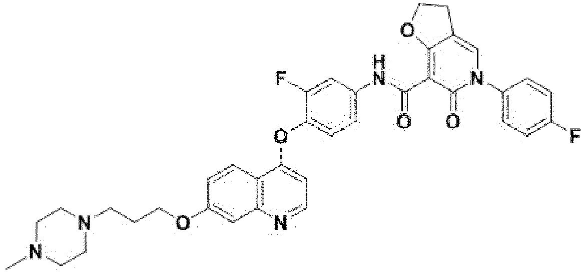

¹H NMR (500 MHz, CDCl₃) δ 12.20 (s, 1H), 8.59 (d, J = 6.5 Hz, 1H), 8.39 (d, J = 9.5 Hz, 1H), 8.12 (m, 2H), 7.41-7.35 (m, 5H), 7.27-7.23 (m, 2H), 7.23 (m, 2H), 7.21 (t, J = 8.5 Hz, 1H), 6.72 (d, J = 6.5 Hz, 1H), 5.02 (t, J = 8.5 Hz, 2H), 4.41 (t, J = 6.0 Hz, 2H), 3.35 (m, 2H) 3.22 (m, 2H), 2.93 (m, 4H), 2.85 (s, 3H), 2.67 (m, 2H), 2.05 (m, 2H)

### Example 161: 7-(2-(3-Fluoro-4-((7-(3-(piperidin-1-yl)propoxy)quinolin-4-yl)oxy)phenyl)acetyl)-5-(4-fluorophenyl)-3,5-dihydrofuro[3,2-c]pyridin-6(2H)-one

The title compound (white solid) was obtained using the same synthetic route as in Example 76, except that 4-chloro-7-hydroxyquinoline was used as a starting material in step 1 and piperidine was used as a starting material in step 7.

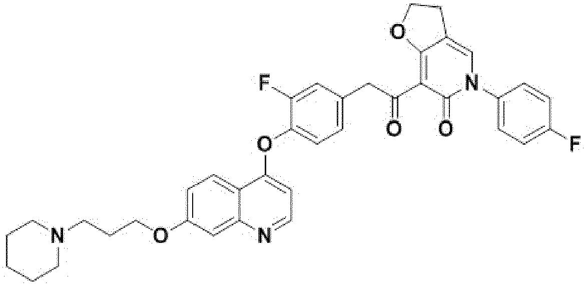

¹H NMR (500 MHz, DMSO-d₆) δ 8.61 (d, J = 5.0 Hz, 1H), 8.24 (d, J = 9.0 Hz, 1H), 8.01-7.98 (dd, J = 13.0, 1.5 Hz, 1H), 7.88 (s, 1H), 7.52-7.49 (m, 2H), 7.43-7.36 (m, 5H) 7.31-7.28 (dd, J = 9.5, 2.5 Hz, 1H), 6.48 (d, J = 5.0 Hz, 1H), 4.84 (t, J = 8.5 Hz, 2H), 4.23 (bs, 2H), 3.50 (bs, 2H), 3.10 (t, J = 8.0 Hz, 2H), 2.90 (bs, 3H), 2.19 (bs, 3H), 1.66 (bs, 5H), 1.40 (bs, 1H)

### Example 162: 7-(2-(3-Fluoro-4-((7-(3-(4-methylpiperidin-1-yl)propoxy)quinolin-4-yl)oxy)phenyl)acetyl)-5-(4-fluorophenyl)-3,5-dihydrofuro[3,2-c]pyridin-6(2H)-one

The title compound (white solid) was obtained using the same synthetic route as in Example 76, except that 4-chloro-7-hydroxyquinoline was used as a starting material in step 1 and 4-methylpiperidine was used as a starting material in step 7.

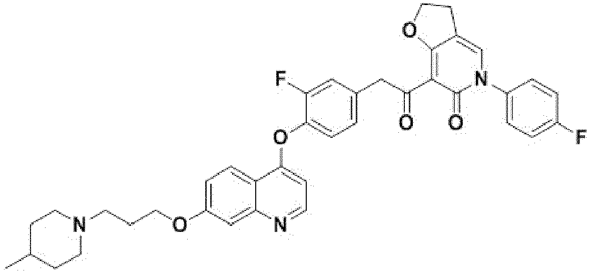

¹H NMR (500 MHz, DMSO-d₆) δ 8.61 (d, J = 5.5 Hz, 1H), 8.25 (d, J = 9.5 Hz, 1H), 8.01-7.98 (dd, J = 13.0, 5.5 Hz, 1H), 7.88 (s, 1H), 7.52-7.49 (dd, J = 9.0, 5.0 Hz, 2H), 7.44-7.42 (m, 2H), 7.40-7.36 (m, 3H), 7.31-7.28 (dd, J =9.0, 2.0 Hz, 1H), 6.49 (d, J = 5.0 Hz, 1H), 4.86 (t, J = 8.5 Hz, 2H), 4.24 (s, 2H), 3.51 (s, 2H), 2.93 (s, 2H), 2.22 (s, 3H), 1.79 (s, 3H), 1.60 (s, 2H), 1.36 (s, 2H), 0.92 (d, J = 5.5 Hz, 3H)

### Example 163: N-(3-Fluoro-4-((6-(methylcarbamoyl)-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4-chloro-7-hydroxy-N-methylquinoline-6-carboxamide of Preparation Example 20 was used as a starting material in step 1 and 1,2-dibromoethane was used as a starting material in step 6.

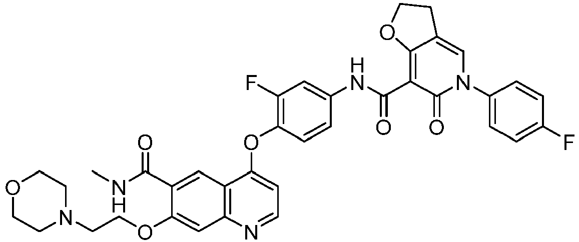

¹H NMR (400 MHz, CDCl₃) δ 12.04 (s, 1H), 9.28 (s, 1H), 8.63 (d, J = 5.6 Hz, 1H), 8.56 (d, J = 4.0 Hz, 1H), 8.00 (dd, J = 12.4, 2.4 Hz, 1H), 7.45 (s, 1H), 7.38-7.34 (m, 4H), 7.24-7.22 (m, 2H), 7.15 (t, J = 8.8 Hz, 1H), 6.43 (d, J = 5.6 Hz, 1H), 4.99 (t, J = 8.4 Hz, 2H), 4.36 (t, J = 4.8 Hz, 2H), 3.76 (t, J = 4.8 Hz, 4H), 3.18 (t, J = 8.8 Hz, 2H), 3.09 (d, J = 4.8 Hz, 3H), 2.92 (t, J = 5.2 Hz, 2H), 2.58 (s, 4H)

### Example 164: N-(3-Fluoro-4-((6-(methylcarbamoyl)-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4-chloro-7-hydroxy-N-methylquinoline-6-carboxamide of Preparation Example 20 was used as a starting material in step 1.

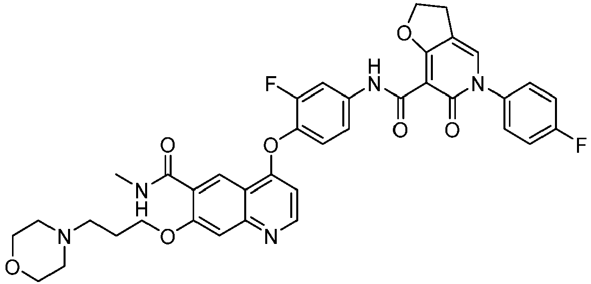

¹H NMR (500 MHz, DMSO-d₆) δ 11.92 (s, 1H), 8.66 (d, J = 5.5 Hz, 1H), 8.53 (s, 1H), 8.27 (bs, 1H), 8.02-7.99 (dd, J = 2.5 Hz, J = 12.0 Hz, 1H), 7.88 (s, 1H), 7.52-7.49 (m, 3H), 7.46-7.42 (m, 2H), 7.40-7.36 (m, 2H), 6.52 (d, J = 5.0 Hz, 1H), 4.85 (t, J = 8.0 Hz, 2H), 4.29 (bs, 2H), 3.59 (bs, 4H), 3.10 (t, J = 8.5 Hz, 2H), 2.85 (d, J = 4.5 Hz, 3H), 2.38 (bs, 4H), 2.01 (bs, 2H), 1.24 (d, J = 6.0 Hz, 2H)

### Example 165: N-(3-Fluoro-4-((6-methoxy-7-(methylcarbamoyl)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4-chloro-6-methoxy-N-methylquinoline-7-carboxamide of Preparation Example 21 was used as a starting material in step 1.

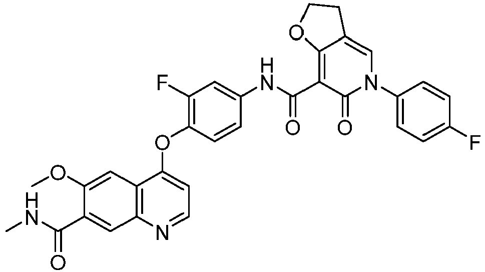

¹H NMR (400 MHz, DMSO-d₆) δ 11.91 (s, 1H), 8.59 (d, J = 5.2 Hz, 1H), 8.40-8.39 (m, 1H), 8.20 (s, 1H), 8.00-8.03 (m, 1H), 7.88 (s, 1H), 7.67 (s, 1H), 7.53-7.50 (m, 2H), 7.45-7.44 (m, 2H), 7.41-7.37 (m, 2H), 6.65 (d, J = 5.2 Hz, 1H), 4.85 (t, J = 8.4 Hz, 2H), 4.01 (s, 3H), 3.11 (t, J = 8.0 Hz, 2H), 2.84 (d, J = 5.2 Hz, 3H)

### Example 166: N-(3-Fluoro-4-((7-(methylcarbamoyl)-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4-chloro-6-hydroxy-N-methylquinoline-7-carboxamide of Preparation Example 22 was used as a starting material in step 1.

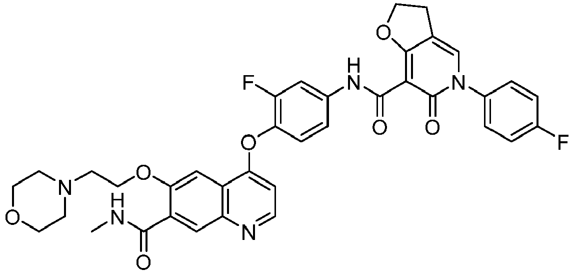

¹H NMR (400 MHz, DMSO-d₆) δ 11.91 (s, 1H), 8.64-8.62 (m, 1H), 8.60 (d, J = 4.8 Hz, 1H), 8.36 (s, 1H), 8.03-8.00 (m, 1H), 7.88 (s, 1H), 7.75 (s, 1H), 7.53-7.49 (m, 2H), 7.45-7.37 (m, 4H), 6.65 (d, J = 5.2 Hz, 1H), 4.85 (t, J = 8.4 Hz, 2H), 4.41 (t, J = 5.2 Hz, 2H), 3.61 (t, J = 4.8 Hz, 4H), 3.11 (t, J = 7.6 Hz, 2H), 2.91 (d, J = 4.8 Hz, 3H), 2.82 (s, 2H), 2.5 (m, 4H, overlapped with DMSO)

### Example 167: N-(3-Fluoro-4-((6-fluoro-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4-chloro-6-fluoroquinolin-7-ol of Preparation Example 23 was used as a starting material in step 1 and 1,2-dibromoethane was used as a starting material in step 6.

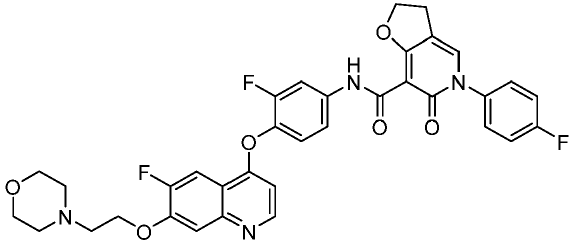

¹H NMR (400 MHz, DMSO-d₆) δ 11.91 (s, 1H), 8.61 (d, J = 5.6 Hz, 1H), 8.02 (d, J = 11.2 Hz, 1H), 7.98 (d, J = 2.4 Hz, 1H), 7.88 (s, 1H),7.66 (d, J = 8.4 Hz, 1H), 7.52-7.49 (m, 2H), 7.45-7.36 (m, 4H), 6.54 (d, J = 4.8 Hz, 1H), 4.84 (t, J = 8.0 Hz, 2H), 4.36 (t, J = 5.6 Hz, 2H), 3.59 (t, J = 4.4 Hz, 4H), 3.10 (t, J = 8.0 Hz, 2H), 2.81 (t, J = 5.6 Hz, 2H), 2.51-2.49 (m, 4H, overlapped with DMSO)

### Example 168: N-(3-Fluoro-4-((6-fluoro-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4-chloro-6-fluoroquinolin-7-ol of Preparation Example 23 was used as a starting material in step 1.

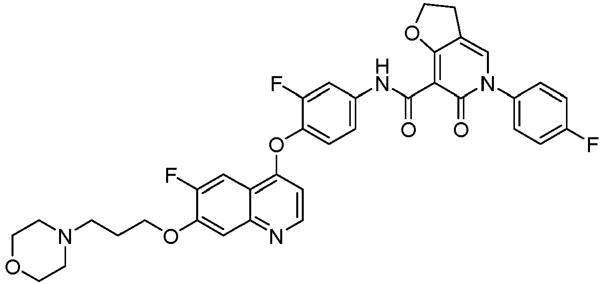

¹H NMR (400 MHz, DMSO-d₆) δ 11.91 (s, 1H), 8.61 (d, J = 5.6 Hz, 1H), 8.01 (d, J = 11.2 Hz, 1H), 7.98 (d, J = 2.4 Hz, 1H), 7.87 (s, 1H), 7.62 (d, J = 8.4 Hz, 1H), 7.52-7.50 (m, 2H), 7.45-7.36 (m, 4H), 7.62 (d, J = 5.2 Hz, 1H), 4.84 (t, J = 8.0 Hz, 2H), 4.29 (t, J = 6.0 Hz, 2H), 3.58 (t, J = 4.4 Hz, 4H), 3.10 (t, J = 8.0 Hz, 2H), 2.50-2.46 (m, 4H, overlapped with DMSO), 2.39 (bs, 2H), 1.99 (t, J = 6.8 Hz, 2H)

### Example 169: N-(3-Fluoro-4-((7-(2-morpholinoethoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 4-chloro-6-(trifluoromethyl)quinolin-7-ol of Preparation Example 24 was used as a starting material in step 1 and 1,2-dibromoethane was used as a starting material in step 6.

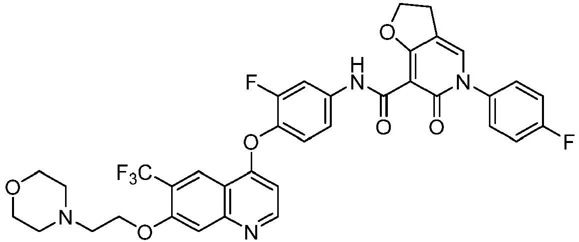

¹H NMR (400 MHz, DMSO-d₆) δ 11.92 (s, 1H), 8.75 (d, J = 5.2 Hz, 1H), 8.55 (s, 1H), 8.03 (d, J = 14.4 Hz, 1H), 7.88 (s, 1H), 7.70 (s, 1H), 7.53-7.48 (m, 2H), 7.46-7.45 (m, 2H), 7.40-7.36 (m, 2H), 6.60 (d, J = 5.2 Hz, 1H), 4.85 (t, J = 8.4 Hz, 2H), 4.41 (t, J = 5.6 Hz, 2H), 3.57 (t, J = 4.4 Hz, 4H), 3.10 (t, J = 8.0 Hz, 2H), 2.80 (t, J = 5.2 Hz, 2H), 2.52-2.49 (m, 4H, overlapped with DMSO)

### Example 170: N-(3-Fluoro-4-((7-(3-morpholinopropoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 4-chloro-6-(trifluoromethyl)quinolin-7-ol of Preparation Example 24 was used as a starting material in step 1.

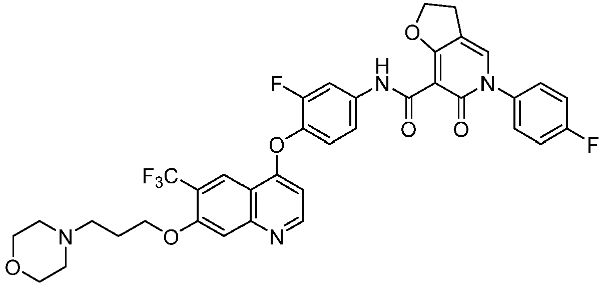

¹H NMR (400 MHz, DMSO-d₆) δ 11.92 (s,1H), 8.75 (d, J = 5.6 Hz, 1H), 8.55 (s,1H), 8.03 (d, J = 14.4 Hz, 1H), 7.88 (s, 1H), 7.66 (s, 1H), 7.53-7.50 (m, 2H), 7.46-7.45 (m, 2H), 7.40-7.36 (m, 2H), 6.60 (d, J = 4.8 Hz, 1H), 4.85 (t, J = 8.4 Hz, 2H), 4.33 (t, J = 5.6 Hz, 2H), 3.58 (t, J = 4.4 Hz, 4H), 3.10 (t, J = 8.0 Hz, 2H), 2.38 (bs, 4H), 1.97 (t, J = 6.8 Hz, 2H), 1.35 (d, J = 6.4 Hz, 2H)

### Example 171: N-(4-((6-Chloro-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4,6-dichloroquinolin-7-ol of Preparation Example 25 was used as a starting material in step 1 and 1,2-dibromoethane was used as a starting material in step 6.

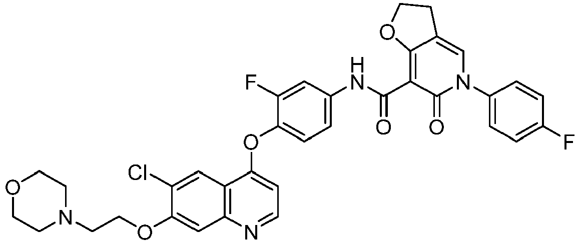

¹H NMR (400 MHz, DMSO-d₆) δ 11.92 (s, 1H), 8.64 (d, J = 5.6 Hz, 1H), 8.32 (s, 1H), 8.00 (dd, J = 12.4, 1.6 Hz, 1H), 7.88 (br, 1H), 7.62 (s, 1H), 7.53-7.49 (m, 2H), 7.44-7.36 (m, 4H), 6.53 (d, J = 5.2 Hz, 1H), 4.85 (t, J = 8.4 Hz, 2H), 4.38 (t, J = 5.6 Hz, 2H), 3.59 (t, J = 4.4 Hz, 4H), 3.11 (t, J = 8.0 Hz, 2H), 2.83 (t, J = 5.6 Hz, 2H), 2.56 (t, J = 4.4 Hz, 4H)

### Example 172: N-(4-((6-Chloro-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 76, except that 4,6-dichloroquinolin-7-ol of Preparation Example 25 was used as a starting material in step 1.

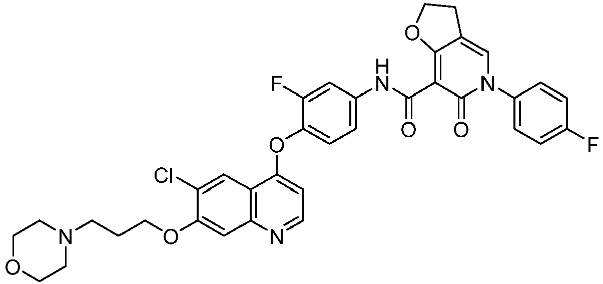

¹H NMR (400 MHz, DMSO-d₆) δ 11.92 (s, 1H), 8.63 (d, J = 4.8 Hz, 1H), 8.33 (s, 1H), 8.00 (dd, J = 13.2,1.6 Hz, 1H), 7.88 (s, 1H), 7.58 (s, 1H), 7.53-7.49 (m, 2H), 7.44-7.36 (m, 4H), 6.53 (d, J = 4.8 Hz, 1H), 4.85 (t, J = 8.4 Hz, 2H), 4.30 (t, J = 6.4 Hz, 2H), 3.58 (t, J = 4.8 Hz, 4H), 3.39-3.35 (br, 2H), 3.11 (t, J = 7.6 Hz, 2H), 2.39 (br, 4H), 2.03-1.97 (m, 2H)

### Example 173: N-(4-((6-Cyano-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4-chloro-7-hydroxyquinoline-6-carbonitrile of Preparation Example 26 was used as a starting material in step 1 and 1,2-dibromoethane was used as a starting material in step 6.

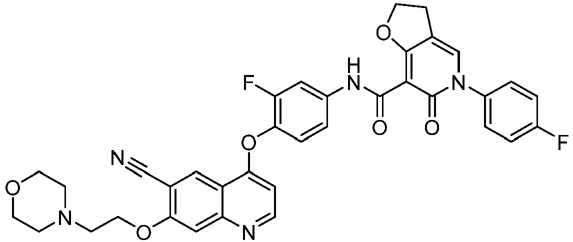

¹H NMR (400 MHz, DMSO-d₆) δ 11.93 (s, 1H), 8.80 (s, 1H), 8.75 (d, J = 5.6 Hz, 1H), 8.04-8.00 (m, 1H), 7.88 (s, 1H), 7.67 (s, 1H), 7.53-7.50 (m, 2H), 7.45-7.44 (m, 2H), 7.39 (t, J = 8.4 Hz, 2H), 6.60 (d, J = 5.2 Hz, 1H), 4.85 (t, J = 8.4 Hz, 2H), 4.43 (t, J = 5.2 Hz, 2H), 3.59 (t, J = 4.8 Hz, 4H), 3.11 (t, J = 8.4 Hz, 2H), 2.83 (t, J = 5.2 Hz, 2H), 2.56 (t, J = 4.4 Hz, 4H)

### Example 174: N-(4-((6-Cyano-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4-chloro-7-hydroxyquinoline-6-carbonitrile of Preparation Example 26 was used as a starting material in step 1.

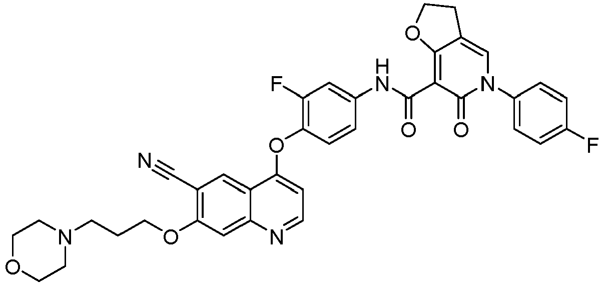

¹H NMR (400 MHz, CDCl₃) δ 12.09 (s, 1H), 8.70 (s, 1H), 8.67 (d, J = 5.2 Hz, 1H), 8.03 (dd, J = 12.8, 2.0 Hz, 1H), 7.47 (s, 1H), 7.37-7.35 (m, 4H), 7.25-7.23 (m, 2H), 7.15 (t, J = 8.8 Hz, 1H), 6.46 (d, J = 5.6 Hz, 1H), 4.99 (t, J = 8.4 Hz, 2H), 4.29 (t, J = 6.0 Hz, 2H), 3.73 (t, J = 4.0 Hz, 4H), 3.19 (t, J = 7.2 Hz, 2H), 2.63 (t, J = 6.8 Hz, 2H), 2.52 (s, 4H), 2.14-2.11 (m, 2H)

### Example 175: N-(3-Fluoro-4-((7-methoxy-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4-chloro-7-methoxyquinolin-6-ol of Preparation Example 27 was used as a starting material in step 1 and 1,2-dibromoethane was used as a starting material in step 6.

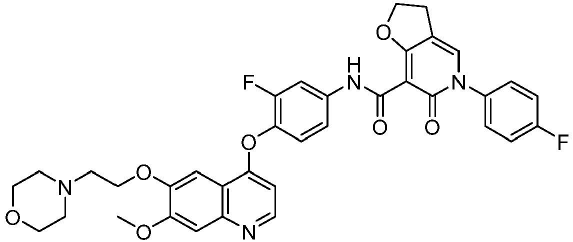

¹H NMR (400 MHz, DMSO-d₆) δ 11.89 (s, 1H), 8.47 (d, J = 5.2 Hz, 1H), 8.01 (dd, J = 13.2,2.4 Hz, 1H), 7.88 (s, 1H), 7.59 (s, 1H), 7.53-7.49 (m, 2H), 7.43-7.36 (m, 5H), 6.46 (d, J = 4.4 Hz, 1H), 4.85 (t, J = 8.0 Hz, 2H), 4.26 (t, J = 6.0 Hz, 2H), 3.95 (s, 3H), 3.57 (t, J = 4.8 Hz, 4H), 3.11 (t, J = 8.0 Hz, 2H), 2.77 (t, J = 6.0 Hz, 2H), 2.5 (m, 4H, overlapped with DMSO)

### Example 176: N-(3-Fluoro-4-((7-methoxy-6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 76, except that 4-chloro-7-methoxyquinolin-6-ol of Preparation Example 27 was used as a starting material in step 1.

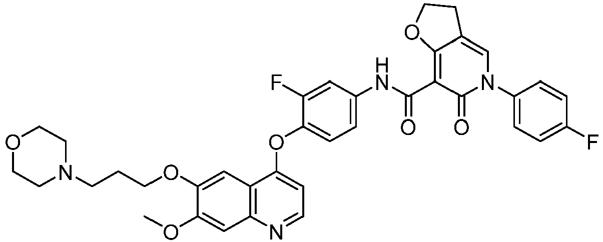

¹H NMR (400 MHz, DMSO-d₆) δ 11.89 (s, 1H), 8.47 (d, J = 5.2 Hz, 1H), 8.00 (d, J = 12.8 Hz, 1H), 7.88 (s, 1H), 7.53-7.37 (m, 8H), 6.46 (d, J = 5.2 Hz, 1H), 4.85 (t, J = 8.4 Hz, 2H), 4.19 (t, J = 6.4 Hz, 2H), 3.95 (s, 3H), 3.56 (s, 4H), 3.11 (t, J = 8.0 Hz, 2H), 2.44 (t, J = 6.8 Hz, 2H), 2.38 (s, 4H), 1.97 (t, J = 6.8 Hz, 2H)

### Example 177: N-(3-Fluoro-4-((6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide

The title compound (brown solid) was obtained using the same synthetic route as in Example 76, except that 4-chloro-6-hydroxyquinoline was used as a starting material in step 1 and 1,2-dibromoethane was used as a starting material in step 6.

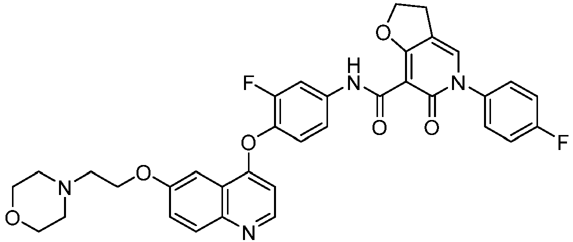

¹H NMR (500 MHz, DMSO-d₆) δ 11.90 (s, 1H), 8.53 (d, J = 5.0 Hz, 1H), 8.01 (dd, J = 13.5, 2.5 Hz, 1H), 7.94 (d, J = 9.5 Hz, 1H), 7.88 (s, 1H), 7.62 (d, J = 3.0 Hz, 1H), 7.53-7.37 (m, 7H), 6.59 (d, J = 5.0 Hz, 1H), 4.85 (t, J = 8.0 Hz, 2H), 4.27 (t, J = 6.0 Hz, 2H), 3.58 (t, J = 4.5 Hz, 4H), 3.11 (t, J = 8.0 Hz, 2H), 2.77 (t, J = 5.5 Hz, 2H), 2.5 (m, 4H, overlapped with DMSO)

### Example 178: N-(3-Fluoro-4-((6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide

The title compound (brown solid) was obtained using the same synthetic route as in Example 76, except that 4-chloro-6-hydroxyquinoline was used as a starting material in step 1.

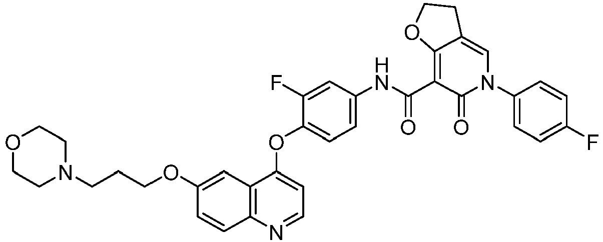

¹H NMR (500 MHz, DMSO-d₆) δ 11.90 (s, 1H), 8.53 (d, J = 5.0 Hz, 1H), 8.02-7.99 (m, 1H), 7.94 (d, J = 9.0 Hz, 1H), 7.88 (t, J = 1.5 Hz, 1H), 7.57 (d, J = 2.5 Hz, 1H), 7.53-7.50 (m, 2H), 7.47-7.37 (m, 5H), 6.59 (d, J = 5.0 Hz, 1H), 4.85 (t, J = 8.0 Hz, 2H), 4.20 (t, J = 6.5 Hz, 2H), 3.56 (t, J = 4.5 Hz, 4H), 3.11 (t, J = 7.5 Hz, 2H), 2.46 (t, J = 7.0 Hz, 2H), 2.38 (s, 4H), 1.96 (m, 2H)

### Example 179: N-(4-((6-Amino-7-methoxyquinolin-4-yl)oxy)-3-fluorophenyl)-5-(4-fluorophenyl)-6-oxo-2,3,5,6-tetrahydrofuro[3,2-c]pyridine-7-carboxamide

### Step 1: Synthesis of 3-fluoro-4-((7-methoxy-6-nitroquinolin-4-yl)oxy)aniline

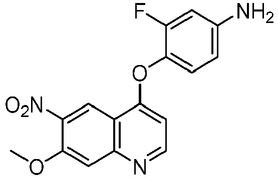

4-Amino-2-fluorophenol (220 mg, 1.73 mmol) was dissolved in dimethylformamide (3.0 ml). Then, 1 M potassium t-butoxide (2.16 ml, 2.16 mmol) was added thereto, and stirring was performed at room temperature for 30 minutes. After 30 minutes, 4-chloro-7-methoxy-6-nitroquinoline (340 mg, 1.44 mmol) of Preparation Example 28 was added thereto, and stirring was performed at a temperature of 90°C for 1 hour. The reaction mixture was extracted with water and ethyl acetate. The separated organic layer was dried over anhydrous sodium sulfate, filtered, and concentrated under reduced pressure. The resulting residue was purified by column chromatography to obtain the title compound (240 mg, yield: 50%, yellow solid).

¹H NMR (400 MHz, DMSO-d₆) δ 8.78 (s, 1H),8.77 (d, J = 5.6 Hz, 1H), 7.72 (s, 1H), 7.11 (t, J = 9.2 Hz, 1H), 6.57-6.54 (m, 2H), 6.48-6.45 (dd, J = 2.0 Hz, J = 8.8 Hz, 1H), 5.55 (s, 2H), 4.06 (s, 3H)

### Step 2: Synthesis of N-(3-fluoro-4-((7-methoxy-6-nitroquinolin-4-yl)oxy)phenyl)-5-(4-fluorophenyl)-6-oxo-2,3,5,6-tetrahydrofuro[3,2-c]pyridine-7-carboxamide

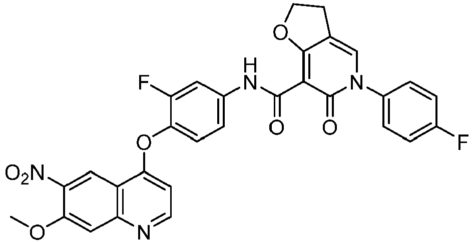

The carboxylic acid compound (333 mg, 1.21 mmol) prepared in Preparation Example 1 and 1-[bis(dimethylamino)methylene]-1H-1,2,3-triazolo[4,5-b]pyridinium-3-oxide hexafluorophosphate (HATU, 460 mg, 1.21 mmol) were dissolved in dimethylformamide (2 ml). Then, N,N-diisopropyl ethylamine (0.54 ml, 3.34 mmol) and 3-fluoro-4-((7-methoxy-6-nitroquinolin-4-yl)oxy)aniline (200 mg, 0.6 mmol) prepared in step 1 were sequentially added thereto, and stirring was performed at room temperature for 24 hours. The reaction was quenched with saturated aqueous sodium bicarbonate solution, and extraction was performed with ethyl acetate. Then, the separated organic layer was dried over anhydrous magnesium sulfate and concentrated under reduced pressure. The resulting residue was purified by column chromatography to obtain the title compound (200 mg, yield: 56%, yellow solid).

¹H NMR (400 MHz, DMSO-d₆) δ 11.9 (s, 1H), 8.82 (s, 1H), 8.78 (d, J = 5.2 Hz, 1H), 8.04 (d, J = 14.4 Hz, 1H), 7.87 (s, 1H), 7.75 (s, 1H), 7.52-7.49 (m, 2H), 7.47-7.45 (m, 2H), 7.40-7.36 (m, 2H), 6.64 (d, J = 5.6 Hz, 1H), 4.85 (t, J = 8.8 Hz, 2H), 4.07 (s, 3H), 3.10 (t, J = 8.0 Hz, 2H)

### Step 3: Synthesis of N-(4-((6-amino-7-methoxyquinolin-4-yl)oxy)-3-fluorophenyl)-5-(4-fluorophenyl)-6-oxo-2,3,5,6-tetrahydrofuro[3,2-c]pyridine-7-carboxamide

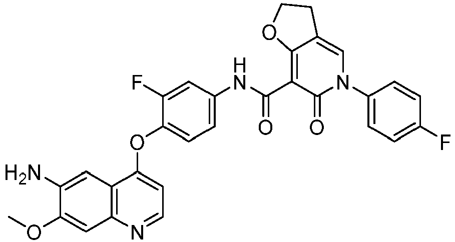

N-(3-Fluoro-4-((7-methoxy-6-nitroquinolin-4-yl)oxy)phenyl)-5-(4-fluorophenyl)-6-oxo-2,3,5,6-tetrahydrofuro[3,2-c]pyridine-7-carboxamide (50 mg, 0.08 mmol) prepared in step 2 was dissolved in ethanol (0.6 ml) and water (0.3 ml). Then, iron (14.2 mg, 0.25 mmol) and ammonium chloride (45.6 mg, 0.85 mmol) were sequentially added thereto at room temperature. The temperature was raised to a temperature of 90°C, and stirring was performed for 5 hours. After completion of the reaction, the residue was filtered using a Celite pad and concentrated under reduced pressure. The resulting residue was extracted with dichloromethane. The separated organic layer was concentrated under reduced pressure. The resulting residue was purified by column chromatography to obtain the title compound (41 mg, yield: 86%, yellow solid).

¹H NMR (400 MHz, DMSO-d₆) δ 11.8 (s, 1H), 8.27 (d, J = 5.2 Hz, 1H), 7.98-7.95 (dd, J = 2.0 Hz, J = 13.2 Hz, 1H), 7.87 (s, 1H), 7.52-7.49 (m, 2H), 7.40-7.30 (m, 4H), 7.25 (s, 2H), 6.35 (d, J = 4.8 Hz, 1H), 5.49 (s, 2H), 4.84 (t, J = 8.0 Hz, 2H), 3.92 (s, 3H), 3.12 (t, J = 7.6 Hz, 2H)

### Example 180: N-(3-Fluoro-4-((7-methoxy-6-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide

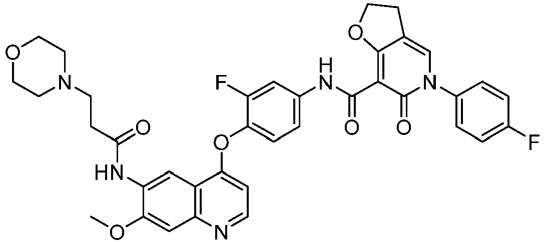

3-Morpholinopropanoic acid (14.16 mg, 0.08 mmol) and 1-[bis(dimethylamino)methylene]-1H-1,2,3-triazolo[4,5-b]pyridinium-3-oxide hexafluorophosphate (HATU, 33.8 mg, 0.08 mmol) were dissolved in dimethylformamide (0.2 ml). Then, N,N-diisopropyl ethylamine (0.03 ml, 0.21 mmol) and N-(4-((6-amino-7-methoxyquinolin-4-yl)oxy)-3-fluorophenyl)-5-(4-fluorophenyl)-6-oxo-2,3,5,6-tetrahydrofuro[3,2-c]pyridine-7-carboxamide (20 mg, 0.03 mmol) prepared in Example 179 were sequentially added thereto, and stirring was performed at room temperature for 24 hours or longer. The reaction was quenched with saturated aqueous sodium bicarbonate solution, and extraction was performed with ethyl acetate. The separated organic layer was dried over anhydrous magnesium sulfate and concentrated under reduced pressure. The resulting residue was purified by column chromatography and preparative thin layer chromatography (PTLC) to obtain the title compound (5 mg, yield: 24%, white solid).

¹H NMR (400 MHz, MeOD-d₆) δ 9.25 (s, 1H), 8.48 (d, J = 5.6 Hz, 1H), 8.01-7.97 (dd, J = 2.0 Hz, J = 12.8Hz, 1H), 7.73 (s,1H), 7.49-7.46 (m, 2H), 7.44 (s, 1H), 7.36-7.27 (m, 4H), 6.98-6.95 (dd, J = 1.2 Hz, J = 8.8Hz, 1H), 6.51 (d, J = 4.8 Hz, 1H), 4.95-4.89 (m, 2H, overlapped with DMSO), 4.12 (s, 3H), 3.81 (t, J = 4.4 Hz, 4H), 3.20 (t, J = 7.2 Hz, 2H), 2.79-2.69 (m, 4H), 2.61 (bs, 4H)

### Example 181: N-(4-((7-Amino-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

The title compound (pink solid) was obtained using the same synthetic route as in Example 179, except that 4-chloro-6-methoxy-7-nitroquinoline of Preparation Example 29 was used as a starting material in step 1.

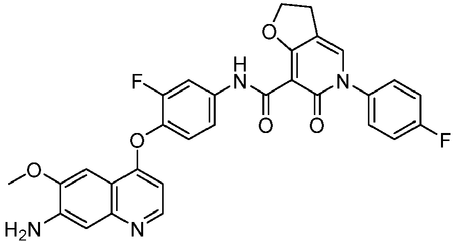

¹H NMR (400 MHz, CDCl₃) δ 12.02 (s, 1H), 8.41 (d, J = 5.6 Hz, 1H), 8.01-7.98 (dd, J = 2.0 Hz, J = 12.8 Hz, 1H), 7.49 (s, 1H), 7.37-7.33 (m, 4H), 7.24-7.21 (m, 3H), 7.13 (t, J = 8.4 Hz, 1H), 6.34 (d, J = 5.6 Hz, 1H), 4.98 (t, J = 8.4 Hz, 2H), 4.37 (bs, 2H), 4.02 (s, 3H), 3.18 (t, J = 8.8 Hz, 2H)

### Example 182: N-(3-Fluoro-4-((6-methoxy-7-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide

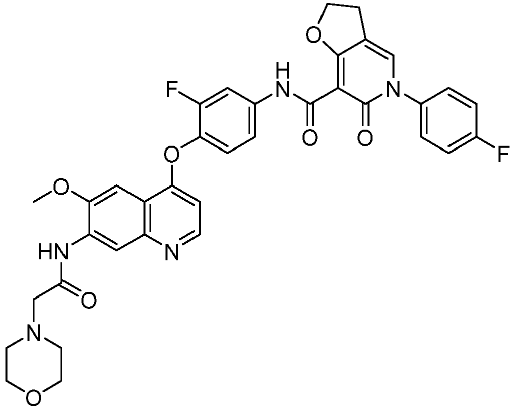

The title compound (yield: 60%, yellow solid) was obtained using the same synthetic route as in Example 180, except that N-(4-((7-amino-6-methoxyquinolin-4-yl)oxy)-3-fluorophenyl)-5-(4-fluorophenyl)-6-oxo-2,3,5,6-tetrahydrofuro[3,2-c]pyridine-7-carboxamide prepared in Example 181 and morpholin-4-yl-acetic acid were used as starting materials.

¹H NMR (400 MHz, CDCl₃) δ 12.09 (s, 1H, NH), 10.18 (s, 1H, NH), 9.11 (s, 1H), 8.56 (d, J = 5.6 Hz, 1H), 8.04 (dd, J = 12.8, 2.0 Hz, 1H),7.63 (s,1H), 7.37-7.35 (m, 5H), 7.17 (t, J = 8.8 Hz, 2H), 6.52 (d, J = 5.6 Hz, 1H), 4.98 (t, J = 8.4 Hz, 2H), 4.10 (s,3H), 3.83 (d, J = 3.2 Hz, 4H), 3.24 (s, 2H), 3.18 (t, J = 8.0 Hz, 2H), 2.69 (d, J = 3.2 Hz, 4H)

### Example 183: N-(3-Fluoro-4-((6-methoxy-7-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide

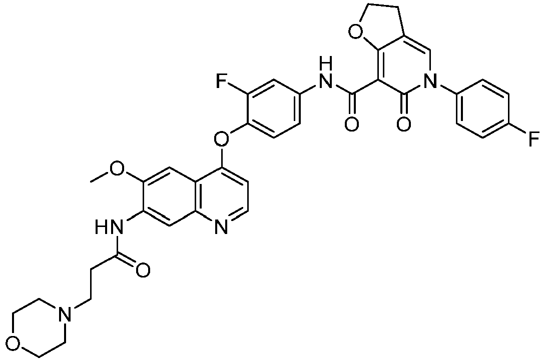

The title compound (yield: 60%, yellow solid) was obtained using the same synthetic route as in Example 180, except that N-(4-((7-amino-6-methoxyquinolin-4-yl)oxy)-3-fluorophenyl)-5-(4-fluorophenyl)-6-oxo-2,3,5,6-tetrahydrofuro[3,2-c]pyridine-7-carboxamide prepared in Example 181 was used as a starting material.

¹H NMR (400 MHz, CDCl₃) δ 12.04 (s, 1H, NH), 10.66 (s, 1H, NH), 9.14 (s, 1H), 8.54 (d, J = 5.2 Hz, 1H), 8.02 (dd, J = 12.8, 2.0 Hz, 1H),7.60 (s, 1H), 7.37-7.36 (m, 4H), 7.24-7.20 (m, 2H), 7.15 (t, J = 8.4 Hz, 1H), 6.44 (d, J = 5.2 Hz, 1H), 4.98 (t, J = 8.0 Hz, 2H), 4.07 (s, 3H), 3.85 (t, J = 4.4 Hz, 4H), 3.17 (t, J = 8.4 Hz, 2H), 3.17 (t, J = 5.2 Hz, 2H), 2.67-2.62 (m, 6H)

### Example 184: N-(4-((7-Acetamido-6-methoxyquinolin-4-yl)oxy)-3-fluorophenyl)-5-(4-fluorophenyl)-6-oxo-2,3,5,6-tetrahydrofuro[3,2-c]pyridine-7-carboxamide

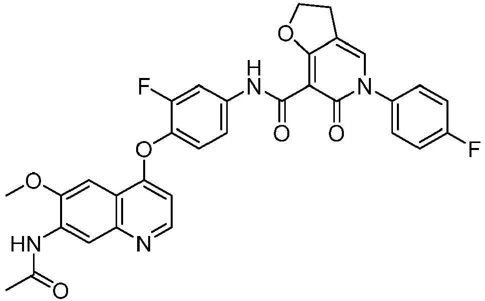

The title compound (yield: 60%, yellow solid) was obtained using the same synthetic route as in Example 180, except that N-(4-((7-amino-6-methoxyquinolin-4-yl)oxy)-3-fluorophenyl)-5-(4-fluorophenyl)-6-oxo-2,3,5,6-tetrahydrofuro[3,2-c]pyridine-7-carboxamide prepared in Example 181 and acetic anhydride were used as starting materials.

¹H NMR (400 MHz, DMSO-d₆) δ 11.89 (s, 1H, NH), 9.50 (s, 1H, NH), 8.78 (s, 1H), 8.50 (d, J = 5.2 Hz, 1H), 8.01 (dd, J = 13.2,\ 2.0 Hz, 1H),7.87 (s, 1H), 7.59 (s, 1H), 7.52-7.49 (m, 2H), 7.45-7.36 (m, 4H), 6.50 (d, J = 5.2 Hz, 1H), 4.85 (t, J = 8.4 Hz, 2H), 4.04 (s, 3H), 3.10 (t, J = 7.6 Hz, 2H), 2.21 (s, 3H)

### Example 185: N-(3-Fluoro-4-((7-methoxy-6-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide

### Step 1: Synthesis of 6-bromo-4-(2-fluoro-4-nitrophenoxy)-7-methoxyquinoline

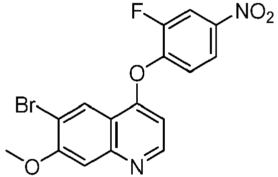

6-Bromo-4-chloro-7-methoxyquinoline (400 mg, 1.46 mmol) of Preparation Example 30 was dissolved in chlorobenzene (10 ml). Then, 2-fluoro-4-nitrophenol (461 mg, 2.930 mmol) was sequentially added thereto, and stirring was performed overnight at a temperature of 140°C. The reaction mixture was cooled to room temperature, and extracted with ethyl acetate. The separated organic layer was dried over anhydrous sodium sulfate, filtered, and concentrated under reduced pressure. The resulting residue was purified by column chromatography to obtain the title compound (220 mg, yield: 38%, yellow solid).

¹H NMR (400 MHz, CD₃OD) δ 8.66 (d, J = 4.8 Hz, 1H), 8.56 (s, 1H), 8.35-8.32 (dd, J = 2.4 Hz, J = 10.4Hz, 1H), 8.26 (d, J = 9.2 Hz, 1H), 7.64 (d, J = 8.4 Hz, 1H), 7.48 (s, 1H), 6.72 (d, J = 5.2 Hz, 1H), 4.07 (s, 3H)

### Step 2: Synthesis of 4-(2-fluoro-4-nitrophenoxy)-7-methoxy-N-(2-morpholinoethyl)quinolin-6-amine

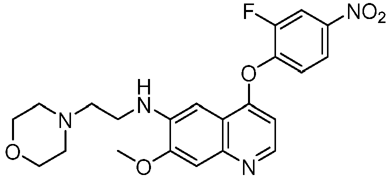

6-Bromo-4-(2-fluoro-4-nitrophenoxy)-7-methoxyquinoline (100 mg, 0.25 mmol) prepared in step 1, tris(dibenzylideneacetone)dipalladium (0) (2.3 mg, 0.002 mmol), and rac-BINAP (4.7 mg, 0.007 mmol) were mixed. The mixture was dissolved in toluene (3 ml) under nitrogen gas. 4-(2-Aminoethyl)morpholine (0.04 ml, 0.30 mmol) was added thereto, and stirring was performed at a temperature of 90°C for 4 hours. The reaction mixture was cooled to room temperature, and extracted with ethyl acetate. The separated organic layer was dried over anhydrous sodium sulfate, filtered, and concentrated under reduced pressure. The resulting residue was purified by column chromatography to obtain the title compound (90 mg, yield: 81%, yellow solid).

¹H NMR (400 MHz, CD₃OD) δ 8.34 (d, J = 5.6 Hz, 1H), 8.31-8.28 (dd, J = 2.0 Hz, J = 10.0 Hz, 1H), 8.18-8.15 (m, 1H), 7.42 (t, J = 8.0 Hz, 1H), 7.27 (s, 1H), 6.98 (s, 1H), 6.71 (d, J = 4.8 Hz, 1H), 4.06 (s, 3H), 3.71 (t, J = 4.8 Hz, 4H), 3.36 (t, J = 6.4 Hz, 2H), 2.69 (t, J = 6.4 Hz, 2H), 2.51 (bs, 4H)

### Step 3: Synthesis of 4-(4-amino-2-fluorophenoxy)-7-methoxy-N-(2-morpholinoethyl)quinolin-6-amine

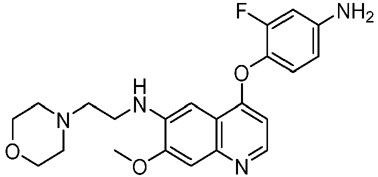

4-(2-Fluoro-4-nitrophenoxy)-7-methoxy-N-(2-morpholinoethyl)quinolin-6-amine (90 mg, 0.20 mmol) prepared in step 2 was dissolved in ethanol (1.0 ml) and water (0.5 ml). Then, iron (34 mg, 0.60 mmol) and ammonium chloride (107 mg, 2.00 mmol) were sequentially added thereto at room temperature. The temperature was raised to a temperature of 80°C, and stirring was performed for 2 hours and 30 minutes. After completion of the reaction, the reaction mixture was filtered using a Celite pad and concentrated under reduced pressure. The resulting residue was extracted with dichloromethane. The separated organic layer was concentrated under reduced pressure. The resulting residue was purified by column chromatography to obtain the title compound (63 mg, yield: 76%, yellow solid).

¹H NMR (400 MHz, CD₃OD) δ 8.23 (d, J = 5.2 Hz, 1H), 7.22 (s, 1H), 7.17 (s, 1H), 7.01 (t, J = 8.8 Hz, 1H), 6.63-6.59 (dd, J = 2.4 Hz, J = 12.8 Hz, 1H), 6.57-6.54 (dd, J = 2.4 Hz, J = 8.0 Hz, 1H), 6.40 (d, J = 5.2 Hz, 1H), 4.04 (s,3H), 3.72 (t, J = 4.8 Hz, 4H), 3.41 (t, J = 6.4 Hz, 2H), 2.74 (t, J = 6.4 Hz, 2H), 2.55 (bs, 4H)

### Step 4: Synthesis of N-(3-fluoro-4-((7-methoxy-6-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide

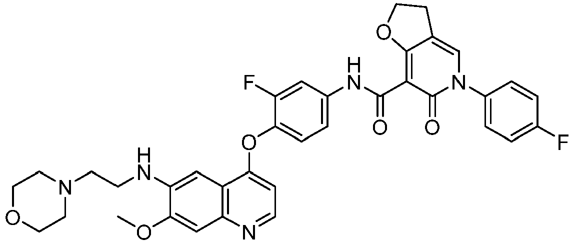

The carboxylic acid compound of Preparation Example 1 (26 mg, 0.09 mmol) and 1-[bis(dimethylamino)methylene]-1H-1,2,3-triazolo[4,5-b]pyridinium-3-oxide hexafluorophosphate (HATU, 37 mg, 0.09 mmol) were dissolved in dimethylformamide (0.2 ml). Then, N,N-diisopropyl ethylamine (0.04 ml, 0.26 mmol) and 4-(4-amino-2-fluorophenoxy)-7-methoxy-N-(2-morpholinoethyl)quinolin-6-amine (20 mg, 0.04 mmol) prepared in step 3 were sequentially added thereto, and stirring was performed at room temperature for 24 hours. The reaction was quenched with saturated aqueous sodium bicarbonate solution, and extraction was performed with ethyl acetate. The separated organic layer was dried over anhydrous magnesium sulfate and concentrated under reduced pressure. The resulting residue was purified by column chromatography to obtain the title compound (16 mg, yield: 50%, yellow solid).

¹H NMR (400 MHz, CDCl₃) δ 12.0 (s, 1H), 8.36 (d, J = 5.2 Hz, 1H), 8.00-7.97 (dd, J = 2.0 Hz, J = 12.8 Hz, 1H), 7.41-7.32 (m, 5H), 7.26-7.22 (m, 2H, overlapped with DMSO), 7.14-7.10 (m, 2H), 6.40 (d, J = 5.2 Hz, 1H), 4.98 (t, J = 8.4 Hz, 2H),4.03 (s, 3H), 3.74 (t, J = 4.0 Hz, 4H), 3.35 (bs, 2H), 3.17 (t, J = 8.4 Hz, 2H), 2.74 (t, J = 5.6 Hz, 2H), 2.51 (bs, 4H)

### Example 186: N-(3-Fluoro-4-((7-methoxy-6-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide

The title compound (yellow solid) was obtained using the same synthetic route as in Example 185, except that N-(3-aminopropyl)morpholine was used as a starting material in step 2.

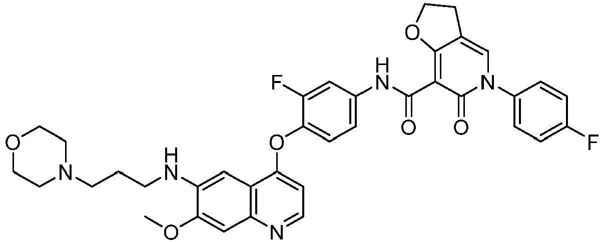

¹H NMR (400 MHz, CDCl₃) δ 12.0 (s, 1H), 8.34 (d, J = 5.6 Hz, 1H), 8.00-7.96 (dd, J = 2.4 Hz, J = 12.8 Hz, 1H), 7.41-7.29 (m, 5H), 7.26-7.21 (m, 2H, overlapped with DMSO), 7.14-7.10 (m, 2H), 6.39 (d, J = 5.2 Hz, 1H), 4.98 (t, J = 8.4 Hz, 2H), 4.02 (s, 3H), 3.76 (t, J = 4.8 Hz, 4H), 3.38-3.34 (dd, J = 6.0 Hz, J = 11.2 Hz, 2H), 3.76 (t, J = 8.0 Hz, 2H), 2.54 (t, J = 6.4 Hz, 2H), 2.50 (bs, 4H), 1.91 (t, J = 6.4 Hz, 2H)

### Example 187: N-(3-Fluoro-4-((6-methoxy-7-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 185, except that 6-bromo-4-(2-fluoro-4-nitrophenoxy)-7-methoxyquinoline was used as a starting material in step 1.

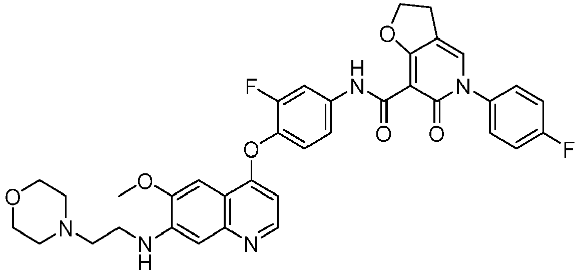

¹H NMR (400 MHz, DMSO-d₆) δ 11.87 (s, 1H, NH), 8.33 (d, J = 4.8 Hz, 1H), 7.99 (dd, J = 13.2,2.4 Hz, 1H), 7.87 (s, 1H), 7.52-7.49 (m, 2H),7.42-7.35 (m, 5H), 6.87 (s, 1H), 6.27 (d, J = 4.8 Hz, 1H), 5.71 (t, J = 5.6 Hz, 1H), 4.84 (t, J = 8.0 Hz, 2H), 3.98 (s, 3H), 3.60 (t, J = 4.8 Hz, 4H), 3.32-3.29 (m, 2H, overlapped with H₂O), 3.10 (t, J = 8.0 Hz, 2H), 2.62 (t, J = 6.4 Hz, 2H), 2.44 (bs, 4H)

### Example 188: N-(3-Fluoro-4-((6-methoxy-7-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide

The title compound (white solid) was obtained using the same synthetic route as in Example 185, except that 6-bromo-4-(2-fluoro-4-nitrophenoxy)-7-methoxyquinoline was used as a starting material in step 1 and N-(3-aminopropyl)morpholine was used as a starting material in step 2.

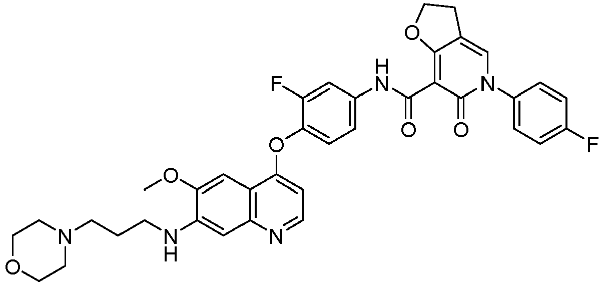

¹H NMR (400 MHz, DMSO-d₆) δ 11.86 (s, 1H, NH), 8.32 (d, J = 5.2 Hz, 1H), 7.99 (dd, J = 13.6, 2.4 Hz, 1H), 7.87 (s, 1H), 7.52-7.49 (m, 2H),7.40-7.34 (m, 5H), 6.83 (s, 1H), 6.34 (t, J = 5.2 Hz, 1H), 6.25 (d, J = 5.6 Hz, 1H), 4.84 (t, J = 8.0 Hz, 2H), 3.97 (s, 3H), 3.63 (t, J = 4.4 Hz, 4H), 3.29-3.24 (m, 2H), 3.10 (t, J = 8.0 Hz, 2H), 2.45-2.39 (m, 6H), 1.81 (t, J = 6.4 Hz, 2H)

### Experimental Example 1: Measurement of enzyme inhibitory activity against RON tyrosine kinase

To measure enzyme inhibitory activity of the example compounds against RON, IC₅₀ values of the compounds were measured using fluorescence resonance energy transfer (FRET).

Specifically, first, RON (Carna Bioscience) was diluted in a kinase assay buffer (50 mM HEPES pH 7.5, 1 mM EGTA, 10 mM MgCl₂, 2 mM DTT, and 0.01% Tween-20) to be at a concentration (0.4 nM) that is twice the final reaction concentration. The dilute was added to a 384-well-plate at 5 µl per well. Each of the example compounds was prepared at a concentration of 1 mM in 100% DMSO and diluted 8 times in a stepwise manner through 10-fold dilution. Each compound was diluted in a kinase assay buffer to be at a concentration that is 4 times the final reaction concentration. The dilute was added to a 384-well-plate at 2.5 µl per well, and incubation was performed at room temperature for 15 minutes.

Thereafter, an ATP solution (Sigma Aldrich) prepared at a concentration of 0.25 M was diluted in a kinase assay buffer to be at a concentration (60 µM) that is 4 times the final reaction concentration. Dilution of a tyrosine peptide substrate (Perkin-Elmer) was performed therein to be at a concentration (400 nM) that is 4 times the final reaction concentration. The dilute was added to a 384-well-plate at 2.5 µl per well. Reaction was allowed to proceed for 60 minutes by incubating the 384-well-plate at room temperature. Thereafter, EDTA (Sigma Aldrich) was diluted in a TR-FRET detection buffer (Perkin-Elmer) to be at a concentration (20 mM) that is twice the final reaction concentration. Dilution of a Europium-containing phosphotyrosine antibody (Perkin-Elmer) was performed in the diluted EDTA solution to be at a concentration (4 nM) that is twice the final reaction concentration, so that a stop solution for RON reaction was prepared.

The stop solution was added to the 384-well-plate at 10 µl per well to quench the reaction, and incubation was performed at room temperature for 60 minutes. Then, the 384-well-plate was read on a Victor X5 (Perkin-Elmer) plate reader. Here, the excitation wavelength was 340 nm, and the emission wavelengths were 620 nm and 665 nm. An IC₅₀ value of the compound was derived using GraphPad Prism 5 program, in which the IC₅₀ was derived by expressing emission energy at the wavelength of 665 nm as a function of the compound concentration. The results are shown in Table 1 below.

**[Table 1]**

| **Example** | **IC₅₀, nM** | **Example** | **IC₅₀, nM** | **Example** | **IC₅₀, nM** |
|---|---|---|---|---|---|
| **1** | 0.82 | **58** | 24.96 | **162** | 1.18 |
| **2** | 1.25 | **59** | 2.24 | **163** | 0.10 |
| **3** | 2.39 | **60** | 13.05 | **164** | 1.46 |
| **4** | 0.35 | **61** | 156.00 | **165** | 0.45 |
| **5** | 5.87 | **62** | 12.29 | **166** | 1.90 |
| **6** | 1.43 | **63** | 20.63 | **167** | 0.83 |
| **7** | 34.90 | **64** | 14.19 | **168** | 0.45 |
| **8** | 0.66 | **65** | 1.72 | **169** | >100 |
| **9** | 0.50 | **66** | 8.63 | **170** | >100 |
| **10** | 0.92 | **67** | 15.00 | **171** | 7.13 |
| **40** | 3.21 | **68** | 1.57 | **172** | 7.44 |
| **41** | 2.86 | **69** | 3.29 | **173** | 0.58 |
| **42** | 29.99 | **70** | 3.66 | **174** | 1.61 |
| **43** | 5.13 | **71** | 8.24 | **175** | 1.24 |
| **44** | 1.87 | **72** | 46.19 | **176** | 0.10 |
| **45** | 1.50 | **73** | 17.53 | **177** | 0.83 |
| **46** | 2.39 | **74** | 9.75 | **178** | 0.45 |
| **47** | 0.47 | **75** | 2.8 µM | **179** | 0.47 |
| **48** | 0.85 | **76** | 1.79 | **180** | 6.83 |
| **49** | 2.98 | **77** | 9.32 | **181** | 5.92 |
| **50** | 1.18 | **78** | 2.63 | **182** | 3.35 |
| **51** | 0.55 | **79** | 0.78 | **183** | 2.50 |
| **52** | 1.83 | **80** | 1.30 | **184** | 0.92 |
| **53** | 1.03 | **157** | 1.32 | **185** | 4.67 |
| **54** | > 1 µM | **158** | 1.99 | **186** | 7.52 |
| **55** | 2.24 | **159** | 11.86 | **187** | 3.86 |
| **56** | 11.85 | **160** | 1.01 | **188** | 3.23 |
| **57** | 18.17 | **161** | 0.61 | | |

As shown in Table 1, it was identified that most of the example compounds according to the present invention exhibited an excellent activity inhibitory effect on RON kinase with an IC₅₀ value of 10 nM or less.

### Experimental Example 2: Identification of cell growth inhibitory ability of example compounds in RON-activated cancer cell line

The cancer cell line KM12C (80015, KCLB, MEM+10%FBS), in which RON is activated, was treated with each of the example compounds at a concentration of 10 µM, and then reaction was allowed to proceed for 48 hours. 24 hours after completion of the reaction, 20 µl of MTS solution (CellTiter 96 Aqueous One Solution Cell Proliferation Assay, product number: G3581, Promega) was added to each well, and then incubation was performed at a temperature of 37°C in 5% CO2 incubator for 4 hours. Then, an IC₅₀ value of the compound was derived by measuring its absorbance value at a wavelength of 490 nm using a Victor X5 plate reader. Here, as a positive control, N-(4-(2-amino-3-chloropyridin-4-yloxy)-3-fluorophenyl)-4-ethoxy-1 -(4-fluorophenyl)-2-oxo-1,2-dihydropyridine-3-carboxamide, which is the compound of Example 1 in WO 2008/058229 A1, was used. The results are shown in Table 2 below.

**[Table 2]**

| **Example** | **ICso, µM** | **Example** | **ICso, µM** | **Example** | **ICso, µM** |
|---|---|---|---|---|---|
| | **Cancer cell line KM12C** | | **Cancer cell line KM12C** | | **Cancer cell line KM12C** |
| **Positive control** | 0.133 | **47** | 0.002 | **158** | 0.005 |
| **1** | 0.002 | **48** | 0.001 | **160** | 0.006 |
| **2** | 0.067 | **50** | 0.002 | **161** | 0.007 |
| **3** | 0.003 | **51** | 0.002 | **163** | 0.002 |
| **4** | 0.008 | **52** | 0.001 | **164** | 0.001 |
| **5** | 0.004 | **52** | 0.001 | **165** | 0.003 |
| **6** | 0.002 | **53** | 0.003 | **166** | 0.016 |
| **8** | 0.0003 | **62** | 1.674 | **167** | 0.006 |
| **9** | 0.0006 | **63** | 2.276 | **168** | 0.025 |
| **14** | 0.002 | **71** | 0.011 | **171** | 0.008 |
| **16** | 0.001 | **73** | 0.008 | **172** | 0.015 |
| **17** | 0.001 | **76** | 0.004 | **173** | 0.003 |
| **18** | 0.003 | **77** | 0.010 | **174** | 0.004 |
| **19** | 0.001 | **78** | 0.002 | **175** | 0.002 |
| **20** | 0.001 | **79** | 0.001 | **176** | 0.002 |
| **24** | 0.001 | **80** | 0.003 | **177** | 0.006 |
| **25** | 0.001 | **87** | 0.003 | **178** | 0.025 |
| **27** | 0.002 | **88** | 0.003 | **179** | 0.003 |
| **28** | 0.003 | **91** | 0.050 | **180** | 0.003 |
| **29** | 0.001 | **95** | 0.005 | **181** | 0.002 |
| **30** | 0.001 | **102** | 0.001 | **182** | 0.008 |
| **31** | 0.002 | **105** | 0.004 | **183** | 0.003 |
| **32** | 0.006 | **124** | 0.005 | **184** | 0.002 |
| **34** | 0.003 | **125** | 0.003 | **185** | 0.001 |
| **35** | 0.004 | **134** | 0.002 | **186** | 0.003 |
| **36** | 0.005 | **137** | 0.002 | **187** | 0.006 |
| **41** | 0.002 | **142** | 0.001 | **188** | 0.008 |
| **46** | 0.006 | **145** | 0.002 | | |

As shown in Table 2, it was identified that most of the example compounds exhibited excellent cell growth inhibitory capacity in the cancer cell line KM12C, in which RON is activated, as compared with the positive control.

### Experimental Example 3: Measurement of cell-killing efficacy of example compounds in RON-activated cancer cell line

The cancer cell line KM12C (80015, KCLB, MEM+10% FBS), in which RON is activated, was dispensed into each 60 mm² dish at 1×10⁵ cells. After 24 hours, the dish was treated with each of the example compounds at a concentration of 1 µM or 5 µM while performing medium exchange, and then reaction was allowed to proceed for 48 hours. After 48 hours, centrifugation was performed for 4 minutes at 1,500 rpm to collect cells and cell fluid. Thereafter, living cells and dead cells were quantified (n=2) using a trypan blue solution (15250-061, Gibco), and statistical analysis was performed using Excel-T-test. Here, as a negative control, only a solvent (0.1% DMSO) was used; and as a positive control, N-(4-(2-amino-3-chloropyridin-4-yloxy)-3-fluorophenyl)-4-ethoxy-1-(4-fluorophenyl)-2-oxo-1,2-dihydropyridine-3-carboxamide, which is the compound of Example 1 in WO 2008/058229 A1, was used. The results are shown in Table 3 below.

**[Table 3]**

| **Example** | **Cell death (%)** | | **Example** | **Cell death (%)** | |
|---|---|---|---|---|---|
| | **Cancer cell line KM12C** | | | **Cancer cell line KM12C** | |
| | **1 µM** | **5 µM** | | **1 µM** | **5 µM** |
| **Positive control** | 9.94 | 12.77 | **52** | 10.12 | 24.71 |
| **1** | 10.32 | 68.58 | **64** | 13.12 | 97.12 |
| **2** | 38.36 | 93.42 | **76** | 35.74 | 38.29 |
| **3** | 24.61 | 34.03 | **78** | 23.51 | 23.93 |
| **5** | 21.20 | 25.90 | **80** | 19.54 | 19.94 |
| **6** | 29.30 | 34.30 | **87** | 40.70 | 60.10 |
| **8** | 32.39 | 46.88 | **88** | 18.80 | 24.70 |
| **10** | 22.04 | 21.88 | **91** | 12.65 | 14.30 |
| **14** | 13.60 | 21.50 | **95** | 19.65 | 25.65 |
| **16** | 20.45 | 24.25 | **102** | 23.40 | 29.70 |
| **17** | 14.00 | 27.70 | **105** | 33.80 | 45.20 |
| **18** | 10.95 | 11.30 | **124** | 16.30 | 57.30 |
| **19** | 15.70 | 21.90 | **125** | 22.30 | 39.60 |
| **20** | 8.12 | 35.50 | **134** | 35.50 | 47.40 |
| **24** | 18.55 | 16.90 | **137** | 18.20 | 22.35 |
| **25** | 17.50 | 21.00 | **142** | 34.00 | 40.00 |
| **27** | 17.30 | 29.00 | **145** | 25.70 | 45.40 |
| **28** | 9.60 | 10.30 | **158** | 24.14 | 37.12 |
| **29** | 22.60 | 24.50 | **160** | 12.68 | 19.36 |
| **30** | 24.80 | 25.90 | **161** | 23.45 | 22.04 |
| **31** | 40.90 | 52.00 | **162** | 16.40 | 22.57 |
| **32** | 13.50 | 20.45 | **164** | 19.39 | 29.69 |
| **34** | 13.65 | 15.90 | **165** | 7.30 | 32.90 |
| **35** | 19.60 | 28.10 | **166** | 11.40 | 14.90 |
| **36** | 17.10 | 30.00 | **172** | 3.19 | 11.55 |
| **40** | 29.84 | 37.26 | **176** | 4.10 | 20.20 |
| **41** | 16.00 | 21.40 | **180** | 18.15 | 20.60 |
| **44** | 16.00 | 21.40 | **181** | 14.60 | 36.80 |
| **45** | 16.30 | 18.90 | **182** | 25.10 | 44.40 |
| **46** | 23.74 | 27.38 | **183** | 27.30 | 40.30 |
| **47** | 18.00 | 30.90 | **184** | 13.80 | 54.20 |
| **48** | 18.89 | 15.49 | **185** | 32.45 | 63.75 |
| **49** | 9.98 | 11.25 | **187** | 20.40 | 60.00 |
| **50** | 19.38 | 32.28 | **188** | 25.40 | 57.30 |

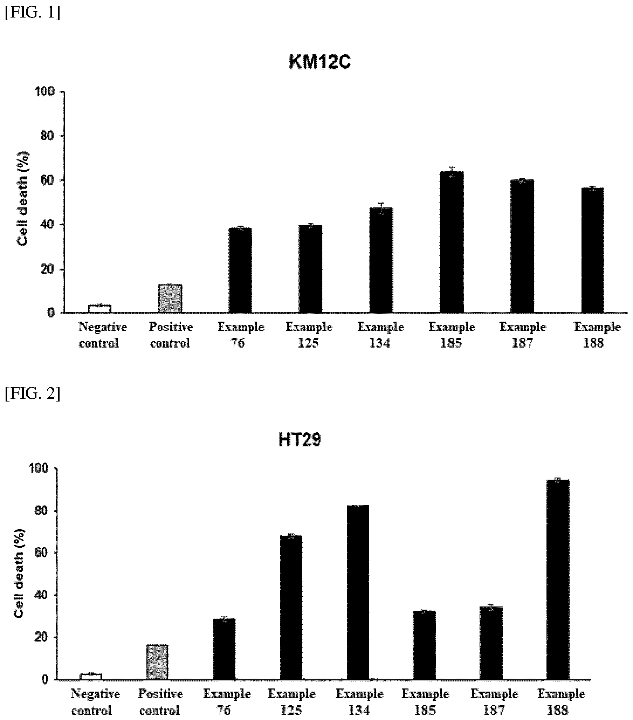

As shown in Table 3, it was identified that the example compounds had an excellent cell-killing effect, as compared with the positive control, in the cancer cell line KM12C in which RON is activated. In particular, the compounds of Examples 76, 125, 134, 185, 187, and 188 showed a high cell-killing effect (see FIG. 1).

### Experimental Example 4: Measurement of cell-killing efficacy of example compounds in RON-mutated cancer cell lines

Each of two colorectal cancer cell lines, that is, the cancer cell line HT29 (30038, KCLB, RPMI + 10% FBS), in which mutated RON (Δ160) is expressed, and the cancer cell line SW620 (CCL-227, ATCC, RPMI+10% FBS), in which mutated RON (Δ155) is expressed, was dispensed into each 60 mm² dish at 1 × 10⁵ cells. After 24 hours, the dish was treated with each of the example compounds at a concentration of 1 µM or 5 µM while performing medium exchange, and then reaction was allowed to proceed for 48 hours. After 48 hours, centrifugation was performed for 4 minutes at 1,500 rpm to collect cells and cell fluid. Thereafter, living cells and dead cells were quantified (n=2) using a trypan blue solution (15250-061, Gibco), and statistical analysis was performed using Excel-T-test. Here, as a negative control, only a solvent (0.1% DMSO) was used; and as a positive control, N-(4-(2-amino-3-chloropyridin-4-yloxy)-3-fluorophenyl)-4-ethoxy-1-(4-fluorophenyl)-2-oxo-1,2-dihydropyridine-3-carboxamide, which is the compound of Example 1 in WO 2008/058229 A1, was used. The results are shown in Table 4 below.

**[Table 4]**

| **Example** | **Cell death (%)** | | | | **Example** | **Cell death (%)** | | | |
|---|---|---|---|---|---|---|---|---|---|
| | **Cancer cell line HT29** | | **Cancer cell line SW620** | | | **Cancer cell line HT29** | | **Cancer cell line SW620** | |
| | **1 µM** | **5 µM** | **1 µM** | **5 µM** | | **1 µM** | **5 µM** | **1 µM** | **5 µM** |
| **Positive control** | 10.40 | 16.10 | 3.46 | 9.28 | **125** | 39.35 | 67.85 | 60.90 | 91.50 |
| **1** | 9.35 | 38.18 | N.D | N.D | **134** | 47.05 | 82.25 | 76.10 | 82.80 |
| **2** | 15.45 | 80.49 | N.D | N.D | **142** | N.D | N.D | 74.00 | 67.60 |
| **8** | 4.88 | 16.78 | 5.98 | 7.83 | **145** | 16.45 | 81.40 | 11.00 | 87.10 |
| **20** | N.D | N.D | 17.35 | 91.25 | **158** | 15.25 | 30.91 | 11.60 | 92.30 |
| **31** | N.D | N.D | 15.40 | 47.00 | **181** | 10.20 | 99.10 | 11.30 | 35.00 |
| **40** | 9.17 | 22.87 | 8.50 | 9.20 | **182** | 7.60 | 10.20 | N.D | N.D |
| **52** | 10.53 | 16.05 | 13.55 | 81.85 | **183** | 7.80 | 87.50 | N.D | N.D |
| **64** | 16.02 | 18.79 | N.D | N.D | **184** | 7.90 | 23.20 | 5.00 | 29.80 |
| **76** | 21.90 | 28.57 | 46.37 | 67.80 | **185** | 5.10 | 32.40 | 28.10 | 70.20 |
| **87** | N.D | N.D | 77.10 | 70.50 | **187** | 8.40 | 34.30 | 23.95 | 50.50 |
| **105** | N.D | N.D | 60.40 | 84.10 | **188** | 28.70 | 94.40 | 26.95 | 31.65 |
| **124** | 15.65 | 62.15 | 6.20 | 98.70 | | | | | |

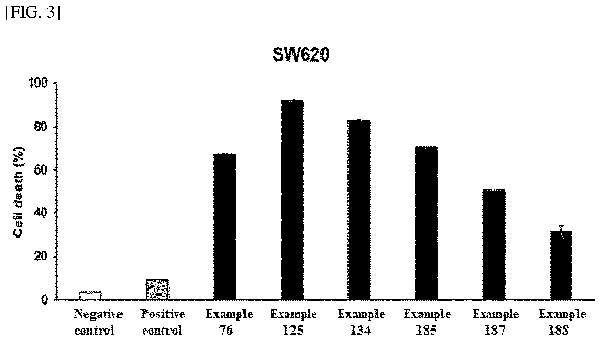

As shown in Table 4, it was identified that the Example compounds exhibited an excellent cell-killing effect, as compared with the positive control, even in the cancer cell line HT29, in which mutated RON (Δ160) is expressed, and the cancer cell line SW620, in which mutated RON (Δ155) is expressed. In particular, the compounds of Examples 76, 125, 134, 185, 187, and 188 showed a high cell-killing effect not only in the RON-activated cancer cell line but also in the RON-mutated cancer cell lines (see FIGS. 2 and 3).

## Claims

1. A compound represented by Formula 1, or a pharmaceutically acceptable salt thereof: 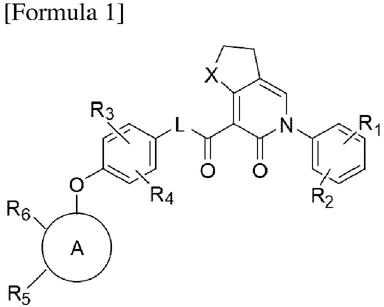 in the formula,
L is -NH- or -CH₂-,
R₁ to R₄ are each independently hydrogen, halogen, hydroxy, cyano, C₁₋₄ alkyl, C₁₋₄ alkoxy, C₁₋₄ haloalkyl, C₂₋₄ alkenyl, C₂₋₄ alkynyl, C₃₋₇ cycloalkyl, C₆₋₁₀ aryl, 5- to 9-membered heteroaryl, or 3- to 9-membered heterocycloalkyl,
X is O, S, -CH(-Rx)-, or -N(-Rx)-,
Rx is hydrogen, C₁₋₄ alkyl, C₁₋₄ alkoxy, C₁₋₄ haloalkyl, C₂₋₄ alkenyl, C₂₋₄ alkynyl, C6-₁₀ aryl, C₆₋₁₀ aryl-C₁₋₄ alkyl, or 3- to 9-membered heterocycloalkyl,
A is a 3- to 12-membered heterocyclic ring, and
R₅ and R₆ are each independently hydrogen, nitro, amino, halogen, hydroxy, cyano, C₁₋₆ alkyl, C₁₋₆ haloalkyl, C₂₋₆ alkenyl, C₂₋₆ alkynyl, C₁₋₆ alkoxy, amino-C₁₋₆ alkoxy, aminocarbonyl, C₁₋₆ alkylaminocarbonyl, diC₁₋₆ alkylaminocarbonyl, C₁₋₆ alkylcarbonylamino, C₁₋₆ alkylamino, C₁₋₆ alkylamino-C₁₋₆ alkoxy, C₆₋₁₀ aryl, C₆₋₁₀ aryl-C₁₋₄ alkyl, or 5- to 9-membered heteroaryl,
wherein R₅ and R₆ are each independently optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3- to 9-membered cycloalkyl; or 3- to 9-membered heterocycloalkyl,
the cycloalkyl or the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heterocyclic ring, the heteroaryl, and the heterocycloalkyl each independently contain one or more heteroatoms selected from the group consisting of N, O, and S.

2. The compound of claim 1, wherein R₁ to R₄ are each independently hydrogen, C₁₋₄ haloalkyl, or halogen; or a pharmaceutically acceptable salt thereof.

3. The compound of claim 1, wherein R₁ is hydrogen, trifluoromethyl, or fluoro; R₂ is hydrogen; R₃ is fluoro; and R₄ is hydrogen; or a pharmaceutically acceptable salt thereof.

4. The compound of claim 1, wherein X is O or -CH(-Rx)- and Rx is hydrogen or C₁₋₆ alkyl; or a pharmaceutically acceptable salt thereof.

5. The compound of claim 1, wherein the 3- to 12-membered heterocyclic ring contains one or more aromatic rings; or a pharmaceutically acceptable salt thereof.

6. The compound of claim 1, wherein A is quinoline, quinazoline, pyridine, pyrimidine, thienopyridine, pyrrolopyridine, pyrazolopyridine, imidazopyridine, pyrrolopyrimidine, dihydropyrrolopyrimidine, furopyridine, pyrazolopyrimidine, purine, or indazole; or a pharmaceutically acceptable salt thereof.

7. The compound of claim 1, wherein R₅ and R₆ are each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylcarbonylamino, C₁₋₆ alkylcarbonylamino, C₁₋₆ alkylamino, C₁₋₆ alkylamino-C₁₋₆ alkoxy, or 5- to 9- membered heteroaryl,
wherein R₅ and R₆ are each independently optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3- to 9-membered cycloalkyl; or 3- to 9-membered heterocycloalkyl,
the cycloalkyl or the heterocycloalkyl optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heteroaryl and the heterocycloalkyl each independently contain one or more heteroatoms selected from the group consisting of N, O, and S; or a pharmaceutically acceptable salt thereof.

8. The compound of claim 7, wherein the heteroaryl is pyridinyl, imidazolyl, or pyrazolyl; the heterocycloalkyl is azetidinyl, pyrrolidinyl, tetrahydropyranyl, morpholino, morpholinyl, dioxidothiomorpholino, piperazinyl, piperidinyl, or oxetanyl; and the cycloalkyl is cyclobutyl, cyclopentyl, or cyclohexyl; or a pharmaceutically acceptable salt thereof.

9. The compound of claim 1, wherein R₁ and R₂ are each independently hydrogen, C₁₋₄ haloalkyl, or halogen; R₃ and R₄ are each independently hydrogen or halogen; X is O or - CH(-Rx)- and Rx is hydrogen or C₁₋₄ alkyl; A is quinoline, quinazoline, pyridine, pyrimidine, thienopyridine, pyrrolopyridine, pyrazolopyridine, imidazopyridine, pyrrolopyrimidine, dihydropyrrolopyrimidine, furopyridine, pyrazolopyrimidine, purine, or indazole; and R₅ and R₆ are each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylcarbonylamino, C₁₋₆ alkylcarbonylamino, C₁₋₆ alkylamino, C₁₋₆ alkylamino-C₁₋₆ alkoxy, or 5- to 9-membered heteroaryl,
wherein R₅ and R₆ are each independently be optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3- to 9-membered cycloalkyl; or 3- to 9-membered heterocycloalkyl;
the cycloalkyl or the heterocycloalkyl optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl; and
the heteroaryl and the heterocycloalkyl each independently contain one or more heteroatoms selected from the group consisting of N, O, and S; or a pharmaceutically acceptable salt thereof.

10. The compound of claim 1, wherein the compound of Formula 1 has a structure of Formula 1a; or a pharmaceutically acceptable salt thereof: 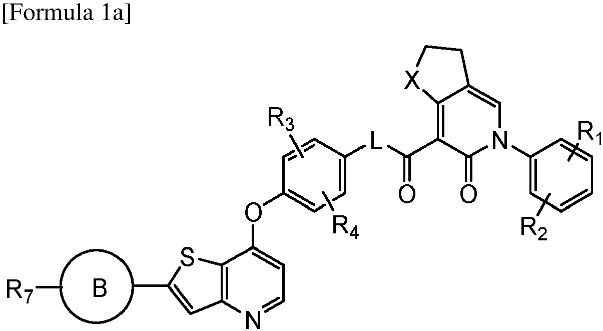 in the formula,
X, L, R₁, R₂, R₃, and R₄ are as defined in claim 1,
B is a 5- or 6-membered aromatic heterocyclic ring containing 1 or 2 N atoms, and
R₇ is C₁₋₆ alkyl; or C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl,
wherein the cycloalkyl or the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heterocycloalkyl contains 1 to 4 heteroatoms selected from the group consisting of N, O, and S.

11. The compound of claim 10, wherein the compound represented by Formula 1a is a compound selected from the group consisting of the following compounds; or a pharmaceutically acceptable salt thereof:
1) N-(3-fluoro-4-((2-(5-(((2-methoxyethyl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7 -carboxamide;
2) N-(3-fluoro-4-((2-(5-(((2-methoxyethyl)amino)methyl)pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
3) N-(3-fluoro-4-((2-(pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
4) N-(3-fluoro-4-((2-(pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
5) N-(3-fluoro-4-((2-(5-(morpholinomethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
6) N-(3-fluoro-4-((2-(5-(morpholinomethyl)pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
7) N-(3-fluoro-4-((2-(5-morpholinomethyl)pyridin-2-yl)thieno[3,2-b]pyridin-7-yl)phenyl)-2-(4-fluorophenyl)-3-oxo-3,5,6,7-tetrahydro-2H-cyclopenta[c]pyridine-4-carboxamide;
8) N-(3-fluoro-4-((2-(1-methyl-1H-imidazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
9) N-(3-fluoro-4-((2-(1-methyl-1H-imidazol-4-yl)thieno[3,2-b]pyridin-7-yl)oxy)phenyl)-6-oxo-5-phenyl-2,3,5,6-tetrahydrofuro[3,2-c]pyridine-7-carboxamide;
10) N-(3-fluoro-4-((2-(1-methyl-1H-pyrazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
11) N-(3-fluoro-4-((2-(5-((3-hydroxyazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
12) N-(3-fluoro-4-((2-(5-((3-(hydroxymethyl)azetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
13) N-(3-fluoro-4-((2-(5-((3-hydroxy-3-methylazetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
14) N-(3-fluoro-4-((2-(5-((3-methoxyazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
15) N-(3-fluoro-4-((2-(5-((3-(methoxymethyl)azetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
16) N-(3-fluoro-4-((2-(5-((3-methoxy-3-methylazetidin-l-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
17) N-(3-fluoro-4-((2-(5-((3-fluoroazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
18) N-(4-((2-(5-((3,3-difluoroazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
19) N-(4-((2-(5-((3-cyanoazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
20) N-(4-((2-(5-((3-(dimethylamino)azetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
21) N-(3-fluoro-4-((2-(5-((3-methylazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
22) N-(4-((2-(5-((3,3-dimethylazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
23) N-(3-fluoro-4-((2-(5-((3-hydroxypyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7 -carboxamide;
24) N-(3-fluoro-4-((2-(5-((3-hydroxy-3-methylpyrrolidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
25) N-(3-fluoro-4-((2-(5-((3-methoxypyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
26) N-(3-fluoro-4-((2-(5-(pyrrolidin-1-ylmethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
27) N-(3-fluoro-4-((2-(5-((3-fluoropyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
28) N-(4-((2-(5-((3,3-difluoropyrrolidin-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide;
29) N-(3-fluoro-4-((2-(5-((4-hydroxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
30) N-(3-fluoro-4-((2-(5-((3-hydroxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
31) N-(3-fluoro-4-((2-(5-((4-methoxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
32) N-(3-fluoro-4-((2-(5-((3-methoxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
33) N-(3-fluoro-4-((2-(5-(piperidin-1-ylmethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
34) N-(3-fluoro-4-((2-(5-((4-fluoropiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7 -carboxamide;
35) N-(4-((2-(5-((4,4-difluoropiperidm-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide;
36) N-(3-fluoro-4-((2-(5-((4-methylpiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
37) N-(4-((2-(5-((4,4-dimethylpiperidin-l-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
38) N-(3-fluoro-4-((2-(5-((((3-hydroxycyclohexyl)methyl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide; and
39) N-(3-fluoro-4-((2-(5-(((tetrahydro-2H-pyran-4-yl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide.

12. The compound of claim 1, wherein the compound of Formula 1 has a structure of Formula 1b; or a pharmaceutically acceptable salt thereof: 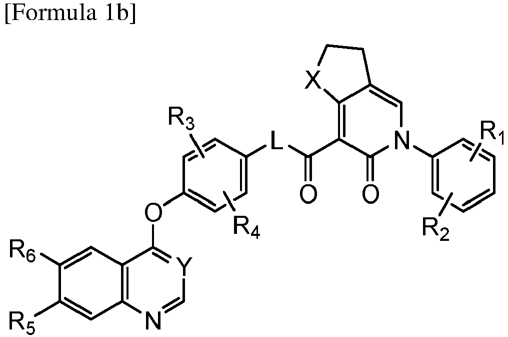 in the formula,
X, L, R₁, R₂, R₃, and R₄ are as defined in claim 1,
Y is -N= or -CH=, and
R₅ and R₆ are each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylcarbonylamino, C₁₋₆ alkylcarbonylamino, C₁₋₆ alkylamino, or C₁₋₆ alkylamino-C₁₋₆ alkoxy,
wherein R₅ and R₆ are each independently optionally substituted with 3- to 9-membered cycloalkyl or 3- to 9-membered heterocycloalkyl,
the cycloalkyl or the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heterocycloalkyl contains 1 to 4 heteroatoms selected from the group consisting of N, O, and S.

13. The compound of claim 12, wherein the compound represented by Formula 1b is a compound selected from the group consisting of the following compounds; or a pharmaceutically acceptable salt thereof:
40) N-(4-((6,7-dimethoxyquinolin-4-yl)oxy)-3-fluorophenyl)-5-(4-fluorophenyl)-6-oxo-2,3,5,6-tetrahydrofuro[3,2-c]pyridine-7-carboxamide;
41) N-(4-((6,7-dimethoxyquinolin-4-yl)oxy)-3-fluorophenyl)-6-oxo-5-phenyl-2,3,5,6-tetrahydrofuro[3,2-c]pyridine-7-carboxamide;
42) N-(4-((6,7-dimethoxyquinolin-4-yl)oxy)-3-fluorophenyl)-6-oxo-5-(4-(trifluoromethyl)phenyl)-2,3,5,6-tetrahydrofuro[3,2-c]pyridine-7-carboxamide;
43) N-(4-((6,7-dimethoxyquinolin-4-yl)oxy)-3-fluorophenyl)-6-oxo-5-(3-fluorophenyl)-2,3,5,6-tetrahydrofuro[3,2-c]pyridine-7-carboxamide;
44) N-(3-fluoro-4-((6-methoxyquinolin-4-yl)oxy)phenyl)-5-(4-fluorophenyl)-6-oxo-2,3,5,6-tetrahydrofuro[3,2-c]pyridine-7-carboxamide;
45) N-(3-fluoro-4-((7-methoxyquinolin-4-yl)oxy)phenyl)-5-(4-fluorophenyl)-6-oxo-2,3,5,6-tetrahydrofuro[3,2-c]pyridine-7-carboxamide;
46) N-(4-((6,7-dimethoxyquinazolin-4-yl)oxy)-3-fluorophenyl)-5-(4-fluorophenyl)-6-oxo-2,3,5,6-tetrahydrofuro[3,2-c]pyridine-7-carboxamide;
47) N-(4-((6-carbamoyl-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
48) N-(3-fluoro-4-((7-methoxy-6-(methylcarbamoyl)quinolin-4-yl)oxy)phenyl)-5-(4-fluorophenyl)-6-oxo-2,3,5,6-tetrahydrofuro[3,2-c]pyridine-7-carboxamide;
49) N-(4-((6-(dimethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
50) N-(3-fluoro-4-((7-methoxy-6-((2-morpholinoethyl)carbamoyl)quinolin-4-yl)oxy)phenyl)-5-(4-fluorophenyl)-6-oxo-2,3,5,6-tetrahydrofuro[3,2-c]pyridine-7-carboxamide;
51) N-(4-((6-(ethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
52) N-(4-((6-acetamido-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
53) N-(3-fluoro-4-((7-methoxy-6-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide;
76) N-(3-fluoro-4-((6-methoxy-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
77) N-(3-fluoro-4-((6-methoxy-7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide;
78) N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
79) N-(3-fluoro-4-((6-methoxy-7-(2-morpholinoethoxy)quinolin-4-yl)oxy)phenyl)-6-oxo-5-phenyl-2,3,5,6-tetrahydrofuro[3,2-c]pyridine-7-carboxamide;
80) N-(3-fluoro-4-((6-methoxy-7-(3-(4-methylpiperazin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
81) N-(3-fluoro-4-((7-(3-(3-hydroxyazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
82) N-(3-fluoro-4-((7-(3-(3-hydroxy-3-methylazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
83) N-(3-fluoro-4-((7-(3-(3-(hydroxymethyl)azetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
84) N-(3-fluoro-4-((6-methoxy-7-(3-(3-methoxyazetidin-1-yl)propoxy)qumolin-4-yl)oxy)phenyl)-5-(4-fluorophenyl)-6-oxo-2,3,5,6-tetrahydrofuro[3,2-c]pyridine-7-carboxamide;
85) N-(3-fluoro-4-((6-methoxy-7-(3-(3-methoxy-3-methylazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
86) N-(3-fluoro-4-((7-(3-(3-fluoroazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
87) N-(4-((7-(3-(3,3-difluoroazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
88) N-(4-((7-(3-(3-cyanoazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
89) N-(3-fluoro-4-((6-methoxy-7-(3-(3-methylazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
90) N-(3-fluoro-4-((7-(3-(3-hydroxypyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
91) N-(3-fluoro-4-((7-(3-(3-hydroxy-3-methylpyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
92) N-(3-fluoro-4-((6-methoxy-7-(3-(3-methoxypyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
93) N-(3-fluoro-4-((6-methoxy-7-(3-(pyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
94) N-(3-fluoro-4-((7-(3-(3-fluoropyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
95) N-(4-((7-(3-(3,3-difluoropyrrolidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
96) N-(3-fluoro-4-((7-(3-(4-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
97) N-(3-fluoro-4-((7-(3-(3-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
98) N-(3-fluoro-4-((7-(3-(4-hydroxy-4-methylpiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
99) N-(3-fluoro-4-((6-methoxy-7-(3-(4-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
100) N-(3-fluoro-4-((6-methoxy-7-(3-(3-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
101) N-(3-fluoro-4-((6-methoxy-7-(3-(4-oxopiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
102) N-(4-((7-(3-(1,1-dioxidothiomorpholino)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
103) N-(3-fluoro-4-((6-methoxy-7-(3-(piperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
104) N-(3-fluoro-4-((7-(3-(4-fluoropiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
105) N-(4-((7-(3-(4,4-difluoropiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
106) N-(3-fluoro-4-((6-methoxy-7-(3-(4-methylpiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
107) N-(4-((7-(3-(4,4-dimethylpiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
108) N-(3-fluoro-4-((7-(3-((3-hydroxycyclobutyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
109) N-(3-fluoro-4-((6-methoxy-7-(3-((3-methoxycyclobutyl)amino)propoxy)quinolin-4-yl)oxy)phenyl)-5-(4-fluorophenyl)-6-oxo-2,3,5,6-tetrahydrofuro[3,2-c]pyridine-7-carboxamide;
110) N-(3-fluoro-4-((6-methoxy-7-(3-(oxetan-3-ylamino)propoxy)quinolin-4-yl)oxy)phenyl)-5-(4-fluorophenyl)-6-oxo-2,3,5,6-tetrahydrofuro[3,2-c]pyridine-7-carboxamide;
111) N-(3-fluoro-4-((6-methoxy-7-(3-((oxetan-3-ylmethyl)amino)propoxy)quinolin-4-yl)oxy)phenyl)-5-(4-fluorophenyl)-6-oxo-2,3,5,6-tetrahydrofuro[3,2-c]pyridine-7-carboxamide;
112) N-(3-fluoro-4-((7-(3-((3-hydroxycyclopentyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
113) N-(3-fluoro-4-((7-(3-((3-hydroxycyclohexyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
114) N-(3-fluoro-4-((7-(3-(((3-hydroxycyclohexyl)methyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
115) N-(3-fluoro-4-((6-methoxy-7-(3-((tetrahydro-2H-pyran-4-yl)amino)propoxy)quinolin-4-yl)oxy)phenyl)-5-(4-fluorophenyl)-6-oxo-2,3,5,6-tetrahydrofuro[3,2-c]pyridine-7-carboxamide;
116) N-(3-fluoro-4-((6-methoxy-7-(3-(((tetrahydro-2H-pyran-4-yl)methyl)amino)propoxy)quinolin-4-yl)oxy)phenyl)-5-(4-fluorophenyl)-6-oxo-2,3,5,6-tetrahydrofuro[3,2-c]pyridine-7-carboxamide;
117) N-(3-fluoro-4-((7-(2-(3-hydroxyazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
118) N-(3-fluoro-4-((7-(2-(3-hydroxy-3-methylazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
119) N-(3-fluoro-4-((7-(2-(3-(hydroxymethyl)azetidin-l-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
120) N-(3-fluoro-4-((6-methoxy-7-(2-(3-methoxyazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
121) N-(3-fluoro-4-((6-methoxy-7-(2-(3-methoxy-3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
122) N-(3-fluoro-4-((6-methoxy-7-(2-(3-(methoxymethyl)azetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
123) N-(3-fluoro-4-((7-(2-(3-fluoroazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
124) N-(4-((7-(2-(3,3-difluoroazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
125) N-(4-((7-(2-(3-ethynylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
126) N-(3-fluoro-4-((6-methoxy-7-(2-(3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
127) N-(4-((7-(2-(3,3-dimethylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
128) N-(4-((7-(2-(3-(dimethylamino)azetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
129) N-(3-fluoro-4-((7-(2-(3-hydroxypyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
130) N-(3-fluoro-4-((7-(2-(3-hydroxy-3-methylpyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
131) N-(3-fluoro-4-((6-methoxy-7-(2-(3-methoxypyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
132) N-(3-fluoro-4-((6-methoxy-7-(2-(pyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
133) N-(3-fluoro-4-((7-(2-(3-fluoropyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
134) N-(4-((7-(2-(3,3-difluoropyrrolidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
135) N-(3-fluoro-4-((7-(2-(4-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
136) N-(3-fluoro-4-((7-(2-(3-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
137) N-(3-fluoro-4-((7-(2-(4-hydroxy-4-methylpiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
138) N-(3-fluoro-4-((6-methoxy-7-(2-(4-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
139) N-(3-fluoro-4-((6-methoxy-7-(2-(3-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
140) N-(3-fluoro-4-((6-methoxy-7-(2-(4-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
141) N-(3-fluoro-4-((6-methoxy-7-(2-(3-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
142) N-(4-((7-(2-(1,1-dioxidothiomorpholino)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
143) N-(3-fluoro-4-((6-methoxy-7-(2-(piperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
144) N-(3-fluoro-4-((7-(2-(4-fluoropiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
145) N-(4-((7-(2-(4,4-difluoropiperidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
146) N-(3-fluoro-4-((6-methoxy-7-(2-(4-methylpiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
147) N-(4-((7-(2-(4,4-dimethylpiperidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
148) N-(3-fluoro-4-((7-(2-((3-hydroxycyclobutyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
149) N-(3-fluoro-4-((6-methoxy-7-(2-((3-methoxycyclobutyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
150) N-(3-fluoro-4-((6-methoxy-7-(2-(oxetan-3-ylamino)ethoxy)quinolin-4-yl)oxy)phenyl)-5-(4-fluorophenyl)-6-oxo-2,3,5,6-tetrahydrofuro[3,2-c]pyridine-7-carboxamide;
151) N-(3-fluoro-4-((6-methoxy-7-(2-((oxetan-3-ylmethyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
152) N-(3-fluoro-4-((7-(2-((4-hydroxycyclohexyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
153) N-(3-fluoro-4-((7-(2-((3-hydroxycyclohexyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
154) N-(3-fluoro-4-((7-(2-(((3-hydroxycyclohexyl)methyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
155) N-(3-fluoro-4-((6-methoxy-7-(2-((tetrahydro-2H-pyran-4-yl)amino)ethoxy)quinolin-4-yl)oxy)phenyl)-5-(4-fluorophenyl)-6-oxo-2,3,5,6-tetrahydrofuro[3,2-c]pyridine-7-carboxamide;
156) N-(3-fluoro-4-((6-methoxy-7-(2-((4-methoxycyclohexyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
157) N-(3-fluoro-4-((7-(2-morpholinylethoxy)quinolin-4-yl)oxy)phenyl)-5-(4-fluorophenyl)-6-oxo-2,3,5,6-tetrahydrofuro[3,2-c]pyridine-7-carboxamide;
158) N-(3-fluoro-4-((7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
159) N-(3-fluoro-4-((7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide;
160) N-(3-fluoro-4-((7-(3-(4-methylpiperazin-1-yl)propoxy)qumolin-4-yl)oxy)phenyl)-5-(4-fluorophenyl)-6-oxo-2,3,5,6-tetrahydrofuro[3,2-c]pyridine-7-carboxamide;
161) 7-(2-(3-fluoro-4-((7-(3-(piperidin-1-yl)propoxy)quinolin-4-yl)oxy)phenyl)acetyl)-5-(4-fluorophenyl)-3,5-dihydrofuro[3,2-c]pyridin-6(2H)-one;
162) 7-(2-(3-fluoro-4-((7-(3-(4-methylpiperidin-1-yl)propoxy)quinolin-4-yl)oxy)phenyl)acetyl)-5-(4-fluorophenyl)-3,5-dihydrofuro[3,2-c]pyridin-6(2H)-one;
163) N-(3-fluoro-4-((6-(methylcarbamoyl)-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
164) N-(3-fluoro-4-((6-(methylcarbamoyl)-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
165) N-(3-fluoro-4-((6-methoxy-7-(methylcarbamoyl)quinolin-4-yl)oxy)phenyl)-5-(4-fluorophenyl)-6-oxo-2,3,5,6-tetrahydrofuro[3,2-c]pyridine-7-carboxamide;
166) N-(3-fluoro-4-((7-(methylcarbamoyl)-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
167) N-(3-fluoro-4-((6-fluoro-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
168) N-(3-fluoro-4-((6-fluoro-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
169) N-(3-fluoro-4-((7-(2-morpholinoethoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide;
170) N-(3-fluoro-4-((7-(3-morpholinopropoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide;
171) N-(4-((6-chloro-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide;
172) N-(4-((6-chloro-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide;
173) N-(4-((6-cyano-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide;
174) N-(4-((6-cyano-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide;
175) N-(3-fluoro-4-((7-methoxy-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
176) N-(3-fluoro-4-((7-methoxy-6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
177) N-(3-fluoro-4-((6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
178) N-(3-fluoro-4-((6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
179) N-(4-((6-amino-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
180) N-(3-fluoro-4-((7-methoxy-6-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide;
181) N-(4-((7-amino-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
182) N-(3-fluoro-4-((6-methoxy-7-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide;
183) N-(3-fluoro-4-((6-methoxy-7-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide;
184) N-(4-((7-acetamido-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
185) N-(3-fluoro-4-((7-methoxy-6-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide;
186) N-(3-fluoro-4-((7-methoxy-6-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide;
187) N-(3-fluoro-4-((6-methoxy-7-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide; and
188) N-(3-fluoro-4-((6-methoxy-7-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide.

14. The compound of claim 1, wherein the compound of Formula 1 has a structure of Formula 1c; or a pharmaceutically acceptable salt thereof: 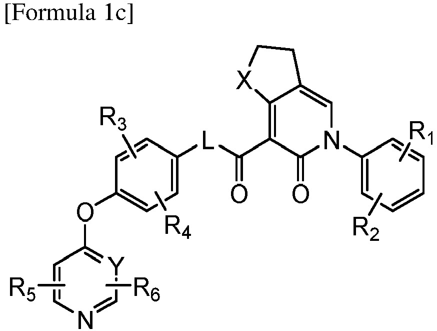 in the formula,
X, L, R₁, R₂, R₃, and R₄ are as defined in claim 1,
Y is -N= or -CH=, and
R₅ and R₆ are each independently hydrogen, amino, halogen, aminocarbonyl, or C₁₋₆ alkylamino,
wherein R₅ and R₆ are each independently optionally substituted with 3- to 9-membered heterocycloalkyl,
the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heterocycloalkyl contains 1 to 4 heteroatoms selected from the group consisting of N, O, and S.

15. The compound of claim 14, wherein the compound represented by Formula 1c is a compound selected from the group consisting of the following compounds; or a pharmaceutically acceptable salt thereof:
54) N-(4-((2-carbamoyl-3-chloropyridin-4-yl)oxy)-3-fluorophenyl)-5-(4-fluorophenyl)-6-oxo-2,3,5,6-tetrahydrofuro[3,2-c]pyridine-7-carboxamide;
55) N-(4-((2-amino-3-chloropyridin-4-yl)oxy)-3-fluorophenyl)-5-(4-fluorophenyl)-6-oxo-2,3,5,6-tetrahydrofuro[3,2-c]pyridine-7-carboxamide;
56) N-(4-((2-aminopyridin-4-yl)oxy)-3-fluorophenyl)-5-(4-fluorophenyl)-6-oxo-2,3,5,6-tetrahydrofuro[3,2-c]pyridine-7-carboxamide;
57) N-(4-((2-aminopyrimidin-4-yl)oxy)-3-fluorophenyl)-5-(4-fluorophenyl)-6-oxo-2,3,5,6-tetrahydrofuro[3,2-c]pyridine-7-carboxamide;
58) N-(4-((6-aminopyrimidin-4-yl)oxy)-3-fluorophenyl)-5-(4-fluorophenyl)-6-oxo-2,3,5,6-tetrahydrofuro[3,2-c]pyridine-7-carboxamide;
59) N-(3-fluoro-4-((4-((tetrahydro-2H-pyran-4-yl)amino)pyridin-2-yl)oxy)phenyl)-5-(4-fluorophenyl)-6-oxo-2,3,2,6-tetrahydrofuro[3,2-c]pyridine-7-carboxamide;
60) N-(3-fluoro-4-((2-((tetrahydro-2H-pyran-4-yl)amino)pyridin-4-yl)oxy)phenyl)-5-(4-fluorophenyl)-6-oxo-2,3,5,6-tetrahydrofuro[3,2-c]pyridine-7-carboxamide;
61) N-(3-fluoro-4-((6-((1-methylpiperidin-4-yl)amino)pyrimidin-4-yl)oxy)phenyl)-5-(4-fluorophenyl)-6-oxo-2,3,5,6-tetrahydrofuro[3,2-c]pyridine-7-carboxamide;
62) N-(4-((3-chloro-2-((3-morpholinopropyl)amino)pyridin-4-yl)oxy)-3-fluorophenyl)-5-(4-fluorophenyl)-6-oxo-2,3,5,6-tetrahydrofuro[3,2-c]pyridine-7-carboxamide; and
63) N-(3-fluoro-4-((2-((1-methylpiperidin-4-yl)amino)pyridin-4-yl)oxy)phenyl)-5-(4-fluorophenyl)-6-oxo-2,3,5,6-tetrahydrofuro[3,2-c]pyridine-7-carboxamide.

16. The compound of claim 1, wherein A is pyrrolopyridine, pyrazolopyridine, imidazopyridine, pyrrolopyrimidine, dihydropyrrolopyrimidine, furopyridine, pyrazolopyrimidine, purine, or indazole; and R₅ and R₆ are each independently be hydrogen or 5- to 9-membered heteroaryl,
wherein the heteroaryl contains one or more heteroatoms selected from the group consisting of N, O, and S; or a pharmaceutically acceptable salt thereof.

17. The compound of claim 16, wherein the compound represented by Formula 1 is a compound selected from the group consisting of the following compounds; or a pharmaceutically acceptable salt thereof:
64) N-(3-fluoro-4-((1-methyl-6-(1H-pyrazol-4-yl)-1H-indazol-5-yl)oxy)phenyl)-5-(4-fluorophenyl)-2-oxo-2,3,5,6-tetrahydrofuro[3,2-c]pyridine-7-carboxamide;
65) N-(4-((1H-pyrrolo[2,3-b]pyridin-4-yl)oxy)-3-fluorophenyl)-5-(4-fluorophenyl)-6-oxo-2,3,5,6-tetrahydrofuro[3,2-c]pyridine-7-carboxamide;
66) N-(4-((3H-imidazo[4,5-b]pyridin-7-yl)oxy)-3-fluorophenyl)-5-(4-fluorophenyl)-6-oxo-2,3,5,6-tetrahydrofuro[3,2-c]pyridine-7-carboxamide;
67) N-(3-fluoro-4-((2-methyl-3H-imidazo[4,5-b]pyridin-7-yl)oxy)phenyl)-5-(4-fluorophenyl)-6-oxo-2,3,5,6-tetrahydrofuro[3,2-c]pyridine-7-carboxamide;
68) N-(4-((1H-pyrazolo[3,4-b]pyridin-4-yl)oxy)-3-fluorophenyl)-5-(4-fluorophenyl)-6-oxo-2,3,5,6-tetrahydrofuro[3,2-c]pyridine-7-carboxamide;
69) N-(3-fluoro-4-((furo[3,2-b]pyridin-7-yl)oxy)phenyl)-5-(4-fluorophenyl)-6-oxo-2,3,5,6-tetrahydrofuro[3,2-c]pyridine-7-carboxamide;
70) N-(3-fluoro-4-((2-methyl-1H-pyrrolo[2,3-b]pyridin-4-yl)oxy)-3-fluorophenyl)-5-(4-fluorophenyl)-6-oxo-2,3,5,6-tetrahydrofuro[3,2-c]pyridine-7-carboxamide;
71) N-(4-((7H-pyrrolo[2,3-d]pyrimidin-4-yl)oxy)-3-fluorophenyl)-5-(4-fluorophenyl)-6-oxo-2,3,5,6-tetrahydrofuro[3,2-c]pyridine-7-carboxamide;
72) N-(4-((9H-purin-6-yl)oxy)-3-fluorophenyl)-5-(4-fluorophenyl)-6-oxo-2,3,5,6-tetrahydrofuro[3,2-c]pyridine-7-carboxamide;
73) N-(4-((1H-pyrazolo[3,4-d]pyrimidin-4-yl)oxy)-3-fluorophenyl)-5-(4-fluorophenyl)-6-oxo-2,3,5,6-tetrahydrofuro[3,2-c]pyridine-7-carboxamide;
74) N-(4-((6,7-dihydro-5H-pyrrolo[2,3-d]pyrimidin-4-yl)oxy)-3-fluorophenyl)-5-(4-fluorophenyl)-6-oxo-2,3,5,6-tetrahydrofuro[3,2-c]pyridine-7-carboxamide; and
75) N-(3-fluoro-4-(pyrazolo[1,5-a]pyrimidin-7-yloxy)phenyl)-5-(4-fluorophenyl)-6-oxo-2,3,5,6-tetrahydro[3,2-c]pyridine-7-carboxamide.

18. The compound of claim 1, wherein the compound represented by Formula 1 is a compound selected from the group consisting of the following compounds; or a pharmaceutically acceptable salt thereof:
1) N-(3-fluoro-4-((2-(5-(((2-methoxyethyl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
2) N-(3-fluoro-4-((2-(5-(((2-methoxyethyl)amino)methyl)pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
3) N-(3-fluoro-4-((2-(pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
4) N-(3-fluoro-4-((2-(pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
5) N-(3-fluoro-4-((2-(5-(morpholinomethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
6) N-(3-fluoro-4-((2-(5-(morpholinomethyl)pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
7) N-(3-fluoro-4-((2-(5-morpholinomethyl)pyridin-2-yl)thieno[3,2-b]pyridin-7-yl)phenyl)-2-(4-fluorophenyl)-3-oxo-3,5,6,7-tetrahydro-2H-cyclopenta[c]pyridine-4-carboxamide;
8) N-(3-fluoro-4-((2-(1-methyl-1H-imidazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
9) N-(3-fluoro-4-((2-(1-methyl-1H-imidazol-4-yl)thieno[3,2-b]pyridin-7-yl)oxy)phenyl)-6-oxo-5-phenyl-2,3,5,6-tetrahydrofuro[3,2-c]pyridine-7-carboxamide;
10) N-(3-fluoro-4-((2-(1-methyl-1H-pyrazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
11) N-(3-fluoro-4-((2-(5-((3-hydroxyazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
12) N-(3-fluoro-4-((2-(5-((3-(hydroxymethyl)azetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
13) N-(3-fluoro-4-((2-(5-((3-hydroxy-3-methylazetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
14) N-(3-fluoro-4-((2-(5-((3-methoxyazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7 -carboxamide;
15) N-(3-fluoro-4-((2-(5-((3-(methoxymethyl)azetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
16) N-(3-fluoro-4-((2-(5-((3-methoxy-3-methylazetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
17) N-(3-fluoro-4-((2-(5-((3-fluoroazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7 -carboxamide;
18) N-(4-((2-(5-((3,3-difluoroazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
19) N-(4-((2-(5-((3-cyanoazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
20) N-(4-((2-(5-((3-(dimethylamino)azetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
21) N-(3-fluoro-4-((2-(5-((3-methylazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
22) N-(4-((2-(5-((3,3-dimethylazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
23) N-(3-fluoro-4-((2-(5-((3-hydroxypyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7 -carboxamide;
24) N-(3-fluoro-4-((2-(5-((3-hydroxy-3-methylpyrrolidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
25) N-(3-fluoro-4-((2-(5-((3-methoxypyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
26) N-(3-fluoro-4-((2-(5-(pyrrolidin-1-ylmethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
27) N-(3-fluoro-4-((2-(5-((3-fluoropyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7 -carboxamide;
28) N-(4-((2-(5-((3,3-difluoropyrrolidin-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide;
29) N-(3-fluoro-4-((2-(5-((4-hydroxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7 -carboxamide;
30) N-(3-fluoro-4-((2-(5-((3-hydroxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7 -carboxamide;
31) N-(3-fluoro-4-((2-(5-((4-methoxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7 -carboxamide;
32) N-(3-fluoro-4-((2-(5-((3-methoxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7 -carboxamide;
33) N-(3-fluoro-4-((2-(5-(piperidin-1-ylmethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
34) N-(3-fluoro-4-((2-(5-((4-fluoropiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
35) N-(4-((2-(5-((4,4-difluoropiperidin-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide;
36) N-(3-fluoro-4-((2-(5-((4-methylpiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7 -carboxamide;
37) N-(4-((2-(5-((4,4-dimethylpiperidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
38) N-(3-fluoro-4-((2-(5-((((3-hydroxycyclohexyl)methyl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
39) N-(3-fluoro-4-((2-(5-(((tetrahydro-2H-pyran-4-yl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
40) N-(4-((6,7-dimethoxyquinolin-4-yl)oxy)-3-fluorophenyl)-5-(4-fluorophenyl)-6-oxo-2,3,5,6-tetrahydrofuro[3,2-c]pyridine-7-carboxamide;
41) N-(4-((6,7-dimethoxyquinolin-4-yl)oxy)-3-fluorophenyl)-6-oxo-5-phenyl-2,3,5,6-tetrahydrofuro[3,2-c]pyridine-7-carboxamide;
42) N-(4-((6,7-dimethoxyquinolin-4-yl)oxy)-3-fluorophenyl)-6-oxo-5-(4-(trifluoromethyl)phenyl)-2,3,5,6-tetrahydrofuro[3,2-c]pyridine-7-carboxamide;
43) N-(4-((6,7-dimethoxyquinolin-4-yl)oxy)-3-fluorophenyl)-6-oxo-5-(3-fluorophenyl)-2,3,5,6-tetrahydrofuro[3,2-c]pyridine-7-carboxamide;
44) N-(3-fluoro-4-((6-methoxyquinolin-4-yl)oxy)phenyl)-5-(4-fluorophenyl)-6-oxo-2,3,5,6-tetrahydrofuro[3,2-c]pyridine-7-carboxamide;
45) N-(3-fluoro-4-((7-methoxyquinolin-4-yl)oxy)phenyl)-5-(4-fluorophenyl)-6-oxo-2,3,5,6-tetrahydrofuro[3,2-c]pyridine-7-carboxamide;
46) N-(4-((6,7-dimethoxyquinazolin-4-yl)oxy)-3-fluorophenyl)-5-(4-fluorophenyl)-6-oxo-2,3,5,6-tetrahydrofuro[3,2-c]pyridine-7-carboxamide;
47) N-(4-((6-carbamoyl-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
48) N-(3-fluoro-4-((7-methoxy-6-(methylcarbamoyl)quinolin-4-yl)oxy)phenyl)-5-(4-fluorophenyl)-6-oxo-2,3,5,6-tetrahydrofuro[3,2-c]pyridine-7-carboxamide;
49) N-(4-((6-(dimethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
50) N-(3-fluoro-4-((7-methoxy-6-((2-morpholinoethyl)carbamoyl)quinolin-4-yl)oxy)phenyl)-5-(4-fluorophenyl)-6-oxo-2,3,5,6-tetrahydrofuro[3,2-c]pyridine-7-carboxamide;
51) N-(4-((6-(ethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
52) N-(4-((6-acetamido-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
53) N-(3-fluoro-4-((7-methoxy-6-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide;
54) N-(4-((2-carbamoyl-3-chloropyridin-4-yl)oxy)-3-fluorophenyl)-5-(4-fluorophenyl)-6-oxo-2,3,5,6-tetrahydrofuro[3,2-c]pyridine-7-carboxamide;
55) N-(4-((2-amino-3-chloropyridin-4-yl)oxy)-3-fluorophenyl)-5-(4-fluorophenyl)-6-oxo-2,3,5,6-tetrahydrofuro[3,2-c]pyridine-7-carboxamide;
56) N-(4-((2-aminopyridin-4-yl)oxy)-3-fluorophenyl)-5-(4-fluorophenyl)-6-oxo-2,3,5,6-tetrahydrofuro[3,2-c]pyridine-7-carboxamide;
57) N-(4-((2-aminopyrimidin-4-yl)oxy)-3-fluorophenyl)-5-(4-fluorophenyl)-6-oxo-2,3,5,6-tetrahydrofuro[3,2-c]pyridine-7-carboxamide;
58) N-(4-((6-aminopyrimidin-4-yl)oxy)-3-fluorophenyl)-5-(4-fluorophenyl)-6-oxo-2,3,5,6-tetrahydrofuro[3,2-c]pyridine-7-carboxamide;
59) N-(3-fluoro-4-((4-((tetrahydro-2H-pyran-4-yl)amino)pyridin-2-yl)oxy)phenyl)-5-(4-fluorophenyl)-6-oxo-2,3,2,6-tetrahydrofuro[3,2-c]pyridine-7-carboxamide;
60) N-(3-fluoro-4-((2-((tetrahydro-2H-pyran-4-yl)amino)pyridin-4-yl)oxy)phenyl)-5-(4-fluorophenyl)-6-oxo-2,3,5,6-tetrahydrofuro[3,2-c]pyridine-7-carboxamide;
61) N-(3-fluoro-4-((6-((1-methylpiperidin-4-yl)amino)pyrimidin-4-yl)oxy)phenyl)-5-(4-fluorophenyl)-6-oxo-2,3,5,6-tetrahydrofuro[3,2-c]pyridine-7-carboxamide;
62) N-(4-((3-chloro-2-((3-morpholinopropyl)amino)pyridin-4-yl)oxy)-3-fluorophenyl)-5-(4-fluorophenyl)-6-oxo-2,3,5,6-tetrahydrofuro[3,2-c]pyridine-7-carboxamide;
63) N-(3-fluoro-4-((2-((1-methylpiperidin-4-yl)amino)pyridin-4-yl)oxy)phenyl)-5-(4-fluorophenyl)-6-oxo-2,3,5,6-tetrahydrofuro[3,2-c]pyridine-7-carboxamide;
64) N-(3-fluoro-4-((1-methyl-6-(1H-pyrazol-4-yl)-1H-indazol-5-yl)oxy)phenyl)-5-(4-fluorophenyl)-2-oxo-2,3,5,6-tetrahydrofuro[3,2-c]pyridine-7-carboxamide;
65) N-(4-((1H-pyrrolo[2,3-b]pyridin-4-yl)oxy)-3-fluorophenyl)-5-(4-fluorophenyl)-6-oxo-2,3,5,6-tetrahydrofuro[3,2-c]pyridine-7-carboxamide;
66) N-(4-((3H-imidazo[4,5-b]pyridin-7-yl)oxy)-3-fluorophenyl)-5-(4-fluorophenyl)-6-oxo-2,3,5,6-tetrahydrofuro[3,2-c]pyridine-7-carboxamide;
67) N-(3-fluoro-4-((2-methyl-3H-imidazo[4,5-b]pyridin-7-yl)oxy)phenyl)-5-(4-fluorophenyl)-6-oxo-2,3,5,6-tetrahydrofuro[3,2-c]pyridine-7-carboxamide;
68) N-(4-((1H-pyrazolo[3,4-b]pyridin-4-yl)oxy)-3-fluorophenyl)-5-(4-fluorophenyl)-6-oxo-2,3,5,6-tetrahydrofuro[3,2-c]pyridine-7-carboxamide;
69) N-(3-fluoro-4-((furo[3,2-b]pyridin-7-yl)oxy)phenyl)-5-(4-fluorophenyl)-6-oxo-2,3,5,6-tetrahydrofuro[3,2-c]pyridine-7-carboxamide;
70) N-(3-fluoro-4-((2-methyl-1H-pyrrolo[2,3-b]pyridin-4-yl)oxy)-3-fluorophenyl)-5-(4-fluorophenyl)-6-oxo-2,3,5,6-tetrahydrofuro[3,2-c]pyridine-7-carboxamide;
71) N-(4-((7H-pyrrolo[2,3-d]pyrimidin-4-yl)oxy)-3-fluorophenyl)-5-(4-fluorophenyl)-6-oxo-2,3,5,6-tetrahydrofuro[3,2-c]pyridine-7-carboxamide;
72) N-(4-((9H-purin-6-yl)oxy)-3-fluorophenyl)-5-(4-fluorophenyl)-6-oxo-2,3,5,6-tetrahydrofuro[3,2-c]pyridine-7-carboxamide;
73) N-(4-((1H-pyrazolo[3,4-d]pyrimidin-4-yl)oxy)-3-fluorophenyl)-5-(4-fluorophenyl)-6-oxo-2,3,5,6-tetrahydrofuro[3,2-c]pyridine-7-carboxamide;
74) N-(4-((6,7-dihydro-5H-pyrrolo[2,3-d]pyrimidin-4-yl)oxy)-3-fluorophenyl)-5-(4-fluorophenyl)-6-oxo-2,3,5,6-tetrahydrofuro[3,2-c]pyridine-7-carboxamide;
75) N-(3-fluoro-4-(pyrazolo[1,5-a]pyrimidin-7-yloxy)phenyl)-5-(4-fluorophenyl)-6-oxo-2,3,5,6-tetrahydro[3,2-c]pyridine-7-carboxamide;
76) N-(3-fluoro-4-((6-methoxy-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
77) N-(3-fluoro-4-((6-methoxy-7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide;
78) N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
79) N-(3-fluoro-4-((6-methoxy-7-(2-morpholinoethoxy)quinolin-4-yl)oxy)phenyl)-6-oxo-5-phenyl-2,3,5,6-tetrahydrofuro[3,2-c]pyridine-7-carboxamide;
80) N-(3-fluoro-4-((6-methoxy-7-(3-(4-methylpiperazin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
81) N-(3-fluoro-4-((7-(3-(3-hydroxyazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
82) N-(3-fluoro-4-((7-(3-(3-hydroxy-3-methylazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
83) N-(3-fluoro-4-((7-(3-(3-(hydroxymethyl)azetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
84) N-(3-fluoro-4-((6-methoxy-7-(3-(3-methoxyazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
85) N-(3-fluoro-4-((6-methoxy-7-(3-(3-methoxy-3-methylazetidin-l-yl)propoxy)quinolin-4-yl)oxy)phenyl)-5-(4-fluorophenyl)-6-oxo-2,3,5,6-tetrahydrofuro[3,2-c]pyridine-7-carboxamide;
86) N-(3-fluoro-4-((7-(3-(3-fluoroazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
87) N-(4-((7-(3-(3,3-difluoroazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
88) N-(4-((7-(3-(3-cyanoazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
89) N-(3-fluoro-4-((6-methoxy-7-(3-(3-methylazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
90) N-(3-fluoro-4-((7-(3-(3-hydroxypyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
91) N-(3-fluoro-4-((7-(3-(3-hydroxy-3-methylpyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
92) N-(3-fluoro-4-((6-methoxy-7-(3-(3-methoxypyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
93) N-(3-fluoro-4-((6-methoxy-7-(3-(pyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
94) N-(3-fluoro-4-((7-(3-(3-fluoropyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
95) N-(4-((7-(3-(3,3-difluoropyrrolidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
96) N-(3-fluoro-4-((7-(3-(4-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
97) N-(3-fluoro-4-((7-(3-(3-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
98) N-(3-fluoro-4-((7-(3-(4-hydroxy-4-methylpiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
99) N-(3-fluoro-4-((6-methoxy-7-(3-(4-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
100) N-(3-fluoro-4-((6-methoxy-7-(3-(3-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
101) N-(3-fluoro-4-((6-methoxy-7-(3-(4-oxopiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
102) N-(4-((7-(3-(1,1-dioxidothiomorpholino)propoxy)-6-methoxyqumolin-4-yl)oxy)-3-fluorophenyl)-5-(4-fluorophenyl)-6-oxo-2,3,5,6-tetrahydrofuro[3,2-c]pyridine-7-carboxamide;
103) N-(3-fluoro-4-((6-methoxy-7-(3-(piperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
104) N-(3-fluoro-4-((7-(3-(4-fluoropiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
105) N-(4-((7-(3-(4,4-difluoropiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
106) N-(3-fluoro-4-((6-methoxy-7-(3 -(4-methylpiperidin- 1 -yl)propoxy)quinolin-4-yl)oxy)phenyl)-5-(4-fluorophenyl)-6-oxo-2,3,5,6-tetrahydrofuro[3,2-c]pyridine-7-carboxamide;
107) N-(4-((7-(3-(4,4-dimethylpiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
108) N-(3 -fluoro-4-((7-(3 -((3-hydroxycyclobutyl)amino)propoxy)-6-methoxyquinolin-4-yl)oxy)phenyl)-5-(4-fluorophenyl)-6-oxo-2,3,5,6-tetrahydrofuro[3,2-c]pyridine-7 -carboxamide;
109) N-(3-fluoro-4-((6-methoxy-7-(3-((3-methoxycyclobutyl)amino)propoxy)quinolin-4-yl)oxy)phenyl)-5-(4-fluorophenyl)-6-oxo-2,3,5,6-tetrahydrofuro[3,2-c]pyridine-7-carboxamide;
110) N-(3-fluoro-4-((6-methoxy-7-(3-(oxetan-3-ylamino)propoxy)quinolin-4-yl)oxy)phenyl)-5-(4-fluorophenyl)-6-oxo-2,3,5,6-tetrahydrofuro[3,2-c]pyridine-7-carboxamide;
111) N-(3-fluoro-4-((6-methoxy-7-(3-((oxetan-3-ylmethyl)amino)propoxy)quinolin-4-yl)oxy)phenyl)-5-(4-fluorophenyl)-6-oxo-2,3,5,6-tetrahydrofuro[3,2-c]pyridine-7-carboxamide;
112) N-(3-fluoro-4-((7-(3-((3-hydroxycyclopentyl)amino)propoxy)-6-methoxyquinolin-4-yl)oxy)phenyl)-5-(4-fluorophenyl)-6-oxo-2,3,5,6-tetrahydrofuro[3,2-c]pyridine-7 -carboxamide;
113) N-(3-fluoro-4-((7-(3-((3-hydroxycyclohexyl)amino)propoxy)-6-methoxyquinolin-4-yl)oxy)phenyl)-5-(4-fluorophenyl)-6-oxo-2,3,5,6-tetrahydrofuro[3,2-c]pyridine-7 -carboxamide;
114) N-(3-fluoro-4-((7-(3-(((3-hydroxycyclohexyl)methyl)amino)propoxy)-6-methoxyquinolin-4-yl)oxy)phenyl)-5-(4-fluorophenyl)-6-oxo-2,3,5,6-tetrahydrofuro[3,2-c]pyridine-7 -carboxamide;
115) N-(3-fluoro-4-((6-methoxy-7-(3-((tetrahydro-2H-pyran-4-yl)amino)propoxy)quinolin-4-yl)oxy)phenyl)-5-(4-fluorophenyl)-6-oxo-2,3,5,6-tetrahydrofuro[3,2-c]pyridine-7-carboxamide;
116) N-(3-fluoro-4-((6-methoxy-7-(3-(((tetrahydro-2H-pyran-4-yl)methyl)amino)propoxy)quinolin-4-yl)oxy)phenyl)-5-(4-fluorophenyl)-6-oxo-2,3,5,6-tetrahydrofuro[3,2-c]pyridine-7-carboxamide;
117) N-(3-fluoro-4-((7-(2-(3-hydroxyazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
118) N-(3-fluoro-4-((7-(2-(3-hydroxy-3-methylazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
119) N-(3-fluoro-4-((7-(2-(3-(hydroxymethyl)azetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
120) N-(3-fluoro-4-((6-methoxy-7-(2-(3-methoxyazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
121) N-(3-fluoro-4-((6-methoxy-7-(2-(3-methoxy-3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
122) N-(3-fluoro-4-((6-methoxy-7-(2-(3-(methoxymethyl)azetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
123) N-(3-fluoro-4-((7-(2-(3-fluoroazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
124) N-(4-((7-(2-(3,3-difluoroazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
125) N-(4-((7-(2-(3-ethynylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
126) N-(3-fluoro-4-((6-methoxy-7-(2-(3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
127) N-(4-((7-(2-(3,3-dimethylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
128) N-(4-((7-(2-(3-(dimethylamino)azetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
129) N-(3-fluoro-4-((7-(2-(3-hydroxypyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
130) N-(3-fluoro-4-((7-(2-(3-hydroxy-3-methylpyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
131) N-(3-fluoro-4-((6-methoxy-7-(2-(3-methoxypyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
132) N-(3-fluoro-4-((6-methoxy-7-(2-(pyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
133) N-(3-fluoro-4-((7-(2-(3-fluoropyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
134) N-(4-((7-(2-(3,3-difluoropyrrolidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
135) N-(3-fluoro-4-((7-(2-(4-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
136) N-(3-fluoro-4-((7-(2-(3-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
137) N-(3-fluoro-4-((7-(2-(4-hydroxy-4-methylpiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
138) N-(3-fluoro-4-((6-methoxy-7-(2-(4-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
139) N-(3-fluoro-4-((6-methoxy-7-(2-(3-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
140) N-(3-fluoro-4-((6-methoxy-7-(2-(4-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
141) N-(3-fluoro-4-((6-methoxy-7-(2-(3-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
142) N-(4-((7-(2-(1,1-dioxidothiomorpholino)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
143) N-(3-fluoro-4-((6-methoxy-7-(2-(piperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
144) N-(3-fluoro-4-((7-(2-(4-fluoropiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
145) N-(4-((7-(2-(4,4-difluoropiperidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
146) N-(3-fluoro-4-((6-methoxy-7-(2-(4-methylpiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
147) N-(4-((7-(2-(4,4-dimethylpiperidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
148) N-(3-fluoro-4-((7-(2-((3-hydroxycyclobutyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
149) N-(3-fluoro-4-((6-methoxy-7-(2-((3-methoxycyclobutyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
150) N-(3-fluoro-4-((6-methoxy-7-(2-(oxetan-3-ylamino)ethoxy)quinolin-4-yl)oxy)phenyl)-5-(4-fluorophenyl)-6-oxo-2,3,5,6-tetrahydrofuro[3,2-c]pyridine-7-carboxamide;
151) N-(3-fluoro-4-((6-methoxy-7-(2-((oxetan-3-ylmethyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
152) N-(3-fluoro-4-((7-(2-((4-hydroxycyclohexyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
153) N-(3-fluoro-4-((7-(2-((3-hydroxycyclohexyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
154) N-(3-fluoro-4-((7-(2-(((3-hydroxycyclohexyl)methyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
155) N-(3-fluoro-4-((6-methoxy-7-(2-((tetrahydro-2H-pyran-4-yl)amino)ethoxy)quinolin-4-yl)oxy)phenyl)-5-(4-fluorophenyl)-6-oxo-2,3,5,6-tetrahydrofuro[3,2-c]pyridine-7-carboxamide;
156) N-(3-fluoro-4-((6-methoxy-7-(2-((4-methoxycyclohexyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
157) N-(3-fluoro-4-((7-(2-morpholinylethoxy)quinolin-4-yl)oxy)phenyl)-5-(4-fluorophenyl)-6-oxo-2,3,5,6-tetrahydrofuro[3,2-c]pyridine-7-carboxamide;
158) N-(3-fluoro-4-((7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
159) N-(3-fluoro-4-((7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide;
160) N-(3-fluoro-4-((7-(3-(4-methylpiperazin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
161) 7-(2-(3-fluoro-4-((7-(3-(piperidin- 1 -yl)propoxy)quinolin-4-yl)oxy)phenyl)acetyl)-5-(4-fluorophenyl)-3,5-dihydrofuro[3,2-c]pyridin-6(2H)-one;
162) 7-(2-(3-fluoro-4-((7-(3-(4-methylpiperidin-1-yl)propoxy)quinolin-4-yl)oxy)phenyl)acetyl)-5-(4-fluorophenyl)-3,5-dihydrofuro[3,2-c]pyridin-6(2H)-one;
163) N-(3-fluoro-4-((6-(methylcarbamoyl)-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
164) N-(3-fluoro-4-((6-(methylcarbamoyl)-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
165) N-(3-fluoro-4-((6-methoxy-7-(methylcarbamoyl)quinolin-4-yl)oxy)phenyl)-5-(4-fluorophenyl)-6-oxo-2,3,5,6-tetrahydrofuro[3,2-c]pyridine-7-carboxamide;
166) N-(3-fluoro-4-((7-(methylcarbamoyl)-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
167) N-(3-fluoro-4-((6-fluoro-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
168) N-(3-fluoro-4-((6-fluoro-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
169) N-(3-fluoro-4-((7-(2-morpholinoethoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide;
170) N-(3-fluoro-4-((7-(3-morpholinopropoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide;
171) N-(4-((6-chloro-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide;
172) N-(4-((6-chloro-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide;
173) N-(4-((6-cyano-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide;
174) N-(4-((6-cyano-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide;
175) N-(3-fluoro-4-((7-methoxy-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
176) N-(3-fluoro-4-((7-methoxy-6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
177) N-(3-fluoro-4-((6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
178) N-(3-fluoro-4-((6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
179) N-(4-((6-amino-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
180) N-(3-fluoro-4-((7-methoxy-6-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide;
181) N-(4-((7-amino-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
182) N-(3-fluoro-4-((6-methoxy-7-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide;
183) N-(3-fluoro-4-((6-methoxy-7-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide;
184) N-(4-((7-acetamido-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
185) N-(3-fluoro-4-((7-methoxy-6-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide;
186) N-(3-fluoro-4-((7-methoxy-6-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide;
187) N-(3-fluoro-4-((6-methoxy-7-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide; and
188) N-(3-fluoro-4-((6-methoxy-7-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide.

19. A pharmaceutical composition for preventing or treating a disease related to protein kinase activity, comprising as an active ingredient:
the compound of any one of claims 1 to 18 or a pharmaceutically acceptable salt thereof.

20. The pharmaceutical composition of claim 19, wherein the protein kinase is RON (Recepteur d'Origine Nantais).

21. The pharmaceutical composition of claim 19, wherein the disease related to protein kinase activity is cancer, psoriasis, rheumatoid arthritis, inflammatory bowel disease, or chronic obstructive pulmonary disease.

22. The pharmaceutical composition of claim 21, wherein the cancer is any one selected from the group consisting of breast cancer, lung cancer, gastric cancer, prostate cancer, uterine cancer, ovarian cancer, kidney cancer, pancreatic cancer, liver cancer, colorectal cancer, skin cancer, head and neck cancer, and thyroid cancer.

23. A use of the compound of any one of claims 1 to 18 or a pharmaceutically acceptable salt thereof, for preventing or treating a disease related to protein kinase activity.

24. A use of the compound of any one of claims 1 to 18 or a pharmaceutically acceptable salt thereof, for manufacture of a medicament for preventing or treating a disease related to protein kinase activity.

25. A use of the compound of any one of claims 1 to 18 or a pharmaceutically acceptable salt thereof, for inhibiting protein kinase activity.

26. A use of the compound of any one of claims 1 to 18 or a pharmaceutically acceptable salt thereof, for manufacture of a medicament for inhibiting protein kinase activity.

27. A method for preventing or treating a disease related to protein kinase activity, comprising:
a step of administering, to a subject in need thereof, the compound of any one of claims 1 to 18 or a pharmaceutically acceptable salt thereof.

28. A method for inhibiting protein kinase activity, comprising:
a step of administering, to a subject in need thereof, the compound of any one of claims 1 to 18 or a pharmaceutically acceptable salt thereof.
